(19) **Europäisches Patentamt**
**European Patent Office**
**Office européen des brevets**

(11) **EP 2 163 616 A2**

(12) **EUROPEAN PATENT APPLICATION**

(43) Date of publication:
**17.03.2010 Bulletin 2010/11**

(51) Int Cl.:
*C12N 9/50* (2006.01)    *C12N 15/57* (2006.01)
*C11D 3/386* (2006.01)

(21) Application number: **09152523.8**

(22) Date of filing: **22.03.2004**

(84) Designated Contracting States:
**AT BE BG CH CY CZ DE DK EE ES FI FR GB GR HU IE IT LI LU MC NL PL PT RO SE SI SK TR**

(30) Priority: **21.03.2003  DK 200300435**

(62) Document number(s) of the earlier application(s) in accordance with Art. 76 EPC:
**04722236.9 / 1 608 744**

(27) Previously filed application:
**22.03.2004 PCT/DK2004/000194**

(71) Applicant: **Novozymes A/S**
**2880 Bagsvaerd (DK)**

(72) Inventors:
- **Svendsen, Allan**
  **2970 Hoersholm (DK)**
- **Minning, Stefan**
  **1902 Frederiksberg (DK)**

Remarks:
This application was filed on 11-02-2009 as a divisional application to the application mentioned under INID code 62.

(54) **Jp170 subtilase variants**

(57)    A method for non-targeted complex sample analysis which involves the following steps. A first step involves providing a database (16) containing identifying data of known molecules. A second step involves introducing a complex sample containing multiple unidentified molecules into a Fourier Transform Ion Cyclotron Mass Spectrometer (12) to obtain data regarding the molecules in the complex sample. A third step involves comparing the collected data regarding the molecules in the complex sample with the identifying data of known molecules in order to arrive at an identification through comparison of the molecules in the sample.

SEQUENCE LISTING

<110>   Novozymes A/S

<120>   Novel subtilases

<130>   10321.204-WO

<160>   7

<170>   PatentIn version 3.2

<210>   1
<211>   433
<212>   PRT
<213>   Bacillus sp. JP170


<220>
<221>   PEPTIDE
<222>   (1)..(433)
<223>   JP170 subtilase

<400>   1

Asn Asp Val Ala Arg Gly Ile Val Lys Ala Asp Val Ala Gln Asn Asn
1               5                   10                  15


Phe Gly Leu Tyr Gly Gln Gly Gln Ile Val Ala Val Ala Asp Thr Gly
                20                  25                  30


Leu Asp Thr Gly Arg Asn Asp Ser Ser Met His Glu Ala Phe Arg Gly
            35                  40                  45


Lys Ile Thr Ala Leu Tyr Ala Leu Gly Arg Thr Asn Asn Ala Asn Asp
        50                  55                  60


Pro Asn Gly His Gly Thr His Val Ala Gly Ser Val Leu Gly Asn Ala
65                  70                  75                  80


Thr Asn Lys Gly Met Ala Pro Gln Ala Asn Leu Val Phe Gln Ser Ile
                85                  90                  95


Met Asp Ser Gly Gly Gly Leu Gly Gly Leu Pro Ala Asn Leu Gln Thr
                100                 105                 110


Leu Phe Ser Gln Ala Tyr Ser Ala Gly Ala Arg Ile His Thr Asn Ser
            115                 120                 125


Trp Gly Ala Pro Val Asn Gly Ala Tyr Thr Thr Asp Ser Arg Asn Val
        130                 135                 140


Asp Asp Tyr Val Arg Lys Asn Asp Met Thr Ile Leu Phe Ala Ala Gly
145                 150                 155                 160


Asn Glu Gly Pro Gly Ser Gly Thr Ile Ser Ala Pro Gly Thr Ala Lys
                165                 170                 175

```
Asn Ala Ile Thr Val Gly Ala Thr Glu Asn Leu Arg Pro Ser Phe Gly
            180             185                 190

Ser Tyr Ala Asp Asn Ile Asn His Val Ala Gln Phe Ser Ser Arg Gly
        195             200             205

Pro Thr Arg Asp Gly Arg Ile Lys Pro Asp Val Met Ala Pro Gly Thr
    210             215                 220

Tyr Ile Leu Ser Ala Arg Ser Ser Leu Ala Pro Asp Ser Ser Phe Trp
225             230             235                 240

Ala Asn His Asp Ser Lys Tyr Ala Tyr Met Gly Gly Thr Ser Met Ala
            245             250                 255

Thr Pro Ile Val Ala Gly Asn Val Ala Gln Leu Arg Glu His Phe Val
            260             265                 270

Lys Asn Arg Gly Val Thr Pro Lys Pro Ser Leu Leu Lys Ala Ala Leu
        275             280             285

Ile Ala Gly Ala Ala Asp Val Gly Leu Gly Phe Pro Asn Gly Asn Gln
    290             295             300

Gly Trp Gly Arg Val Thr Leu Asp Lys Ser Leu Asn Val Ala Phe Val
305             310             315                 320

Asn Glu Thr Ser Pro Leu Ser Thr Ser Gln Lys Ala Thr Tyr Ser Phe
            325             330                 335

Thr Ala Gln Ala Gly Lys Pro Leu Lys Ile Ser Leu Val Trp Ser Asp
            340             345             350

Ala Pro Gly Ser Thr Thr Ala Ser Leu Thr Leu Val Asn Asp Leu Asp
        355             360             365

Leu Val Ile Thr Ala Pro Asn Gly Thr Lys Tyr Val Gly Asn Asp Phe
    370             375             380

Thr Ala Pro Tyr Asp Asn Asn Trp Asp Gly Arg Asn Asn Val Glu Asn
385             390             395                 400

Val Phe Ile Asn Ala Pro Gln Ser Gly Thr Tyr Thr Val Glu Val Gln
            405             410             415

Ala Tyr Asn Val Pro Val Ser Pro Gln Thr Phe Ser Leu Ala Ile Val
            420             425             430

His
```

<210> 2
<211> 433
<212> PRT
<213> Bacillus sp. Y


<220>
<221> PEPTIDE
<222> (1)..(433)
<223> Subtilase Y

<400> 2

Asn Asp Val Ala Arg Gly Ile Val Lys Ala Asp Val Ala Gln Asn Asn
1               5                   10                  15


Tyr Gly Leu Tyr Gly Gln Gly Gln Leu Val Ala Val Ala Asp Thr Gly
                20                  25                  30


Leu Asp Thr Gly Arg Asn Asp Ser Ser Met His Glu Ala Phe Arg Gly
            35                  40                  45


Lys Ile Thr Ala Leu Tyr Ala Leu Gly Arg Thr Asn Asn Ala Ser Asp
        50                  55                  60


Pro Asn Gly His Gly Thr His Val Ala Gly Ser Val Leu Gly Asn Ala
65                  70                  75                  80


Leu Asn Lys Gly Met Ala Pro Gln Ala Asn Leu Val Phe Gln Ser Ile
                85                  90                  95


Met Asp Ser Ser Gly Gly Leu Gly Gly Leu Pro Ser Asn Leu Asn Thr
            100                 105                 110


Leu Phe Ser Gln Ala Trp Asn Ala Gly Ala Arg Ile His Thr Asn Ser
            115                 120                 125


Trp Gly Ala Pro Val Asn Gly Ala Tyr Thr Ala Asn Ser Arg Gln Val
        130                 135                 140


Asp Glu Tyr Val Arg Asn Asn Asp Met Thr Val Leu Phe Ala Ala Gly
145                 150                 155                 160


Asn Glu Gly Pro Asn Ser Gly Thr Ile Ser Ala Pro Gly Thr Ala Lys
                165                 170                 175


Asn Ala Ile Thr Val Gly Ala Thr Glu Asn Tyr Arg Pro Ser Phe Gly
            180                 185                 190


Ser Ile Ala Asp Asn Pro Asn His Ile Ala Gln Phe Ser Ser Arg Gly
        195                 200                 205


Ala Thr Arg Asp Gly Arg Ile Lys Pro Asp Val Thr Ala Pro Gly Thr
        210                 215                 220

```
Phe Ile Leu Ser Ala Arg Ser Ser Leu Ala Pro Asp Ser Ser Phe Trp
225             230             235                 240

Ala Asn Tyr Asn Ser Lys Tyr Ala Tyr Met Gly Gly Thr Ser Met Ala
                245             250                 255

Thr Pro Ile Val Ala Gly Asn Val Ala Gln Leu Arg Glu His Phe Ile
            260             265                 270

Lys Asn Arg Gly Ile Thr Pro Lys Pro Ser Leu Ile Lys Ala Ala Leu
            275             280             285

Ile Ala Gly Ala Thr Asp Val Gly Leu Gly Tyr Pro Ser Gly Asp Gln
    290             295                 300

Gly Trp Gly Arg Val Thr Leu Asp Lys Ser Leu Asn Val Ala Tyr Val
305             310             315                 320

Asn Glu Ala Thr Ala Leu Ala Thr Gly Gln Lys Ala Thr Tyr Ser Phe
            325             330                 335

Gln Ala Gln Ala Gly Lys Pro Leu Lys Ile Ser Leu Val Trp Thr Asp
            340             345                 350

Ala Pro Gly Ser Thr Thr Ala Ser Tyr Thr Leu Val Asn Asp Leu Asp
        355             360                 365

Leu Val Ile Thr Ala Pro Asn Gly Gln Lys Tyr Val Gly Asn Asp Phe
    370             375                 380

Ser Tyr Pro Tyr Asp Asn Asn Trp Asp Gly Arg Asn Asn Val Glu Asn
385             390             395                 400

Val Phe Ile Asn Ala Pro Gln Ser Gly Thr Tyr Ile Ile Glu Val Gln
                405             410                 415

Ala Tyr Asn Val Pro Ser Gly Pro Gln Arg Phe Ser Leu Ala Ile Val
            420             425                 430

His
```

```
<210>   3
<211>   433
<212>   PRT
<213>   Bacillus sp. SD-521


<220>
<221>   PEPTIDE
<222>   (1)..(433)
<223>   Subtilase SD-521
```

<400>  3

```
Asn Asp Val Ala Arg Gly Ile Val Lys Ala Asp Val Ala Gln Asn Asn
1               5                   10                  15

Tyr Gly Leu Tyr Gly Gln Gly Gln Val Val Ala Val Ala Asp Thr Gly
            20              25                  30

Leu Asp Thr Gly Arg Asn Asp Ser Ser Met His Glu Ala Phe Arg Gly
        35              40                  45

Lys Ile Thr Ala Leu Tyr Ala Leu Gly Arg Thr Asn Asn Ala Asn Asp
    50              55                  60

Pro Asn Gly His Gly Thr His Val Ala Gly Ser Val Leu Gly Asn Ala
65              70                  75                  80

Leu Asn Lys Gly Met Ala Pro Gln Ala Asn Leu Val Phe Gln Ser Ile
            85                  90                  95

Met Asp Ser Ser Gly Gly Leu Gly Gly Leu Pro Ser Asn Leu Asn Thr
            100             105             110

Leu Phe Ser Gln Ala Trp Asn Ala Gly Ala Arg Ile His Thr Asn Ser
            115             120             125

Trp Gly Ala Pro Val Asn Gly Ala Tyr Thr Ala Asn Ser Arg Gln Val
    130             135             140

Asp Glu Tyr Val Arg Asn Asn Asp Met Thr Val Leu Phe Ala Ala Gly
145             150             155             160

Asn Glu Gly Pro Asn Ser Gly Thr Ile Ser Ala Pro Gly Thr Ala Lys
            165             170             175

Asn Ala Ile Thr Val Gly Ala Thr Glu Asn Tyr Arg Pro Ser Phe Gly
            180             185             190

Ser Leu Ala Asp Asn Pro Asn His Ile Ala Gln Phe Ser Ser Arg Gly
            195             200             205

Ala Thr Arg Asp Gly Arg Ile Lys Pro Asp Val Thr Ala Pro Gly Thr
    210             215             220

Phe Ile Leu Ser Ala Arg Ser Ser Leu Ala Pro Asp Ser Ser Phe Trp
225             230             235             240

Ala Asn Tyr Asn Ser Lys Tyr Ala Tyr Met Gly Gly Thr Ser Met Ala
            245             250             255

Thr Pro Ile Val Ala Gly Asn Val Ala Gln Leu Arg Glu His Phe Ile
```

<pre>
                260                    265                      270

      Lys Asn Arg Gly Ile Thr Pro Lys Pro Ser Leu Ile Lys Ala Ala Leu
              275                  280                    285

      Ile Ala Gly Ala Thr Asp Val Gly Leu Gly Tyr Pro Ser Gly Asp Gln
          290                  295                  300

      Gly Trp Gly Arg Val Thr Leu Asp Lys Ser Leu Asn Val Ala Tyr Val
      305                  310                  315                  320

      Asn Glu Ala Thr Ala Leu Ala Thr Gly Gln Lys Ala Thr Tyr Ser Phe
                  325                  330                  335

      Gln Ala Gln Ala Gly Lys Pro Leu Lys Ile Ser Leu Val Trp Thr Asp
                  340                  345                  350

      Ala Pro Gly Ser Thr Thr Ala Ser Tyr Thr Leu Val Asn Asp Leu Asp
                  355                  360                  365

      Leu Val Ile Thr Ala Pro Asn Gly Gln Lys Tyr Val Gly Asn Asp Phe
          370                  375                  380

      Ser Tyr Pro Tyr Asp Asn Asn Trp Asp Gly Arg Asn Asn Val Glu Asn
      385                  390                  395                  400

      Val Phe Ile Asn Ala Pro Gln Ser Gly Thr Tyr Thr Ile Glu Val Gln
                  405                  410                  415

      Ala Tyr Asn Val Pro Ser Gly Pro Gln Arg Phe Ser Leu Ala Ile Val
                  420                  425                  430

      His


<210>   4
<211>   275
<212>   PRT
<213>   Bacillus amyloliquefaciens


<220>
<221>   PEPTIDE
<222>   (1)..(275)

<220>
<221>   PEPTIDE
<222>   (1)..(275)
<223>   Subtilase BPN'

<400>   4

Ala Gln Ser Val Pro Tyr Gly Val Ser Gln Ile Lys Ala Pro Ala Leu
1                   5                   10                  15
</pre>

His Ser Gln Gly Tyr Thr Gly Ser Asn Val Lys Val Ala Val Ile Asp
20              25              30

Ser Gly Ile Asp Ser Ser His Pro Asp Leu Lys Val Ala Gly Gly Ala
35              40              45

Ser Met Val Pro Ser Glu Thr Asn Pro Phe Gln Asp Asn Asn Ser His
50              55              60

Gly Thr His Val Ala Gly Thr Val Ala Ala Leu Asn Asn Ser Ile Gly
65              70              75              80

Val Leu Gly Val Ala Pro Ser Ala Ser Leu Tyr Ala Val Lys Val Leu
85              90              95

Gly Ala Asp Gly Ser Gly Gln Tyr Ser Trp Ile Ile Asn Gly Ile Glu
100             105             110

Trp Ala Ile Ala Asn Asn Met Asp Val Ile Asn Met Ser Leu Gly Gly
115             120             125

Pro Ser Gly Ser Ala Ala Leu Lys Ala Ala Val Asp Lys Ala Val Ala
130             135             140

Ser Gly Val Val Val Val Ala Ala Ala Gly Asn Glu Gly Thr Ser Gly
145             150             155             160

Ser Ser Ser Thr Val Gly Tyr Pro Gly Lys Tyr Pro Ser Val Ile Ala
165             170             175

Val Gly Ala Val Asp Ser Ser Asn Gln Arg Ala Ser Phe Ser Ser Val
180             185             190

Gly Pro Glu Leu Asp Val Met Ala Pro Gly Val Ser Ile Gln Ser Thr
195             200             205

Leu Pro Gly Asn Lys Tyr Gly Ala Tyr Asn Gly Thr Ser Met Ala Ser
210             215             220

Pro His Val Ala Gly Ala Ala Ala Leu Ile Leu Ser Lys His Pro Asn
225             230             235             240

Trp Thr Asn Thr Gln Val Arg Ser Ser Leu Glu Asn Thr Thr Thr Lys
245             250             255

Leu Gly Asp Ser Phe Tyr Tyr Gly Lys Gly Leu Ile Asn Val Gln Ala
260             265             270

Ala Ala Gln
275

```
<210> 5
<211> 7
<212> PRT
<213> Partiel sequence


<220>
<221> peptide
<222> (1)..(7)

<400> 5

Leu Asn Asn Ser Ile Gly Val
1               5


<210> 6
<211> 3
<212> PRT
<213> Partiel sequence


<220>
<221> peptide
<222> (1)..(3)

<400> 6

Ser Ser Asn
1


<210> 7
<211> 311
<212> PRT
<213> Bacillus sp. TY145


<220>
<221> peptide
<222> (1)..(311)

<400> 7

Ala Val Pro Ser Thr Gln Thr Pro Trp Gly Ile Lys Ser Ile Tyr Asn
1               5                   10                  15


Asp Gln Ser Ile Thr Lys Thr Thr Gly Gly Ser Gly Ile Lys Val Ala
            20                  25                  30


Val Leu Asp Thr Gly Val Tyr Thr Ser His Leu Asp Leu Ala Gly Ser
            35                  40                  45


Ala Glu Gln Cys Lys Asp Phe Thr Gln Ser Asn Pro Leu Val Asp Gly
        50                  55                  60


Ser Cys Thr Asp Arg Gln Gly His Gly Thr His Val Ala Gly Thr Val
65                  70                  75                  80


Leu Ala His Gly Gly Ser Asn Gly Gln Gly Val Tyr Gly Val Ala Pro
                85                  90                  95
```

```
Gln Ala Lys Leu Trp Ala Tyr Lys Val Leu Gly Asp Asn Gly Ser Gly
            100             105                 110

Tyr Ser Asp Asp Ile Ala Ala Ala Ile Arg His Val Ala Asp Glu Ala
        115             120                 125

Ser Arg Thr Gly Ser Lys Val Val Ile Asn Met Ser Leu Gly Ser Ser
        130             135                 140

Ala Lys Asp Ser Leu Ile Ala Ser Ala Val Asp Tyr Ala Tyr Gly Lys
145                 150                 155                 160

Gly Val Leu Ile Val Ala Ala Ala Gly Asn Ser Gly Ser Gly Ser Asn
            165                 170                 175

Thr Ile Gly Phe Pro Gly Gly Leu Val Asn Ala Val Ala Val Ala Ala
            180             185                 190

Leu Glu Asn Val Gln Gln Asn Gly Thr Tyr Arg Val Ala Asp Phe Ser
        195             200                 205

Ser Arg Gly Asn Pro Ala Thr Ala Gly Asp Tyr Ile Ile Gln Glu Arg
        210             215                 220

Asp Ile Glu Val Ser Ala Pro Gly Ala Ser Val Glu Ser Thr Trp Tyr
225                 230                 235                 240

Thr Gly Gly Tyr Asn Thr Ile Ser Gly Thr Ser Met Ala Thr Pro His
            245                 250                 255

Val Ala Gly Leu Ala Ala Lys Ile Trp Ser Ala Asn Thr Ser Leu Ser
            260             265                 270

His Ser Gln Leu Arg Thr Glu Leu Gln Asn Arg Ala Lys Val Tyr Asp
        275             280                 285

Ile Lys Gly Gly Ile Gly Ala Gly Thr Gly Asp Asp Tyr Ala Ser Gly
    290             295                 300

Phe Gly Tyr Pro Arg Val Lys
305             310
```

**Description**

**FIELD OF THE INVENTION**

The present invention relates to JP170 and BPN' like subtilases and to methods of construction such variants with altered properties, such as stability (e.g. thermostability or storage stability), $Ca^{2+}$ dependency, pH dependent activity, improved performance in washing and cleaning applications.

**BACKGROUND OF THE INVENTION**

Enzymes have been used within the detergent industry as part of washing formulations for more than 30 years. Proteases are from a commercial perspective the most relevant enzyme in such formulations, but other enzymes including lipases, amylases, cellulases or mixtures of enzymes are also often used.

To improve the cost and/or the performance of proteases there is an ongoing search for proteases with altered properties, such as increased activity at low temperatures, increased thermostability, increased specific activity at a given pH, altered $Ca^{2+}$ dependency, increased stability in the presence of other detergent ingredients (e.g. bleach, surfactants etc.) etc.

The search for proteases with altered properties include both discovery of naturally occurring proteases, i.e. so called wild-type proteases but also alteration of well-known proteases by e.g. genetic manipulation of the nucleic acid sequence encoding said proteases. Knowledge of the relationship between the three-dimensional structure and the function of a protein has improved the ability to evaluate which areas of a protein to alter to affect a specific characteristic of the protein.

One family of proteases, which are often used in detergents, are the subtilases. This family has previously been further grouped into 6 different sub-groups by Siezen RJ and Leunissen JAM, 1997, Protein Science, 6, 501-523. One of these sub-groups is the Subtilisin family which includes subtilases such as BPN', subtilisin 309 (SAVINASE®, NOVOZYMES A/S), subtilisin Carlsberg (ALCALASE®, NOVOZYMES A/S), subtilisin S41 (a subtilase from the psychrophilic Antarctic *Bacillus* TA41, Davail S et al. 1994, The Journal of Biological Chemistry, 269(26), 99. 1 7448-17453), subtilisin S 39 (a subtilase from the psychrophilic Antarctic *Bacillus* TA39, Narinx E et al. 1997, Protein Engineering, 10 (11), pp. 1271-1279) and TY145 (a subtilase from Bacillus sp. TY145, NCIMB 40339 described in WO 92/17577).

The groupings indicated above were made based on primary sequence alignments, with only little consideration of three-dimensional structure. However, despite sequence homologies between subtilases belonging to the Subtilisin subgroup, modelling of the three-dimensional structure of one subtilase on the basis of the three-dimensional structure of another subtilase (such as the subtilisin BPN' that was used by Siezen and Leunissen) may result in an incorrect three-dimensional model structure because of structural differences.

Recently the three-dimensional structure of subtilase TY145 have been elucidated and it was found that there are several differences between this and the three-dimensional structure of BPN' also belonging to the Subtilisin subgroup of subtilases (PCT/DK2004/000066).

The differences between the three-dimensional structures of TY145 and BPN' are confirmed by the three-dimensional structure of the s ubtilase " sphericase" f rom *Bacillus sphaericus* (PDB NO:1 EA7, Protein Data Bank). The overall structure and many details of this subtilase are very homologous with the TY145 subtilase structure.

The subtilase JP170 and subtilases similar to JP170 are already known in the art, but the three-dimensional structure has not been disclosed for such subtilases.

The JP170 subtilase was described as protease A in WO 88/01293 to Novozymes. Later the patent application WO 98/56927 to Novozymes Biotech disclosed the amino acid (polypeptide) sequence of JP170 and the DNA sequence encoding JP170. In EP 204 342 the protease Ya was disclosed, and JP7-62152 and JP 4197182 to Lion Corp. disclosed the DNA sequence encoding protease Ya produced by *Bacillus sp.* Y that is homologous to JP170. In addition US 6,376,227 to Kao Corp. discloses physical characteristics as well as DNA and polypeptide sequences of alkaline proteases KP43, K P1790 and KP9860 which are also homologous to JP170. Recently variants of the KP43, KP9860, SD-521 and Ya proteases among others were disclosed in EP 1209233. These proteases are highly homologous, and an alignment of KP43, KP9860, SD-521, Y and JP170 revealed at least 90% homology. Therefore JP170, Ya and SD-521 represent these proteases in the alignment of Fig. 1 of the present specification.

**BRIEF DESCRIPTION OF THE FIGURES**

Figure 1 shows an alignment of three JP170 type proteases: (a) SD-521 (EP 1 209 233), (b) protease Ya (WO 99/67370), and (c) JP170 (WO 98/56927, mature sequence from Appendix 1).

Figure 2 shows a superposition of the 3D structures of the proteases JP170 and Savinase (BLSAVI), with indication of calcium binding sites. In the figure J P170 is indicated in light grey with three ion-binding sites, and Savinase in a dark structure with two ion-binding sites.

Figure 3 shows a matrix of homology between amino acid sequences of subtilases pertaining to various subtilase subgroups. The sequences are identified by sequence database accession numbers and their derivation.

1: aam50084; Subtilase derived from *Bacillus sp.* strain SD-521
2: aaw89547; Subtilase derived from *Bacillus sp.* JP170
3: q45681; Subtilase derived from *B. subtilis* (BSTA41)
4: p28842; Psychrophilic subtilisin derived from Antarctic *Bacillus* strain (BSTA39)
5: abb77095; Subtilase derived from *Bacillus sp.* (TY145)
6: p00783; Subtilase derived from *Bacillus subtilis var. amylosacchariticus* (BSAMY)
7: p29142; Subtilase derived from *Bacillus stearothermophilus* (BSSJ)
8: p35835; Subtilase derived from *Bacillus subtilis var. natto.* (BSNAT)
9: p07518; Subtilase derived from *Bacillus pumilus (B. mesentericus)* (BPMES)
10: p00782; Subtilase derived from *Bacillus amyloliquefaciens* (BPN')
11: p00780; Subtilase derived from *Bacillus licheniformis* (BLSCAR)
12: p41363; Subtilase derived from *Bacillus halodurans* (BHSAH)
13: aaw62222; Subtilase derived from *Bacillus lentus* (BLS147)
14: p29600; Subtilase derived from *Bacillus lentus* (BLSAVI, BLS309)
15: p27693; Subtilase derived from *Bacillus alcalophilus* (BAALKP)
16: q99405; Subtilase derived from *Bacillus sp.* strain KSM-K16 (BSKSMK)
17: p29599; Subtilase derived from *Bacillus lentus* (BLSUBL).

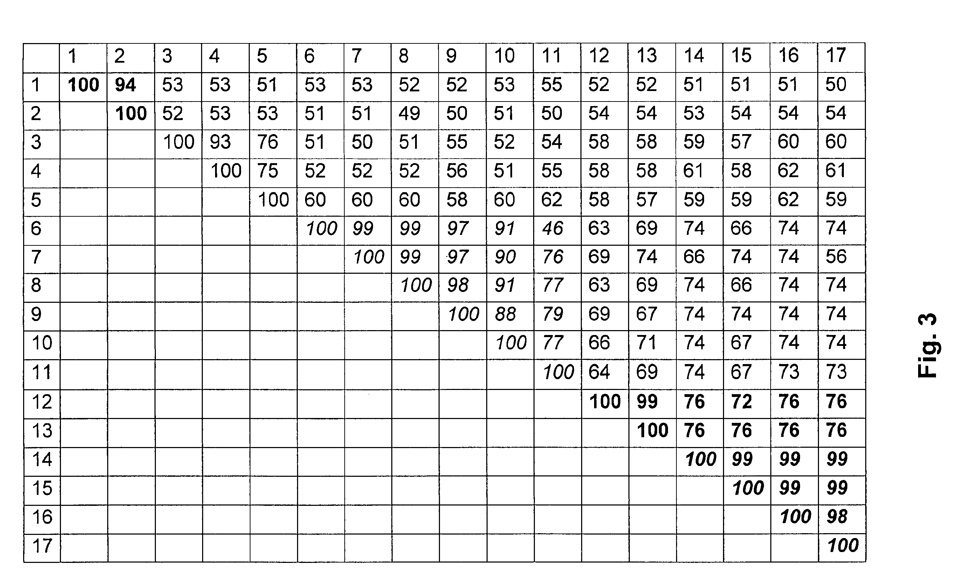

Sequences 1 and 2 belong to the JP170 type, sequences 3 to 5 belong to the TY145 type, sequences 6 to 11 belong to the "true subtilisins" or I-S1 type, and sequences 12 to 17 belong to the "highly alkaline" subtilisins or I-S2 type. From Fig. 3 it is clear that these types are quite distinct.

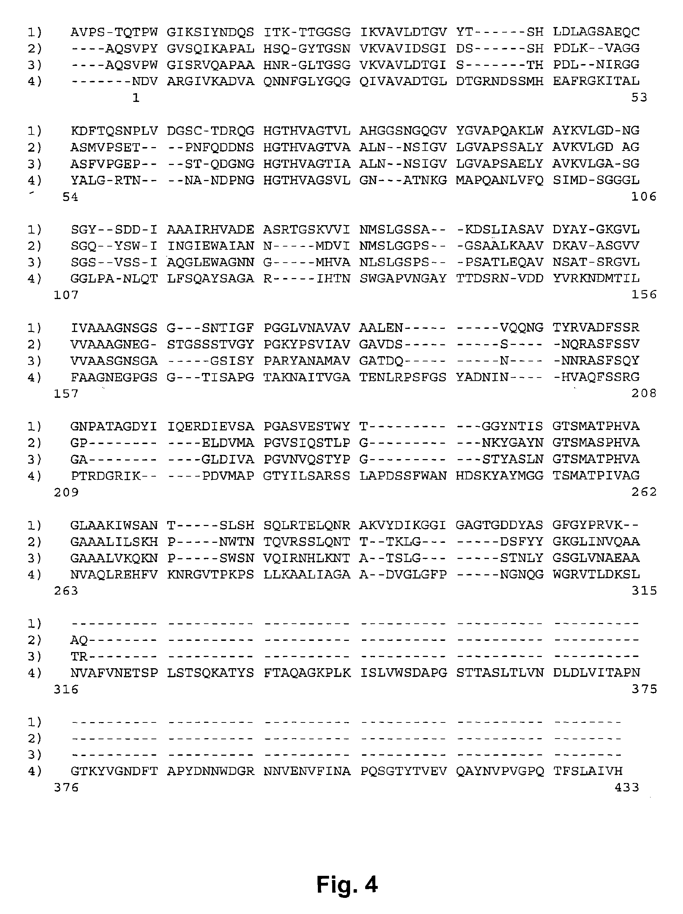

Figure 4 shows a three-dimensional alignment of the subtilases: (1) Ty145; (2) BPN'; (3) Sayinase; and (4) JP170.

By 3D sequences is meant that the position of homologous residues are chosen by superposition of the 3D structures and subsequently the amino acid sequences are aligned based on these homologous positions.

**BRIEF DESCRIPTION OF THE APPENDIX**

APPENDIX 1 shows the structural coordinates for the s olved crystal structure of JP170.

**SEQUENCE LISTING**

In the appended sequence listing the following amino acid sequences are provided:

Subtilase JP170 (SEQ ID NO:1)
Subtilase Y (SEQ ID NO:2)
Subtilase SD-521 (SEQ ID NO:3)
Subtilase BPN' (SEQ ID NO:4)
Partiel sequence (SEQ ID NO:5)
Partiel sequence (SEQ ID NO:6)
Subtilase TY145 (SEQ ID NO:7)

**BRIEF DESCRIPTION OF THE INVENTION**

Now the inventors of the present invention disclose the three-dimensional structure of the subtilase JP170. This subtilase has large structural differences to the structures of the subtilisins BPN' and TY145.

Based on these differences the inventors have modified the amino acid sequence of subtilases having a JP170 type structure and subtilases having a BPN' type structure to obtain variants with improved properties. The variants have altered properties, such as increased activity at low temperatures, increased thermostability, increased specific activity at a given pH, altered $Ca^{2+}$ dependency, increased stability in the presence of other detergent ingredients (e.g. bleach, surfactants etc.) etc.

Accordingly, the object of the present invention is to provide a method for constructing subtilases having altered properties, in particular to provide a method for constructing subtilases having altered properties as described above.

Thus the present invention relates to a method for constructing a variant of a parent subtilase, wherein the variant has at least one altered property as compared to said parent subtilase, which method comprises:

a) analyzing the three-dimensional structure of the subtilase to identify, on the basis of an evaluation of structural considerations in relation to a JP170 three dimensional structure, at least one amino acid residue or at least one structural region of the subtilase, which is of relevance for altering said property;

b) modifying the DNA of the polynucleotide encoding the parent to construct a polynucleotide encoding a variant subtilase, which in comparison to the parent subtilase, has been modified by deletion, substitution or insertion of the amino acid residue or structural part identified in i) so as to alter said property;

c) expressing the variant subtilase in a suitable host, and

d) testing the resulting subtilase variant for said property.

More specifically the invention relates to a method of producing a subtilase variant, wherein the variant has at least one altered property as compared to a parent subtilase, which method comprises:

a) producing a model structure of the parent subtilase on the three-dimensional structure of BPN', TY145 or JP170; or producing an actually determined three-dimensional structure of the parent subtilase,

b) comparing the model or actual three-dimensional structure of the parent subtilase to the JP170 structure by superimposing the structures through matching the CA, CB, C, O, and N atoms of the active site residues,

c) identifying on the basis of the comparison in step b) at least one structural part of the parent subtilase, wherein an alteration in said structural part is predicted to result in an altered property;

d) modifying the nucleic acid sequence encoding the parent subtilase to produce a nucleic acid sequence encoding at least one deletion or substitution of one or more amino acids at a position corresponding to said structural part, or at least one insertion of one or more amino acid residues in positions corresponding to said structural part;

e) performing steps c) and d) iteratively N times, where N is an integer with the value of one or more;

f) preparing the variant resulting from steps a) - e);

g) testing the properties of said variant; and

h) optionally repeating steps a) - g) recursively; and

i) selecting a subtilase variant having at least one altered property as compared to the parent subtilase.

j) expressing the modified nucleic acid sequence in a host cell to produce the variant subtilase;

k) isolating the produced subtilase variant;

l) purifying the isolated subtilase variant and

m) recovering the purified subtilase variant.

Although it has been described in the following that modification of the parent subtilase in certain regions and/or positions is expected to confer a particular effect to the thus produced subtilase variant, it should be noted that modification of the parent subtilase in any of such regions may also give rise to any other of the above-mentioned effects. For example, any of the regions and/or positions mentioned as being of particular interest with respect to, e.g., improved thermostability, may also give rise to, e.g., higher activity at a lower pH, an altered pH optimum, or increased specific activity, such as increased peptidase activity.

Further aspects of the present invention relates to variants of a subtilase, the DNA encoding such variants and methods of preparing the variants. Still further aspects of the present invention relates to the use of the variants for various industrial purposes, in particular as an additive in detergent compositions. Other aspects of the present invention will be apparent from the below description as well as from the appended claims.

## DEFINITIONS

Prior to discussing this invention in further detail, the following terms and conventions will first be defined.

For a detailed description of the nomenclature of amino acids and nucleic acids, we refer to WO 00/71691 page 5, hereby incorporated by reference. A description of the nomenclature of modifications introduced in a polypeptide by genetic manipulation can b e found in WO 00/71691 page 7-12, hereby incorporated by reference.

The term "subtilases" refer to a sub-group of serine protease according to Siezen et al., Protein Engng. 4 (1991) 719-737 and Siezen et al. Protein Science 6 (1997) 501-523. Serine proteases or serine peptidases is a subgroup of proteases characterised by having a serine in the active site, which forms a covalent adduct with the substrate. Further the subtilases (and the serine proteases) are characterised by having two active site amino acid residues apart from the serine, namely a histidine and an aspartic acid residue.

Subtilases are defined by homology analysis of more than 170 amino acid sequences of serine proteases previously referred to as subtilisin-like proteases. The subtilases may be divided into 6 sub-divisions, i.e. the Subtilisin family, the Thermitase family, the Proteinase K family, the Lantibiotic peptidase family, the Kexin family and the Pyrolysin family.

The Subtilisin family (EC 3.4.21.62) may be further divided into 3 sub-groups, i.e. IS1 ("true" subtilisins), I-S2 (highly alkaline proteases) and intracellular subtilisins. Definitions or grouping of enzymes may vary or change, however, in the context of the present invention the above division of subtilases into sub-division or sub-groups shall be understood as those described by Siezen et al., Protein Engng. 4 (1991) 719-737 and Siezen et al. Protein Science 6 (1997) 501-523.

The term "parent" is in the context of the present invention to be understood as a protein, which is modified to create a protein variant. The parent protein may be a naturally occurring (wild-type) polypeptide or it may be a variant thereof prepared by any suitable means. For instance, the parent protein may be a variant of a naturally occurring protein which has been modified by substitution, chemical modification, deletion or truncation of one or more amino acid residues, or by addition or insertion of one or more amino acid residues to the amino acid sequence, of a naturally-occurring polypeptide. Thus the term "parent subtilase" refers to a subtilase which is modified to create a subtilase variant.

The term "variant" is in the context of the present invention to be understood as a protein which has been modified as compared to a parent protein at one or more amino acid residues.

The term "modification(s)" or "modified" is in the context of the present invention to be understood as to include chemical modification of a protein as well as genetic manipulation of the DNA encoding a protein. The modification(s) may be replacement(s) of the amino acid side chain(s), substitution(s), deletion(s) and/or insertions in or at the amino acid(s) of interest. Thus the term "modified protein", e.g. "modified subtilase", is to be understood a s a protein which contains modification(s) compared to a parent protein, e.g. subtilase.

"Homology" or "homologous to" is in the context of the present invention to be understood in its conventional meaning and the "homology" between two amino acid sequences should be determined by use of the "Similarity" defined by the GAP program from the University of Wisconsin Genetics Computer Group (UWGCG) package using default settings for alignment parameters, comparison matrix, gap and gap extension penalties. Default values for GAP penalties, i.e. GAP creation penalty of 3.0 and GAP extension penalty of 0.1 (Program Manual for the Wisconsin Package, Version 8, August 1994, Genetics Computer Group, 575 Science Drive, Madison, Wisconsin, USA 53711). The method is also described in S.B. Needleman and C.D. Wunsch, Journal of Molecular Biology, 48, 443-445 (1970). Identities can be extracted from the same calculation. The homology between two amino acid sequences can also be determined by "identity" or "similarity" using the GAP routine of the UWGCG package version 9.1 with default setting for alignment parameters, comparison matrix, gap and gap extension penalties can also be applied using the following parameters: gap creation penalty = 8 and gap extension penalty = 8 and all other parameters kept at their default values. The output from the routine is besides the amino acid alignment the calculation of the "Percent Identity" and the "Similarity" between the two sequences. The numbers calculated using UWGCG package version 9.1 is slightly different from the version 8.

The term "position" is in the context of the present invention to be understood as the number of an amino acid in a peptide or polypeptide when counting from the N-terminal end of said peptide/polypeptide. The position numbers used in the present invention refer to different subtilases depending on which subgroup the subtilase belongs to.

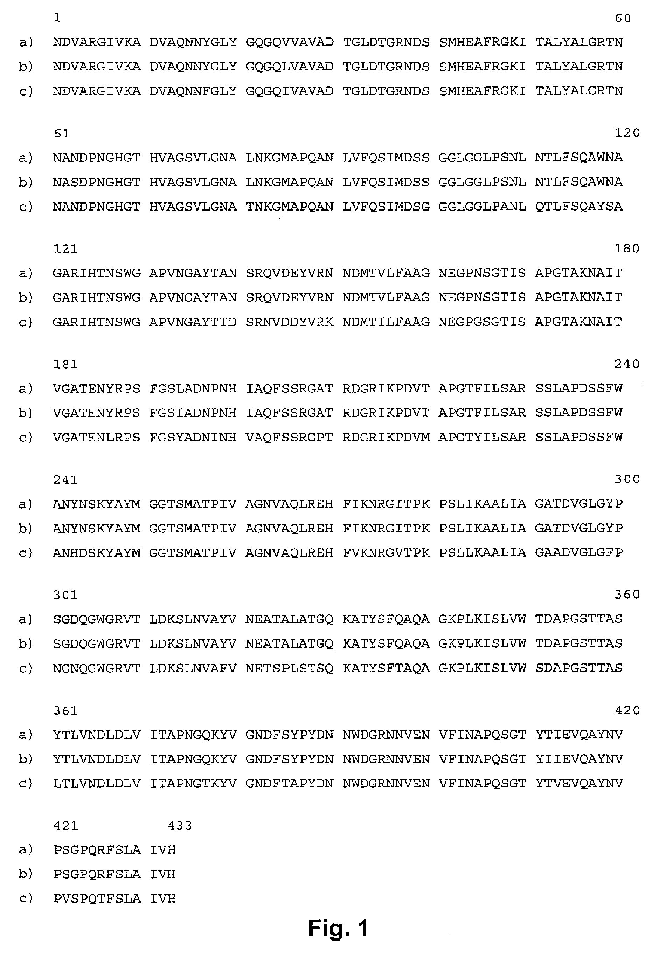

As mentioned above the alkaline subtilases KP43, KP1790, KP9860, Y, SD-521 and E1 belong to the JP170 subgroup, based on sequence homology. Due to the extensive homology only subtilase Ya and SD-521 are in Fig. 1 aligned with JP170. The JP170 subtilase, Y subtilase and SD-521 subtilase are numbered according to SEQ ID NO:1, SEQ ID NO:2 and SEQ ID NO:3, respectively.

The invention, however, is not limited to variants of these particular subtilases but extends to parent subtilases, especially of the JP170 type, containing amino acid residues at positions which are "equivalent" to the particular identified residues in the JP170 subtilase.

A residue (amino acid) position of a JP170 type subtilase is equivalent to a residue (position) of the JP170 subtilase, if it is either homologous (i.e., corresponding in position in either primary or tertiary structure) or analogous to a specific residue or portion of that residue in the JP170 subtilase (i.e., having the same or similar functional capacity to combine, react, or interact chemically).

In order to establish homology to primary structure, the amino acid sequence of a precursor protease is directly compared to the JP170 subtilase primary sequence by aligning the a mino acid sequence of an isolated or parent wild type enzyme with a suitable well-known (standard) enzyme of the same group or class of enzymes to define a frame of reference. This type of numbering has been used in numerous patent applications relating to subtilisins of the I-S1 and I-S2 subgroups with subtilisin BPN' as the standard subtilisin.

If nothing else is indicated herein, in the present instance the JP170 subtilase has been chosen as standard.

In order to establish homology to the tertiary structure (3D structure) of JP170, the 3D structure based alignment in Fig. 1 has been provided. By using this alignment the amino acid sequence of a precursor JP170 type subtilase may be directly correlated to the JP170 primary sequence. For a novel JP170 type subtilase, the (3D based) position corresponding to a position in JP170 is found by

i) identifying the JP170 type subtilase from the alignment of Fig. 1 that is most homologous to the novel sequence,
ii) aligning the novel sequence with the sequence identified to find the corresponding position in the JP170 type subtilase from Fig. 1, and
iii) establishing from Fig. 1 the corresponding position in JP170.

For comparison and finding the most homologous sequence the GAP program from GCG package as described below are used.

The alignment can as indicated above be obtained by the GAP routine of the GCG package version 8 to number the variants using the following parameters: gap creation penalty = 3 and gap extension penalty = 0.1 and all other parameters kept at their default values.

The alignment may define a number of deletions and insertions in relation to the sequence of JP170. In the alignment deletions are indicated by asterixes (*) in the referenced sequence, and the referenced enzyme will be considered to have a gap at the position in question. Insertions are indicated by asterixes (*) in the JP170 sequence, and the positions in the referenced enzyme are given as the position number of the last amino acid residue where a corresponding amino acid residue exists in the standard enzyme with a lower case letter appended in alphabetical order, e.g. 82a, 82b, 82c, 82d.

In case the referenced enzyme contains a N- or C-terminal extension in comparison to JP170; an N-terminal extension is given the position number 0a, 0b, etc. in the direction of the N-terminal; and a C-terminal extension will be given either the position number of the C-terminal amino acid residue of JP170 with a lower case letter appended in alphabetical order, or simply a continued consecutive numbering.

Thus for comparisons JP170 type subtilases are numbered by reference to the positions of the JP170 subtilase (SEQ ID NO: 1) as provided in Fig. 1. The position is then indicated as "corresponding to JP170".

Subtilases belonging to the BPN' subgroup refers to the positions of Subtilisin Novo (BPN') from *B. amyloliquefaciens* (SEQ ID NO:4).

Subtilases belonging to the TY145 subgroup refers to the positions of the TY145 subtilase (SEQ ID NO:7), see also PCT/DK2004/000066.

## DETAILED DESCRIPTION OF THE INVENTION

Despite the great homology of the subtilases described above the inventors of the present invention have elucidated the three-dimensional structure of JP170, SEQ ID NO:1 by X-ray crystallography and found that there are several differences between this and the three-dimensional structure of BPN'. The inventors of the present invention have further compared the sequence homology of subtilases belonging to the Subtilisin subgroup. This is shown in Figure 3 of the present invention.

On the basis of this comparison the inventors of the present invention suggest to divide the Subtilisin subgroup so that the JP170 type subtilases become a separate sub-group in addition to the subgroups of BPN' subtilases and TY145 subtilases PCT/DK2004/000066.

### JP170 type subtilases

The term "JP170 subtilase" or "JP170 type subtilase" should in the context of the present invention be understood as a subtilase belonging to the Subtilisin group according to Siezen et al. Protein Science 6 (1997) 501-523 and which has at least 58% homology to JP170, SEQ ID NO:1. In particular a JP170 type subtilase may have at least 60% homology to SEQ ID NO:1, such as at least 65%, at least 70%, at least 75%, at least 80%, at least 85%, at least 90%, at least 91 %, at least 92%, at least 93%, at least 94%, at least 95%, at least 96%, at least 97%, at least 98% or at least 99% homology to JP170, i.e. to SEQ ID NO:1. Thus, among others the alkaline proteases KP43, KP1790, KP9860, Protease Ya, Protease E-1 and SD-521 are subtilases belonging to the JP170 subgroup of subtilases.

A JP170 subtilase suitable for the purpose described herein may be a subtilase homologous to the three-dimensional structure of JP170, i.e. it may be homologous to the three-dimensional structure defined by the structure coordinates in Appendix 1.

As it is well-known to a person skilled in the art that a set of structure coordinates for a protein or a portion thereof is a relative set of points that define a shape in three dimensions, it is possible that an entirely different set of coordinates could define an identical or a similar shape. Moreover, slight variations in the individual coordinates may have little or no effect on the overall shape.

These variations in coordinates may be generated because of mathematical manipulations of the structure coordinates. For example, the structure coordinates of JP170 (Appendix 1) may be manipulated by crystallographic permutations of the structure coordinates, fractionalization of the structure coordinates, integer additions or subtractions to sets of the structure coordinates, inversion of the structure coordinates or any combination of the above. Alternatively, said variations may be due to differences in the primary amino acid sequence.

If such variations are within an acceptable standard error, such as 0.8 Å, as compared to the structure coordinates of Appendix 1, said three-dimensional structure is within the context of the present invention to be understood as being homologous to the structure of Appendix 1. The standard error may typically be measured as the root mean square deviation of e.g. conserved backbone residues, where the term "root mean square deviation" (RMS) means the square root of the arithmetic mean of the squares of the deviations from the mean.

As it is also well-known to a person skilled in the art that within a group of proteins which have a homologous structure there may be variations in the three-dimensional structure in certain areas, sub-structures or domains of the structure, e.g. loops, which are not or at least only of a small importance to the functional domains of the structure, but which may result in a big root mean square deviation of the conserved residue backbone atoms between said structures.

Thus it is well known that a set of structure coordinates is unique to the crystallised protein. No other three dimensional structure will have the exact same set of coordinates, be it a homologous structure or even the same protein crystallised in a different manner. There are natural fluctuations in the coordinates. The overall structure and the inter-atomic relationship can be found to be similar. The similarity can be discussed in terms of root mean square deviation of each atom of a structure from each "homologous" atom of another structure. However, only identical proteins have the exact same number of atoms. Therefore, proteins having a similarity below 100% will normally have a different number of atoms, and thus the root mean square deviation can not be calculated on all atoms, but only the ones that are considered "homologous". A precise description of the similarity based on the coordinates is thus difficult to describe and difficult to compute for homologous proteins. Regarding the present invention, similarities in 3D structure of different subtilases can be described by the content of homologous structural elements, and/or the similarity in amino acid or DNA sequence. For sequences having no deletions or insertions a RMS for the CA carbon atoms can be calculated.

Optionally a JP170 type subtilase is further characterised as comprising the following structural characteristics:

　　a) a twisted beta-sheet with 7 strands,
　　b) six alpha helices,
　　c) at least three ion-binding sites, and

not comprising the Strong and Weak ion-binding site of the BPN' like subtilases

Further the isolated nucleic acid sequence encoding a JP170 subtilase of the invention hybridizes with a complementary strand of a nucleic acid sequence encoding the amino acid sequence of SEQ ID NO:1 preferably under low stringency conditions, at least under medium stringency conditions, at least under medium/high stringency conditions, at least under high stringency conditions, at least under very high stringency conditions.

Suitable experimental conditions for determining hybridization at low, medium, or high stringency conditions between a nucleotide probe and a homologous DNA or RNA sequence involves presoaking of the filter containing the DNA fragments or RNA to hybridize in 5 x SSC (Sodium chloride/Sodium citrate, Sambrook et al. 1989) for 10 min, and prehybridization of the filter in a solution of 5 x SSC, 5 x Denhardt's solution (Sambrook et al. 1989), 0.5 % SDS and 100 $\mu$g/ml of denatured sonicated salmon sperm DNA (Sambrook et al. 1989), followed by hybridization in the same solution containing a concentration of 10ng/ml of a random-primed (Feinberg, A. P. and Vogelstein, B. (1983) Anal. Biochem. 132:6-13), $^{32}$P-dCTP-labeled (specific activity > 1 x 10$^9$ cpm/$\mu$g) probe for 12 hours at ca. 45°C. The filter is then washed twice for 30 minutes in 2 x SSC, 0.5 % SDS at least * 55°C (low stringency), more preferably at least 60°C (medium stringency), still more preferably at least 65°C (medium/high stringency), even more preferably at least 70°C (high stringency), and even more preferably at least 75°C (very high stringency).

<u>BPN' subtilases</u>

A BPN' subtilase or BPN' type subtilase is in the context of the present invention to be understood as a subtilase belonging to the Subtilisin group according Siezen et al. Siezen et al. Protein Science 6 (1997) 501-523 and which has at least 61% homology to SEQ ID NO:4. In particular a BPN' subtilase may have at least 65%, such as at least 70%, at least 75%, at least 80%, at least 85%, at least 90%, at least 91 %, at least 92%, at least 93%, at least 94%, at least 95%, at least 96%, at least 97%, at least 98% or at least 99% homology to BPN', i.e. to SEQ ID NO:4.

Further the isolated nucleic acid sequence encoding a BPN' subtilase of the invention hybridizes with a complementary strand of the nucleic acid sequence encoding the amino acid sequence of SEQ ID NO:4 preferably under low stringency conditions, at least under medium stringency conditions, at least under medium/high stringency conditions, at least under high stringency conditions, at least under very high stringency conditions.

In one embodiment of the present invention a BPN' subtilase suitable for the purpose described herein may be a subtilase homologous to the three-dimensional structure of BPN' as defined by the structure coordinates given in PDB Nos. 1 SBT and 1 GNS (Protein Data Bank), or one of the several other structures of BPN' that are accessible from the Protein Data Bank. Variations between homologous structures may occur for several reasons as described above. Thus a BPN' subtilase within the context of the present invention is to be understood as any subtilase having the structural characteristics pertaining to the BPN' subtilases as described above, and in addition such subtilases do preferably not have further structural characteristics which are not present in the BPN' subtilases as described herein. In the context of the present invention a BPN' type subtilase has two ion-binding sites. A BPN' like subtilase may, in the context of the present invention, belong to branch I-S of the subtilisins i.e. to branch I-S1, the "true" subtilisins or I-S2, the highly alkaline proteases (Siezen et al., Protein Engng. 4 (1991) 719-737).

Examples of BPN' type subtilases include the subtilisin 309 (PDB NO:1SVN, SAVINASE®, NOVOZYMES A/S) and subtilisin Carlsberg (ALCALASE®, NOVOZYMES A/S), among others.

In connection with Figure 1 of R.J. Siezeri and J.A.M Leunissen (Protein science, Vol. 6 (3), pp. 501-523, 1997) page 502 a structure of subtilases is described as: A subtilase consists of 6-8 helices, 11 strands of which 7 are central in a twisted beta-sheet. Two ion-binding sites are mentioned, the so called "Strong" and "Weak" calcium-binding sites. It was later discovered that for some structures (subtilisin DY PDB no. 1 BH6, 1998), the Weak calcium-binding site was shown to be a Na (sodium) binding site when the calcium concentration in the crystallization medium was low. Thus, in the following we refer to ion-binding sites instead of calcium-binding sites.

TY145 subtilases

A TY145 subtilase or TY145 type subtilase is in the context of the present invention to be understood as a subtilase which has at least 63% homology to SEQ ID NO:7. In particular said TY145 subtilase may have at least 65%, such as at least 70%, at least 74%, at least 80%, at least 83%, at least 90%, at least 91%, at least 92%, at least 93%, at least 94%, at least 95%, at least 96%, at least 97%, at least 98% or at least 99% homology to TY145, i.e. to SEQ ID NO:7.

In one embodiment of the present invention a TY145 subtilase suitable for the purpose described herein may be a subtilase homologous to the three-dimensional structure of TY145 as defined by the structure coordinates given in PCT/DK2004/000066. Variations between homologous structures may occur for several reasons as described above. Thus a TY145 subtilase within the context of the present invention is to be understood as any subtilase having the structural characteristics pertaining to the TY145 subtilases as described above, and in addition such subtilases do preferably not have further structural characteristics which are not present in the TY145 subtilases as described herein.

Typically a TY145 subtilase further comprises the following structural characteristics:

> a) a twisted beta-sheet with 7 strands,
> b) six alpha helices,
> c) at least three ion-binding sites, wherein the Strong ion-binding site of the BPN' type subtilases is not present,

Examples of subtilases of the T Y145 type include the T Y145 subtilase, the psychrophilic subtilisin protease S41 derived from the Antarctic Bacillus TA41, herein also called TA41 subtilase (Davail S et al., 1994, J. Biol. Chem., 269, 17448-17453), and the psychrophilic subtilisin protease S39 derived from the Antarctic Bacillus TA39, herein also called TA39 subtilase (Narinx E et al., 1997, Protein Engineering, 10 (11), 1271-1279).

**Three-dimensional structure of JP170 subtilases**

The JP170 subtilase was used to elucidate the three-dimensional structure forming the basis for the present invention.

The structure of JP170 was solved in accordance with the principle for x-ray crystallographic methods, for example, as given in X-Ray Structure Determination, Stout, G.K. and Jensen, L.H., John Wiley & Sons, Inc. NY, 1989.

The structural coordinates for the solved crystal structure of JP170 are given in standard PDB format (Protein Data Bank, Brookhaven National Laboratory, Brookhaven, CT) as set forth in Appendix 1. It is to be understood that Appendix 1 forms part of the present application. In the context of Appendix 1, the following abbreviations are used: CA refers to c-alpha (carbon atoms) or to calcium ions, (however to avoid misunderstandings we normally use the full names "c-alpha atoms" and "calcium" or "ion" in the present specification). Amino acid residues are given in their standard three-letter code. The attached structural coordinates contain the protease structure, and an inhibitor structure Cl2 as well as water molecules. The protease coordinates has a chain identification called A, whereas the Cl2 inhibitor is called B, the calcium ions are called C, and the water is W. In the following the positions of the mentioned residues refer to the sequence of JP170 as disclosed in SEQ ID NO:1.

The JP170 structure consists of two domains, a catalytic domain and a C-terminal domain.

The structure of the catalytic domain shows the same overall fold as found in the S8 family of subtilisins. The structure comprises a twisted beta-sheet with 7 strands arranged in the following sequential order S2, S3, S1, S4, S5, S6, S7.

There are six alpha helices in the catalytic domain structure of which number H1 contains residues 9-17, H 2 contains residues 6 8-76, H 3 contains residues 110-119, H 4 contains residues 139-150, H5 contains residues 253-273 and H6 contains residues 281-291.

The C-terminal domain comprises a strand motif, a so called "beta sandwich" consisting of sheets a and b. The sheet in this domain is combined of strands in an antiparallel fashion, whereas the strand in the catalytic domain is combined in parallel. The sequential order of the strands can be denoted as: S1a-S1b-S3a-S3b-S4b-S4a-S2b-S2a with the beta sandwich organised as to the two sheets S1a, S3a, S4a, S2a and S1 b, S3b, S4b, S2b.

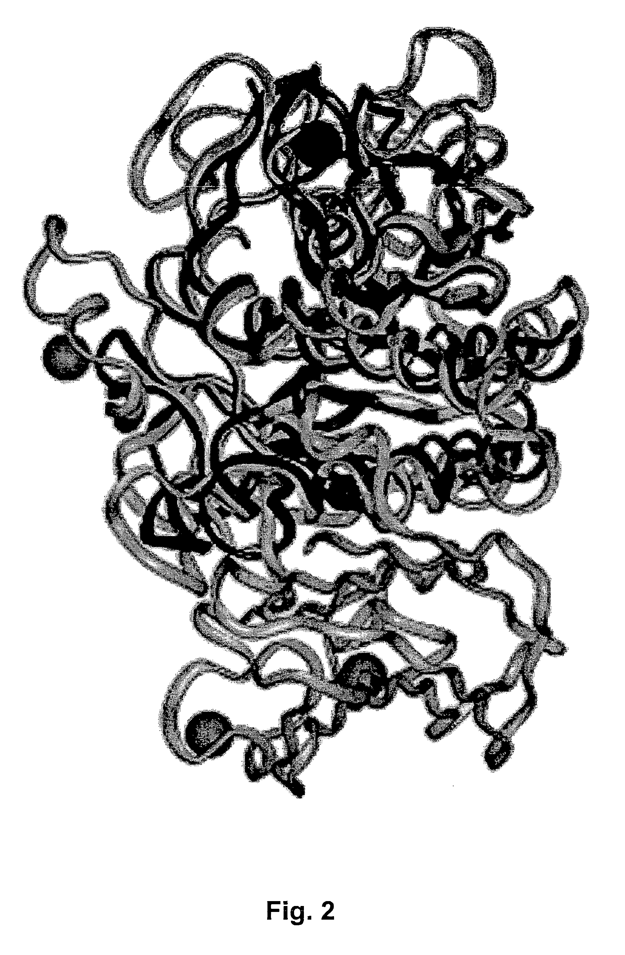

The JP170 subtilases were found to lack the well-known Strong and Weak ion-binding sites of the BPN' subtilases. However, the JP170 subtilases have three ion-binding sites which are not present in the BPN' subtilisin structures. This can be seen in the structural alignment presented in Fig. 2. These three ion-binding sites are hereinafter referred to as Site 1, which is placed in the catalytic domain, and Site 2 and 3 which are placed in the non-catalytic C-terminal domain.

Thus in relation to the atomic coordinates disclosed in Appendix 1, the ion-binding sites of JP170 are located at:

Site 1 - calcium atom named A601 CA
Site 2 - calcium atom named A603 CA, and
Site 3 - calcium atom named A602 CA in the PDB table (Appendix 1).

The position of an ion-binding site can be defined by the distance to four specific atoms in the core structure. The distance from the ion-binding site to the c-alpha atoms of the three active site residues has been chosen. Throughout the subtilases the residues Ser, His and Asp in the active site are highly conserved. In JP170 they are Asp30, His68 and Ser254. The fourth distance chosen is the distance to the c-alpha atom of the amino acid residue coming first after the active site serine residue in the sequence (herein after called "next to Ser"); in the 3D structure of JP170 it is Met255.

In a preferred embodiment of the present invention, the distance between:

a) ion-binding site 1 and

i) Asp c-alpha atom is 26.70-28.70Å,
ii) His c-alpha atom is 22.10-24.10Å,
iii) Ser c-alpha atom is 16.95-18.95Å,
iv) next to Ser c-alpha atom is 15.30-17.30Å,

b) ion-binding site 2 and

i) Asp c-alpha atom is 33.50-35.50Å,
ii) His c-alpha atom is 37-39/Å,
iii) Ser c-alpha atom is 29.40-31.40Å,
iv) next to Ser c-alpha atom is 30.70-32.70Å,

c) ion-binding site 3 and

i) Asp c-alpha atom is 41.50-43.50Å,
ii) His c-alpha atom is 42.90-44.90Å,
iii) Ser c-alpha atom is 34.50-36.50Å,
iv) next to Ser c-alpha atom is 35-37Å.

Below the specific distances between the four chosen c-alpha atoms and the three ion binding sites of the JP170 subtilase; and the distances between the ion binding sites are given in A:

|  | site 1 | site 2 | site 3 |
| --- | --- | --- | --- |
| Asp30 | 27.69 | 34.49 | 42.48 |
| His68 | 23.12 | 38.03 | 43.87 |
| Ser254 | 17.95 | 30.41 | 35.51 |
| Met255 | 16.34 | 31.68 | 36.02 |
| site 1 | 0 | 35.29 | 32.92 |
| site 2 | 35.29 | 0 | 14.08 |
| site 3 | 32.92 | 14.08 | 0 |

However, these distances may vary from one subtilase to the other. The present distances are given with a calcium ion in the structure. If a sodium ion was bound instead the distances would be shifted a little bit. Generally the distances can vary $\pm 0.80$Å, preferably $\pm 0.70$Å, $\pm 0.60$Å, $\pm 0.50$Å, $\pm 0.40$Å, or most preferably $\pm 0.30$Å.

Further, in the JP170 like subtilases, the peptide structure circumscribing ion-binding site 1 up to a distance of 10 Å from the metal ion is composed of the amino acid residues placed in positions 183-189, 191-204 and 224-225.

The peptide structure circumscribing ion-binding site 2 up to a distance of 10 Å from the metal ion is composed of residues 378-393.

The peptide structure circumscribing ion-binding site 3 up to a distance of 10 Å from the metal ion is composed of residues 348, 350, 352, 363-370, 380-383, 391-400 and 414-420.

**Comparison to the I-S1 and I-S2 subgroups (BPN' like subtilases)**

In comparison to the BPN' like subtilase structures the structure of the JP170 like subtilases can be divided into a "core subtilase-like" region, an "intermediate" region and a "nonhomologous" region.

The active site can be found in the core subtilase-like region, which is structurally closely related to the BPN' structures. The core subtilase-like region is composed of residues 17-34, 197-209 and 216-232, and contains the alpha-helix H3 and the central alpha-helix H5 in which the active site serine residue is situated in the N-terminal part. The core subtilase-like region has an RMS lower than 1.2.

Outside the core subtilase-like region the structure of the JP170 like subtilase differs from the BPN' structures to a greater extent.

The intermediate region consists of residues 42-46, 150-186, 245-272 and 278-296. The intermediate region has an RMS bigger than 1.2 and less than 1.8. The relationships between the three-dimensional structure and functionality are potentially difficult to predict in this region of the JP170 like subtilases.

The nonhomologous region consists of residues 1-16, 35-41, 47-149, 187-196, 210-215, 233-244, 273-277 and 297-316. The nonhomologous region has a RMS higher than 1.8. The relationships between the three-dimensional structure and functionality are very difficult to predict in this region of the JP170 like subtilases.

Many loops in the 3D structure of the JP170 like subtilases differ significantly from the BPN' type structures, both in length and in content of amino acid residues. The following loops or protein sequence stretches of JP170 are compared to Savinase (BPN' numbering in parenthesis, (cf. Fig. 4)):

G32-H43 (G34-H39)
E44-Y54 (P40-A48)
G57-G67 (V51-G63)
N79-N82 (I75-V81)
I96-P107 (V95-S105)
A108-S119 (106-N117)
A131-Y137 (S128-S132)
T138-D152 (A133-G146)
E162-1169 (S156-I165)
G173-T180 (A169-A176)
E185-N199 (D181-N184)
G208-D218 (G193-0197)
S232-K246 (G211-T213)
D294-N303 (S256-L262)

The loops N79-N82 (I75-V81) and G208-D218 (G193-D197) are in contact with a ion-binding site in Savinase, but not in JP170. Similarly the loop E185-N199 (D181-N184) is in contact with a ion-binding site in JP170, but not in Savinase. This knowledge opens for possibilities of adding or removing ion-binding sites to subtilases of the JP170 and BPN' like types.

A good example of the difference is the loop S232-K246 in JP170 which has 15 residues compared to the corresponding BPN' type loop G211-T213 (in Savinase), which has only three residues. In the JP170 like subtilases, the loop folds back to the substrate binding site, especially the P' parts of the substrate binding site. The loop is situated close to the substrate as illustrated by the CI2 inhibitor bound in the 3D structure attached (Appendix 1).

The location of loop S232-K246 in JP170 can be described in relation to the four specific residues as described above. The distance from the CA atom of residue W240 in the loop to the CA atoms of the active site residues are:

| Residue | H68 | D30 | S254 | M255 |
|---------|-----|-----|------|------|

(continued)

Distance, Å      11.45      18.51      13.06      11.94

As mentioned above, distances like these can vary ±0.80Å, preferably ±0.70Å, ±0.60Å, ±0.50Å, ±0.40Å, or most preferably ±0.30Å.

Furthermore, distances from the residues of JP170 loop S232-K246 to atoms of the Cl2 inhibitor can be calculated. These distances are:

    from CA atom of W240 to CA atom of R62 in Cl2 is 7.49Å,
    from CA atom of F239 to CA atom of R62 in Cl2 is 8.39Å,
    from CA atom of S238 to CA atom of R62 in Cl2 is 8.42Å,
    from CA atom of S237 to CA atom of R62 in Cl2 is 9.44Å,
    from CA atom of S238 to CA atom of E60 in Cl2 is 9.42Å.

The distances from JP170 active site residue S254 to atoms of the Cl2 inhibitor, as placed in the 3D coordinates of Appendix 1, are:

    from CA atom of S254 to CA atom of E60 in Cl2 is 5.25Å,
    from CA atom of S254 to CA atom of R62 in Cl2 is 11.55Å,
    from CA atom of S254 to CA atom of T58 in Cl2 is 7.06Å,
    from CA atom of S254 to CA atom of M59 in Cl2 is 4.71Å.

The distances can vary ±0.80Å, preferably ±0.70Å, ±0.60Å, ±0.50Å, ±0.40Å, or most preferably ±0.30Å.

A preferred JP170 like subtilase variant has a deletion in the region S232-K246, and the subsequent insertion of one or more residues to partly or completely remove the loop. Preferred variants comprises the deletion of L233-S245 + insertion of Asn, deletion of L233-D244 + insertion of Gly or deletion of S232-D244 + insertion of Gly.

Similar considerations can be made in respect of differences to the TY145 structure.

**Homology building of JP170, BPN' and TY145 subtilases**

A model structure of a JP170 type subtilase, a BPN' type subtilase or a TY145 type subtilase can be built using the Homology program or a comparable program, e.g., Modeller (both from Molecular Simulations, Inc., San Diego, CA). The principle is to align the amino acid sequence of a protein for which the 3D structure is known with the amino acid sequence of a protein for which a model 3D structure has to be constructed. The structurally conserved regions can then be built on the basis of consensus sequences. In areas lacking homology, loop structures can be inserted, or sequences can be deleted with subsequent b onding of the necessary residues using, e.g., the program Homology. Subsequent relaxing and optimization of the structure should be done using either Homology or another molecular simulation program, e.g., CHARMm from Molecular Simulations.

**Methods for designing JP170, BPN', and TY145 subtilase variants**

Comparisons of the molecular dynamics of different proteins can give a hint as to which domains are important or connected to certain properties pertained by each protein.

Thus the present invention relates to a method for constructing a variant of a parent subtilase, wherein the variant has at least one altered property as compared to said parent subtilase, which method comprises:

    a) analyzing the three-dimensional structure of the parent subtilase to identify, on the basis of an evaluation of structural considerations in relation to a JP170 three dimensional structure, at least one amino acid residue or at least one structural region of the subtilase, which is of relevance for altering said property;
    b) modifying the DNA of the polynucleotide encoding the parent to construct a polynucleotide encoding a variant subtilase, which in comparison to the parent subtilase, has been modified by deletion, substitution or insertion of the amino acid residue or structural part identified in i) so as to alter said property;
    c) expressing the variant subtilase in a suitable host, and
    d) testing the resulting subtilase variant for said property.

More specifically the invention relates to a method of producing a subtilase variant, wherein the variant has at least one altered property as compared to a parent subtilase, which method comprises:

a) producing a model structure of the parent subtilase on the three-dimensional structure of BPN', TY145 or JP170; or producing an actually determined three-dimensional structure of the parent subtilase,

b) comparing the model or actual three-dimensional structure of the parent subtilase to the JP170 structure by superimposing the structures through matching the CA, CB, C, O, and N atoms of the active site residues,

c) identifying on the basis of the comparison in step b) at least one structural part of the parent subtilase, wherein an alteration in said structural part is predicted to result in an altered property;

d) modifying the nucleic acid sequence encoding the parent subtilase to produce a nucleic acid sequence encoding at least one deletion or substitution of one or more amino acids at a position corresponding to said structural part, or at least one insertion of one or more amino acid residues in positions corresponding to said structural part;

e) performing steps c) and d) iteratively N times, where N is an integer with the value of one or more;

f) preparing the variant resulting from steps a) - e);

g) testing the properties of said variant; and

h) optionally repeating steps a) - g) recursively; and

i) selecting a subtilase variant having at least one altered property as compared to the parent subtilase.

j) expressing the modified nucleic acid sequence in a host cell to produce the variant subtilase;

k) isolating the produced subtilase variant;

l) purifying the isolated subtilase variant and

m) recovering the purified subtilase variant.

The present invention thus generally relates to the use of the JP170 structure as provided herein for the identification of desired modifications in subtilases of any of the three subtilisin types, the BPN' types (I-S1 and I-S2 subgroups), the TY145 types and the JP170 types through modelling the 3-D structure of a parent subtilase to the type it belongs to and subsequent comparison thereof to the JP170 3-D structure, or in instances where the 3-D structure of the parent subtilase is actually known by comparison thereof to the JP170 3-D structure.

Based on this comparison at least one residue in the parent subtilase is selected for modification by substitution, deletion or insertion in order to provide a subtilase variant with altered properties in comparison to the parent subtilase.

In one embodiment the parent subtilase may therefore belong to the sub-group IS1, preferably selected from the group consisting of ABSS168, BASBPN, BSSDY, and BLSCAR, or functional variants thereof having retained the characteristic of sub-group IS1.

In another embodiment the parent subtilase belongs to the sub-group I-S2, preferably selected from the g roup consisting of BLS147, BLS309, BAPB92, and BYSYAB, or functional variants thereof having retained the characteristic of sub-group I-S2.

In a further embodiment the parent subtilase belongs to the TY145 type subgroup, preferably selected from the group comprising TY145, protease S41 also called TA41 protease S39 also called TA39 subtilase, etc.

Specifically the parent subtilase belongs to the JP170 type subgroup, preferably selected from the group comprising JP170, KP43, KP9860, Protease E-1, Protease Ya, Protease SD-521, etc.

A further embodiment of the invention relates to a method of producing a JP170 type subtilase variant, wherein the variant has at least one altered property as compared to a parent subtilase, which method comprises:

a) producing a model structure of the parent JP170 type subtilase on the three-dimensional structure of JP170; or producing an actually determined three-dimensional structure of the parent subtilase,

b) comparing the model or actual three-dimensional structure of the parent JP170 type subtilase to the BPN' or TY145 structure by superimposing the structures through matching the CA, CB, C, O, and N atoms of the active site residues,

c) identifying on the basis of the comparison in step b) at least one structural part of the parent JP170 type subtilase, wherein an alteration in said structural part is predicted to result in an altered property;

d) modifying the nucleic acid sequence encoding the parent JP170 type subtilase to produce a nucleic acid sequence encoding at least one deletion or substitution of one or more amino acids at a position corresponding to said structural part, or at least one insertion of one or more amino acid residues in positions corresponding to said structural part;

e) performing steps c) and d) iteratively N times, where N is an integer with the value of one or more;

f) preparing the JP170 type subtilase variant resulting from steps a) - e);

g) testing the properties of said variant; and

h) optionally repeating steps a) - g) recursively; and

i) selecting a JP170 type subtilase variant having at least one altered property as compared to the parent subtilase.

j) expressing the modified nucleic acid sequence in a host cell to produce the variant subtilase;

k) isolating the produced JP170 type subtilase variant;

l) purifying the isolated subtilase variant and

m) recovering the purified subtilase variant.

The invention also comprises the protease variants produced by the above methods.

**Stability - alteration of ion-binding sites**

As described above the three-dimensional structure of JP170 subtilases as provided in Appendix 1 indicates the presence of three ion-binding sites not present in the BPN' subtilisin structures, thus lacking the Strong and Weak ion-binding site of the BPN' subtilases. Stability of ion-binding sites is important for the functionality of the enzyme. Therefore alterations of the ion-binding sites are likely to result in alterations of the stability of the enzyme.

Improved stability

Stabilisation of a JP170 subtilase may possibly be obtained by alterations in the positions close to the ion-binding sites. Thus in one embodiment of the method of the invention step (c) above identifies amino acid residue positions located at a distance of 10Å or less to the ion-binding site of the JP170 type parent, preferably positions located at a distance of 6 Å or less.

Thus a preferred variant of the present invention has a modification in one or more of the positions located at a distance of 10Å to the ion-binding sites of JP170 (SEQ ID NO:1). These positions are:

| Site 1: | 183-189 | (i.e. positions 183, 184, 185, 186, 187, 188, 189), |
| | 191-204 | (i.e. positions 191, 192, 193, 194, 195, 196, 197, 198, 199, 200, 201, 202, 203, 204), |
| | 224-225; | |
| Site 2: | 378-393 | (i.e. positions 378, 379, 380, 381, 382, 383, 384, 385, 386, 387, 388, 389, 390, 391, 392, 393); |
| Site 3: | 348, 350, 352, | |
| | 363-370 | (i.e. positions 363, 364, 365, 366, 367, 368, 369, 370), |
| | 380-383 | (i.e. positions 380, 381, 382, 383), |
| | 391-400 | (i.e. positions 391, 392, 393, 394, 395, 396, 397, 398, 399, 400), |
| | 414-420 | (i.e. positions 414, 415, 416, 417, 418, 419, 420). |

Corresponding positions in other JP170 type subtilases may be identified as disclosed above or by using Fig. 1 herein..

In detergent compositions calcium chelaters contribute to removal of calcium from the subtilases with subsequent inactivation of the enzyme as the result. To decrease the inactivation due to calcium removal of e.g. calcium chelaters variants with improved calcium stability was constructed.

Preferred variants stabilised in ion-binding site 1 are S193Q, Y; H200D,N and H200D,N+D196N.

Preferred variants stabilised in ion-binding site 2 are N390D and N391 D, and preferred variants stabilised in ion-binding site 3 are G394N,Q,F,Y,S and W392S,N,Q.

**Alteration of thermostability**

A variant with improved stability (typically increased thermostability) may be obtained by substitution with proline, introduction of a disulfide bond, altering a hydrogen bond contact, altering charge distribution, introduction of a salt bridge, filling in an internal structural cavity with one or more a mino acids with bulkier s ide groups (in e.g. regions which are structurally mobile), substitution of histidine residues with other amino acids, removal of a deamidation site, or by helix capping.

Regions with increased mobility

The following regions of JP170 have an increased mobility in the crystal structure of the enzyme, and it is presently believed that these regions can be responsible for stability or activity of JP170. Especially thermostabilisation may possibly be obtained by altering the highly mobile regions. Improvements of the enzyme can be obtained by mutation in the below regions and positions. Introducing e.g. larger residues or residues having more atoms in the side chain could increase the stability, or, e.g., introduction of residues having fewer atoms in the side chain could be important for the mobility and thus the activity profile of the enzyme.

Two methods are used extract the highly mobile regions from a 3D structure. One is a molecular dynamics calculation of the isotropic fluctuations by using the program CHARMm from MSI (Molecular Simulations Inc.), and the other is an analysis of the B-factors. The B-factors are listed in Appendix 1 and give a value to the uncertainty of determination of the location of the various atoms of the structure. The uncertainty relates to the mobility of the atoms in the molecules in the crystal lattice. This mobility reflects the thermal motion of the atoms and thus indicates possible sites for thermostabilisation of the enzyme.

Thus, by analysing the B-factors taken from the coordinate file in Appendix 1, (see "X-Ray Structure Determination, Stout, G.K. and Jensen, L.H., John Wiley & Sons, Inc. NY, 1989") the following mobile regions in the JP170 structure were determined:

| | |
|---|---|
| 13-18 | (i.e. positions 13, 14, 15, 16, 17, 18), |
| 37-43 | (i.e. positions 37, 38, 39, 40, 41, 42, 43), |
| 47-50 | (i.e. positions 47, 48, 49, 50), |
| 57-59 | (i.e. positions 57, 58, 59), |
| 96-103 | (i.e. positions 96, 97, 98, 99, 100, 101, 102, 103), |
| 131-134 | (i.e. positions 131, 132, 133, 134), |
| 152-153 | |
| 162-166 | (i.e. positions 162, 163, 164, 165, 166), |
| 188-195 | (i.e. positions 188, 189, 190, 191, 192, 193, 194, 195), |
| 210 | |
| 234-246 | (i.e. positions 234, 235, 236, 237, 238, 239, 240, 241, 242, 243, 244, 245, 246), |
| 372-378 | (i.e. positions 372, 373, 374, 375, 376, 377, 378), |
| 387-392 | (i.e. positions 387, 388, 389, 390, 391, 392), |
| 406-407 | |
| 419. | |

Molecular dynamics simulations at 300K and 400K of JP170 provided the following highly mobile regions:

| | |
|---|---|
| 37-42 | (i.e. positions 37, 38, 39, 40, 41, 42), |
| 57-60 | (i.e. positions 57, 58, 59, 60), |
| 66-67, | |
| 98-103 | (i.e. positions 98, 99, 100, 101, 102, 103), |
| 107-111 | (i.e. positions 107, 108, 109, 110, 111), |
| 188-193 | (i.e. positions 188, 189, 190, 191, 192, 193), |
| 236-240 | (i.e. positions 236, 237, 238, 239, 240), |
| 326-332 | (i.e. positions 326, 327, 328, 329, 330, 331, 332), |
| 337-342 | (i.e. positions 337, 338, 339, 340, 341, 342), |
| 355-360 | (i.e. positions 355, 356, 357, 358, 359, 360), |
| 372-377 | (i.e. positions 372, 373, 374, 375, 376, 377), |
| 384-388 | (i.e. positions 384, 385, 386, 387, 388), |
| 404-411 | (i.e. positions 404, 405, 406, 407, 408, 409, 410, 411). |

Thus, a preferred JP170 subtilase variant of the present invention has been modified in one or more of the above mentioned positions of SEQ ID NO:1. Further preferred variants comprises one or more alterations in the regions 57-60, 66-67, 107-111, 236-240, 326-332, 355-360, 372-377, 384-388, 404-411. Especially preferred is variant W240H, Y and variants modified in the region 355-360, such as variants comprising one or more of the modifications: G355A, S; S356T,N; T357N,Q,D,E,P; T358S; A359S,T,N,Q and S360T,N.

Variants modified in the region 355-360 may be produced in accordance with the method for random mutagenesis by use of the DOPE program as described herein. To obtain variants comprising 1-3 modifications in region 355-360 one may introduce the substitutions with the following frequencies:

| wild-type | modified |
|---|---|
| 95% | 5% G355A,S |
| 90% | 10% S356T,N |
| 80% | 20% T357N,Q,D,E,P |
| 90% | 10% T358S |
| 80% | 20% A359S,T,N,Q |

(continued)

| wild-type | modified |
|---|---|
| 80% | 20% S360T,N. |

Disulfide bonds:

A JP170 variant of the present invention with improved stability, e.g. thermostability, as compared to the parent JP170 subtilase may be obtained by introducing new interdomain or intra-domain bonds, such as by establishing inter- or intra-domain disulfide bridges.

Thus a further aspect of the present invention relates to a method for producing a variant of a parent JP170, wherein step (c) identifies amino acid residue positions in the parent JP170 type subtilase, the modification of which may create at least one disulfide bridge by insertion of or substitution with at least one Cys residue.

The below mentioned amino acid residues identified in the amino acid sequence of SEQ ID NO:1 are considered as being suitable for cysteine replacement. With one or more of these substitutions with cysteine, disulfide bridges may possibly form in a variant of JP170. The substitutions are: G21C+A86C, V26C+A265C, G57C+G105C, G74C+A229C, Q111C+N143C, G160C+S170C, A286C+V349C, A27C+A122C, A45C+G78C, V72C+P258C, G78C+A229C, D98C+G104C, Q111C+Y147C, G135C+G167C, R142C+P354C, V144C+A178C, G182C+P217C, A183C+G223C, A195C+Y225C, F271C+P279C, A287C+A430C, A293C+T310C, E322C+S428C, S324C+A332C, S327C+P424C, D352C+N397C, G355C+T362C, G291C+S314C.

Preferred variants comprise one or more of the substitutions: G21C+A86C, V26C+A265C, G57C+G105C, G74C+A229C, Q111C+Y143C, G160C+S170C, A286C+V349C, A4C+P222C and A27C+A117C.

Similar residues suitable for cysteine replacement in subtilases homologous with JP170 can be elucidated by finding the homologous positions in the alignment of Figure 1. Concerning another JP170 like sequence the homologous positions suitable for cysteine replacement can be selected by aligning said JP170 like sequence with all of the sequences of Figure 1 using the GAP analysis method as described above. The suitable residues can then be selected in accordance with the homologous positions in the most homologous of SEQ ID's NO:1, 2 and 3 which are the sequences of the subtilases aligned in Figure 1.

**Surface charge distribution**

A variant with improved stability (typically improved thermostability) as compared to the parent subtilase may be obtained by changing the surface charge distribution of the subtilase. For example, when the pH is lowered to about 5 or below histidine residues typically become positively charged and, consequently, unfavorable electrostatic interactions on the protein surface may occur. By engineering the surface charge of the subtilase one may avoid such unfavorable electrostatic interactions that in turn lead to a higher stability of the subtilase.

Charged amino acid residues are (a) positively charged: Lys, Arg, His (pH<5), Tyr (pH>9) and Cys (pH>10) and (b) negatively charged: Asp and Glu.

Therefore, a further aspect of the present invention relates to method for constructing a variant of a parent subtilase, the method comprising:

a) identifying, on the surface of the parent subtilase, preferably a JP170 like or a BPN' like subtilase, at least one amino acid residue selected from the group consisting of Asp, Glu, Arg, Lys and His;
b) substituting, on the surface of the parent subtilase, at least one amino acid residue selected from the group consisting of Asp, Glu, Arg, Lys and His with an uncharged amino acid residue;
c) optionally repeating steps a) and b) recursively;
d) optionally, making alterations each of which is an insertion, a deletion or a substitution of an amino acid residue at one or more positions other than b);
e) preparing the variant resulting from steps a) - d);
f) testing the stability of said variant; and
g) optionally repeating steps a) - f) recursively; and
h) selecting a subtilase variant having increased stability as compared to the parent subtilase.

As will be understood by the skilled person it may also, in some cases, be advantageous to substitute an uncharged amino acid residue with an amino acid residue bearing a charge or, alternatively, it may in some cases be advantageous to substitute an amino acid residue bearing a charge with an amino acid residue bearing a charge of opposite sign. Thus, the above-mentioned method may easily be employed by the skilled person also for these purposes. In the case of substituting an uncharged amino acid residue with an amino acid residue bearing a charge the above-mentioned method may be employed the only difference being steps a) and b) which will then read:

a) identifying, on the surface of the parent subtilase, at least one uncharged amino acid residue;
b) substituting, on the surface of the parent subtilase, at least one uncharged amino acid residue with a charged amino acid residue selected from the group consisting of Asp, Glu, Arg, Lys and His.

Also in the case of changing the sign of an amino acid residue present on the surface of the subtilase the above method may be employed. Again, compared to the above method, the only difference being steps a) and b) which, in this case, read:

a) identifying, on the surface of the parent subtilase, at least one charged amino acid residue selected from the group consisting of Asp, Glu, Arg, Lys and His;
b) substituting, on the surface of the parent subtilase, at least one charged amino acid residue identified in step a) with an amino acid residue having an opposite charge.

Thus, Asp may be substituted with Arg, Lys or His; Glu may be substituted with Arg, Lys or His; Arg may be substituted with Asp or Glu; Lys may be substituted with Asp or Glu; and His may be substituted with Asp or Glu.

In order to determine the amino acid residues of a subtilase, which are present on the surface of the enzyme, the surface accessible area are measured using the DSSP program (Kabsch and Sander, Biopolymers (1983), 22, 2577-2637). All residues having a surface accessibilty higher than 0 0, 0.10, 0.20, 0.30, 0.35, 0.40, 0.45, 0.50, 0.55 or 0.60 are regarded a surface residue.

Among the amino acid residues found on the surface of JP170 using the above method are N79, N316, L381, K246, K9, K313 and K83. We consider the substitutions N79D, N316D and L381 D of particular interest for stabilisation by introduction of salt bridges, whereas the substitutions K246R, K9R, K313R and K83R are of particular interest for the stabilisation at high pH.

Similar substitutions may be introduced in equivalent positions of other JP170 like subtilases.

**Substitution with proline residues**

Improved thermostability of a subtilase can be obtained by subjecting the subtilase in question to analysis for secondary structure, identifying residues in the subtilase having dihedral angles $\phi$ (phi) and $\psi$ (psi) confined to the intervals [-90°<$\phi$<-40° and -180°<$\psi$<180°], preferably the intervals [-90°<$\phi$-40° and 120°<$\psi$<180°] or [-90°<$\phi$-40° and -50°<$\psi$<10°] and excluding residues located in regions in which the subtilase is characterized by possessing $\alpha$-helical or $\beta$-sheet structure.

After the dihedral angles $\phi$ (phi) and $\psi$ (psi) for the amino acids have been calculated, based on the atomic structure in the crystalline subtilases, it is possible to select position(s) which has/have dihedral phi and psi angles favorable for substitution with a proline residue. The aliphatic side chain of proline residues is bonded covalently to the nitrogen atom of the peptide g roup. The resulting cyclic five-membered ring consequently imposes a rigid constraint on the rotation about the N-C$_\alpha$ bond of the peptide backbone and simultaneously prevents the formation of hydrogen bonding to the backbone N-atom. For these structural reasons, proline residues are generally not compatible with $\alpha$-helical and $\beta$-sheet secondary conformations.

If a proline residue is not already at the identified position(s), the naturally occurring amino acid residue is substituted with a proline residue, preferably by site directed mutagenesis applied on a gene encoding the subtilase in question.

In the group of JP170 type subtilases proline residues can advantageously be introduced at positions 22, 44, 110, 139, 140, 166, 198, 201, 203, 231, 282, 356, 357 and 378. Accordingly, a preferred JP170 variant has one or more of the substitutions: Q22P, E44P, L110P, T139P, D140P, S166P I198P, V201P, Q203P, S231P, S282P, S356P, T357P and K378P. Especially preferred are variants comprising one or more of: E44P, Q203P and S356P.

**Improved activity of JP170 subtilases**

As mentioned, the JP170 subtilases differ greatly from the BPN' like subtilases in having a long apparently non-catalytic C-terminal. A possible truncation of JP170 is the removal of approx. 115 residues including two ion-binding sites, which can be obtained by deletion of or within the region 311-433, which is the non-catalytic C-terminal. Preferred deletions comprises the regions 317-433 or 315-433. Preferably the new C-terminal will be within the region of 311-325. Further, the deletion can be optimised with additional substitutions, such as one or more of L283N,Q; A290S,N and W306H,Y,K.

Preferred truncations comprise:

a) deletion of region 317-433 and the substitutions L283N + A290S + W306H,
b) deletion of region 315-433 and the substitutions L283N + A290S + W306H.

**Substrate binding site**

The substrate binding site is identified by the residues in contact with a substrate model, such as the Cl2 inhibitor. The 3D structure coordinates of the JP170 subtilase with Cl2 bound in the active site are provided in Appendix 1. Without being limited to any theory, it is presently believed that binding between a substrate and an enzyme is supported by favorable interactions found within a sphere 10 Å from the substrate molecule. Examples of such favorable bonds are hydrogen bonds, strong electrostatic interaction and/or hydrophobic interactions.

The following residues of the JP170 subtilase (SEQ ID NO:1), are within a distance of 10Å from the Cl2 inhibitor which is bound to the substrate binding site. These residues are thus believed to be involved in interactions with said substrate:

| | |
|---|---|
| 29-32, | (i.e. residues 29, 30, 31, 32) |
| 64-72, | (i.e. residues 64, 65, 66, 67, 68, 69, 70, 71, 72) |
| 93, | |
| 96-98, | (i.e. residues 96, 97, 98) |
| 100-110, | (i.e. residues 100, 101, 102, 103, 104, 105, 106, 107, 108, 109, 110) |
| 113-114, | |
| 127-136, | (i.e. residues 127, 128, 129, 130, 131, 132, 133, 134, 135, 136) |
| 138-141, | (i.e. residues 138, 139, 140, 141) |
| 144, 157, 174, | |
| 180-183, | (i.e. residues 180, 181, 182, 183) |
| 191, 193-194, | |
| 202-207, | (i.e. residues 202, 203, 204, 205, 206, 207) |
| 211, | |
| 223-226, | (i.e. residues 223, 224, 225, 226) |
| 234-241, | (i.e. residues 234, 235, 236, 237, 238, 239, 240, 241) |
| 249-258 | (i.e. residues 249, 250, 251, 252, 253, 254, 255, 256, 257, 258). |

In an embodiment of the present invention a variant comprises a modification in one or more of the above mentioned positions. A preferred variant is W129L.

**JP170 with extra ion-binding site**

The Strong ion-binding site from the BPN' subtilases can be transplanted into JP170 (or other subtilases in JP170 subgroup) by deletion of N79-N82 and subsequent insertion of LNNSIGV (SEQ ID NO:5), followed by the substitution A45D,N and optionally the substitutions E44P,T and/or R47Q.

**Removal of ion-binding site in JP170**

By removing an ion-binding site it is possible to decrease the enzymes dependency of calcium in the media. The ion-binding sites in JP170 or other JP170 type subtilases can be removed with guidance from information about the three-dimensional structures of other related subtilases, such as a BPN' type subtilase, such as Savinase or BPN', and a TY145 type subtilase.

Removal of ion-binding site 1 can be done by deletion of N186-N199 and subsequent insertion of at least three amino acid residues - or stated differently by the substitution of a region comprising from 3 to 6 amino acid residues for the region comprising 14 amino acid residues in positions 186 to 199, preferably the sequence of the substituting region is SSN (SEQ ID NO:6). Preferably, but not mandatory one or both of the substitutions I7Q and V3Y is further added.

The ion-binding site 1 can be removed from a wild-type JP170 subtilase or a JP170 subtilase truncated as described above.

**Subtilases free of ion-binding sites**

Similarly, information about the three-dimensional structures of JP170 type subtilases and TY145 type subtilases can be used to remove the Strong and Weak ion-binding sites in BPN' type subtilases, or the ion-binding sites in TY145 type subtilases may be removed on the basis of structural information a bout the J P170 and BPN' types of subtilases.

Using Savinase as an example, the removal can be done by altering the loops A194-L196 (weak ion-binding site) and L75-L82 (strong ion-binding site) either by a) insertion or deletion of a number of amino acid residues in the loops or b) by deletion of the entire loop or part of the loop and subsequent insertion of a number of residues from a corresponding loop of a JP170 or TY145 like subtilase.

Preferably the ion-binding sites of Savinase can be removed by either

> i) full or partial deletion of the region A194-L196 (BPN' numbers) and insertion of three or more residues chosen from JP170 positions P209-P217, and
>
> ii) full or partial deletion of the region L75-L82 (BPN' numbers) and insertion of at least one residue chosen from TY145 positions H83-Y92

or

> i) full or partial deletion of the region A194-L196 (BPN' numbers) and insertion of three or more residues chosen from JP170 positions P209-P217 and
>
> ii) full or partial deletion of the region L75-L82 (BPN' numbers) and insertion of at least one residues chosen from JP170 positions N79-K83.

**Removal of critical oxidation sites**

In order to increase the stability of a JP170 type subtilase protease it may be advantageous to substitute or delete critical oxidation sites, such as methionines, with other amino acid residues which are not subject to oxidation.

Accordingly, in a further embodiment the present invention relates to an RP-II protease variant, in which one or more amino acid residues susceptible to oxidation, especially methionine residues exposed to the surface of the molecule, is/are deleted or replaced with another amino acid residue less susceptible to oxidation. The amino acid residue less susceptible to oxidation may for instance be selected from the group consisting of A, E, N, Q, I, L, S and K.

Specific such variants comprises at least one of the deletions or substitutions M42{*,S,A,N,Q,K}; M85{*,S,A, N,Q,K}; M97{*,S,A,N,Q,K}; M153{*,S,A,N,Q,K}; M220{*,S,A,N,Q,K}; M250{*,S,A,N,Q,K}; and M255{*,S,A,N,Q,K} of the JP170 protease; the deletions or substitutions M42{*,S,A,N,Q,K}; M85{*,S,A,N,Q,K}; M97{*,S,A,N,Q,K}; M153{*,S,A,N, Q,K}; M250{*,S,A,N,Q,K}; and M255{*,S,A,N,Q,K} of the SD-521 and Ya proteases.

Stabilization by modification of Asn-Gly pairs

It is known that at alkaline pH, the side chain of Asn may interact with the NH group of a sequential neighbouring amino acid to form an isoAsp residue where the backbone goes through the Asp side chain. This will leave the backbone more vulnerable to proteolysis. The deamidation is much more likely to occur if the residue that follows is a Gly. Changing the Asn in front of the Gly or the Gly will prevent this from happening and thus improve the stability, especially as concerns thermo- and storage stability.

The invention consequently further relates to a subtilase, in which the modificatioins indicated above are either or both residues of any of the Asn-Gly sequence appearing in the amino acid sequence of the parent RP-II protease is/are deleted or substituted with a residue of a different amino acid.

The Asn and/or Gly residue may, for instance, be substituted with a residue of an amino acid selected from the group consisting of A, Q, S, P, T and Y.

More specifically, any of the Asn or Gly residues of the Asn-Gly occupying positions 66-67, 134-135 and/or 375-376 of the SD-521 and Ya protease; and positions 66-67, 134-135, 301-302 and/or 375-376 of the JP170 protease, may be deleted or substituted with a residue of an amino acid selected from the group consisting of A, Q, S, P, T and Y. (positions are indicated in relation to the JP170 protease as indicated in Fig. 1).

Specific variants of JP170 are: N66{*,A,Q,S,P,T,Y}; G67{*,A,Q,S,P,T,Y}; N134{*,A,Q,S,P,T,Y}; G135{*,A,Q,S, P,T,Y}; N301{*,A,Q,S,P,T,Y}; G302{*,A,Q,S,P,T,Y}; N375{*,A,Q,S,P,T,Y}; and G376{*,A,Q,S,P,T,Y}; and combinations thereof, such as N66{*,A,Q,S,P,T,Y}+N134{*,A,Q,S,P,T,Y}, N66{*,A,Q,S,P,T,Y}+N301{*,A,Q,S,P,T,Y}, and N66{*,A,Q, S,P,T,Y}+N375{*,A,Q,S,P,T,Y}, etc.

Specific variants of SD-521 and Ya proteases are: N66{*,A,Q,S,P,T,Y}; G67{*,A,Q,S,P,T,Y}; N134{*,A,Q,S,P, T,Y}; G135{*,A,Q,S,P,T,Y}; and N375{*,A,Q,S,P,T,Y}; G376{*,A,Q,S,P,T,Y}, and combinations thereof as indicated above.

**Modification of Tyrosine residues**

In relation to wash performance it has been found that the modification of certain tyrosine residues to phenylalanine provides an improved wash performance. Without being bound by any specific theory, it is believed that titration of these Tyr residues in the alkaline wash liquor has negative effects that are alleviated by replacing the Tyr residues with other residues, especially Phe or Trp, particularly Phe.

In JP170 tyrosine residues may be modified in positions: 20, 54, 118, 137, 147, 194, 225, 247, 249, 334, 379, 388, 411, and 418.

In SD-521 and Ya proteases the tyrosine residues may be modified in positions: 17, 20, 54, 137, 147, 187, 243, 247, 249, 299, 319, 334, 361, 379, 386, 388, 411, and 418.

In relation to JP170 the invention thus relates to the variants: Y17{F,W}, Y20{F,W}, Y54{F,W}, Y137{F,W}, Y147{F,W}, Y187{F,W}, Y243{F,W}, Y247{F,W}, Y249{F,W}, Y299{F,W}, Y319{F,W}, Y334{F,W}, Y361{F,W}, Y379{F, W}, Y386{F,W}, Y388{F,W}, Y411{F,W}, and Y418{F,W}. Corresponding modifications are easily identified in other JP170 type subtilases.

**Modification of tryptophan residues**

In order to stabilize the protein it may be advantageous to replace or delete tryptophan residues at the surface of the protein, e.g., as described in US 5,118,623. The tryptophan residues may advantageously be substituted for F, T, Q or G. Thus, in a further embodiment the invention relates to JP170 type subtilase variants comprising one or more of the following substitutions: For the SD-521 and Ya proteases positions 1 18, 1 29, 240, 306, 350, and 392; and for the JP170 protease positions 129, 240, 306, 350, and 392.

Thus, the invention relates to a JP170 variant comprising one or more of the following substitutions W129{F, T,Q,G}, W240{F,T,Q,G}, W306{F,T,Q,G}, W350{F,T,Q,G}, and W392{F,T,Q,G}.

**Combined modifications**

The present invention also encompasses any of the above mentioned subtilase variants in combination with any other modification to the amino acid sequence thereof. Especially combinations with other modifications known in the art to provide improved properties to the enzyme are envisaged.

**Methods of preparing JP170 like or BPN' like subtilase variants**

The subtilase variants, i.e. the JP170 and BPN' variants of the present invention may be produced by any known method within the art and the present invention also relates to nucleic acid encoding a subtilase variant of the present invention, a DNA construct comprising said nucleic acid and a host cell comprising said nucleic acid sequence.

In general natural occurring proteins may be produced by culturing the organism expressing the protein and subsequently purifying the protein or it may be produced by cloning a nucleic acid, e.g. genomic DNA or cDNA, encoding the protein into an expression vector, introducing said expression vector into a host cell, culturing the host cell and purifying the expressed protein.

Typically protein variants may be produced by site-directed mutagenesis of a parent protein, introduction into expression vector, host cell etc. The parent protein may be cloned from a strain producing the polypeptide or from an expression library, i.e. it may be isolated from genomic DNA or prepared from cDNA, or a combination thereof.

In general standard procedures for cloning of genes and/or introducing mutations (random and/or site directed) into said genes may be used in order to obtain a parent subtilase, or subtilase or subtilase variant of the invention. For further description of suitable techniques reference is made to Molecular cloning: A laboratory manual (Sambrook et al. (1989), Cold Spring Harbor lab., Cold Spring Harbor, NY; Ausubel, F. M. et al. (eds.)); Current protocols in Molecular Biology (John Wiley and Sons, 1995; Harwood, C. R., and Cutting, S. M. (eds.)); Molecular Biological Methods for Bacillus (John Wiley and Sons, 1990); DNA Cloning: A Practical Approach, Volumes I and II (D.N. Glover ed. 1985); Oligonucleotide Synthesis (M.J. Gait ed. 1984); Nucleic Acid Hybridization (B.D. Hames & S.J. Higgins eds (1985)); Transcription And Translation (B.D. Hames & S.J. Higgins, eds. (1984)); Animal Cell Culture (R.I. Freshney, ed. (1986)); Immobilized Cells And Enzymes (IRL Press, (1986)); A Practical Guide To Molecular Cloning (B. Perbal, (1984)) and WO 96/34946.

Further, variants could be constructed by:

Random Mutagenesis

Random mutagenesis is suitably performed either as localized or region-specific random mutagenesis in at least three parts of the gene translating to the amino acid sequence shown in question, or within the whole gene.

When the mutagenesis is performed by the use of an oligonucleotide, the oligonucleotide may be doped or spiked with the three non-parent nucleotides during the synthesis of the oligonucleotide at the positions that are to be changed. The doping or spiking may be done so that codons for unwanted amino acids are avoided. The doped or spiked oligonucleotide can be incorporated into the DNA encoding the subtilase enzyme by any published technique, using, e.g., PCR, LCR or any DNA polymerase and ligase as deemed appropriate.

Preferably, the doping is carried out using "constant random doping", in which the percentage of wild-type and modification in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% modifications in each position. An additional consideration in the choice of a doping scheme is based on genetic as well as protein-structural constraints. The doping scheme may be made by using the DOPE program which, *inter alia*, ensures that introduction of stop codons is avoided (L.J. Jensen et al. Nucleic Acid Research, 26, 697-702 (1998).

When PCR-generated mutagenesis is used, either a chemically treated or non-treated gene encoding a parent subtilase enzyme is subjected to PCR under conditions that increase the misincorporation of nucleotides (Deshler 1992; Leung et al., Technique, 1, 1989, pp. 11-15).

The DNA sequence to be mutagenized may conveniently be present in a genomic or cDNA library prepared from an organism expressing the parent subtilase. Alternatively, the DNA sequence may be present on a suitable vector such as a plasmid or a bacteriophage, which as such may be incubated with or otherwise exposed to the mutagenising agent. The DNA to be mutagenized may also be present in a host cell either by being integrated in the genome of said cell or by being present on a vector harbored in the cell. Finally, the DNA to be mutagenized may be in isolated form. It will be understood that the DNA sequence to be subjected to random mutagenesis is preferably a cDNA or a genomic DNA sequence.

In some cases it may be convenient to amplify the mutated DNA sequence prior to performing the expression step b) or the screening step c). Such amplification may be performed in accordance with methods known in the art, the presently preferred method being PCR-generated amplification using oligonucleotide primers prepared on the basis of the DNA or amino acid sequence of the parent enzyme.

The mutated DNA sequence may further comprise a DNA sequence encoding functions permitting expression of the mutated DNA sequence.

Localised random mutagenesis

The random mutagenesis may be advantageously localised to a part of the parent subtilase in question. This may, e.g., be advantageous when certain regions of the enzyme have been identified to be of particular importance for a given property of the enzyme, and when modified are expected to result in a variant having improved properties. Such regions may normally be identified when the tertiary structure of the parent enzyme has been elucidated and related to the function of the enzyme.

The localised or region-specific, random mutagenesis is conveniently performed by use of PCR generated mutagenesis techniques as described above or any other suitable technique known in the art. Alternatively, the DNA sequence encoding the part of the DNA sequence to be modified may be isolated, e.g., by insertion into a suitable vector, and said part may be subsequently subjected to mutagenesis by use of any of the mutagenesis methods discussed above.

General method for random mutagenesis by use of the DOPE program

The random mutagenesis may be carried out by the following steps:

1. Select regions of interest for modification in the parent enzyme
2. Decide on mutation sites and non-mutated sites in the selected region
3. Decide on which kind of mutations should be carried out, e.g. with respect to the desired stability and/or performance of the variant to be constructed
4. Select structurally reasonable mutations
5. Adjust the residues selected by step 3 with regard to step 4.
6. Analyse by use of a suitable dope algorithm the nucleotide distribution.
7. If necessary, adjust the wanted residues to genetic code realism, e.g. taking into account constraints resulting from the genetic code, e.g. in order to avoid introduction of stop codons; the skilled person will be aware that some codon combinations cannot be used in practice and will need to be adapted
8. Make primers
9. Perform random mutagenesis by use of the primers

10. Select resulting subtilase variants by screening for the desired improved properties.

Suitable dope algorithms for use in step 6 are well known in the art. One such algorithm is described by Tomandl, D. et al., 1997, Journal of Computer-Aided Molecular Design 11:29-38. Another algorithm is DOPE (Jensen, LJ, Andersen, KV, Svendsen, A, and Kretzschmar, T (1998) Nucleic Acids Research 26:697-702).

Expression vectors

A recombinant expression vector comprising a nucleic acid sequence encoding a subtilase variant of the invention may be any vector that may conveniently be subjected to recombinant DNA procedures a nd which may bring about the expression of the nucleic acid sequence.

The choice of vector will often depend on the host cell into which it is to be introduced. Examples of a suitable vector include a linear or closed circular plasmid or a virus. The vector may be an autonomously replicating vector, i.e., a vector which exists as an extra-chromosomal entity, the replication of which is independent of chromosomal replication, e.g., a plasmid, an extra-chromosomal element, a mini chromosome, or an artificial chromosome. The vector may contain any means for assuring self-replication. Examples of bacterial origins of replication are the origins of replication of plasmids pBR322, pUC19, pACYC177, pACYC184, pUB110, pE194, pTA1060, and pAMβ1. Examples of origin of replications for use in a yeast host cell are the 2 micron origin of replication, the combination of CEN6 and ARS4, and the combination of CEN3 and ARS1. The origin of replication may be one having a mutation which makes it function as temperature-sensitive in the host cell (see, e.g., Ehrlich, 1978, Proceedings of the National Academy of Sciences USA 75:1433).

Alternatively, the vector may be one which, when introduced into the host cell, is integrated into the genome and replicated together with the chromosome(s) into which it has been integrated. Vectors which are integrated into the genome of the host cell may contain any nucleic acid sequence enabling integration into the genome, in particular it may contain nucleic acid sequences facilitating integration into the genome by homologous or non-homologous recombination. The vector system may be a single vector, e.g. plasmid or virus, or two or more vectors, e.g. plasmids or virus', which together contain the total DNA to be introduced into the genome of the host cell, or a transposon.

The vector may in particular be an expression vector in which the DNA sequence encoding the subtilase variant of the invention is operably linked to additional segments or control sequences required for transcription of the DNA. The term, "operably linked" indicates that the segments are arranged so that they function in concert for their intended purposes, e.g. transcription initiates in a promoter and proceeds through the DNA sequence encoding the subtilase variant. Additional segments or control sequences include a promoter, a leader, a polyadenylation sequence, a propeptide sequence, a signal sequence and a transcription terminator. At a minimum the control sequences include a promoter and transcriptional and translational stop signals.

The promoter may be any DNA sequence that shows transcriptional activity in the host cell of choice and may be derived from genes encoding proteins either homologous or heterologous to the host cell.

Examples of suitable promoters for use in bacterial host cells include the promoter of the *Bacillus subtilis* levansucrase gene (sacB), the *Bacillus stearothermophilus* maltogenic amylase gene (amyM), the *Bacillus licheniformis* alpha-amylase gene (amyL), the *Bacillus amyloliquefaciens* alpha-amylase gene (amyQ), the *Bacillus subtilis* alkaline protease gene, or the *Bacillus pumilus* xylosidase gene, the *Bacillus amyloliquefaciens* BAN amylase gene, the *Bacillus licheniformis* penicillinase gene (penP), the *Bacillus subtilis* xylA and xylB genes, and the prokaryotic beta-lactamase gene (Villa-Kamaroff et al., 1978, Proceedings of the National Academy of Sciences USA 75:3727-3731). Other examples include the phage Lambda $P_R$ or $P_L$ promoters or the E. coli lac, trp or tac promoters or the Streptomyces coelicolor agarase gene (dagA). Further promoters are described in "Useful proteins from recombinant bacteria" in Scientific American, 1980, 242:74-94; and in Sambrook et al., 1989, supra.

Examples of suitable promoters for use in a filamentous fungal host cell are promoters obtained from the genes encoding *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (glaA), *Rhizomucor miehei* lipase, *Aspergillus oryzae* alkaline protease, *Aspergillus oryzae* triose phosphate isomerase, *Aspergillus nidulans* acetamidase, *Fusarium oxysporum* trypsin-like protease (as described in U.S. Patent No. 4,288,627, which is incorporated herein by reference), and hybrids thereof. Particularly preferred promoters for use in filamentous fungal host cells are the TAKA amylase, NA2-tpi (a hybrid of the promoters from the genes encoding *Aspergillus niger* neutral (-amylase and *Aspergillus oryzae* triose phosphate isomerase), and glaA promoters. Further suitable promoters for use in filamentous fungus host cells are the ADH3 promoter (McKnight et al., The EMBO J. 4 (1985), 2093 - 2099) or the tpiA promoter.

Examples of suitable promoters for use in yeast host cells include promoters from yeast glycolytic genes (Hitzeman et al., J. Biol. Chem. 255 (1980), 12073 - 12080; Alber and Kawasaki, J. Mol. Appl. Gen. 1 (1982), 419 - 434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., Nature 304 (1983), 652 - 654) promoters.

Further useful promoters are obtained from the *Saccharomyces cerevisiae* enolase (ENO-1) gene, the *Saccharomyces cerevisiae* galactokinase gene (GAL1), the *Saccharomyces cerevisiae* alcohol dehydrogenase/glyceraldehyde-3-phosphate dehydrogenase genes (ADH2/GAP), and the *Saccharomyces cerevisiae* 3-phosphoglycerate kinase gene. Other useful promoters for yeast host cells are described by Romanos et al., 1992, Yeast 8:423-488. In a mammalian host cell, useful promoters include viral promoters such as those from Simian Virus 40 (SV40), Rous sarcoma virus (RSV), adenovirus, and bovine papilloma virus (BPV).

Examples of suitable promoters for use in mammalian cells are the SV40 promoter (Subramani et al., Mol. Cell Biol. 1 (1981), 854 -864), the MT-1 (metallothionein gene) promoter (Palmiter et al., Science 222 (1983), 809 - 814) or the adenovirus 2 major late promoter.

An example of a suitable promoter for use in insect cells is the polyhedrin promoter (US 4,745,051; Vasuvedan et al., FEBS Lett. 311, (1992) 7 - 11), the P10 promoter (J.M. Vlak et al., J. Gen. Virology 69, 1988, pp. 765-776), the Autographa californica polyhedrosis virus basic protein promoter (EP 397 485), the baculovirus immediate early gene 1 promoter (US 5,155,037; US 5,162,222), or the baculovirus 39K delayed-early gene promoter (US 5,155,037; US 5,162,222).

The DNA sequence encoding a subtilase variant of the invention may also, if necessary, be operably connected to a suitable terminator.

The recombinant vector of the invention may further comprise a DNA sequence enabling the vector to replicate in the host cell in question.

The vector may also comprise a selectable marker, e.g. a gene the product of which complements a defect in the host cell, or a gene encoding resistance to e.g. antibiotics like ampicillin, kanamycin, chloramphenicol, erythromycin, tetracycline, spectinomycine, neomycin, hygromycin, methotrexate, or resistance to heavy metals, virus or herbicides, or which provides for prototrophy or auxotrophs. Examples of bacterial selectable markers are the dal genes from *Bacillus subtilis* or *Bacillus licheniformis,* resistance. A frequently used mammalian marker is the dihydrofolate reductase gene (DHFR). Suitable markers for yeast host cells are ADE2, HIS3, LEU2, LYS2, MET3, TRP1, and URA3. A selectable marker for use in a filamentous fungal host cell may be selected from the group including, but not limited to, amdS (acetamidase), argB (ornithine carbamoyltransferase), bar (phosphinothricin acetyltransferase), hygB (hygromycin phosphotransferase), niaD (nitrate reductase), pyrG (orotidine-5'-phosphate decarboxylase), s C (sulfate a denyltransferase), trpC (anthranilate synthase), and g lufosinate resistance markers, as well as equivalents from other species. Particularly, for use in an *Aspergillus* cell are the amdS and pyrG markers of *Aspergillus nidulans* or *Aspergillus oryzae* and the bar marker of *Streptomyces hygroscopicus*. Furthermore, selection may be accomplished by co-transformation, e.g., as described in WO 91/17243, where the selectable marker is on a separate vector.

To direct a subtilase variant of the present invention into the secretory pathway of the host cells, a secretory signal sequence (also known as a leader sequence, prepro sequence or pre sequence) may be provided in the recombinant vector. The secretory signal sequence is joined to the DNA sequence encoding the enzyme in the correct reading frame. Secretory signal sequences are commonly positioned 5' to the DNA sequence encoding the enzyme. The secretory signal sequence may be that normally associated with the enzyme or may be from a gene encoding another secreted protein.

The procedures used to ligate the DNA sequences coding for the present enzyme, the promoter and optionally the terminator and/or secretory signal sequence, respectively, or to assemble these sequences by suitable PCR amplification schemes, and to insert them into suitable vectors containing the information necessary for replication or integration, are well known to persons skilled in the art (cf., for instance, Sambrook et al.).

More than one copy of a nucleic acid sequence encoding an enzyme of the present invention may be inserted into the host cell to amplify expression of the nucleic acid sequence. Stable amplification of the nucleic acid sequence can be obtained by integrating at least one additional copy of the sequence into the host cell genome using methods well known in the art and selecting for transformants.

The nucleic acid constructs of the present invention may also comprise one or more nucleic acid sequences which encode one or more factors that are advantageous in the expression of the polypeptide, e.g., an activator (e.g., a trans-acting factor), a chaperone, and a processing protease. Any factor that is functional in the host cell of choice may be used in the present invention. The nucleic acids encoding one or more of these factors are not necessarily in tandem with the nucleic acid sequence encoding the polypeptide.

Host cells

The DNA sequence encoding a subtilase variant of the present invention may be either homologous or heterologous to the host cell into which it is introduced. If homologous to the host cell, i.e. produced by the host cell in nature, it will typically be operably connected to another promoter sequence or, if applicable, another secretory signal sequence and/or terminator sequence than in its natural environment. The term "homologous" is intended to include a DNA sequence encoding an enzyme native to the host organism in question. The term "heterologous" is intended to include a DNA sequence not expressed by the host cell in nature. Thus, the DNA sequence may be from another organism, or it may be a synthetic sequence.

The host cell into which the DNA construct or the recombinant vector of the invention is introduced may be any cell that is capable of producing the present subtilase variants, such as prokaryotes, e.g. bacteria or eukaryotes, such as fungal cells, e.g. yeasts or filamentous fungi, insect cells, plant cells or mammalian cells.

Examples of bacterial host cells which, on cultivation, are capable of producing the subtilase variants of the invention are gram-positive bacteria such as strains of *Bacillus,* e.g. strains of *B. subtilis, B. licheniformis, B. lentus, B. brevis, B. stearothermophilus, B. alkalophilus, B. amyloliquefaciens, B. coagulans, B. circulans, B. lautus, B. megaterium* or *B. thuringiensis,* or strains of *Streptomyces,* such as *S. lividans* or *S. murinus,* or gram-negative bacteria such as *Escherichia coli* or *Pseudomonas sp.*

The transformation of the bacteria may be effected by protoplast transformation, electroporation, conjugation, or by using competent cells in a manner known per se (cf. Sambrook et al., supra).

When expressing the subtilase variant in bacteria such as *E. coli,* the enzyme may be retained in the cytoplasm, typically as insoluble granules (known as inclusion bodies), or it may be directed to the periplasmic space by a bacterial secretion sequence. In the former case, the cells are lysed and the granules are recovered and denatured after which the enzyme is refolded by diluting the denaturing agent. In the latter case, the enzyme may be recovered from the periplasmic space by disrupting the cells, e.g. by sonication or osmotic shock, to release the contents of the periplasmic space and recovering the enzyme.

When expressing the subtilase variant in gram-positive bacteria such as *Bacillus* or *Streptomyces* strains, the enzyme may be retained in the cytoplasm, or it may be directed to the extracellular medium by a bacterial secretion sequence. In the latter case, the enzyme may be recovered from the medium as described below.

Examples of host yeast cells include cells of a species of *Candida, Kluyveromyces, Saccharomyces, Schizosaccharomyces, Pichia, Hansehula*, or *Yarrowia*. In a particular embodiment, the yeast host cell is a *Saccharomyces carlsbergensis, Saccharomyces cerevisiae, Saccharomyces diastaticus, Saccharomyces douglasii, Saccharomyces kluyveri, Saccharomyces norbensis* or *Saccharomyces oviformis* cell. Other useful yeast host cells are a *Kluyveromyces lactis, Kluyveromyces fragilis, Hansehula polymorpha, Pichia pastoris, Yarrowia lipolytica, Schizosaccharomyces pombe, Ustilgo maylis, Candida maltose, Pichia guillermondii* and *Pichia methanolio* cell (cf. Gleeson et al., J. Gen. Microbiol. 132, 1986, pp. 3459-3465; US 4,882,279 and US 4,879,231). Since the classification of yeast may change in the future, for the purposes of this invention, yeast shall be defined as described in Biology and Activities of Yeast (Skinner, F.A., Passmore, S.M., and Davenport, R.R., eds, Soc. App. Bacteriol. Symposium Series No. 9, 1980. The biology of yeast and manipulation of yeast genetics are well known in the art (see, e.g., Biochemistry and Genetics of Yeast, Bacil, M., Horecker, B.J., and Stopani, A.O.M., editors, 2nd edition, 1987; The Yeasts, Rose, A.H., and Harrison, J.S., editors, 2nd edition, 1987; and The Molecular Biology of the Yeast Saccharomyces, Strathern et al., editors, 1981). Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153:163; and Hinnen et al., 1978, Proceedings of the National Academy of Sciences USA 75:1920.

Examples of filamentous fungal cells include filamentous forms of the subdivision Eumycota and Oomycota (as defined by Hawksworth et al., 1995, supra), in particular it may of the a cell of a species of *Acremonium,* such as *A. chrysogenum, Aspergillus,* such as *A. awamori, A. foetidus, A. japonicus, A. niger, A. nidulans* or *A. oryzae, Fusarium,* such as *F. bactridioides, F. cerealis, F. crookwellense, F. culmorum, F. graminearum, F. graminum, F. heterosporum, F. negundi, F. reticulatum, F. roseum, F. sambucinum, F. sarcochroum, F. sulphureum, F. trichothecioides* or *F. oxysporum, Humicola,* such as *H. insolens* or *H. lanuginose, Mucor,* such as *M. miehei, Myceliophthora,* such as *M. thermophilum, Neurospora,* such as *N. crassa, Penicillium,* such as *P. purpurogenum, Thielavia,* such as *T. terrestris, Tolypocladium,* or *Trichoderma*, such as *T. harzianum, T. koningii, T. longibrachiatum, T.* reesei or *T. viride,* or a teleomorph or synonym thereof. The use of *Aspergillus spp.* for the expression of proteins is described in, e.g., EP 272 277, EP 230 023.

Examples of insect cells include a *Lepidoptera* cell line, such as *Spodoptera frugiperda* cells or *Trichoplusia ni* cells (cf. US 5,077,214). Culture conditions may suitably be as described in WO 89/01029 or WO 89/01028. Transformation of insect cells and production of heterologous polypeptides therein may be performed as described in US 4,745,051; US 4, 775, 624; US 4,879,236; US 5,155,037; US 5,162,222; EP 397,485).

Examples of mammalian cells include Chinese hamster ovary (CHO) cells, HeLa cells, baby hamster kidney (BHK) cells, COS cells, or any number of other immortalized cell lines available, e.g., from the American Type Culture Collection. Methods of transfecting mammalian cells and expressing DNA sequences introduced in the cells are described in e.g. Kaufman and Sharp, J. Mol. Biol. 159 (1982), 601 - 621; Southern and Berg, J. Mol. Appl. Genet. 1 (1982), 327 - 341; Loyter et al., Proc. Natl. Acad. Sci. USA 79 (1982), 422 - 426; Wigler et al., Cell 14 (1978), 725; Corsaro and Pearson, Somatic Cell Genetics 7 (1981), 603, Ausubel et al., Current Protocols in Molecular Biology, John Wiley and Sons, Inc., N.Y., 1987, Hawley-Nelson et al., Focus 15 (1993), 73; Ciccarone et al., Focus 15 (1993), 80; Graham and van der Eb, Virology 52 (1973), 456; and Neumann et al., EMBO J. 1 (1982), 841 - 845. Mammalian cells may be transfected by direct uptake using the calcium phosphate precipitation method of Graham and Van der Eb (1978, Virology 52:546).

Methods for expression and isolation of proteins

To express an enzyme of the present invention the above mentioned host cells transformed or transfected with a vector comprising a nucleic acid sequence encoding an enzyme of the present invention are typically cultured in a suitable nutrient medium under conditions permitting the production of the desired molecules, after which these are recovered from the cells, or the culture broth.

The medium used to culture the host cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The media may be prepared using procedures known in the art (see, e.g., references for bacteria and yeast; Bennett, J.W. and LaSure, L., editors, More Gene Manipulations in Fungi, Academic Press, CA, 1991).

If the enzymes of the present invention are secreted into the nutrient medium, they may be recovered directly from the medium. If they are not secreted, they may be recovered from cell lysates. The enzymes of the present invention may be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. ion exchange chromatography, gelfiltration chromatography, affinity chromatography, or the like, dependent on the enzyme in question.

The enzymes of the invention may be detected using methods known in the art that are specific for these proteins. These detection methods include use of specific antibodies, formation of a product, or disappearance of a substrate. For example, an enzyme assay may be used to determine the activity of the molecule. Procedures for determining various kinds of activity are known in the art.

The enzymes of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF), differential solubility (e.g., ammonium sulfate precipitation), or extraction (see, e.g., Protein Purification, J-C Janson and Lars Ryden, editors, VCH Publishers, New York, 1989).

When an expression vector comprising a DNA sequence encoding an enzyme of the present invention is transformed/transfected into a heterologous host cell it is possible to enable heterologous recombinant production of the enzyme. An advantage of using a heterologous host cell is that it is possible to make a highly purified enzyme composition, characterized in being free from homologous impurities, which are often present when a protein or peptide is expressed in a homologous host cell. In this context homologous impurities mean any impurity (e.g. other polypeptides than the enzyme of the invention) which originates from the homologous cell where the enzyme of the invention is originally obtained from.

**DETERGENT APPLICATIONS**

The enzyme of the invention may be added to and thus become a component of a detergent composition.

The detergent composition of the invention m ay f or example be formulated a s a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations, especially for automatic dish washing (ADW).

In a specific aspect, the invention provides a detergent additive comprising the enzyme of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts. Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase™, Savinase™, Primase™, Duralase™, Esperase™, and Kannase™ (Novozymes A/S), Maxatase™, Maxacal™, Maxapem™, Properase™, Purafect™, Purafect OxP™, FN2™, and FN3™ (Genencor International Inc.).

Lipases: Suitable lipases include those of bacterial or fungal origin. Chemically modified or protein engineered mutants are included. Examples of useful lipases include lipases from *Humicola* (synonym *Thermomyces*), e.g. from *H. lanuginosa* (*T. lanuginosus*) as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 1 05, WO 95/35381, WO 96/00292, WO 95/30744, WO 94/25578, WO 95/14783, WO 95/22615, WO 97/04079 and WO 97/07202.

Preferred commercially available lipase enzymes include Lipolase™ and Lipolase Ultra™ (Novozymes A/S).

Amylases: Suitable amylases (α and/or β) include those of bacterial or fungal origin. Chemically modified or protein engineered mutants are included. Amylases include, for example, α-amylases obtained from *Bacillus,* e.g. a special strain of *B. licheniformis*, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Fungamyl™ and BAN™ (Novozymes A/S), Rapidase™ and Purastar™ (from Genencor International Inc.).

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, and Carezyme™ (Novozymes A/S), Clazinase™, and Puradax HA™ (Genencor International Inc.), and KAC-500(B)™ (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from *Coprinus*, e.g. from *C. cinereus*, and variants thereof as those described in WO 93/24618, WO 95/10602, and WO 98/15257.

Commercially available peroxidases include Guardzyme™ (Novozymes A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or non-aqueous.

The detergent composition comprises one or more surfactants, which may be non-ionic including semi-polar and/or anionic and/or cationic and/or zwitterionic. The surfactants are typically present at a level of from 0.1 % to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic a cid, d iethylenetriaminepentaacetic a cid, alkyl- o r alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including c lays, foam boosters, suds suppressors, a nti-corrosion agents, soil-suspending agents, anti-soil redeposition agents, dyes, bactericides, optical brighteners, hydrotropes, tarnish inhibitors, or perfumes.

In the detergent compositions any enzyme, in particular the enzyme of the invention, may be added in an amount corresponding to 0.01-100 mg of enzyme protein per litre of wash liquor, preferably 0.05-5 mg of enzyme protein per litre of wash liquor, in particular 0.1-1 mg of enzyme protein per litre of wash liquor.

The enzyme of the invention may additionally be incorporated in the detergent formulations disclosed in WO 97/07202 which is hereby incorporated as reference.

## MATERIALS AND METHODS

### Textiles

Standard textile pieces are obtained from EMPA St. Gallen, Lerchfeldstrasse 5, CH-9014 St. Gallen, Switzerland. Especially type EMPA 116 (cotton textile stained with blood, milk and ink) and EMPA 117 (polyester/cotton textile stained with blood, milk and ink). Other atandard textile pieces are obtained from wfk-Cleaning Technology Research Institute, Christenfeld 10, D-41379 Brüggen-Bracht, Germany. Especially type wfk10N (cotton textile stained with egg/pigment), wfk10eggEG (cotton textile stained with egg yolk). Denaturation of wfk10N occurs in an autoclave.

### Method for producing a subtilase variant

The present invention provides a method of producing an isolated enzyme according to the invention, wherein a suitable host cell, which has been transformed with a DNA sequence encoding the enzyme, is cultured under conditions permitting the production of the enzyme, and the resulting enzyme is recovered from the culture.

When an expression vector comprising a DNA sequence encoding the enzyme is transformed into a heterologous host cell it is possible to enable heterologous recombinant production of the enzyme of the invention. Thereby it is possible to make a highly purified subtilase composition, characterized in being free from homologous impurities.

The medium used to culture the transformed host cells may be any conventional medium suitable for growing the host cells in question. The expressed subtilase may conveniently be secreted into the culture medium and may be recovered there-from by well-known procedures including separating the cells from the medium by centrifugation or filtration, precipitating proteinaceous components of the medium by means of a salt such as ammonium sulfate, followed by chromatographic procedures such as ion exchange chromatography, affinity chromatography, or the like.

**EXAMPLE 1**

Removal of ion-binding sites from BPN' like subtilases

The below mentioned regions in JP170 and TY145 have been selected for transfer from JP170 and TY145 to Savinase. By use of the molecular methods of preparing subtilase variants as described herein, the Savinase regions (BPN' numbering) are deleted and the JP170 and TY145 regions are inserted instead. Since the Savinase regions are in contact with ion-binding sites, the purpose of the modifications is to remove the ion-binding site from Savinase.

Savinase     region A194-L196
JP170          region P209-P217 and
Savinase     region L75-L82
TY145          region H83-Y92,

alternatively the modification can be

Savinase     region A194-L196
JP170          region P209-P217 and
Savinase     region L75-L82
JP170          region N79-K83.

Construction and expression of enzyme variants:

Site-directed mutagenesis:

Subtilase JP170 site-directed variants of the invention comprising specific insertions/deletions/substitutions are made by traditional cloning of DNA fragments (Sambrook et al., Molecular Cloning: A Laboratory Manual, 2nd Ed., Cold Spring Harbor, 1989) produced by PCR with oligos containing the desired mutations.
The template plasmid DNA may be pSX222, or an analogue of this containing a variant of subtilisin JP170. Mutations are introduced by oligo directed mutagenesis to the construction of variants.
The subtilisin JP170 variants were transformed into *E. coli.* DNA purified from an over night culture of these transformants is transformed into *B. subtilis* by restriction endonuclease digestion, purification of DNA fragments, ligation, transformation of *B. subtilis.* Transformation of *B. subtilis* is performed as described by Dubnau et al., 1971, J. M ol. Biol. 56, pp. 209-221.

Site-directed mutagenesis in order to introduce mutations in a specific region:

The overall strategy used to perform site-directed mutagenesis is:
Mutagenic primers (oligonucleotides) are synthesized corresponding to the DNA sequence flanking the sites of mutation, separated by the DNA base pairs defining the insertions / deletions / substitutions.
Subsequently, the resulting mutagenic primers are used in a PCR reaction with the modified plasmid pSX222. The resulting PCR fragment is purified and extended in a second PCR-reaction, the resulting PCR product is purified and extended in a third PCR-reaction before being digested by endonucleases and cloned into the *E. coli - B. subtilis* shuttle vector pSX222. The PCR reactions are performed under normal conditions. The plasmid DNA is transformed into E. *coli* by well-known techniques and one *E. coli* colony is sequenced to confirm the mutation designed.
In order to purify subtilase variants of the invention, the pSX222 expression plasmid comprising a variant of the invention was transformed into a competent *B. subtilis* strain and fermented as described above.

**EXAMPLE 2**

Purification and assessment of enzyme concentration

After fermentation, purification of subtilisin variants is accomplished using Hydrophobic Charge Induction Chromatography (HCIC) and subsequent vacuum filtration.
To capture the enzyme, the HCIC uses a cellulose matrix to which 4-Mercapto-EthylPyridine (4-MEP) is bound.
Beads of the cellulose matrix sized 80-100 $\mu$m are mixed with a media containing yeast and the transformed *B. subtilis* capable of secreting the subtilisin variants and incubated at pH 9.5 in Unifilter® microplates.

As 4-MEP is hydrophobic at pH > 7 and the subtilisin variants are hydrophobic at pH 9.5 a hydrophobic association is made between the secreted enzyme and the 4-MEP on the beads. After incubation the media and cell debris is removed by vacuum filtration while the beads and enzyme are kept on the filter.

To elute the enzyme from the beads the pH is now lowered by washing the filter with a n elution buffer (pH 5). Hereby the enzymes p art from the beads a nd can be retrieved from the buffer.

The concentration of the purified subtilisin enzyme variants is assessed by active site titration (AST).

The purified enzyme is incubated with the high affinity inhibitor Cl-2A at different concentrations to inhibit a varying amount of the active sites. The protease and inhibitor binds to each other at a 1:1 ratio and accordingly the enzyme concentration can be directly related to the concentration of inhibitor, at which all protease is inactive. To measure the residual protease activity, a substrate (0.6 mM Suc-Ala-Ala-Pro-Phe-pNA in Tris/HCl buffer) is added after the incubation with inhibitor and during the following 4 minutes the development of the degradation product pNA (paranitrophenol) is measured periodically at 405 nm on an Elisa Reader.

Each of the variants listed below were constructed as described above.

H243K; S238K; L233T; L233S; L233D; Y247R; H200D; H200A; H200G; E185D; S193Q; S193Y; N390D; G394N; G394F; W240H; G355A; G355S; N316D; N79D; K246R; K83R; H200D+D196N; H243E.

**EXAMPLE 3**

**Automatic Mechanical Stress Assay (AMSA).**

Description of AMSA-test method:

Washing experiments are performed in order to asses the wash performance of selected JP170 subtilase variants in detergent compositions. Subtilases of the present application were tested using the Automatic Mechanical Stress Assay (AMSA). With the AMSA, the wash performance of a large quantity of small volume enzyme-detergent solutions can be examined. The AMSA plate has a number of slots for test solutions and a lid firmly squeezing the textile swatch to be washed against all the slot openings. During the washing time, the plate, test solutions, textile and lid are vigorously shaken to bring the test solution in contact with the textile and apply mechanical stress in a regular, periodic oscillating manner. For further description see WO 02/42740 especially the paragraph "Special method embodiments" at page 23-24.

The experiment was conducted under the experimental conditions specified below:

| | |
|---|---|
| Commercial detergent base | European 3in1 AD.W type |
| Detergent dosage | 5 - 5.5 g/L |
| Test solution volume | 160 $\mu$L |
| pH | As is |
| Wash time | 20 minutes |
| Temperature | 50°C |
| Water hardness | 25°dH |
| Enzyme concentration in test solution | 0.25mg/L, 0.5mg/L, 1 mg/L, and 2.,5 mg/L for wfk10N; 1mg/L, 2,5mg/L, 4mg/L, and 6mg/L for denatured wfk10N. |
| Test material | Wfk10N |
| Water hardness was adjusted to 9°dH by addition of $CaCl_2$, $MgCl_2$, and $NaHCO_3$ ($Ca^{2+}$:$Mg^{2+}$ = 4:1) to the test system. After washing the textile pieces were flushed in tap water and dried. | |

The performance of the enzyme variant is measured as the brightness of the colour of the textile samples washed with that specific protease. Brightness can also be expressed as the intensity of the light reflected from the textile sample when illuminated with white light. When the textile is stained the intensity of the reflected light is lower, than that of a c lean textile. Therefore the intensity of the reflected light can be used to measure wash performance of a shuffled protease.

Colour measurements are made with a professional flatbed scanner (PFU DL2400pro, obtainable from: J.M. Thomsen, Dorfgade 2, Dorf, Dronninglund, DK-9330), which is used to capture an image of the washed textile samples.

The scans are made with a resolution of 200 dpi and with an output colour dept of 24 bits. In order to get accurate results, the scanner is frequently calibrated with a *Kodak reflective IT8 target*.

To extract a value f or the light intensity from the s canned i mages, a special d e-signed software application is used (*Novozymes Color Vector Analyzer*). The program retrieves the 24 bit pixel values from the image and converts them into values for red, green and blue (RGB). The intensity value (Int) is calculated by adding the RGB values together as vectors and then taking the length of the resulting vector:

$$Int = \sqrt{r^2 + g^2 + b^2}.$$

Detergents

Detergents for wash performance tests of the subtilases of the invention can be obtained by purchasing fully formulated commercial detergents at the market and subsequently inactivate the enzymatic components by heat treatment (5 minutes at 85°C in aqueous solution). Moreover a commercial detergent base without enzymes can be purchased directly from the manufacturer. Further a suitable model detergent can be purchased and used for wash performance tests.

The proteases may also be tested in a model detergent composition comprising

| | |
|---|---|
| Sodium Tripolyphosphate | 23.0% |
| Sodium Citrate Dihydrate | 22.3% |
| Sodium Perborate Monohydrate | 6.0% |
| Tetraacetyl Ethylendiamine | 2.0% |
| Sodium Disilicate (noncrystaline) | 5.0% |
| Linear Fatty Alcohol Ethoxylate | 2.0% |
| (non-ionic surfactant, low foaming) | |
| Maleic acid/Acrylic acid copolymer | 4.0% |
| (Sodium salt, 50% active on Sodium Carbonate) | |
| Sodium Carbonate, anhydrous add to | 100% |

Using the above test method in combination with a commercially available detergent the results shown below were obtained. As it appears, the subtilases according to the invention exhibits improved wash performance on egg stains in comparison to the wiild type JP170 subtilase with SEQ ID NO:1.

| Mutations | AMSA | "peptide binding loop" |
|---|---|---|
| WT JP170 | REF | |
| S193Q | ≥ | S193Y (Ca-site 1) |
| S193Y | ≥ | N390D (Ca-site 2) |
| N390D | ≥ | G394N (Ca-site 3) |
| G394N | ≥ | G394F (Ca-site 3) |
| G394F | ≥ | W240H (temperature stability) |
| W240H | ≥ | G355A (temperature stability) |
| G355A | ≥ | G355S (temperature stability) |
| K246R | ≥ | K83R (surface charge distribution) |
| H200D+D196N | ≥ | (Ca-site 1) |

**APPENDIX 1**

```
REMARK Complex of JP170 and CI2A inhibitor
REMARK Contents of asymmetric unit subtilisin 2x (433 a.a. x 2)
REMARK CI2A inhibitor 2x (a.a. 16 - 83 and 21 - 83)
REMARK small peptide (autodigestion product, a.a. KPSLL, 280 - 284)
REMARK Ca ions 6x, H2O 1115 x
REMARK
REMARK Crystallization conditions: (AMB) Hanging drop vapour diffusion
REMARK method where the drop consists of 2 μl of 15 - 20 mg.ml-1
REMARK protein concentration, 10 mM Na cacodylate - HCl buffer, pH 6.5
REMARK and 1 μl of the well solution, 20% w/v PEG 4000, 0.1 M Hepes
REMARK buffer, pH 7.5, 10% v/v isopropanol.
HEADER        ----                                      XX-XXX-XX    xxxx
COMPND        ---
REMARK   3
REMARK   3 REFINEMENT.
REMARK   3    PROGRAM       : REFMAC 5.1.24
REMARK   3    AUTHORS       : MURSHUDOV,VAGIN,DODSON
REMARK   3
REMARK   3     REFINEMENT TARGET : MAXIMUM LIKELIHOOD
REMARK   3
REMARK   3   DATA USED IN REFINEMENT.
REMARK   3    RESOLUTION RANGE HIGH (ANGSTROMS) :   1.90
REMARK   3    RESOLUTION RANGE LOW  (ANGSTROMS) :  19.96
REMARK   3    DATA CUTOFF            (SIGMA(F)) : NONE
REMARK   3    COMPLETENESS FOR RANGE        (%) :  76.65
REMARK   3    NUMBER OF REFLECTIONS             :    59444
REMARK   3
REMARK   3   FIT TO DATA USED IN REFINEMENT.
REMARK   3    CROSS-VALIDATION METHOD          : NULL
REMARK   3    FREE R VALUE TEST SET SELECTION  : NULL
REMARK   3    R VALUE       (WORKING + TEST SET) : 0.12256
REMARK   3    R VALUE             (WORKING SET) :  0.12256
REMARK   3    FREE R VALUE                      : NULL
REMARK   3    FREE R VALUE TEST SET SIZE    (%) : NULL
REMARK   3    FREE R VALUE TEST SET COUNT       : NULL
REMARK   3
REMARK   3   FIT IN THE HIGHEST RESOLUTION BIN.
REMARK   3    TOTAL NUMBER OF BINS USED         :      20
REMARK   3    BIN RESOLUTION RANGE HIGH         :    1.901
REMARK   3    BIN RESOLUTION RANGE LOW          :    1.950
REMARK   3    REFLECTION IN BIN    (WORKING SET) :     940
REMARK   3    BIN R VALUE          (WORKING SET) :    0.149
REMARK   3    BIN FREE R VALUE SET COUNT         :        0
REMARK   3    BIN FREE R VALUE                   : -999.000
REMARK   3
REMARK   3   NUMBER OF NON-HYDROGEN ATOMS USED IN REFINEMENT.
REMARK   3    ALL ATOMS              :     8694
REMARK   3
REMARK   3   B VALUES.
REMARK   3    FROM WILSON PLOT          (A**2) : NULL
REMARK   3    MEAN B VALUE       (OVERALL, A**2) :  16.479
REMARK   3    OVERALL ANISOTROPIC B VALUE.
REMARK   3     B11 (A**2) :      0.05
REMARK   3     B22 (A**2) :      0.06
REMARK   3     B33 (A**2) :     -0.11
REMARK   3     B12 (A**2) :      0.00
REMARK   3     B13 (A**2) :      0.00
REMARK   3     B23 (A**2) :      0.00
REMARK   3
REMARK   3   ESTIMATED OVERALL COORDINATE ERROR.
REMARK   3    ESU BASED ON R VALUE                          (A):   0.151
```

```
REMARK   3    ESU BASED ON FREE R VALUE                      (A): NULL
REMARK   3    ESU BASED ON MAXIMUM LIKELIHOOD                (A):   0.052
REMARK   3    ESU FOR B VALUES BASED ON MAXIMUM LIKELIHOOD (A**2):   1.828
REMARK   3
REMARK   3  CORRELATION COEFFICIENTS.
REMARK   3    CORRELATION COEFFICIENT FO-FC      :   0.969
REMARK   3    CORRELATION COEFFICIENT FO-FC FREE : NULL
REMARK   3
REMARK   3  RMS DEVIATIONS FROM IDEAL VALUES         COUNT    RMS     WEIGHT
REMARK   3    BOND LENGTHS REFINED ATOMS        (A): 7733 ; 0.014 ; 0.021
REMARK   3    BOND LENGTHS OTHERS               (A): 6857 ; 0.001 ; 0.020
REMARK   3    BOND ANGLES REFINED ATOMS   (DEGREES): 10540 ; 1.478 ; 1.936
REMARK   3    BOND ANGLES OTHERS          (DEGREES): 15972 ; 0.815 ; 3.000
REMARK   3    TORSION ANGLES, PERIOD 1    (DEGREES):   997 ;15.784 ; 5.000
REMARK   3    CHIRAL-CENTER RESTRAINTS       (A**3): 1197 ; 0.106 ; 0.200
REMARK   3    GENERAL PLANES REFINED ATOMS      (A): 8819 ; 0.007 ; 0.020
REMARK   3    GENERAL PLANES OTHERS             (A): 1500 ; 0.008 ; 0.020
REMARK   3    NON-BONDED CONTACTS REFINED ATOMS (A): 1552 ; 0.221 ; 0.300
REMARK   3    NON-BONDED CONTACTS OTHERS        (A): 8282 ; 0.265 ; 0.300
REMARK   3    NON-BONDED TORSION OTHERS         (A): 4417 ; 0.089 ; 0.500
REMARK   3    H-BOND (X...Y) REFINED ATOMS      (A): 1391 ; 0.198 ; 0.500
REMARK   3    POTENTIAL METAL-ION REFINED ATOMS (A):   25 ; 0.145 ; 0.500
REMARK   3    SYMMETRY VDW REFINED ATOMS        (A):   10 ; 0.129 ; 0.300
REMARK   3    SYMMETRY VDW OTHERS               (A):   57 ; 0.268 ; 0.300
REMARK   3    SYMMETRY H-BOND REFINED ATOMS     (A):   87 ; 0.272 ; 0.500
REMARK   3
REMARK   3  ISOTROPIC THERMAL FACTOR RESTRAINTS.    COUNT    RMS     WEIGHT
REMARK   3    MAIN-CHAIN BOND REFINED ATOMS  (A**2): 4985 ; 0.697 ; 1.500
REMARK   3    MAIN-CHAIN ANGLE REFINED ATOMS (A**2): 8031 ; 1.205 ; 2.000
REMARK   3    SIDE-CHAIN BOND REFINED ATOMS  (A**2): 2746 ; 1.963 ; 3.000
REMARK   3    SIDE-CHAIN ANGLE REFINED ATOMS (A**2): 2509 ; 3.180 ; 4.500
REMARK   3
REMARK   3  NCS RESTRAINTS STATISTICS
REMARK   3    NUMBER OF NCS GROUPS : NULL
REMARK   3
REMARK   3
REMARK   3  TLS DETAILS
REMARK   3    NUMBER OF TLS GROUPS  : NULL
REMARK   3
REMARK   3
REMARK   3  BULK SOLVENT MODELLING.
REMARK   3    METHOD USED : BABINET MODEL WITH MASK
REMARK   3    PARAMETERS FOR MASK CALCULATION
REMARK   3    VDW PROBE RADIUS   :   1.40
REMARK   3    ION PROBE RADIUS   :   0.80
REMARK   3    SHRINKAGE RADIUS   :   0.80
REMARK   3
REMARK   3  OTHER REFINEMENT REMARKS:
REMARK   3  HYDROGENS HAVE BEEN ADDED IN THE RIDING POSITIONS
REMARK   3
CISPEP  1 GLY A  163    PRO A  164                0.00
CISPEP  2 ALA A  171    PRO A  172                0.00
CISPEP  3 PHE A  191    GLY A  192                0.00
CISPEP  4 ASN A  199    HIS A  200                0.00
CISPEP  5 GLY A  208    PRO A  209                0.00
CISPEP  6 LYS A  216    PRO A  217                0.00
CISPEP  7 ASP A  236    SER A  237                0.00
CISPEP  8 ASP A  244    SER A  245                0.00
CISPEP  9 PHE A  299    PRO A  300                0.00
CISPEP 10 SER A  327    THR A  328                0.00
CISPEP 11 ALA A  386    PRO A  387                0.00
CISPEP 12 GLU A  414    VAL A  415                0.00
CISPEP 13 GLY A  423    PRO A  424                0.00
                            --
```

```
LINK                ASN B 316              LYS B 318              gap
LINK                GLU B 330              ALA B 332              gap
LINK                LEU B 337              LYS B 340              gap
LINK                GLU D 330              ALA D 332              gap
LINK                LEU D 337              LYS D 340              gap
CISPEP   14 GLY C   163      PRO C   164                0.00
CISPEP   15 ALA C   171      PRO C   172                0.00
CISPEP   16 PHE C   191      GLY C   192                0.00
CISPEP   17 ASN C   199      HIS C   200                0.00
CISPEP   18 GLY C   208      PRO C   209                0.00
CISPEP   19 LYS C   216      PRO C   217                0.00
CISPEP   20 ASP C   236      SER C   237                0.00
CISPEP   21 ASP C   244      SER C   245                0.00
CISPEP   22 PHE C   299      PRO C   300                0.00
CISPEP   23 SER C   327      THR C   328                0.00
CISPEP   24 ALA C   386      PRO C   387                0.00
CISPEP   25 GLU C   414      VAL C   415                0.00
CISPEP   26 GLY C   423      PRO C   424                0.00
CRYST1   58.387  151.411    64.054  90.00 117.11  90.00 P 1 21 1
SCALE1      0.017127  0.000000  0.008768       0.00000
SCALE2      0.000000  0.006605  0.000000       0.00000
SCALE3      0.000000  0.000000  0.017539       0.00000
HETATM    1  N   ASN A   1      18.066  20.808  -3.996  1.00 14.87       A    N
HETATM    2  C9  ASN A   1      18.461  22.053  -3.689  1.00 14.47       A    C
HETATM    3  O10 ASN A   1      19.168  22.251  -2.661  1.00 13.33       A    O
HETATM    4  O11 ASN A   1      18.108  23.029  -4.423  1.00 14.69       A    O
HETATM    5  CA  ASN A   1      18.499  19.635  -3.189  1.00 14.35       A    C
HETATM    6  CB  ASN A   1      18.164  18.329  -3.883  1.00 14.69       A    C
HETATM    7  CG  ASN A   1      16.670  18.063  -4.031  1.00 14.08       A    C
HETATM    8  ND2 ASN A   1      16.271  17.100  -5.019  1.00 12.20       A    N
HETATM    9  OD1 ASN A   1      15.768  18.701  -3.206  1.00 14.76       A    O
HETATM   10  C   ASN A   1      19.990  19.659  -2.890  1.00 14.84       A    C
HETATM   11  O   ASN A   1      20.353  19.313  -1.601  1.00 14.20       A    O
ATOM     12  N   ASP A   2      20.881  19.935  -3.834  1.00 15.84       A    N
ATOM     13  CA  ASP A   2      22.306  19.835  -3.520  1.00 16.82       A    C
ATOM     14  CB  ASP A   2      23.178  20.088  -4.763  1.00 17.53       A    C
ATOM     15  CG  ASP A   2      23.121  18.947  -5.783  1.00 18.18       A    C
ATOM     16  OD1 ASP A   2      22.652  17.811  -5.493  1.00 20.58       A    O
ATOM     17  OD2 ASP A   2      23.544  19.106  -6.931  1.00 22.02       A    O
ATOM     18  C   ASP A   2      22.712  20.816  -2.413  1.00 17.23       A    C
ATOM     19  O   ASP A   2      23.671  20.562  -1.703  1.00 18.17       A    O
ATOM     20  N   VAL A   3      22.018  21.952  -2.304  1.00 17.05       A    N
ATOM     21  CA  VAL A   3      22.374  22.945  -1.311  1.00 16.07       A    C
ATOM     22  CB  VAL A   3      21.974  24.356  -1.701  1.00 16.60       A    C
ATOM     23  CG1 VAL A   3      22.327  25.323  -0.560  1.00 16.25       A    C
ATOM     24  CG2 VAL A   3      22.676  24.770  -3.003  1.00 18.81       A    C
ATOM     25  C   VAL A   3      21.749  22.565   0.033  1.00 15.65       A    C
ATOM     26  O   VAL A   3      22.431  22.603   1.090  1.00 14.41       A    O
ATOM     27  N   ALA A   4      20.497  22.119  -0.012  1.00 13.75       A    N
ATOM     28  CA  ALA A   4      19.824  21.664   1.196  1.00 14.03       A    C
ATOM     29  CB  ALA A   4      18.388  21.260   0.881  1.00 13.78       A    C
ATOM     30  C   ALA A   4      20.544  20.512   1.876  1.00 14.28       A    C
ATOM     31  O   ALA A   4      20.548  20.406   3.110  1.00 14.07       A    O
ATOM     32  N   ARG A   5      21.093  19.617   1.064  1.00 13.74       A    N
ATOM     33  CA  ARG A   5      21.807  18.445   1.553  1.00 14.95       A    C
ATOM     34  CB  ARG A   5      22.395  17.709   0.349  1.00 15.61       A    C
ATOM     35  CG  ARG A   5      23.452  16.639   0.631  1.00 17.28       A    C
ATOM     36  CD  ARG A   5      23.873  15.945  -0.672  1.00 20.73       A    C
ATOM     37  NE  ARG A   5      24.802  14.852  -0.459  1.00 21.95       A    N
ATOM     38  CZ  ARG A   5      26.128  14.986  -0.513  1.00 24.69       A    C
ATOM     39  NH1 ARG A   5      26.687  16.173  -0.793  1.00 25.62       A    N
ATOM     40  NH2 ARG A   5      26.898  13.933  -0.290  1.00 22.96       A    N
ATOM     41  C   ARG A   5      22.918  18.840   2.515  1.00 14.83       A    C
```

| ATOM | 42 | O | ARG | A | 5 | 23.135 | 18.195 | 3.546 | 1.00 | 14.86 | A | O |
|------|-----|-----|-----|---|----|--------|--------|-------|------|-------|---|---|
| ATOM | 43 | N | GLY | A | 6 | 23.641 | 19.897 | 2.166 | 1.00 | 15.33 | A | N |
| ATOM | 44 | CA | GLY | A | 6 | 24.677 | 20.416 | 3.044 | 1.00 | 15.34 | A | C |
| ATOM | 45 | C | GLY | A | 6 | 24.094 | 21.124 | 4.257 | 1.00 | 15.01 | A | C |
| ATOM | 46 | O | GLY | A | 6 | 24.609 | 20.980 | 5.362 | 1.00 | 14.62 | A | O |
| ATOM | 47 | N | ILE | A | 7 | 23.018 | 21.879 | 4.062 | 1.00 | 14.44 | A | N |
| ATOM | 48 | CA | ILE | A | 7 | 22.411 | 22.613 | 5.168 | 1.00 | 13.98 | A | C |
| ATOM | 49 | CB | ILE | A | 7 | 21.266 | 23.505 | 4.698 | 1.00 | 13.68 | A | C |
| ATOM | 50 | CG1 | ILE | A | 7 | 21.813 | 24.676 | 3.864 | 1.00 | 13.35 | A | C |
| ATOM | 51 | CD1 | ILE | A | 7 | 20.794 | 25.294 | 2.972 | 1.00 | 14.08 | A | C |
| ATOM | 52 | CG2 | ILE | A | 7 | 20.511 | 24.072 | 5.873 | 1.00 | 12.51 | A | C |
| ATOM | 53 | C | ILE | A | 7 | 21.970 | 21.664 | 6.305 | 1.00 | 15.04 | A | C |
| ATOM | 54 | O | ILE | A | 7 | 22.273 | 21.906 | 7.469 | 1.00 | 13.35 | A | O |
| ATOM | 55 | N | VAL | A | 8 | 21.320 | 20.558 | 5.952 | 1.00 | 15.03 | A | N |
| ATOM | 56 | CA | VAL | A | 8 | 20.795 | 19.628 | 6.969 | 1.00 | 14.89 | A | C |
| ATOM | 57 | CB | VAL | A | 8 | 19.419 | 19.047 | 6.545 | 1.00 | 14.63 | A | C |
| ATOM | 58 | CG1 | VAL | A | 8 | 18.472 | 20.135 | 6.246 | 1.00 | 14.63 | A | C |
| ATOM | 59 | CG2 | VAL | A | 8 | 19.526 | 18.151 | 5.333 | 1.00 | 15.54 | A | C |
| ATOM | 60 | C | VAL | A | 8 | 21.770 | 18.511 | 7.356 | 1.00 | 14.75 | A | C |
| ATOM | 61 | O | VAL | A | 8 | 21.438 | 17.645 | 8.168 | 1.00 | 15.23 | A | O |
| ATOM | 62 | N | LYS | A | 9 | 22.983 | 18.568 | 6.804 | 1.00 | 14.02 | A | N |
| ATOM | 63 | CA | LYS | A | 9 | 24.061 | 17.627 | 7.118 | 1.00 | 14.55 | A | C |
| ATOM | 64 | CB | LYS | A | 9 | 24.374 | 17.560 | 8.621 | 1.00 | 15.28 | A | C |
| ATOM | 65 | CG | LYS | A | 9 | 24.553 | 18.888 | 9.299 | 1.00 | 18.34 | A | C |
| ATOM | 66 | CD | LYS | A | 9 | 25.757 | 19.608 | 8.810 | 1.00 | 23.66 | A | C |
| ATOM | 67 | CE | LYS | A | 9 | 26.025 | 20.904 | 9.618 | 1.00 | 28.33 | A | C |
| ATOM | 68 | NZ | LYS | A | 9 | 27.283 | 21.559 | 9.079 | 1.00 | 31.91 | A | N |
| ATOM | 69 | C | LYS | A | 9 | 23.798 | 16.226 | 6.616 | 1.00 | 13.77 | A | C |
| ATOM | 70 | O | LYS | A | 9 | 24.391 | 15.256 | 7.132 | 1.00 | 13.96 | A | O |
| ATOM | 71 | N | ALA | A | 10 | 22.979 | 16.109 | 5.569 | 1.00 | 13.98 | A | N |
| ATOM | 72 | CA | ALA | A | 10 | 22.816 | 14.830 | 4.886 | 1.00 | 14.34 | A | C |
| ATOM | 73 | CB | ALA | A | 10 | 21.649 | 14.866 | 3.848 | 1.00 | 14.47 | A | C |
| ATOM | 74 | C | ALA | A | 10 | 24.141 | 14.437 | 4.205 | 1.00 | 14.55 | A | C |
| ATOM | 75 | O | ALA | A | 10 | 24.409 | 13.264 | 4.015 | 1.00 | 13.73 | A | O |
| ATOM | 76 | N | ASP | A | 11 | 24.967 | 15.423 | 3.860 | 1.00 | 16.04 | A | N |
| ATOM | 77 | CA | ASP | A | 11 | 26.278 | 15.153 | 3.265 | 1.00 | 17.11 | A | C |
| ATOM | 78 | CB | ASP | A | 11 | 26.899 | 16.419 | 2.667 | 1.00 | 17.53 | A | C |
| ATOM | 79 | CG | ASP | A | 11 | 27.059 | 17.547 | 3.680 | 1.00 | 19.89 | A | C |
| ATOM | 80 | OD1 | ASP | A | 11 | 27.845 | 18.461 | 3.375 | 1.00 | 23.81 | A | O |
| ATOM | 81 | OD2 | ASP | A | 11 | 26.434 | 17.635 | 4.773 | 1.00 | 20.19 | A | O |
| ATOM | 82 | C | ASP | A | 11 | 27.219 | 14.489 | 4.285 | 1.00 | 17.57 | A | C |
| ATOM | 83 | O | ASP | A | 11 | 27.941 | 13.540 | 3.947 | 1.00 | 17.08 | A | O |
| ATOM | 84 | N | VAL | A | 12 | 27.153 | 14.945 | 5.528 | 1.00 | 17.31 | A | N |
| ATOM | 85 | CA | VAL | A | 12 | 27.926 | 14.338 | 6.607 | 1.00 | 17.86 | A | C |
| ATOM | 86 | CB | VAL | A | 12 | 27.850 | 15.193 | 7.893 | 1.00 | 18.12 | A | C |
| ATOM | 87 | CG1 | VAL | A | 12 | 28.577 | 14.533 | 9.081 | 1.00 | 18.00 | A | C |
| ATOM | 88 | CG2 | VAL | A | 12 | 28.385 | 16.633 | 7.631 | 1.00 | 19.36 | A | C |
| ATOM | 89 | C | VAL | A | 12 | 27.428 | 12.898 | 6.835 | 1.00 | 18.14 | A | C |
| ATOM | 90 | O | VAL | A | 12 | 28.233 | 11.956 | 6.925 | 1.00 | 18.38 | A | O |
| ATOM | 91 | N | ALA | A | 13 | 26.117 | 12.696 | 6.870 | 1.00 | 17.13 | A | N |
| ATOM | 92 | CA | ALA | A | 13 | 25.572 | 11.353 | 7.076 | 1.00 | 17.08 | A | C |
| ATOM | 93 | CB | ALA | A | 13 | 24.070 | 11.400 | 7.101 | 1.00 | 17.00 | A | C |
| ATOM | 94 | C | ALA | A | 13 | 26.044 | 10.394 | 5.981 | 1.00 | 17.57 | A | C |
| ATOM | 95 | O | ALA | A | 13 | 26.472 | 9.237 | 6.254 | 1.00 | 16.79 | A | O |
| ATOM | 96 | N | GLN | A | 14 | 25.934 | 10.862 | 4.740 | 1.00 | 17.24 | A | N |
| ATOM | 97 | CA | GLN | A | 14 | 26.420 | 10.107 | 3.582 | 1.00 | 17.55 | A | C |
| ATOM | 98 | CB | GLN | A | 14 | 25.972 | 10.825 | 2.309 | 1.00 | 17.74 | A | C |
| ATOM | 99 | CG | GLN | A | 14 | 24.485 | 10.673 | 2.031 | 1.00 | 17.61 | A | C |
| ATOM | 100 | CD | GLN | A | 14 | 23.995 | 11.535 | 0.887 | 1.00 | 20.02 | A | C |
| ATOM | 101 | OE1 | GLN | A | 14 | 24.788 | 11.949 | 0.028 | 1.00 | 19.60 | A | O |
| ATOM | 102 | NE2 | GLN | A | 14 | 22.679 | 11.789 | 0.850 | 1.00 | 19.07 | A | N |
| ATOM | 103 | C | GLN | A | 14 | 27.949 | 9.876 | 3.576 | 1.00 | 18.95 | A | C |
| ATOM | 104 | O | GLN | A | 14 | 28.413 | 8.729 | 3.489 | 1.00 | 18.61 | A | O |

```
ATOM    105  N    ASN A  15     28.730  10.950   3.658  1.00 19.73      A    N
ATOM    106  CA   ASN A  15     30.185  10.847   3.469  1.00 20.71      A    C
ATOM    107  CB   ASN A  15     30.828  12.222   3.244  1.00 20.45      A    C
ATOM    108  CG   ASN A  15     30.404  12.869   1.959  1.00 22.21      A    C
ATOM    109  OD1  ASN A  15     30.098  12.201   0.976  1.00 25.39      A    O
ATOM    110  ND2  ASN A  15     30.390  14.182   1.953  1.00 23.97      A    N
ATOM    111  C    ASN A  15     30.865  10.185   4.653  1.00 20.49      A    C
ATOM    112  O    ASN A  15     31.705   9.362   4.469  1.00 21.06      A    O
ATOM    113  N    ASN A  16     30.495  10.559   5.869  1.00 21.00      A    N
ATOM    114  CA   ASN A  16     31.148  10.056   7.073  1.00 21.90      A    C
ATOM    115  CB   ASN A  16     31.205  11.146   8.136  1.00 22.29      A    C
ATOM    116  CG   ASN A  16     32.100  12.313   7.751  1.00 26.21      A    C
ATOM    117  OD1  ASN A  16     32.261  13.260   8.533  1.00 32.71      A    O
ATOM    118  ND2  ASN A  16     32.672  12.268   6.567  1.00 28.57      A    N
ATOM    119  C    ASN A  16     30.491   8.811   7.692  1.00 21.95      A    C
ATOM    120  O    ASN A  16     31.152   8.065   8.404  1.00 22.21      A    O
ATOM    121  N    PHE A  17     29.203   8.578   7.438  1.00 20.66      A    N
ATOM    122  CA   PHE A  17     28.550   7.392   8.003  1.00 20.24      A    C
ATOM    123  CB   PHE A  17     27.415   7.815   8.938  1.00 21.09      A    C
ATOM    124  CG   PHE A  17     27.890   8.591  10.134  1.00 19.81      A    C
ATOM    125  CD1  PHE A  17     28.110   7.953  11.348  1.00 24.93      A    C
ATOM    126  CE1  PHE A  17     28.556   8.679  12.459  1.00 25.33      A    C
ATOM    127  CZ   PHE A  17     28.779  10.016  12.344  1.00 23.90      A    C
ATOM    128  CE2  PHE A  17     28.564  10.651  11.155  1.00 22.65      A    C
ATOM    129  CD2  PHE A  17     28.111   9.936  10.052  1.00 20.02      A    C
ATOM    130  C    PHE A  17     28.061   6.385   6.977  1.00 19.13      A    C
ATOM    131  O    PHE A  17     27.607   5.336   7.337  1.00 20.18      A    O
ATOM    132  N    GLY A  18     28.205   6.685   5.692  1.00 17.84      A    N
ATOM    133  CA   GLY A  18     27.740   5.790   4.640  1.00 17.25      A    C
ATOM    134  C    GLY A  18     26.220   5.654   4.496  1.00 16.27      A    C
ATOM    135  O    GLY A  18     25.755   4.667   3.948  1.00 14.47      A    O
ATOM    136  N    LEU A  19     25.453   6.651   4.955  1.00 15.24      A    N
ATOM    137  CA   LEU A  19     23.980   6.550   5.007  1.00 14.35      A    C
ATOM    138  CB   LEU A  19     23.456   7.222   6.270  1.00 14.71      A    C
ATOM    139  CG   LEU A  19     24.013   6.680   7.569  1.00 15.58      A    C
ATOM    140  CD1  LEU A  19     23.691   7.633   8.721  1.00 16.09      A    C
ATOM    141  CD2  LEU A  19     23.417   5.294   7.793  1.00 15.86      A    C
ATOM    142  C    LEU A  19     23.305   7.203   3.820  1.00 13.82      A    C
ATOM    143  O    LEU A  19     23.183   8.427   3.775  1.00 13.96      A    O
ATOM    144  N    TYR A  20     22.874   6.400   2.854  1.00 13.81      A    N
ATOM    145  CA   TYR A  20     22.156   6.917   1.714  1.00 14.22      A    C
ATOM    146  CB   TYR A  20     22.841   6.499   0.386  1.00 14.36      A    C
ATOM    147  CG   TYR A  20     24.254   7.034   0.241  1.00 14.09      A    C
ATOM    148  CD1  TYR A  20     25.351   6.353   0.792  1.00 16.48      A    C
ATOM    149  CE1  TYR A  20     26.661   6.858   0.663  1.00 16.91      A    C
ATOM    150  CZ   TYR A  20     26.859   8.041  -0.034  1.00 18.57      A    C
ATOM    151  OH   TYR A  20     28.126   8.567  -0.171  1.00 21.21      A    O
ATOM   .152  CE2  TYR A  20     25.788   8.735  -0.575  1.00 17.45      A    C
ATOM    153  CD2  TYR A  20     24.495   8.217  -0.461  1.00 16.03      A    C
ATOM    154  C    TYR A  20     20.715   6.433   1.702  1.00 14.55      A    C
ATOM    155  O    TYR A  20     19.994   6.688   0.723  1.00 14.48      A    O
ATOM    156  N    GLY A  21     20.297   5.710   2.746  1.00 14.18      A    N
ATOM    157  CA   GLY A  21     18.947   5.172   2.802  1.00 14.23      A    C
ATOM    158  C    GLY A  21     18.749   3.775   2.207  1.00 14.56      A    C
ATOM    159  O    GLY A  21     17.611   3.315   2.054  1.00 13.53      A    O
ATOM    160  N    GLN A  22     19.838   3.084   1.883  1.00 14.57      A    N
ATOM    161  CA   GLN A  22     19.722   1.726   1.334  1.00 14.82      A    C
ATOM    162  CB   GLN A  22     21.095   1.130   0.978  1.00 15.45      A    C
ATOM    163  CG   GLN A  22     21.054  -0.151   0.150  1.00 17.91      A    C
ATOM    164  CD   GLN A  22     20.669  -1.376   0.976  1.00 21.79      A    C
ATOM    165  OE1  GLN A  22     20.892  -1.414   2.185  1.00 22.42      A    O
ATOM    166  NE2  GLN A  22     20.091  -2.379   0.317  1.00 23.11      A    N
ATOM    167  C    GLN A  22     19.011   0.831   2.331  1.00 14.04      A    C
```

| | | | | | | | | | | | | |
|---|---|---|---|---|---|---|---|---|---|---|---|---|
| ATOM | 168 | O | GLN | A | 22 | 19.341 | 0.824 | 3.516 | 1.00 | 14.39 | A | O |
| ATOM | 169 | N | GLY | A | 23 | 18.019 | 0.110 | 1.836 | 1.00 | 14.26 | A | N |
| ATOM | 170 | CA | GLY | A | 23 | 17.236 | -0.859 | 2.628 | 1.00 | 14.81 | A | C |
| ATOM | 171 | C | GLY | A | 23 | 15.957 | -0.245 | 3.176 | 1.00 | 14.12 | A | C |
| ATOM | 172 | O | GLY | A | 23 | 15.086 | -0.948 | 3.718 | 1.00 | 14.17 | A | O |
| ATOM | 173 | N | GLN | A | 24 | 15.836 | 1.077 | 3.057 | 1.00 | 13.54 | A | N |
| ATOM | 174 | CA | GLN | A | 24 | 14.620 | 1.773 | 3.500 | 1.00 | 13.27 | A | C |
| ATOM | 175 | CB | GLN | A | 24 | 14.963 | 3.090 | 4.182 | 1.00 | 12.64 | A | C |
| ATOM | 176 | CG | GLN | A | 24 | 15.806 | 2.945 | 5.450 | 1.00 | 13.46 | A | C |
| ATOM | 177 | CD | GLN | A | 24 | 15.150 | 2.100 | 6.505 | 1.00 | 15.72 | A | C |
| ATOM | 178 | OE1 | GLN | A | 24 | 14.015 | 2.387 | 6.921 | 1.00 | 14.73 | A | O |
| ATOM | 179 | NE2 | GLN | A | 24 | 15.839 | 1.026 | 6.927 | 1.00 | 13.89 | A | N |
| ATOM | 180 | C | GLN | A | 24 | 13.619 | 2.022 | 2.352 | 1.00 | 13.19 | A | C |
| ATOM | 181 | O | GLN | A | 24 | 14.005 | 2.126 | 1.184 | 1.00 | 13.48 | A | O |
| ATOM | 182 | N | ILE | A | 25 | 12.324 | 2.066 | 2.692 | 1.00 | 13.28 | A | N |
| ATOM | 183 | CA | ILE | A | 25 | 11.280 | 2.319 | 1.720 | 1.00 | 13.25 | A | C |
| ATOM | 184 | CB | ILE | A | 25 | 10.404 | 1.077 | 1.507 | 1.00 | 13.64 | A | C |
| ATOM | 185 | CG1 | ILE | A | 25 | 11.267 | -0.108 | 1.030 | 1.00 | 15.44 | A | C |
| ATOM | 186 | CD1 | ILE | A | 25 | 10.508 | -1.518 | 0.962 | 1.00 | 14.73 | A | C |
| ATOM | 187 | CG2 | ILE | A | 25 | 9.303 | 1.387 | 0.503 | 1.00 | 13.37 | A | C |
| ATOM | 188 | C | ILE | A | 25 | 10.447 | 3.491 | 2.209 | 1.00 | 13.24 | A | C |
| ATOM | 189 | O | ILE | A | 25 | 9.884 | 3.430 | 3.285 | 1.00 | 12.93 | A | O |
| ATOM | 190 | N | VAL | A | 26 | 10.438 | 4.573 | 1.432 | 1.00 | 12.43 | A | N |
| ATOM | 191 | CA | VAL | A | 26 | 9.656 | 5.754 | 1.737 | 1.00 | 12.49 | A | C |
| ATOM | 192 | CB | VAL | A | 26 | 10.480 | 7.034 | 1.585 | 1.00 | 12.90 | A | C |
| ATOM | 193 | CG1 | VAL | A | 26 | 9.671 | 8.231 | 2.059 | 1.00 | 11.57 | A | C |
| ATOM | 194 | CG2 | VAL | A | 26 | 11.796 | 6.928 | 2.395 | 1.00 | 15.53 | A | C |
| ATOM | 195 | C | VAL | A | 26 | 8.465 | 5.823 | 0.804 | 1.00 | 12.34 | A | C |
| ATOM | 196 | O | VAL | A | 26 | 8.601 | 5.646 | -0.418 | 1.00 | 11.99 | A | O |
| ATOM | 197 | N | ALA | A | 27 | 7.297 | 6.044 | 1.387 | 1.00 | 12.40 | A | N |
| ATOM | 198 | CA | ALA | A | 27 | 6.080 | 6.289 | 0.624 | 1.00 | 12.49 | A | C |
| ATOM | 199 | CB | ALA | A | 27 | 4.846 | 5.650 | 1.284 | 1.00 | 11.39 | A | C |
| ATOM | 200 | C | ALA | A | 27 | 5.892 | 7.790 | 0.546 | 1.00 | 12.17 | A | C |
| ATOM | 201 | O | ALA | A | 27 | 6.077 | 8.501 | 1.526 | 1.00 | 11.39 | A | O |
| ATOM | 202 | N | VAL | A | 28 | 5.540 | 8.243 | -0.643 | 1.00 | 11.79 | A | N |
| ATOM | 203 | CA | VAL | A | 28 | 5.168 | 9.612 | -0.910 | 1.00 | 11.63 | A | C |
| ATOM | 204 | CB | VAL | A | 28 | 6.054 | 10.176 | -2.003 | 1.00 | 11.56 | A | C |
| ATOM | 205 | CG1 | VAL | A | 28 | 5.629 | 11.625 | -2.440 | 1.00 | 12.77 | A | C |
| ATOM | 206 | CG2 | VAL | A | 28 | 7.514 | 10.079 | -1.594 | 1.00 | 11.95 | A | C |
| ATOM | 207 | C | VAL | A | 28 | 3.729 | 9.580 | -1.458 | 1.00 | 11.23 | A | C |
| ATOM | 208 | O | VAL | A | 28 | 3.470 | 8.936 | -2.459 | 1.00 | 10.72 | A | O |
| ATOM | 209 | N | ALA | A | 29 | 2.817 | 10.294 | -0.831 | 1.00 | 10.64 | A | N |
| ATOM | 210 | CA | ALA | A | 29 | 1.468 | 10.435 | -1.365 | 1.00 | 11.32 | A | C |
| ATOM | 211 | CB | ALA | A | 29 | 0.441 | 10.151 | -0.298 | 1.00 | 11.33 | A | C |
| ATOM | 212 | C | ALA | A | 29 | 1.326 | 11.842 | -1.909 | 1.00 | 11.35 | A | C |
| ATOM | 213 | O | ALA | A | 29 | 1.404 | 12.826 | -1.161 | 1.00 | 11.19 | A | O |
| ATOM | 214 | N | ASP | A | 30 | 1.186 | 11.937 | -3.229 | 1.00 | 11.71 | A | N |
| ATOM | 215 | CA | ASP | A | 30 | 1.266 | 13.221 | -3.917 | 1.00 | 11.52 | A | C |
| ATOM | 216 | CB | ASP | A | 30 | 2.718 | 13.715 | -3.958 | 1.00 | 11.37 | A | C |
| ATOM | 217 | CG | ASP | A | 30 | 2.802 | 15.221 | -3.852 | 1.00 | 12.40 | A | C |
| ATOM | 218 | OD1 | ASP | A | 30 | 3.385 | 15.726 | -2.871 | 1.00 | 12.15 | A | O |
| ATOM | 219 | OD2 | ASP | A | 30 | 2.226 | 15.973 | -4.682 | 1.00 | 14.39 | A | O |
| ATOM | 220 | C | ASP | A | 30 | 0.665 | 13.113 | -5.327 | 1.00 | 12.41 | A | C |
| ATOM | 221 | O | ASP | A | 30 | 0.068 | 12.086 | -5.671 | 1.00 | 12.89 | A | O |
| ATOM | 222 | N | THR | A | 31 | 0.811 | 14.162 | -6.151 | 1.00 | 12.52 | A | N |
| ATOM | 223 | CA | THR | A | 31 | -0.004 | 14.263 | -7.353 | 1.00 | 11.62 | A | C |
| ATOM | 224 | CB | THR | A | 31 | 0.302 | 15.554 | -8.182 | 1.00 | 11.72 | A | C |
| ATOM | 225 | OG1 | THR | A | 31 | 1.709 | 15.702 | -8.423 | 1.00 | 11.44 | A | O |
| ATOM | 226 | CG2 | THR | A | 31 | -0.099 | 16.789 | -7.424 | 1.00 | 11.85 | A | C |
| ATOM | 227 | C | THR | A | 31 | 0.126 | 13.041 | -8.225 | 1.00 | 12.59 | A | C |
| ATOM | 228 | O | THR | A | 31 | -0.868 | 12.341 | -8.494 | 1.00 | 12.63 | A | O |
| ATOM | 229 | N | GLY | A | 32 | 1.360 | 12.810 | -8.665 | 1.00 | 12.03 | A | N |
| ATOM | 230 | CA | GLY | A | 32 | 1.694 | 11.788 | -9.617 | 1.00 | 12.81 | A | C |

| ATOM | 231 | C | GLY | A | 32 | 3.202 | 11.763 | -9.729 | 1.00 | 13.22 | A | C |
| ATOM | 232 | O | GLY | A | 32 | 3.885 | 12.607 | -9.135 | 1.00 | 12.83 | A | O |
| ATOM | 233 | N | LEU | A | 33 | 3.711 | 10.813 | -10.501 | 1.00 | 13.41 | A | N |
| ATOM | 234 | CA | LEU | A | 33 | 5.139 | 10.622 | -10.678 | 1.00 | 13.86 | A | C |
| ATOM | 235 | CB | LEU | A | 33 | 5.625 | 9.397 | -9.899 | 1.00 | 13.74 | A | C |
| ATOM | 236 | CG | LEU | A | 33 | 7.148 | 9.234 | -9.900 | 1.00 | 14.12 | A | C |
| ATOM | 237 | CD1 | LEU | A | 33 | 7.768 | 10.273 | -8.964 | 1.00 | 13.99 | A | C |
| ATOM | 238 | CD2 | LEU | A | 33 | 7.497 | 7.818 | -9.437 | 1.00 | 15.42 | A | C |
| ATOM | 239 | C | LEU | A | 33 | 5.517 | 10.505 | -12.151 | 1.00 | 13.89 | A | C |
| ATOM | 240 | O | LEU | A | 33 | 5.374 | 9.444 | -12.765 | 1.00 | 14.14 | A | O |
| ATOM | 241 | N | ASP | A | 34 | 6.009 | 11.612 | -12.696 | 1.00 | 14.55 | A | N |
| ATOM | 242 | CA | ASP | A | 34 | 6.455 | 11.701 | -14.087 | 1.00 | 14.62 | A | C |
| ATOM | 243 | CB | ASP | A | 34 | 7.899 | 11.201 | -14.224 | 1.00 | 14.72 | A | C |
| ATOM | 244 | CG | ASP | A | 34 | 8.516 | 11.532 | -15.598 | 1.00 | 15.30 | A | C |
| ATOM | 245 | OD1 | ASP | A | 34 | 9.260 | 10.694 | -16.148 | 1.00 | 14.31 | A | O |
| ATOM | 246 | OD2 | ASP | A | 34 | 8.268 | 12.602 | -16.207 | 1.00 | 17.33 | A | O |
| ATOM | 247 | C | ASP | A | 34 | 5.470 | 11.016 | -15.060 | 1.00 | 14.94 | A | C |
| ATOM | 248 | O | ASP | A | 34 | 4.297 | 11.415 | -15.124 | 1.00 | 15.39 | A | O |
| ATOM | 249 | N | THR | A | 35 | 5.927 | 10.013 | -15.816 | 1.00 | 16.25 | A | N |
| ATOM | 250 | CA | THR | A | 35 | 5.083 | 9.340 | -16.813 | 1.00 | 16.50 | A | C |
| ATOM | 251 | CB | THR | A | 35 | 5.912 | 8.471 | -17.786 | 1.00 | 17.03 | A | C |
| ATOM | 252 | OG1 | THR | A | 35 | 6.700 | 7.514 | -17.051 | 1.00 | 17.34 | A | O |
| ATOM | 253 | CG2 | THR | A | 35 | 6.922 | 9.300 | -18.593 | 1.00 | 17.53 | A | C |
| ATOM | 254 | C | THR | A | 35 | 4.005 | 8.437 | -16.229 | 1.00 | 17.26 | A | C |
| ATOM | 255 | O | THR | A | 35 | 3.111 | 7.992 | -16.946 | 1.00 | 15.49 | A | O |
| ATOM | 256 | N | GLY | A | 36 | 4.104 | 8.104 | -14.948 | 1.00 | 16.59 | A | N |
| ATOM | 257 | CA | GLY | A | 36 | 3.094 | 7.259 | -14.360 | 1.00 | 16.76 | A | C |
| ATOM | 258 | C | GLY | A | 36 | 3.308 | 5.802 | -14.660 | 1.00 | 17.58 | A | C |
| ATOM | 259 | O | GLY | A | 36 | 2.432 | 4.984 | -14.383 | 1.00 | 17.55 | A | O |
| ATOM | 260 | N | ARG | A | 37 | 4.473 | 5.465 | -15.200 | 1.00 | 18.31 | A | N |
| ATOM | 261 | CA | ARG | A | 37 | 4.748 | 4.091 | -15.575 | 1.00 | 19.42 | A | C |
| ATOM | 262 | CB | ARG | A | 37 | 4.763 | 3.940 | -17.088 | 1.00 | 20.37 | A | C |
| ATOM | 263 | CG | ARG | A | 37 | 3.436 | 4.298 | -17.742 | 1.00 | 23.71 | A | C |
| ATOM | 264 | CD | ARG | A | 37 | 3.283 | 3.740 | -19.140 | 1.00 | 31.29 | A | C |
| ATOM | 265 | NE | ARG | A | 37 | 4.324 | 4.233 | -20.024 | 1.00 | 34.29 | A | N |
| ATOM | 266 | CZ | ARG | A | 37 | 4.322 | 5.434 | -20.575 | 1.00 | 38.63 | A | C |
| ATOM | 267 | NH1 | ARG | A | 37 | 5.331 | 5.792 | -21.361 | 1.00 | 39.90 | A | N |
| ATOM | 268 | NH2 | ARG | A | 37 | 3.305 | 6.273 | -20.362 | 1.00 | 40.23 | A | N |
| ATOM | 269 | C | ARG | A | 37 | 6.072 | 3.661 | -14.998 | 1.00 | 19.41 | A | C |
| ATOM | 270 | O | ARG | A | 37 | 7.065 | 4.354 | -15.150 | 1.00 | 18.12 | A | O |
| ATOM | 271 | N | ASN | A | 38 | 6.067 | 2.506 | -14.334 | 1.00 | 19.47 | A | N |
| ATOM | 272 | CA | ASN | A | 38 | 7.254 | 1.998 | -13.703 | 1.00 | 20.37 | A | C |
| ATOM | 273 | CB | ASN | A | 38 | 6.917 | 1.215 | -12.431 | 1.00 | 20.71 | A | C |
| ATOM | 274 | CG | ASN | A | 38 | 8.161 | 0.841 | -11.658 | 1.00 | 21.12 | A | C |
| ATOM | 275 | OD1 | ASN | A | 38 | 9.248 | 1.337 | -11.968 | 1.00 | 18.41 | A | O |
| ATOM | 276 | ND2 | ASN | A | 38 | 8.023 | -0.072 | -10.684 | 1.00 | 20.60 | A | N |
| ATOM | 277 | C | ASN | A | 38 | 7.984 | 1.134 | -14.700 | 1.00 | 21.21 | A | C |
| ATOM | 278 | O | ASN | A | 38 | 7.918 | -0.099 | -14.638 | 1.00 | 21.03 | A | O |
| ATOM | 279 | N | ASP | A | 39 | 8.659 | 1.806 | -15.625 | 1.00 | 21.69 | A | N |
| ATOM | 280 | CA | ASP | A | 39 | 9.363 | 1.158 | -16.718 | 1.00 | 23.22 | A | C |
| ATOM | 281 | CB | ASP | A | 39 | 8.405 | 0.839 | -17.882 | 1.00 | 23.19 | A | C |
| ATOM | 282 | CG | ASP | A | 39 | 7.806 | 2.082 | -18.526 | 1.00 | 24.66 | A | C |
| ATOM | 283 | OD1 | ASP | A | 39 | 6.796 | 1.945 | -19.248 | 1.00 | 26.50 | A | O |
| ATOM | 284 | OD2 | ASP | A | 39 | 8.246 | 3.239 | -18.372 | 1.00 | 27.03 | A | O |
| ATOM | 285 | C | ASP | A | 39 | 10.480 | 2.075 | -17.156 | 1.00 | 24.00 | A | C |
| ATOM | 286 | O | ASP | A | 39 | 10.843 | 3.004 | -16.434 | 1.00 | 23.68 | A | O |
| ATOM | 287 | N | SER | A | 40 | 11.003 | 1.832 | -18.355 | 1.00 | 24.67 | A | N |
| ATOM | 288 | CA | SER | A | 40 | 12.166 | 2.539 | -18.847 | 1.00 | 24.80 | A | C |
| ATOM | 289 | CB | SER | A | 40 | 12.777 | 1.766 | -20.041 | 1.00 | 25.30 | A | C |
| ATOM | 290 | OG | SER | A | 40 | 11.925 | 1.881 | -21.163 | 1.00 | 25.60 | A | O |
| ATOM | 291 | C | SER | A | 40 | 11.815 | 3.984 | -19.228 | 1.00 | 23.51 | A | C |
| ATOM | 292 | O | SER | A | 40 | 12.687 | 4.805 | -19.375 | 1.00 | 24.41 | A | O |
| ATOM | 293 | N | SER | A | 41 | 10.532 | 4.308 | -19.317 | 1.00 | 23.14 | A | N |

```
ATOM   294  CA  SER A  41   10.097   5.670 -19.621  1.00 21.75   A  C
ATOM   295  CB  SER A  41    8.620   5.679 -20.037  1.00 22.84   A  C
ATOM   296  OG  SER A  41    7.725   5.739 -18.919  1.00 21.43   A  O
ATOM   297  C   SER A  41   10.262   6.639 -18.427  1.00 21.13   A  C
ATOM   298  O   SER A  41   10.299   7.863 -18.603  1.00 19.88   A  O
ATOM   299  N   MET A  42   10.359   6.079 -17.223  1.00 19.68   A  N
ATOM   300  CA  MET A  42   10.381   6.882 -15.996  1.00 18.70   A  C
ATOM   301  CB  MET A  42   10.295   5.949 -14.782  1.00 18.20   A  C
ATOM   302  CG  MET A  42   10.451   6.626 -13.423  1.00 17.87   A  C
ATOM   303  SD  MET A  42    9.190   7.804 -13.030  1.00 16.31   A  S
ATOM   304  CE  MET A  42    7.658   6.844 -13.134  1.00 15.38   A  C
ATOM   305  C   MET A  42   11.607   7.779 -15.897  1.00 17.89   A  C
ATOM   306  O   MET A  42   12.728   7.390 -16.223  1.00 17.28   A  O
ATOM   307  N   HIS A  43   11.381   8.998 -15.421  1.00 17.69   A  N
ATOM   308  CA  HIS A  43   12.479   9.903 -15.081  1.00 17.38   A  C
ATOM   309  CB  HIS A  43   11.942  11.020 -14.196  1.00 17.29   A  C
ATOM   310  CG  HIS A  43   12.896  12.155 -13.981  1.00 16.73   A  C
ATOM   311  ND1 HIS A  43   12.576  13.456 -14.321  1.00 16.98   A  N
ATOM   312  CE1 HIS A  43   13.566  14.257 -13.971  1.00 13.61   A  C
ATOM   313  NE2 HIS A  43   14.521  13.523 -13.426  1.00 17.49   A  N
ATOM   314  CD2 HIS A  43   14.113  12.207 -13.397  1.00 13.70   A  C
ATOM   315  C   HIS A  43   13.647   9.209 -14.381  1.00 16.64   A  C
ATOM   316  O   HIS A  43   13.453   8.389 -13.479  1.00 15.82   A  O
ATOM   317  N   GLU A  44   14.858   9.559 -14.818  1.00 16.35   A  N
ATOM   318  CA  GLU A  44   16.112   8.985 -14.358  1.00 16.74   A  C
ATOM   319  CB  GLU A  44   17.293   9.763 -14.988  1.00 17.71   A  C
ATOM   320  CG  GLU A  44   17.268  11.270 -14.753  1.00 18.20   A  C
ATOM   321  CD  GLU A  44   18.445  12.004 -15.418  1.00 22.20   A  C
ATOM   322  OE1 GLU A  44   18.997  11.455 -16.397  1.00 20.94   A  O
ATOM   323  OE2 GLU A  44   18.843  13.110 -14.933  1.00 20.93   A  O
ATOM   324  C   GLU A  44   16.280   8.982 -12.823  1.00 17.11   A  C
ATOM   325  O   GLU A  44   16.944   8.104 -12.259  1.00 17.08   A  O
ATOM   326  N   ALA A  45   15.665   9.954 -12.152  1.00 16.39   A  N
ATOM   327  CA  ALA A  45   15.774  10.061 -10.696  1.00 16.01   A  C
ATOM   328  CB  ALA A  45   15.122  11.354 -10.198  1.00 15.44   A  C
ATOM   329  C   ALA A  45   15.155   8.864  -9.971  1.00 16.39   A  C
ATOM   330  O   ALA A  45   15.538   8.564  -8.857  1.00 14.42   A  O
ATOM   331  N   PHE A  46   14.184   8.218 -10.595  1.00 16.01   A  N
ATOM   332  CA  PHE A  46   13.411   7.139  -9.971  1.00 16.28   A  C
ATOM   333  CB  PHE A  46   11.958   7.562  -9.882  1.00 16.14   A  C
ATOM   334  CG  PHE A  46   11.780   8.959  -9.396  1.00 14.90   A  C
ATOM   335  CD1 PHE A  46   12.036   9.275  -8.078  1.00 14.17   A  C
ATOM   336  CE1 PHE A  46   11.897  10.586  -7.628  1.00 13.19   A  C
ATOM   337  CZ  PHE A  46   11.525  11.592  -8.504  1.00 14.92   A  C
ATOM   338  CE2 PHE A  46   11.291  11.299  -9.809  1.00 16.01   A  C
ATOM   339  CD2 PHE A  46   11.416   9.971 -10.261  1.00 15.93   A  C
ATOM   340  C   PHE A  46   13.466   5.791 -10.697  1.00 17.23   A  C
ATOM   341  O   PHE A  46   13.017   4.764 -10.172  1.00 16.06   A  O
ATOM   342  N   ARG A  47   13.986   5.781 -11.917  1.00 18.87   A  N
ATOM   343  CA  ARG A  47   13.963   4.566 -12.723  1.00 20.32   A  C
ATOM   344  CB  ARG A  47   14.659   4.833 -14.062  1.00 21.00   A  C
ATOM   345  CG  ARG A  47   14.309   3.871 -15.173  1.00 24.13   A  C
ATOM   346  CD  ARG A  47   14.468   4.517 -16.570  1.00 28.53   A  C
ATOM   347  NE  ARG A  47   15.803   5.031 -16.813  1.00 32.22   A  N
ATOM   348  CZ  ARG A  47   16.105   6.229 -17.359  1.00 34.45   A  C
ATOM   349  NH1 ARG A  47   15.171   7.109 -17.703  1.00 33.97   A  N
ATOM   350  NH2 ARG A  47   17.384   6.558 -17.527  1.00 33.82   A  N
ATOM   351  C   ARG A  47   14.674   3.437 -12.000  1.00 20.49   A  C
ATOM   352  O   ARG A  47   15.784   3.619 -11.523  1.00 21.38   A  O
ATOM   353  N   GLY A  48   14.032   2.280 -11.898  1.00 21.45   A  N
ATOM   354  CA  GLY A  48   14.642   1.105 -11.274  1.00 21.59   A  C
ATOM   355  C   GLY A  48   14.583   1.091  -9.741  1.00 21.98   A  C
ATOM   356  O   GLY A  48   15.072   0.145  -9.102  1.00 21.74   A  O
```

| ATOM | 357 | N | LYS | A | 49 | 13.984 | 2.117 | -9.136 | 1.00 | 21.05 | A | N |
| ATOM | 358 | CA | LYS | A | 49 | 13.950 | 2.197 | -7.662 | 1.00 | 20.90 | A | C |
| ATOM | 359 | CB | LYS | A | 49 | 14.915 | 3.305 | -7.180 | 1.00 | 22.03 | A | C |
| ATOM | 360 | CG | LYS | A | 49 | 14.366 | 4.713 | -7.161 | 1.00 | 24.83 | A | C |
| ATOM | 361 | CD | LYS | A | 49 | 15.447 | 5.815 | -6.761 | 1.00 | 27.45 | A | C |
| ATOM | 362 | CE | LYS | A | 49 | 15.957 | 5.680 | -5.358 | 1.00 | 27.82 | A | C |
| ATOM | 363 | NZ | LYS | A | 49 | 17.024 | 4.667 | -5.220 | 1.00 | 28.25 | A | N |
| ATOM | 364 | C | LYS | A | 49 | 12.523 | 2.329 | -7.077 | 1.00 | 19.90 | A | C |
| ATOM | 365 | O | LYS | A | 49 | 12.339 | 2.667 | -5.890 | 1.00 | 19.91 | A | O |
| ATOM | 366 | N | ILE | A | 50 | 11.523 | 1.999 | -7.900 | 1.00 | 18.63 | A | N |
| ATOM | 367 | CA | ILE | A | 50 | 10.121 | 2.078 | -7.533 | 1.00 | 17.48 | A | C |
| ATOM | 368 | CB | ILE | A | 50 | 9.284 | 2.650 | -8.695 | 1.00 | 17.67 | A | C |
| ATOM | 369 | CG1 | ILE | A | 50 | 9.738 | 4.076 | -9.050 | 1.00 | 17.24 | A | C |
| ATOM | 370 | CD1 | ILE | A | 50 | 9.083 | 4.630 | -10.302 | 1.00 | 17.34 | A | C |
| ATOM | 371 | CG2 | ILE | A | 50 | 7.807 | 2.723 | -8.319 | 1.00 | 17.29 | A | C |
| ATOM | 372 | C | ILE | A | 50 | 9.562 | 0.730 | -7.090 | 1.00 | 17.96 | A | C |
| ATOM | 373 | O | ILE | A | 50 | 9.339 | -0.161 | -7.909 | 1.00 | 18.69 | A | O |
| ATOM | 374 | N | THR | A | 51 | 9.355 | 0.583 | -5.784 | 1.00 | 17.09 | A | N |
| ATOM | 375 | CA | THR | A | 51 | 8.731 | -0.601 | -5.218 | 1.00 | 17.60 | A | C |
| ATOM | 376 | CB | THR | A | 51 | 8.700 | -0.423 | -3.690 | 1.00 | 18.38 | A | C |
| ATOM | 377 | OG1 | THR | A | 51 | 10.033 | -0.380 | -3.205 | 1.00 | 17.39 | A | O |
| ATOM | 378 | CG2 | THR | A | 51 | 8.054 | -1.617 | -3.014 | 1.00 | 17.34 | A | C |
| ATOM | 379 | C | THR | A | 51 | 7.301 | -0.746 | -5.646 | 1.00 | 17.50 | A | C |
| ATOM | 380 | O | THR | A | 51 | 6.827 | -1.834 | -5.903 | 1.00 | 18.10 | A | O |
| ATOM | 381 | N | ALA | A | 52 | 6.578 | 0.369 | -5.670 | 1.00 | 16.93 | A | N |
| ATOM | 382 | CA | ALA | A | 52 | 5.179 | 0.338 | -6.052 | 1.00 | 17.34 | A | C |
| ATOM | 383 | CB | ALA | A | 52 | 4.314 | -0.132 | -4.884 | 1.00 | 17.41 | A | C |
| ATOM | 384 | C | ALA | A | 52 | 4.753 | 1.725 | -6.501 | 1.00 | 17.46 | A | C |
| ATOM | 385 | O | ALA | A | 52 | 5.187 | 2.730 | -5.928 | 1.00 | 16.50 | A | O |
| ATOM | 386 | N | LEU | A | 53 | 3.921 | 1.760 | -7.539 | 1.00 | 17.19 | A | N |
| ATOM | 387 | CA | LEU | A | 53 | 3.369 | 2.987 | -8.081 | 1.00 | 16.89 | A | C |
| ATOM | 388 | CB | LEU | A | 53 | 4.004 | 3.309 | -9.430 | 1.00 | 16.66 | A | C |
| ATOM | 389 | CG | LEU | A | 53 | 3.490 | 4.525 | -10.224 | 1.00 | 16.83 | A | C |
| ATOM | 390 | CD1 | LEU | A | 53 | 3.523 | 5.796 | -9.401 | 1.00 | 15.83 | A | C |
| ATOM | 391 | CD2 | LEU | A | 53 | 4.303 | 4.720 | -11.476 | 1.00 | 17.71 | A | C |
| ATOM | 392 | C | LEU | A | 53 | 1.868 | 2.779 | -8.212 | 1.00 | 17.28 | A | C |
| ATOM | 393 | O | LEU | A | 53 | 1.421 | 2.057 | -9.097 | 1.00 | 17.43 | A | O |
| ATOM | 394 | N | TYR | A | 54 | 1.101 | 3.393 | -7.303 | 1.00 | 17.28 | A | N |
| ATOM | 395 | CA | TYR | A | 54 | -0.350 | 3.200 | -7.230 | 1.00 | 16.87 | A | C |
| ATOM | 396 | CB | TYR | A | 54 | -0.774 | 2.944 | -5.789 | 1.00 | 16.88 | A | C |
| ATOM | 397 | CG | TYR | A | 54 | -0.268 | 1.679 | -5.144 | 1.00 | 15.63 | A | C |
| ATOM | 398 | CD1 | TYR | A | 54 | -0.411 | 0.448 | -5.770 | 1.00 | 15.86 | A | C |
| ATOM | 399 | CE1 | TYR | A | 54 | 0.037 | -0.698 | -5.192 | 1.00 | 15.12 | A | C |
| ATOM | 400 | CZ | TYR | A | 54 | 0.666 | -0.647 | -3.946 | 1.00 | 15.32 | A | C |
| ATOM | 401 | OH | TYR | A | 54 | 1.093 | -1.815 | -3.374 | 1.00 | 14.97 | A | O |
| ATOM | 402 | CE2 | TYR | A | 54 | 0.856 | 0.558 | -3.312 | 1.00 | 15.70 | A | C |
| ATOM | 403 | CD2 | TYR | A | 54 | 0.384 | 1.718 | -3.908 | 1.00 | 15.59 | A | C |
| ATOM | 404 | C | TYR | A | 54 | -1.098 | 4.411 | -7.712 | 1.00 | 17.11 | A | C |
| ATOM | 405 | O | TYR | A | 54 | -0.733 | 5.546 | -7.387 | 1.00 | 16.78 | A | O |
| ATOM | 406 | N | ALA | A | 55 | -2.161 | 4.184 | -8.483 | 1.00 | 17.01 | A | N |
| ATOM | 407 | CA | ALA | A | 55 | -3.032 | 5.260 | -8.926 | 1.00 | 17.49 | A | C |
| ATOM | 408 | CB | ALA | A | 55 | -3.355 | 5.094 | -10.437 | 1.00 | 17.43 | A | C |
| ATOM | 409 | C | ALA | A | 55 | -4.323 | 5.272 | -8.100 | 1.00 | 18.26 | A | C |
| ATOM | 410 | O | ALA | A | 55 | -5.174 | 4.400 | -8.269 | 1.00 | 19.60 | A | O |
| ATOM | 411 | N | LEU | A | 56 | -4.481 | 6.267 | -7.230 | 1.00 | 17.69 | A | N |
| ATOM | 412 | CA | LEU | A | 56 | -5.641 | 6.353 | -6.368 | 1.00 | 17.32 | A | C |
| ATOM | 413 | CB | LEU | A | 56 | -5.224 | 6.779 | -4.965 | 1.00 | 16.99 | A | C |
| ATOM | 414 | CG | LEU | A | 56 | -4.452 | 5.752 | -4.129 | 1.00 | 17.97 | A | C |
| ATOM | 415 | CD1 | LEU | A | 56 | -3.120 | 5.532 | -4.719 | 1.00 | 20.33 | A | C |
| ATOM | 416 | CD2 | LEU | A | 56 | -4.329 | 6.225 | -2.662 | 1.00 | 19.18 | A | C |
| ATOM | 417 | C | LEU | A | 56 | -6.662 | 7.360 | -6.867 | 1.00 | 17.28 | A | C |
| ATOM | 418 | O | LEU | A | 56 | -7.839 | 7.192 | -6.653 | 1.00 | 17.95 | A | O |
| ATOM | 419 | N | GLY | A | 57 | -6.204 | 8.430 | -7.485 | 1.00 | 17.26 | A | N |

| ATOM | 420 | CA | GLY A | 57 | -7.068 | 9.541 | -7.802 | 1.00 | 17.53 | A | C |
|------|-----|-----|-------|----|--------|-------|--------|------|-------|---|---|
| ATOM | 421 | C | GLY A | 57 | -7.662 | 9.430 | -9.199 | 1.00 | 17.74 | A | C |
| ATOM | 422 | O | GLY A | 57 | -8.758 | 9.905 | -9.446 | 1.00 | 17.69 | A | O |
| ATOM | 423 | N | ARG A | 58 | -6.921 | 8.825 | -10.109 | 1.00 | 18.54 | A | N |
| ATOM | 424 | CA | ARG A | 58 | -7.361 | 8.659 | -11.502 | 1.00 | 19.44 | A | C |
| ATOM | 425 | CB | ARG A | 58 | -6.572 | 9.555 | -12.466 | 1.00 | 18.56 | A | C |
| ATOM | 426 | CG | ARG A | 58 | -6.873 | 11.036 | -12.371 | 1.00 | 18.16 | A | C |
| ATOM | 427 | CD | ARG A | 58 | -5.685 | 11.912 | -12.787 | 1.00 | 17.99 | A | C |
| ATOM | 428 | NE | ARG A | 58 | -4.505 | 11.593 | -11.990 | 1.00 | 17.16 | A | N |
| ATOM | 429 | CZ | ARG A | 58 | -3.248 | 11.716 | -12.392 | 1.00 | 18.78 | A | C |
| ATOM | 430 | NH1 | ARG A | 58 | -2.967 | 12.194 | -13.591 | 1.00 | 18.96 | A | N |
| ATOM | 431 | NH2 | ARG A | 58 | -2.253 | 11.339 | -11.584 | 1.00 | 17.60 | A | N |
| ATOM | 432 | C | ARG A | 58 | -7.123 | 7.240 | -11.909 | 1.00 | 19.97 | A | C |
| ATOM | 433 | O | ARG A | 58 | -6.007 | 6.754 | -11.878 | 1.00 | 20.06 | A | O |
| ATOM | 434 | N | THR A | 59 | -8.183 | 6.575 | -12.324 | 1.00 | 22.06 | A | N |
| ATOM | 435 | CA | THR A | 59 | -8.091 | 5.180 | -12.688 | 1.00 | 22.88 | A | C |
| ATOM | 436 | CB | THR A | 59 | -9.479 | 4.693 | -13.142 | 1.00 | 24.04 | A | C |
| ATOM | 437 | OG1 | THR A | 59 | -10.330 | 4.643 | -11.984 | 1.00 | 25.24 | A | O |
| ATOM | 438 | CG2 | THR A | 59 | -9.406 | 3.250 | -13.657 | 1.00 | 25.24 | A | C |
| ATOM | 439 | C | THR A | 59 | -7.009 | 4.919 | -13.733 | 1.00 | 22.06 | A | C |
| ATOM | 440 | O | THR A | 59 | -7.020 | 5.482 | -14.835 | 1.00 | 22.93 | A | O |
| ATOM | 441 | N | ASN A | 60 | -6.074 | 4.068 | -13.332 | 1.00 | 21.27 | A | N |
| ATOM | 442 | CA | ASN A | 60 | -4.939 | 3.618 | -14.124 | 1.00 | 21.57 | A | C |
| ATOM | 443 | CB | ASN A | 60 | -5.400 | 2.788 | -15.326 | 1.00 | 22.51 | A | C |
| ATOM | 444 | CG | ASN A | 60 | -5.861 | 1.401 | -14.927 | 1.00 | 24.76 | A | C |
| ATOM | 445 | OD1 | ASN A | 60 | -5.546 | 0.908 | -13.835 | 1.00 | 27.82 | A | O |
| ATOM | 446 | ND2 | ASN A | 60 | -6.624 | 0.773 | -15.801 | 1.00 | 25.97 | A | N |
| ATOM | 447 | C | ASN A | 60 | -4.038 | 4.744 | -14.614 | 1.00 | 20.35 | A | C |
| ATOM | 448 | O | ASN A | 60 | -3.369 | 4.589 | -15.629 | 1.00 | 20.71 | A | O |
| ATOM | 449 | N | ASN A | 61 | -4.023 | 5.852 | -13.897 | 1.00 | 18.43 | A | N |
| ATOM | 450 | CA | ASN A | 61 | -3.217 | 6.996 | -14.300 | 1.00 | 18.53 | A | C |
| ATOM | 451 | CB | ASN A | 61 | -4.095 | 8.062 | -14.972 | 1.00 | 17.54 | A | C |
| ATOM | 452 | CG | ASN A | 61 | -3.278 | 9.194 | -15.580 | 1.00 | 19.62 | A | C |
| ATOM | 453 | OD1 | ASN A | 61 | -3.832 | 10.171 | -16.141 | 1.00 | 22.44 | A | O |
| ATOM | 454 | ND2 | ASN A | 61 | -1.968 | 9.081 | -15.481 | 1.00 | 15.52 | A | N |
| ATOM | 455 | C | ASN A | 61 | -2.520 | 7.586 | -13.088 | 1.00 | 16.75 | A | C |
| ATOM | 456 | O | ASN A | 61 | -3.159 | 8.213 | -12.260 | 1.00 | 16.00 | A | O |
| ATOM | 457 | N | ALA A | 62 | -1.219 | 7.357 | -12.988 | 1.00 | 16.29 | A | N |
| ATOM | 458 | CA | ALA A | 62 | -0.418 | 7.910 | -11.902 | 1.00 | 16.34 | A | C |
| ATOM | 459 | CB | ALA A | 62 | 0.310 | 6.804 | -11.183 | 1.00 | 16.55 | A | C |
| ATOM | 460 | C | ALA A | 62 | 0.584 | 8.948 | -12.405 | 1.00 | 16.52 | A | C |
| ATOM | 461 | O | ALA A | 62 | 1.583 | 9.221 | -11.728 | 1.00 | 15.61 | A | O |
| ATOM | 462 | N | ASN A | 63 | 0.344 | 9.515 | -13.593 | 1.00 | 15.91 | A | N |
| ATOM | 463 | CA | ASN A | 63 | 1.276 | 10.465 | -14.157 | 1.00 | 15.75 | A | C |
| ATOM | 464 | CB | ASN A | 63 | 1.251 | 10.471 | -15.720 | 1.00 | 15.36 | A | C |
| ATOM | 465 | CG | ASN A | 63 | 0.043 | 11.165 | -16.307 | 1.00 | 16.00 | A | C |
| ATOM | 466 | OD1 | ASN A | 63 | -0.617 | 11.982 | -15.643 | 1.00 | 14.50 | A | O |
| ATOM | 467 | ND2 | ASN A | 63 | -0.274 | 10.833 | -17.584 | 1.00 | 15.36 | A | N |
| ATOM | 468 | C | ASN A | 63 | 1.115 | 11.858 | -13.518 | 1.00 | 15.21 | A | C |
| ATOM | 469 | O | ASN A | 63 | 0.168 | 12.108 | -12.762 | 1.00 | 15.63 | A | O |
| ATOM | 470 | N | ASP A | 64 | 2.047 | 12.753 | -13.828 | 1.00 | 15.12 | A | N |
| ATOM | 471 | CA | ASP A | 64 | 2.192 | 14.015 | -13.102 | 1.00 | 15.29 | A | C |
| ATOM | 472 | CB | ASP A | 64 | 3.450 | 13.990 | -12.233 | 1.00 | 14.59 | A | C |
| ATOM | 473 | CG | ASP A | 64 | 3.532 | 15.161 | -11.300 | 1.00 | 15.31 | A | C |
| ATOM | 474 | OD1 | ASP A | 64 | 2.476 | 15.813 | -11.058 | 1.00 | 14.15 | A | O |
| ATOM | 475 | OD2 | ASP A | 64 | 4.626 | 15.516 | -10.776 | 1.00 | 14.37 | A | O |
| ATOM | 476 | C | ASP A | 64 | 2.236 | 15.206 | -14.061 | 1.00 | 15.53 | A | C |
| ATOM | 477 | O | ASP A | 64 | 3.315 | 15.713 | -14.423 | 1.00 | 16.54 | A | O |
| ATOM | 478 | N | PRO A | 65 | 1.065 | 15.644 | -14.476 | 1.00 | 16.18 | A | N |
| ATOM | 479 | CA | PRO A | 65 | 0.950 | 16.813 | -15.343 | 1.00 | 17.33 | A | C |
| ATOM | 480 | CB | PRO A | 65 | -0.509 | 16.776 | -15.807 | 1.00 | 17.19 | A | C |
| ATOM | 481 | CG | PRO A | 65 | -1.225 | 15.953 | -14.808 | 1.00 | 17.73 | A | C |
| ATOM | 482 | CD | PRO A | 65 | -0.249 | 15.043 | -14.172 | 1.00 | 17.21 | A | C |

| ATOM | 483 | C | PRO A | 65 | 1.228 | 18.102 | -14.607 | 1.00 | 17.72 | A | C |
|------|-----|----|-------|-----|--------|--------|---------|------|-------|---|---|
| ATOM | 484 | O | PRO A | 65 | 1.515 | 19.081 | -15.250 | 1.00 | 17.98 | A | O |
| ATOM | 485 | N | ASN A | 66 | 1.150 | 18.065 | -13.279 | 1.00 | 18.27 | A | N |
| ATOM | 486 | CA | ASN A | 66 | 1.314 | 19.217 | -12.426 | 1.00 | 19.24 | A | C |
| ATOM | 487 | CB | ASN A | 66 | 0.536 | 18.958 | -11.111 | 1.00 | 20.48 | A | C |
| ATOM | 488 | CG | ASN A | 66 | 0.790 | 19.993 | -10.068 | 1.00 | 22.89 | A | C |
| ATOM | 489 | OD1 | ASN A | 66 | 1.942 | 20.281 | -9.721 | 1.00 | 23.54 | A | O |
| ATOM | 490 | ND2 | ASN A | 66 | -0.287 | 20.591 | -9.566 | 1.00 | 25.20 | A | N |
| ATOM | 491 | C | ASN A | 66 | 2.806 | 19.457 | -12.153 | 1.00 | 18.71 | A | C |
| ATOM | 492 | O | ASN A | 66 | 3.314 | 20.549 | -12.353 | 1.00 | 18.84 | A | O |
| ATOM | 493 | N | GLY A | 67 | 3.500 | 18.426 | -11.698 | 1.00 | 17.55 | A | N |
| ATOM | 494 | CA | GLY A | 67 | 4.917 | 18.503 | -11.406 | 1.00 | 16.38 | A | C |
| ATOM | 495 | C | GLY A | 67 | 5.234 | 18.455 | -9.916 | 1.00 | 15.32 | A | C |
| ATOM | 496 | O | GLY A | 67 | 6.383 | 18.167 | -9.542 | 1.00 | 15.11 | A | O |
| ATOM | 497 | N | HIS A | 68 | 4.230 | 18.722 | -9.075 | 1.00 | 13.44 | A | N |
| ATOM | 498 | CA | HIS A | 68 | 4.406 | 18.776 | -7.608 | 1.00 | 12.51 | A | C |
| ATOM | 499 | CB | BHIS A | 68 | 3.109 | 19.121 | -6.891 | 0.50 | 12.22 | A | C |
| ATOM | 500 | CB | AHIS A | 68 | 3.048 | 19.078 | -6.930 | 0.50 | 12.48 | A | C |
| ATOM | 501 | CG | BHIS A | 68 | 3.266 | 19.371 | -5.417 | 0.50 | 10.61 | A | C |
| ATOM | 502 | CG | AHIS A | 68 | 3.140 | 19.398 | -5.464 | 0.50 | 10.86 | A | C |
| ATOM | 503 | ND1 | BHIS A | 68 | 2.741 | 18.522 | -4.453 | 0.50 | 5.34 | A | N |
| ATOM | 504 | ND1 | AHIS A | 68 | 3.742 | 18.559 | -4.548 | 0.50 | 7.56 | A | N |
| ATOM | 505 | CE1 | BHIS A | 68 | 3.009 | 19.016 | -3.254 | 0.50 | 6.59 | A | C |
| ATOM | 506 | CE1 | AHIS A | 68 | 3.674 | 19.102 | -3.341 | 0.50 | 2.00 | A | C |
| ATOM | 507 | NE2 | BHIS A | 68 | 3.678 | 20.158 | -3.403 | 0.50 | 7.98 | A | N |
| ATOM | 508 | NE2 | AHIS A | 68 | 3.061 | 20.277 | -3.442 | 0.50 | 6.21 | A | N |
| ATOM | 509 | CD2 | BHIS A | 68 | 3.845 | 20.405 | -4.745 | 0.50 | 5.10 | A | C |
| ATOM | 510 | CD2 | AHIS A | 68 | 2.697 | 20.471 | -4.756 | 0.50 | 8.79 | A | C |
| ATOM | 511 | C | HIS A | 68 | 4.986 | 17.474 | -7.064 | 1.00 | 12.70 | A | C |
| ATOM | 512 | O | HIS A | 68 | 6.025 | 17.471 | -6.401 | 1.00 | 12.91 | A | O |
| ATOM | 513 | N | GLY A | 69 | 4.315 | 16.374 | -7.317 | 1.00 | 13.22 | A | N |
| ATOM | 514 | CA | GLY A | 69 | 4.709 | 15.094 | -6.739 | 1.00 | 13.52 | A | C |
| ATOM | 515 | C | GLY A | 69 | 6.039 | 14.574 | -7.181 | 1.00 | 13.01 | A | C |
| ATOM | 516 | O | GLY A | 69 | 6.751 | 13.894 | -6.418 | 1.00 | 13.80 | A | O |
| ATOM | 517 | N | THR A | 70 | 6.391 | 14.865 | -8.432 | 1.00 | 13.25 | A | N |
| ATOM | 518 | CA | THR A | 70 | 7.651 | 14.425 | -8.970 | 1.00 | 12.89 | A | C |
| ATOM | 519 | CB | THR A | 70 | 7.688 | 14.638 | -10.507 | 1.00 | 13.93 | A | C |
| ATOM | 520 | OG1 | THR A | 70 | 6.592 | 13.940 | -11.116 | 1.00 | 14.34 | A | O |
| ATOM | 521 | CG2 | THR A | 70 | 8.895 | 13.977 | -11.110 | 1.00 | 13.50 | A | C |
| ATOM | 522 | C | THR A | 70 | 8.769 | 15.192 | -8.309 | 1.00 | 12.86 | A | C |
| ATOM | 523 | O | THR A | 70 | 9.816 | 14.622 | -8.013 | 1.00 | 13.63 | A | O |
| ATOM | 524 | N | HIS A | 71 | 8.560 | 16.498 | -8.093 | 1.00 | 12.19 | A | N |
| ATOM | 525 | CA | HIS A | 71 | 9.580 | 17.341 | -7.486 | 1.00 | 11.80 | A | C |
| ATOM | 526 | CB | HIS A | 71 | 9.125 | 18.796 | -7.555 | 1.00 | 11.41 | A | C |
| ATOM | 527 | CG | BHIS A | 71 | 10.185 | 19.784 | -7.212 | 0.50 | 11.89 | A | C |
| ATOM | 528 | CG | AHIS A | 71 | 10.189 | 19.775 | -7.181 | 0.50 | 9.73 | A | C |
| ATOM | 529 | ND1 | BHIS A | 71 | 10.926 | 19.709 | -6.050 | 0.50 | 12.60 | A | N |
| ATOM | 530 | ND1 | AHIS A | 71 | 10.236 | 20.388 | -5.942 | 0.50 | 5.89 | A | N |
| ATOM | 531 | CE1 | BHIS A | 71 | 11.791 | 20.706 | -6.025 | 0.50 | 13.16 | A | C |
| ATOM | 532 | CE1 | AHIS A | 71 | 11.281 | 21.192 | -5.898 | 0.50 | 7.95 | A | C |
| ATOM | 533 | NE2 | BHIS A | 71 | 11.618 | 21.438 | -7.114 | 0.50 | 14.18 | A | N |
| ATOM | 534 | NE2 | AHIS A | 71 | 11.923 | 21.107 | -7.054 | 0.50 | 10.14 | A | N |
| ATOM | 535 | CD2 | BHIS A | 71 | 10.617 | 20.883 | -7.869 | 0.50 | 10.25 | A | C |
| ATOM | 536 | CD2 | AHIS A | 71 | 11.258 | 20.231 | -7.874 | 0.50 | 6.22 | A | C |
| ATOM | 537 | C | HIS A | 71 | 9.806 | 16.875 | -6.018 | 1.00 | 12.35 | A | C |
| ATOM | 538 | O | HIS A | 71 | 10.935 | 16.698 | -5.538 | 1.00 | 12.45 | A | O |
| ATOM | 539 | N | VAL A | 72 | 8.697 | 16.657 | -5.331 | 1.00 | 12.33 | A | N |
| ATOM | 540 | CA | VAL A | 72 | 8.704 | 16.204 | -3.960 | 1.00 | 12.62 | A | C |
| ATOM | 541 | CB | VAL A | 72 | 7.279 | 16.056 | -3.469 | 1.00 | 12.75 | A | C |
| ATOM | 542 | CG1 | VAL A | 72 | 7.248 | 15.256 | -2.202 | 1.00 | 12.50 | A | C |
| ATOM | 543 | CG2 | VAL A | 72 | 6.647 | 17.430 | -3.262 | 1.00 | 12.97 | A | C |
| ATOM | 544 | C | VAL A | 72 | 9.431 | 14.864 | -3.799 | 1.00 | 13.01 | A | C |
| ATOM | 545 | O | VAL A | 72 | 10.333 | 14.707 | -2.947 | 1.00 | 12.02 | A | O |

| ATOM | 546 | N | ALA | A | 73 | 9.054 | 13.888 | -4.615 | 1.00 | 12.23 | A | N |
|------|-----|-----|-----|---|----|--------|--------|--------|------|-------|---|---|
| ATOM | 547 | CA | ALA | A | 73 | 9.664 | 12.572 | -4.521 | 1.00 | 12.29 | A | C |
| ATOM | 548 | CB | ALA | A | 73 | 8.986 | 11.617 | -5.440 | 1.00 | 12.32 | A | C |
| ATOM | 549 | C | ALA | A | 73 | 11.180 | 12.682 | -4.850 | 1.00 | 11.78 | A | C |
| ATOM | 550 | O | ALA | A | 73 | 11.985 | 11.992 | -4.280 | 1.00 | 11.60 | A | O |
| ATOM | 551 | N | GLY | A | 74 | 11.553 | 13.583 | -5.742 | 1.00 | 12.04 | A | N |
| ATOM | 552 | CA | GLY | A | 74 | 12.961 | 13.760 | -6.069 | 1.00 | 11.92 | A | C |
| ATOM | 553 | C | GLY | A | 74 | 13.768 | 14.190 | -4.845 | 1.00 | 12.13 | A | C |
| ATOM | 554 | O | GLY | A | 74 | 14.936 | 13.816 | -4.693 | 1.00 | 11.64 | A | O |
| ATOM | 555 | N | SER | A | 75 | 13.157 | 15.015 | -3.994 | 1.00 | 12.08 | A | N |
| ATOM | 556 | CA | SER | A | 75 | 13.844 | 15.546 | -2.827 | 1.00 | 12.08 | A | C |
| ATOM | 557 | CB | SER | A | 75 | 13.095 | 16.748 | -2.267 | 1.00 | 11.53 | A | C |
| ATOM | 558 | OG | SER | A | 75 | 13.254 | 17.915 | -3.077 | 1.00 | 13.29 | A | O |
| ATOM | 559 | C | SER | A | 75 | 14.033 | 14.477 | -1.739 | 1.00 | 12.05 | A | C |
| ATOM | 560 | O | SER | A | 75 | 14.984 | 14.540 | -0.927 | 1.00 | 12.73 | A | O |
| ATOM | 561 | N | VAL | A | 76 | 13.112 | 13.524 | -1.676 | 1.00 | 11.61 | A | N |
| ATOM | 562 | CA | VAL | A | 76 | 13.272 | 12.407 | -0.748 | 1.00 | 11.87 | A | C |
| ATOM | 563 | CB | VAL | A | 76 | 12.023 | 11.519 | -0.691 | 1.00 | 12.06 | A | C |
| ATOM | 564 | CG1 | VAL | A | 76 | 12.224 | 10.396 | 0.324 | 1.00 | 12.75 | A | C |
| ATOM | 565 | CG2 | VAL | A | 76 | 10.799 | 12.319 | -0.316 | 1.00 | 11.56 | A | C |
| ATOM | 566 | C | VAL | A | 76 | 14.415 | 11.501 | -1.173 | 1.00 | 12.11 | A | C |
| ATOM | 567 | O | VAL | A | 76 | 15.280 | 11.158 | -0.372 | 1.00 | 10.37 | A | O |
| ATOM | 568 | N | LEU | A | 77 | 14.410 | 11.085 | -2.437 | 1.00 | 12.79 | A | N |
| ATOM | 569 | CA | LEU | A | 77 | 15.234 | 9.934 | -2.809 | 1.00 | 12.61 | A | C |
| ATOM | 570 | CB | LEU | A | 77 | 14.532 | 8.627 | -2.425 | 1.00 | 13.05 | A | C |
| ATOM | 571 | CG | LEU | A | 77 | 13.050 | 8.419 | -2.774 | 1.00 | 11.85 | A | C |
| ATOM | 572 | CD1 | LEU | A | 77 | 12.868 | 8.361 | -4.281 | 1.00 | 12.80 | A | C |
| ATOM | 573 | CD2 | LEU | A | 77 | 12.512 | 7.140 | -2.114 | 1.00 | 14.07 | A | C |
| ATOM | 574 | C | LEU | A | 77 | 15.676 | 9.847 | -4.267 | 1.00 | 13.21 | A | C |
| ATOM | 575 | O | LEU | A | 77 | 16.181 | 8.810 | -4.656 | 1.00 | 13.59 | A | O |
| ATOM | 576 | N | GLY | A | 78 | 15.586 | 10.935 | -5.022 | 1.00 | 13.72 | A | N |
| ATOM | 577 | CA | GLY | A | 78 | 16.045 | 10.945 | -6.415 | 1.00 | 14.19 | A | C |
| ATOM | 578 | C | GLY | A | 78 | 17.486 | 10.505 | -6.506 | 1.00 | 14.88 | A | C |
| ATOM | 579 | O | GLY | A | 78 | 18.322 | 10.998 | -5.718 | 1.00 | 14.70 | A | O |
| ATOM | 580 | N | ASN | A | 79 | 17.800 | 9.587 | -7.420 | 1.00 | 15.24 | A | N |
| ATOM | 581 | CA | ASN | A | 79 | 19.172 | 9.066 | -7.520 | 1.00 | 16.71 | A | C |
| ATOM | 582 | CB | ASN | A | 79 | 19.204 | 7.542 | -7.263 | 1.00 | 16.08 | A | C |
| ATOM | 583 | CG | ASN | A | 79 | 20.615 | 7.023 | -6.904 | 1.00 | 16.70 | A | C |
| ATOM | 584 | OD1 | ASN | A | 79 | 21.438 | 7.754 | -6.372 | 1.00 | 15.21 | A | O |
| ATOM | 585 | ND2 | ASN | A | 79 | 20.881 | 5.749 | -7.181 | 1.00 | 15.92 | A | N |
| ATOM | 586 | C | ASN | A | 79 | 19.877 | 9.353 | -8.852 | 1.00 | 18.12 | A | C |
| ATOM | 587 | O | ASN | A | 79 | 20.735 | 8.576 | -9.267 | 1.00 | 18.96 | A | O |
| ATOM | 588 | N | ALA | A | 80 | 19.559 | 10.458 | -9.513 | 1.00 | 18.68 | A | N |
| ATOM | 589 | CA | ALA | A | 80 | 20.316 | 10.838 | -10.723 | 1.00 | 19.08 | A | C |
| ATOM | 590 | CB | ALA | A | 80 | 19.381 | 11.169 | -11.876 | 1.00 | 19.15 | A | C |
| ATOM | 591 | C | ALA | A | 80 | 21.261 | 11.995 | -10.376 | 1.00 | 18.85 | A | C |
| ATOM | 592 | O | ALA | A | 80 | 22.245 | 11.795 | -9.663 | 1.00 | 18.76 | A | O |
| ATOM | 593 | N | THR | A | 81 | 20.973 | 13.194 | -10.841 | 1.00 | 18.39 | A | N |
| ATOM | 594 | CA | THR | A | 81 | 21.647 | 14.370 | -10.305 | 1.00 | 18.18 | A | C |
| ATOM | 595 | CB | THR | A | 81 | 22.229 | 15.222 | -11.444 | 1.00 | 18.72 | A | C |
| ATOM | 596 | OG1 | THR | A | 81 | 21.202 | 15.535 | -12.379 | 1.00 | 17.48 | A | O |
| ATOM | 597 | CG2 | THR | A | 81 | 23.229 | 14.420 | -12.289 | 1.00 | 21.52 | A | C |
| ATOM | 598 | C | THR | A | 81 | 20.650 | 15.185 | -9.470 | 1.00 | 17.64 | A | C |
| ATOM | 599 | O | THR | A | 81 | 19.466 | 14.858 | -9.423 | 1.00 | 17.77 | A | O |
| ATOM | 600 | N | ASN | A | 82 | 21.115 | 16.238 | -8.803 | 1.00 | 16.86 | A | N |
| ATOM | 601 | CA | ASN | A | 82 | 20.271 | 16.947 | -7.842 | 1.00 | 16.82 | A | C |
| ATOM | 602 | CB | ASN | A | 82 | 19.279 | 17.840 | -8.574 | 1.00 | 16.90 | A | C |
| ATOM | 603 | CG | ASN | A | 82 | 19.962 | 18.782 | -9.552 | 1.00 | 18.05 | A | C |
| ATOM | 604 | OD1 | ASN | A | 82 | 19.861 | 18.632 | -10.804 | 1.00 | 20.14 | A | O |
| ATOM | 605 | ND2 | ASN | A | 82 | 20.650 | 19.760 | -9.005 | 1.00 | 12.31 | A | N |
| ATOM | 606 | C | ASN | A | 82 | 19.541 | 15.941 | -6.930 | 1.00 | 16.16 | A | C |
| ATOM | 607 | O | ASN | A | 82 | 18.325 | 15.985 | -6.772 | 1.00 | 16.88 | A | O |
| ATOM | 608 | N | LYS | A | 83 | 20.310 | 15.022 | -6.366 | 1.00 | 15.41 | A | N |

| | | | | | | | | | | | | |
|---|---|---|---|---|---|---|---|---|---|---|---|---|
| ATOM | 609 | CA | LYS | A | 83 | 19.767 | 13.853 | -5.710 | 1.00 | 15.60 | A | C |
| ATOM | 610 | CB | LYS | A | 83 | 20.907 | 12.919 | -5.287 | 1.00 | 15.95 | A | C |
| ATOM | 611 | CG | LYS | A | 83 | 21.665 | 12.168 | -6.415 | 1.00 | 16.19 | A | C |
| ATOM | 612 | CD | LYS | A | 83 | 22.815 | 11.339 | -5.811 | 1.00 | 19.24 | A | C |
| ATOM | 613 | CE | LYS | A | 83 | 23.806 | 10.791 | -6.833 | 1.00 | 21.12 | A | C |
| ATOM | 614 | NZ | LYS | A | 83 | 23.076 | 9.941 | -7.791 | 1.00 | 20.88 | A | N |
| ATOM | 615 | C | LYS | A | 83 | 18.966 | 14.243 | -4.453 | 1.00 | 14.74 | A | C |
| ATOM | 616 | O | LYS | A | 83 | 19.243 | 15.248 | -3.801 | 1.00 | 13.71 | A | O |
| ATOM | 617 | N | GLY | A | 84 | 18.000 | 13.402 | -4.117 | 1.00 | 14.66 | A | N |
| ATOM | 618 | CA | GLY | A | 84 | 17.337 | 13.439 | -2.833 | 1.00 | 14.40 | A | C |
| ATOM | 619 | C | GLY | A | 84· | 18.240 | 13.078 | -1.664 | 1.00 | 14.38 | A | C |
| ATOM | 620 | O | GLY | A | 84 | 19.372 | 12.683 | -1.853 | 1.00 | 14.68 | A | O |
| ATOM | 621 | N | MET | A | 85 | 17.734 | 13.231 | -0.439 | 1.00 | 13.41 | A | N |
| ATOM | 622 | CA | MET | A | 85 | 18.586 | 13.079 | 0.753 | 1.00 | 13.39 | A | C |
| ATOM | 623 | CB | MET | A | 85 | 17.865 | 13.660 | 1.970 | 1.00 | 13.62 | A | C |
| ATOM | 624 | CG | MET | A | 85 | 17.446 | 15.132 | 1.799 | 1.00 | 14.10 | A | C |
| ATOM | 625 | SD | MET | A | 85 | 18.823 | 16.235 | 1.480 | 1.00 | 15.77 | A | S |
| ATOM | 626 | CE | MET | A | 85 | 18.801 | 16.373 | -0.341 | 1.00 | 16.54 | A | C |
| ATOM | 627 | C | MET | A | 85 | 18.946 | 11.600 | 1.022 | 1.00 | 13.55 | A | C |
| ATOM | 628 | O | MET | A | 85 | 19.975 | 11.302 | 1.623 | 1.00 | 13.91 | A | O |
| ATOM | 629 | N | ALA | A | 86 | 18.078 | 10.685 | 0.586 | 1.00 | 13.51 | A | N |
| ATOM | 630 | CA | ALA | A | 86 | 18.290 | 9.250 | 0.774 | 1.00 | 13.69 | A | C |
| ATOM | 631 | CB | ALA | A | 86 | 17.223 | 8.682 | 1.717 | 1.00 | 13.63 | A | C |
| ATOM | 632 | C | ALA | A | 86 | 18.200 | 8.571 | -0.589 | 1.00 | 13.59 | A | C |
| ATOM | 633 | O | ALA | A | 86 | 17.258 | 7.821 | -0.868 | 1.00 | 14.50 | A | O |
| ATOM | 634 | N | PRO | A | 87 | 19.175 | 8.818 | -1.445 | 1.00 | 14.33 | A | N |
| ATOM | 635 | CA | PRO | A | 87 | 19.068 | 8.409 | -2.859 | 1.00 | 14.18 | A | C |
| ATOM | 636 | CB | PRO | A | 87 | 20.236 | 9.152 | -3.515 | 1.00 | 14.93 | A | C |
| ATOM | 637 | CG | PRO | A | 87 | 21.263 | 9.267 | -2.393 | 1.00 | 14.56 | A | C |
| ATOM | 638 | CD | PRO | A | 87 | 20.446 | 9.524 | -1.148 | 1.00 | 13.97 | A | C |
| ATOM | 639 | C | PRO | A | 87 | 19.146 | 6.901 | -3.123 | 1.00 | 14.68 | A | C |
| ATOM | 640 | O | PRO | A | 87 | 18.943 | 6.474 | -4.260 | 1.00 | 15.65 | A | O |
| ATOM | 641 | N | GLN | A | 88 | 19.424 | 6.099 | -2.109 | 1.00 | 15.11 | A | N |
| ATOM | 642 | CA | GLN | A | 88 | 19.436 | 4.639 | -2.266 | 1.00 | 16.35 | A | C |
| ATOM | 643 | CB | GLN | A | 88 | 20.748 | 4.050 | -1.733 | 1.00 | 16.73 | A | C |
| ATOM | 644 | CG | GLN | A | 88 | 21.900 | 4.262 | -2.703 | 1.00 | 19.50 | A | C |
| ATOM | 645 | CD | GLN | A | 88 | 23.267 | 3.916 | -2.161 | 1.00 | 21.39 | A | C |
| ATOM | 646 | OE1 | GLN | A | 88 | 23.427 | 2.933 | -1.439 | 1.00 | 22.79 | A | O |
| ATOM | 647 | NE2 | GLN | A | 88 | 24.272 | 4.709 | -2.547 | 1.00 | 22.41 | A | N |
| ATOM | 648 | C | GLN | A | 88 | 18.228 | 3.976 | -1.621 | 1.00 | 16.49 | A | C |
| ATOM | 649 | O | GLN | A | 88 | 18.080 | 2.754 | -1.644 | 1.00 | 16.50 | A | O |
| ATOM | 650 | N | ALA | A | 89 | 17.347 | 4.786 | -1.044 | 1.00 | 16.72 | A | N |
| ATOM | 651 | CA | ALA | A | 89 | 16.056 | 4.279 | -0.599 | 1.00 | 16.78 | A | C |
| ATOM | 652 | CB | ALA | A | 89 | 15.380 | 5.277 | 0.375 | 1.00 | 17.28 | A | C |
| ATOM | 653 | C | ALA | A | 89 | 15.139 | 3.996 | -1.792 | 1.00 | 16.32 | A | C |
| ATOM | 654 | O | ALA | A | 89 | 15.212 | 4.648 | -2.826 | 1.00 | 16.81 | A | O |
| ATOM | 655 | N | ASN | A | 90 | 14.248 | 3.037 | -1.634 | 1.00 | 15.45 | A | N |
| ATOM | 656 | CA | ASN | A | 90 | 13.264 | 2.756 | -2.658 | 1.00 | 15.40 | A | C |
| ATOM | 657 | CB | ASN | A | 90 | 13.036 | 1.247 | -2.756 | 1.00 | 16.05 | A | C |
| ATOM | 658 | CG | ASN | A | 90 | 14.076 | 0.549 | -3.658 | 1.00 | 19.92 | A | C |
| ATOM | 659 | OD1 | ASN | A | 90 | 15.039 | 1.155 | -4.106 | 1.00 | 25.00 | A | O |
| ATOM | 660 | ND2 | ASN | A | 90 | 13.892 | -0.736 | -3.873 | 1.00 | 28.28 | A | N |
| ATOM | 661 | C | ASN | A | 90 | 11.942 | 3.486 | -2.367 | 1.00 | 14.01 | A | C |
| ATOM | 662 | O | ASN | A | 90 | 11.668 | 3.834 | -1.234 | 1.00 | 12.81 | A | O |
| ATOM | 663 | N | LEU | A | 91 | 11.150 | 3.705 | -3.410 | 1.00 | 13.02 | A | N |
| ATOM | 664 | CA | LEU | A | 91 | 9.964 | 4.542 | -3.381 | 1.00 | 13.78 | A | C |
| ATOM | 665 | CB | LEU | A | 91 | 10.022 | 5.524 | -4.540 | 1.00 | 13.95 | A | C |
| ATOM | 666 | CG | LEU | A | 91 | 8.861 | 6.472 | -4.765 | 1.00 | 12.85 | A | C |
| ATOM | 667 | CD1 | LEU | A | 91 | 8.669 | 7.375 | -3.571 | 1.00 | 15.80 | A | C |
| ATOM | 668 | CD2 | LEU | A | 91 | 9.077 | 7.287 | -6.026 | 1.00 | 13.13 | A | C |
| ATOM | 669 | C | LEU | A | 91 | 8.661 | 3.762 | -3.524 | 1.00 | 14.15 | A | C |
| ATOM | 670 | O | LEU | A | 91 | 8.503 | 2.953 | -4.437 | 1.00 | 15.63 | A | O |
| ATOM | 671 | N | VAL | A | 92 | 7.716 | 4.055 | -2.649 | 1.00 | 13.69 | A | N |

42

| ATOM | 672 | CA | VAL | A | 92 | 6.327 | 3.692 | -2.872 | 1.00 | 14.00 | A | C |
|------|-----|-----|-----|---|-----|--------|--------|---------|------|-------|---|---|
| ATOM | 673 | CB | VAL | A | 92 | 5.737 | 3.031 | -1.662 | 1.00 | 13.15 | A | C |
| ATOM | 674 | CG1 | VAL | A | 92 | 4.197 | 3.018 | -1.767 | 1.00 | 15.32 | A | C |
| ATOM | 675 | CG2 | VAL | A | 92 | 6.260 | 1.621 | -1.546 | 1.00 | 13.02 | A | C |
| ATOM | 676 | C | VAL | A | 92 | 5.615 | 5.001 | -3.175 | 1.00 | 13.45 | A | C |
| ATOM | 677 | O | VAL | A | 92 | 5.687 | 5.942 | -2.376 | 1.00 | 13.53 | A | O |
| ATOM | 678 | N | PHE | A | 93 | 4.984 | 5.107 | -4.346 | 1.00 | 13.19 | A | N |
| ATOM | 679 | CA | PHE | A | 93 | 4.293 | 6.351 | -4.714 | 1.00 | 13.05 | A | C |
| ATOM | 680 | CB | PHE | A | 93 | 4.899 | 6.964 | -5.985 | 1.00 | 13.57 | A | C |
| ATOM | 681 | CG | PHE | A | 93 | 4.484 | 8.388 | -6.206 | 1.00 | 13.50 | A | C |
| ATOM | 682 | CD1 | PHE | A | 93 | 5.331 | 9.439 | -5.861 | 1.00 | 13.00 | A | C |
| ATOM | 683 | CE1 | PHE | A | 93 | 4.941 | 10.748 | -6.023 | 1.00 | 12.71 | A | C |
| ATOM | 684 | CZ | PHE | A | 93 | 3.680 | 11.030 | -6.515 | 1.00 | 11.77 | A | C |
| ATOM | 685 | CE2 | PHE | A | 93 | 2.832 | 9.998 | -6.881 | 1.00 | 12.22 | A | C |
| ATOM | 686 | CD2 | PHE | A | 93 | 3.226 | 8.679 | -6.710 | 1.00 | 12.44 | A | C |
| ATOM | 687 | C | PHE | A | 93 | 2.793 | 6.150 | -4.872 | 1.00 | 13.51 | A | C |
| ATOM | 688 | O | PHE | A | 93 | 2.350 | 5.285 | -5.632 | 1.00 | 13.61 | A | O |
| ATOM | 689 | N | GLN | A | 94 | 2.021 | 6.949 | -4.150 | 1.00 | 13.35 | A | N |
| ATOM | 690 | CA | GLN | A | 94 | 0.567 | 6.903 | -4.197 | 1.00 | 13.55 | A | C |
| ATOM | 691 | CB | GLN | A | 94 | -0.034 | 6.775 | -2.786 | 1.00 | 12.95 | A | C |
| ATOM | 692 | CG | GLN | A | 94 | 0.383 | 5.493 | -2.078 | 1.00 | 13.25 | A | C |
| ATOM | 693 | CD | GLN | A | 94 | 0.065 | 5.494 | -0.589 | 1.00 | 14.07 | A | C |
| ATOM | 694 | OE1 | GLN | A | 94 | 0.598 | 6.311 | 0.157 | 1.00 | 16.83 | A | O |
| ATOM | 695 | NE2 | GLN | A | 94 | -0.813 | 4.578 | -0.158 | 1.00 | 13.83 | A | N |
| ATOM | 696 | C | GLN | A | 94 | 0.118 | 8.195 | -4.841 | 1.00 | 13.81 | A | C |
| ATOM | 697 | O | GLN | A | 94 | 0.197 | 9.289 | -4.236 | 1.00 | 12.11 | A | O |
| ATOM | 698 | N | SER | A | 95 | -0.266 | 8.072 | -6.110 | 1.00 | 14.33 | A | N |
| ATOM | 699 | CA | SER | A | 95 | -0.793 | 9.190 | -6.893 | 1.00 | 14.08 | A | C |
| ATOM | 700 | CB | SER | A | 95 | -0.743 | 8.850 | -8.380 | 1.00 | 13.95 | A | C |
| ATOM | 701 | OG | SER | A | 95 | -1.337 | 9.864 | -9.152 | 1.00 | 11.92 | A | O |
| ATOM | 702 | C | SER | A | 95 | -2.221 | 9.519 | -6.494 | 1.00 | 14.82 | A | C |
| ATOM | 703 | O | SER | A | 95 | -3.150 | 8.743 | -6.780 | 1.00 | 14.73 | A | O |
| ATOM | 704 | N | ILE | A | 96 | -2.404 | 10.681 | -5.852 | 1.00 | 15.21 | A | N |
| ATOM | 705 | CA | ILE | A | 96 | -3.699 | 11.049 | -5.277 | 1.00 | 15.55 | A | C |
| ATOM | 706 | CB | ILE | A | 96 | -3.560 | 11.482 | -3.782 | 1.00 | 15.92 | A | C |
| ATOM | 707 | CG1 | ILE | A | 96 | -2.466 | 12.548 | -3.597 | 1.00 | 16.08 | A | C |
| ATOM | 708 | CD1 | ILE | A | 96 | -2.367 | 13.122 | -2.196 | 1.00 | 17.02 | A | C |
| ATOM | 709 | CG2 | ILE | A | 96 | -3.257 | 10.273 | -2.915 | 1.00 | 16.70 | A | C |
| ATOM | 710 | C | ILE | A | 96 | -4.398 | 12.158 | -6.043 | 1.00 | 15.93 | A | C |
| ATOM | 711 | O | ILE | A | 96 | -5.475 | 12.590 | -5.660 | 1.00 | 14.84 | A | O |
| ATOM | 712 | N | MET | A | 97 | -3.797 | 12.640 | -7.119 | 1.00 | 16.32 | A | N |
| ATOM | 713 | CA | MET | A | 97 | -4.440 | 13.700 | -7.889 | 1.00 | 18.17 | A | C |
| ATOM | 714 | CB | MET | A | 97 | -3.471 | 14.346 | -8.884 | 1.00 | 18.20 | A | C |
| ATOM | 715 | CG | MET | A | 97 | -4.107 | 15.480 | -9.688 | 1.00 | 21.58 | A | C |
| ATOM | 716 | SD | MET | A | 97 | -2.949 | 16.297 | -10.814 | 1.00 | 25.41 | A | S |
| ATOM | 717 | CE | MET | A | 97 | -3.900 | 16.196 | -12.225 | 1.00 | 31.75 | A | C |
| ATOM | 718 | C | MET | A | 97 | -5.647 | 13.114 | -8.641 | 1.00 | 18.32 | A | C |
| ATOM | 719 | O | MET | A | 97 | -5.537 | 12.054 | -9.249 | 1.00 | 18.26 | A | O |
| ATOM | 720 | N | ASP | A | 98 | -6.780 | 13.807 | -8.568 | 1.00 | 19.35 | A | N |
| ATOM | 721 | CA | ASP | A | 98 | -8.020 | 13.369 | -9.217 | 1.00 | 20.45 | A | C |
| ATOM | 722 | CB | ASP | A | 98 | -9.268 | 13.714 | -8.375 | 1.00 | 20.39 | A | C |
| ATOM | 723 | CG | ASP | A | 98 | -9.367 | 15.170 | -8.021 | 1.00 | 21.41 | A | C |
| ATOM | 724 | OD1 | ASP | A | 98 | -9.234 | 16.024 | -8.928 | 1.00 | 22.52 | A | O |
| ATOM | 725 | OD2 | ASP | A | 98 | -9.599 | 15.575 | -6.847 | 1.00 | 20.32 | A | O |
| ATOM | 726 | C | ASP | A | 98 | -8.093 | 14.004 | -10.592 | 1.00 | 20.49 | A | C |
| ATOM | 727 | O | ASP | A | 98 | -7.168 | 14.690 | -10.996 | 1.00 | 19.61 | A | O |
| ATOM | 728 | N | SER | A | 99 | -9.170 | 13.747 | -11.321 | 1.00 | 21.70 | A | N |
| ATOM | 729 | CA | SER | A | 99 | -9.252 | 14.202 | -12.703 | 1.00 | 23.74 | A | C |
| ATOM | 730 | CB | SER | A | 99 | -10.202 | 13.301 | -13.510 | 1.00 | 24.07 | A | C |
| ATOM | 731 | OG | SER | A | 99 | -11.497 | 13.436 | -12.986 | 1.00 | 25.19 | A | O |
| ATOM | 732 | C | SER | A | 99 | -9.727 | 15.660 | -12.749 | 1.00 | 24.80 | A | C |
| ATOM | 733 | O | SER | A | 99 | -9.696 | 16.286 | -13.812 | 1.00 | 27.46 | A | O |
| ATOM | 734 | N | GLY | A | 100 | -10.152 | 16.203 | -11.611 | 1.00 | 24.38 | A | N |

| ATOM | 735 | CA | GLY A 100 | -10.425 | 17.621 | -11.488 | 1.00 | 25.17 | A | C |
|------|-----|-----|-----------|---------|--------|---------|------|-------|---|---|
| ATOM | 736 | C | GLY A 100 | -9.262 | 18.470 | -10.968 | 1.00 | 25.36 | A | C |
| ATOM | 737 | O | GLY A 100 | -9.475 | 19.606 | -10.557 | 1.00 | 26.29 | A | O |
| ATOM | 738 | N | GLY A 101 | -8.047 | 17.933 | -10.964 | 1.00 | 25.13 | A | N |
| ATOM | 739 | CA | GLY A 101 | -6.873 | 18.715 | -10.573 | 1.00 | 24.90 | A | C |
| ATOM | 740 | C | GLY A 101 | -6.541 | 18.760 | -9.076 | 1.00 | 24.55 | A | C |
| ATOM | 741 | O | GLY A 101 | -5.425 | 19.133 | -8.713 | 1.00 | 26.15 | A | O |
| ATOM | 742 | N | GLY A 102 | -7.490 | 18.406 | -8.221 | 1.00 | 22.32 | A | N |
| ATOM | 743 | CA | GLY A 102 | -7.258 | 18.339 | -6.783 | 1.00 | 21.84 | A | C |
| ATOM | 744 | C | GLY A 102 | -6.703 | 17.008 | -6.267 | 1.00 | 20.73 | A | C |
| ATOM | 745 | O | GLY A 102 | -6.172 | 16.204 | -7.021 | 1.00 | 19.11 | A | O |
| ATOM | 746 | N | LEU A 103 | -6.814 | 16.794 | -4.959 | 1.00 | 19.97 | A | N |
| ATOM | 747 | CA | LEU A 103 | -6.225 | 15.634 | -4.294 | 1.00 | 19.19 | A | C |
| ATOM | 748 | CB | LEU A 103 | -5.346 | 16.094 | -3.131 | 1.00 | 18.87 | A | C |
| ATOM | 749 | CG | LEU A 103 | -4.169 | 16.986 | -3.552 | 1.00 | 18.31 | A | C |
| ATOM | 750 | CD1 | LEU A 103 | -3.298 | 17.397 | -2.354 | 1.00 | 17.54 | A | C |
| ATOM | 751 | CD2 | LEU A 103 | -3.341 | 16.297 | -4.607 | 1.00 | 19.64 | A | C |
| ATOM | 752 | C | LEU A 103 | -7.307 | 14.676 | -3.809 | 1.00 | 19.31 | A | C |
| ATOM | 753 | O | LEU A 103 | -7.179 | 14.018 | -2.750 | 1.00 | 18.44 | A | O |
| ATOM | 754 | N | GLY A 104 | -8.371 | 14.586 | -4.604 | 1.00 | 18.93 | A | N |
| ATOM | 755 | CA | GLY A 104 | -9.537 | 13.780 | -4.260 | 1.00 | 18.78 | A | C |
| ATOM | 756 | C | GLY A 104 | -9.259 | 12.298 | -4.234 | 1.00 | 18.26 | A | C |
| ATOM | 757 | O | GLY A 104 | -10.078 | 11.506 | -3.780 | 1.00 | 19.17 | A | O |
| ATOM | 758 | N | GLY A 105 | -8.094 | 11.886 | -4.703 | 1.00 | 17.54 | A | N |
| ATOM | 759 | CA | GLY A 105 | -7.698 | 10.500 | -4.520 | 1.00 | 17.23 | A | C |
| ATOM | 760 | C | GLY A 105 | -7.395 | 10.091 | -3.075 | 1.00 | 16.85 | A | C |
| ATOM | 761 | O | GLY A 105 | -7.319 | 8.895 | -2.731 | 1.00 | 15.88 | A | O |
| ATOM | 762 | N | LEU A 106 | -7.263 | 11.067 | -2.194 | 1.00 | 16.62 | A | N |
| ATOM | 763 | CA | LEU A 106 | -7.137 | 10.729 | -0.777 | 1.00 | 16.79 | A | C |
| ATOM | 764 | CB | LEU A 106 | -6.892 | 11.975 | 0.048 | 1.00 | 16.05 | A | C |
| ATOM | 765 | CG | LEU A 106 | -5.519 | 12.560 | -0.204 | 1.00 | 14.68 | A | C |
| ATOM | 766 | CD1 | LEU A 106 | -5.479 | 13.986 | 0.274 | 1.00 | 16.20 | A | C |
| ATOM | 767 | CD2 | LEU A 106 | -4.425 | 11.707 | 0.507 | 1.00 | 13.15 | A | C |
| ATOM | 768 | C | LEU A 106 | -8.423 | 10.056 | -0.304 | 1.00 | 17.90 | A | C |
| ATOM | 769 | O | LEU A 106 | -9.513 | 10.553 | -0.587 | 1.00 | 18.63 | A | O |
| ATOM | 770 | N | PRO A 107 | -8.318 | 8.932 | 0.387 | 1.00 | 18.42 | A | N |
| ATOM | 771 | CA | PRO A 107 | -9.506 | 8.280 | 0.977 | 1.00 | 19.35 | A | C |
| ATOM | 772 | CB | PRO A 107 | -8.963 | 6.932 | 1.430 | 1.00 | 19.15 | A | C |
| ATOM | 773 | CG | PRO A 107 | -7.537 | 7.286 | 1.774 | 1.00 | 19.19 | A | C |
| ATOM | 774 | CD | PRO A 107 | -7.089 | 8.162 | 0.640 | 1.00 | 18.63 | A | C |
| ATOM | 775 | C | PRO A 107 | -10.070 | 9.036 | 2.178 | 1.00 | 18.88 | A | C |
| ATOM | 776 | O | PRO A 107 | -9.340 | 9.724 | 2.886 | 1.00 | 19.33 | A | O |
| ATOM | 777 | N | ALA A 108 | -11.367 | 8.910 | 2.408 | 1.00 | 19.46 | A | N |
| ATOM | 778 | CA | ALA A 108 | -12.022 | 9.562 | 3.530 | 1.00 | 19.50 | A | C |
| ATOM | 779 | CB | ALA A 108 | -13.514 | 9.168 | 3.585 | 1.00 | 20.76 | A | C |
| ATOM | 780 | C | ALA A 108 | -11.359 | 9.229 | 4.875 | 1.00 | 18.72 | A | C |
| ATOM | 781 | O | ALA A 108 | -11.229 | 10.093 | 5.727 | 1.00 | 19.17 | A | O |
| ATOM | 782 | N | ASN A 109 | -11.007 | 7.964 | 5.069 | 1.00 | 18.94 | A | N |
| ATOM | 783 | CA | ASN A 109 | -10.193 | 7.535 | 6.209 | 1.00 | 19.10 | A | C |
| ATOM | 784 | CB | ASN A 109 | -10.691 | 6.206 | 6.773 | 1.00 | 19.41 | A | C |
| ATOM | 785 | CG | ASN A 109 | -9.990 | 5.834 | 8.073 | 1.00 | 22.66 | A | C |
| ATOM | 786 | OD1 | ASN A 109 | -8.872 | 6.295 | 8.349 | 1.00 | 19.31 | A | O |
| ATOM | 787 | ND2 | ASN A 109 | -10.665 | 5.018 | 8.908 | 1.00 | 25.73 | A | N |
| ATOM | 788 | C | ASN A 109 | -8.731 | 7.392 | 5.804 | 1.00 | 17.97 | A | C |
| ATOM | 789 | O | ASN A 109 | -8.353 | 6.446 | 5.088 | 1.00 | 17.31 | A | O |
| ATOM | 790 | N | LEU A 110 | -7.895 | 8.325 | 6.245 | 1.00 | 16.66 | A | N |
| ATOM | 791 | CA | LEU A 110 | -6.489 | 8.277 | 5.862 | 1.00 | 15.50 | A | C |
| ATOM | 792 | CB | LEU A 110 | -5.738 | 9.502 | 6.406 | 1.00 | 15.65 | A | C |
| ATOM | 793 | CG | LEU A 110 | -6.096 | 10.831 | 5.749 | 1.00 | 13.74 | A | C |
| ATOM | 794 | CD1 | LEU A 110 | -5.294 | 11.932 | 6.373 | 1.00 | 16.06 | A | C |
| ATOM | 795 | CD2 | LEU A 110 | -5.873 | 10.768 | 4.256 | 1.00 | 13.53 | A | C |
| ATOM | 796 | C | LEU A 110 | -5.784 | 7.006 | 6.285 | 1.00 | 15.96 | A | C |
| ATOM | 797 | O | LEU A 110 | -4.750 | 6.660 | 5.719 | 1.00 | 16.04 | A | O |

| ATOM | 798 | N   | GLN A 111  | -6.297 | 6.283  | 7.276  | 1.00 | 16.18 | A | N |
|------|-----|-----|------------|--------|--------|--------|------|-------|---|---|
| ATOM | 799 | CA  | GLN A 111  | -5.635 | 5.034  | 7.655  | 1.00 | 16.61 | A | C |
| ATOM | 800 | CB  | GLN A 111  | -6.317 | 4.377  | 8.871  | 1.00 | 17.82 | A | C |
| ATOM | 801 | CG  | GLN A 111  | -6.337 | 5.320  | 10.077 | 1.00 | 17.25 | A | C |
| ATOM | 802 | CD  | GLN A 111  | -6.584 | 4.625  | 11.399 | 1.00 | 20.40 | A | C |
| ATOM | 803 | OE1 | GLN A 111  | -5.934 | 3.635  | 11.699 | 1.00 | 21.99 | A | O |
| ATOM | 804 | NE2 | GLN A 111  | -7.513 | 5.163  | 12.202 | 1.00 | 19.23 | A | N |
| ATOM | 805 | C   | GLN A 111  | -5.560 | 4.086  | 6.461  | 1.00 | 17.45 | A | C |
| ATOM | 806 | O   | GLN A 111  | -4.601 | 3.323  | 6.312  | 1.00 | 17.74 | A | O |
| ATOM | 807 | N   | THR A 112  | -6.522 | 4.195  | 5.548  | 1.00 | 16.47 | A | N |
| ATOM | 808 | CA  | THR A 112  | -6.483 | 3.418  | 4.309  | 1.00 | 16.12 | A | C |
| ATOM | 809 | CB  | THR A 112  | -7.756 | 3.733  | 3.510  | 1.00 | 16.21 | A | C |
| ATOM | 810 | OG1 | THR A 112  | -8.900 | 3.480  | 4.333  | 1.00 | 17.07 | A | O |
| ATOM | 811 | CG2 | THR A 112  | -7.909 | 2.838  | 2.305  | 1.00 | 16.70 | A | C |
| ATOM | 812 | C   | THR A 112  | -5.252 | 3.711  | 3.442  | 1.00 | 15.86 | A | C |
| ATOM | 813 | O   | THR A 112  | -4.623 | 2.789  | 2.869  | 1.00 | 15.50 | A | O |
| ATOM | 814 | N   | LEU A 113  | -4.933 | 4.995  | 3.303  | 1.00 | 14.75 | A | N |
| ATOM | 815 | CA  | LEU A 113  | -3.742 | 5.413  | 2.558  | 1.00 | 14.05 | A | C |
| ATOM | 816 | CB  | LEU A 113  | -3.677 | 6.941  | 2.557  | 1.00 | 14.27 | A | C |
| ATOM | 817 | CG  | LEU A 113  | -2.549 | 7.597  | 1.807  | 1.00 | 14.53 | A | C |
| ATOM | 818 | CD1 | LEU A 113  | -2.840 | 7.473  | 0.297  | 1.00 | 16.65 | A | C |
| ATOM | 819 | CD2 | LEU A 113  | -2.412 | 9.039  | 2.212  | 1.00 | 13.95 | A | C |
| ATOM | 820 | C   | LEU A 113  | -2.478 | 4.836  | 3.212  | 1.00 | 13.77 | A | C |
| ATOM | 821 | O   | LEU A 113  | -1.625 | 4.238  | 2.550  | 1.00 | 13.55 | A | O |
| ATOM | 822 | N   | PHE A 114  | -2.361 | 5.016  | 4.523  | 1.00 | 12.95 | A | N |
| ATOM | 823 | CA  | PHE A 114  | -1.182 | 4.528  | 5.223  | 1.00 | 13.04 | A | C |
| ATOM | 824 | CB  | PHE A 114  | -1.154 | 5.049  | 6.645  | 1.00 | 12.56 | A | C |
| ATOM | 825 | CG  | PHE A 114  | -1.331 | 6.551  | 6.743  | 1.00 | 11.79 | A | C |
| ATOM | 826 | CD1 | PHE A 114  | -0.639 | 7.402  | 5.902  | 1.00 | 12.07 | A | C |
| ATOM | 827 | CE1 | PHE A 114  | -0.785 | 8.781  | 5.986  | 1.00 | 12.47 | A | C |
| ATOM | 828 | CZ  | PHE A 114  | -1.662 | 9.323  | 6.921  | 1.00 | 13.57 | A | C |
| ATOM | 829 | CE2 | PHE A 114  | -2.365 | 8.470  | 7.754  | 1.00 | 11.94 | A | C |
| ATOM | 830 | CD2 | PHE A 114  | -2.186 | 7.100  | 7.663  | 1.00 | 9.85  | A | C |
| ATOM | 831 | C   | PHE A 114  | -1.060 | 3.003  | 5.171  | 1.00 | 13.86 | A | C |
| ATOM | 832 | O   | PHE A 114  | 0.063  | 2.461  | 5.004  | 1.00 | 12.73 | A | O |
| ATOM | 833 | N   | SER A 115  | -2.196 | 2.306  | 5.277  | 1.00 | 14.04 | A | N |
| ATOM | 834 | CA  | SER A 115  | -2.148 | 0.848  | 5.292  | 1.00 | 14.17 | A | C |
| ATOM | 835 | CB  | BSER A 115 | -3.527 | 0.252  | 5.640  | 0.50 | 13.81 | A | C |
| ATOM | 836 | CB  | ASER A 115 | -3.457 | 0.215  | 5.769  | 0.50 | 14.55 | A | C |
| ATOM | 837 | OG  | BSER A 115 | -3.970 | 0.566  | 6.958  | 0.50 | 10.51 | A | O |
| ATOM | 838 | OG  | ASER A 115 | -4.544 | 0.608  | 4.978  | 0.50 | 18.03 | A | O |
| ATOM | 839 | C   | SER A 115  | -1.677 | 0.296  | 3.943  | 1.00 | 14.11 | A | C |
| ATOM | 840 | O   | SER A 115  | -0.932 | -0.663 | 3.909  | 1.00 | 13.43 | A | O |
| ATOM | 841 | N   | GLN A 116  | -2.108 | 0.890  | 2.832  | 1.00 | 14.73 | A | N |
| ATOM | 842 | CA  | GLN A 116  | -1.656 | 0.442  | 1.513  | 1.00 | 14.53 | A | C |
| ATOM | 843 | CB  | GLN A 116  | -2.394 | 1.234  | 0.417  | 1.00 | 15.81 | A | C |
| ATOM | 844 | CG  | GLN A 116  | -1.947 | 0.951  | -1.038 | 1.00 | 15.88 | A | C |
| ATOM | 845 | CD  | GLN A 116  | -2.601 | 1.886  | -2.007 | 1.00 | 16.75 | A | C |
| ATOM | 846 | OE1 | GLN A 116  | -2.629 | 3.086  | -1.747 | 1.00 | 14.56 | A | O |
| ATOM | 847 | NE2 | GLN A 116  | -3.200 | 1.346  | -3.106 | 1.00 | 14.07 | A | N |
| ATOM | 848 | C   | GLN A 116  | -0.131 | 0.571  | 1.375  | 1.00 | 14.16 | A | C |
| ATOM | 849 | O   | GLN A 116  | 0.554  | -0.336 | 0.861  | 1.00 | 14.37 | A | O |
| ATOM | 850 | N   | ALA A 117  | 0.407  | 1.679  | 1.862  | 1.00 | 13.34 | A | N |
| ATOM | 851 | CA  | ALA A 117  | 1.838  | 1.930  | 1.795  | 1.00 | 13.79 | A | C |
| ATOM | 852 | CB  | ALA A 117  | 2.152  | 3.408  | 2.151  | 1.00 | 13.52 | A | C |
| ATOM | 853 | C   | ALA A 117  | 2.608  | 0.972  | 2.714  | 1.00 | 13.17 | A | C |
| ATOM | 854 | O   | ALA A 117  | 3.666  | 0.472  | 2.344  | 1.00 | 13.07 | A | O |
| ATOM | 855 | N   | TYR A 118  | 2.071  | 0.740  | 3.908  | 1.00 | 13.32 | A | N |
| ATOM | 856 | CA  | TYR A 118  | 2.679  | -0.161 | 4.877  | 1.00 | 13.82 | A | C |
| ATOM | 857 | CB  | TYR A 118  | 1.878  | -0.177 | 6.190  | 1.00 | 14.02 | A | C |
| ATOM | 858 | CG  | TYR A 118  | 2.636  | -0.861 | 7.324  | 1.00 | 17.04 | A | C |
| ATOM | 859 | CD1 | TYR A 118  | 2.472  | -2.216 | 7.589  | 1.00 | 20.14 | A | C |
| ATOM | 860 | CE1 | TYR A 118  | 3.186  | -2.839 | 8.640  | 1.00 | 24.14 | A | C |

| ATOM | 861 | CZ | TYR | A | 118 | 4.041 | -2.071 | 9.409 | 1.00 | 23.61 | A | C |
| ATOM | 862 | OH | TYR | A | 118 | 4.762 | -2.631 | 10.442 | 1.00 | 28.49 | A | O |
| ATOM | 863 | CE2 | TYR | A | 118 | 4.194 | -0.725 | 9.155 | 1.00 | 20.69 | A | C |
| ATOM | 864 | CD2 | TYR | A | 118 | 3.501 | -0.135 | 8.136 | 1.00 | 18.61 | A | C |
| ATOM | 865 | C | TYR | A | 118 | 2.782 | -1.576 | 4.294 | 1.00 | 14.18 | A | C |
| ATOM | 866 | O | TYR | A | 118 | 3.838 | -2.228 | 4.363 | 1.00 | 14.05 | A | O |
| ATOM | 867 | N | SER | A | 119 | 1.705 | -2.024 | 3.669 | 1.00 | 14.24 | A | N |
| ATOM | 868 | CA | SER | A | 119 | 1.684 | -3.358 | 3.064 | 1.00 | 15.13 | A | C |
| ATOM | 869 | CB | SER | A | 119 | 0.288 | -3.660 | 2.544 | 1.00 | 14.77 | A | C |
| ATOM | 870 | OG | SER | A | 119 | -0.609 | -3.744 | 3.638 | 1.00 | 13.56 | A | O |
| ATOM | 871 | C | SER | A | 119 | 2.752 | -3.531 | 1.977 | 1.00 | 15.67 | A | C |
| ATOM | 872 | O | SER | A | 119 | 3.313 | -4.602 | 1.818 | 1.00 | 15.80 | A | O |
| ATOM | 873 | N | ALA | A | 120 | 3.052 | -2.461 | 1.254 | 1.00 | 16.24 | A | N |
| ATOM | 874 | CA | ALA | A | 120 | 4.085 | -2.488 | 0.204 | 1.00 | 15.93 | A | C |
| ATOM | 875 | CB | ALA | A | 120 | 3.847 | -1.352 | -0.759 | 1.00 | 16.16 | A | C |
| ATOM | 876 | C | ALA | A | 120 | 5.504 | -2.405 | 0.767 | 1.00 | 16.13 | A | C |
| ATOM | 877 | O | ALA | A | 120 | 6.474 | -2.473 | 0.030 | 1.00 | 16.93 | A | O |
| ATOM | 878 | N | GLY | A | 121 | 5.626 | -2.249 | 2.083 | 1.00 | 16.23 | A | N |
| ATOM | 879 | CA | GLY | A | 121 | 6.917 | -2.249 | 2.747 | 1.00 | 15.35 | A | C |
| ATOM | 880 | C | GLY | A | 121 | 7.400 | -0.883 | 3.247 | 1.00 | 15.15 | A | C |
| ATOM | 881 | O | GLY | A | 121 | 8.466 | -0.811 | 3.893 | 1.00 | 15.57 | A | O |
| ATOM | 882 | N | ALA | A | 122 | 6.665 | 0.195 | 2.977 | 1.00 | 14.40 | A | N |
| ATOM | 883 | CA | ALA | A | 122 | 7.110 | 1.522 | 3.443 | 1.00 | 14.85 | A | C |
| ATOM | 884 | CB | ALA | A | 122 | 6.273 | 2.632 | 2.831 | 1.00 | 14.84 | A | C |
| ATOM | 885 | C | ALA | A | 122 | 7.057 | 1.635 | 4.964 | 1.00 | 14.35 | A | C |
| ATOM | 886 | O | ALA | A | 122 | 6.078 | 1.230 | 5.574 | 1.00 | 15.21 | A | O |
| ATOM | 887 | N | ARG | A | 123 | 8.077 | 2.223 | 5.570 | 1.00 | 13.62 | A | N |
| ATOM | 888 | CA | ARG | A | 123 | 8.013 | 2.499 | 7.008 | 1.00 | 12.85 | A | C |
| ATOM | 889 | CB | ARG | A | 123 | 9.065 | 1.689 | 7.761 | 1.00 | 12.85 | A | C |
| ATOM | 890 | CG | ARG | A | 123 | 8.870 | 0.162 | 7.597 | 1.00 | 13.74 | A | C |
| ATOM | 891 | CD | ARG | A | 123 | 7.584 | -0.334 | 8.290 | 1.00 | 14.35 | A | C |
| ATOM | 892 | NE | ARG | A | 123 | 7.396 | -1.786 | 8.187 | 1.00 | 14.77 | A | N |
| ATOM | 893 | CZ | ARG | A | 123 | 6.676 | -2.389 | 7.253 | 1.00 | 16.63 | A | C |
| ATOM | 894 | NH1 | ARG | A | 123 | 6.039 | -1.678 | 6.337 | 1.00 | 15.68 | A | N |
| ATOM | 895 | NH2 | ARG | A | 123 | 6.579 | -3.719 | 7.240 | 1.00 | 17.36 | A | N |
| ATOM | 896 | C | ARG | A | 123 | 8.132 | 3.987 | 7.298 | 1.00 | 12.72 | A | C |
| ATOM | 897 | O | ARG | A | 123 | 8.116 | 4.418 | 8.448 | 1.00 | 12.15 | A | O |
| ATOM | 898 | N | ILE | A | 124 | 8.225 | 4.773 | 6.234 | 1.00 | 12.67 | A | N |
| ATOM | 899 | CA | ILE | A | 124 | 8.177 | 6.218 | 6.346 | 1.00 | 12.97 | A | C |
| ATOM | 900 | CB | ILE | A | 124 | 9.554 | 6.814 | 6.025 | 1.00 | 12.64 | A | C |
| ATOM | 901 | CG1 | ILE | A | 124 | 10.619 | 6.262 | 6.985 | 1.00 | 13.71 | A | C |
| ATOM | 902 | CD1 | ILE | A | 124 | 12.068 | 6.395 | 6.480 | 1.00 | 14.82 | A | C |
| ATOM | 903 | CG2 | ILE | A | 124 | 9.478 | 8.348 | 6.061 | 1.00 | 13.97 | A | C |
| ATOM | 904 | C | ILE | A | 124 | 7.160 | 6.695 | 5.324 | 1.00 | 12.91 | A | C |
| ATOM | 905 | O | ILE | A | 124 | 7.132 | 6.195 | 4.210 | 1.00 | 13.07 | A | O |
| ATOM | 906 | N | HIS | A | 125 | 6.365 | 7.696 | 5.671 | 1.00 | 12.94 | A | N |
| ATOM | 907 | CA | HIS | A | 125 | 5.252 | 8.100 | 4.823 | 1.00 | 12.77 | A | C |
| ATOM | 908 | CB | HIS | A | 125 | 3.894 | 7.549 | 5.353 | 1.00 | 13.00 | A | C |
| ATOM | 909 | CG | HIS | A | 125 | 2.806 | 7.650 | 4.334 | 1.00 | 15.91 | A | C |
| ATOM | 910 | ND1 | HIS | A | 125 | 2.428 | 8.850 | 3.783 | 1.00 | 13.47 | A | N |
| ATOM | 911 | CE1 | HIS | A | 125 | 1.547 | 8.632 | 2.821 | 1.00 | 16.40 | A | C |
| ATOM | 912 | NE2 | HIS | A | 125 | 1.312 | 7.333 | 2.756 | 1.00 | 16.49 | A | N |
| ATOM | 913 | CD2 | HIS | A | 125 | 2.072 | 6.699 | 3.705 | 1.00 | 18.35 | A | C |
| ATOM | 914 | C | HIS | A | 125 | 5.223 | 9.620 | 4.828 | 1.00 | 12.62 | A | C |
| ATOM | 915 | O | HIS | A | 125 | 5.053 | 10.202 | 5.893 | 1.00 | 12.04 | A | O |
| ATOM | 916 | N | THR | A | 126 | 5.401 | 10.268 | 3.674 | 1.00 | 12.71 | A | N |
| ATOM | 917 | CA | THR | A | 126 | 5.527 | 11.738 | 3.641 | 1.00 | 12.63 | A | C |
| ATOM | 918 | CB | THR | A | 126 | 6.984 | 12.142 | 3.302 | 1.00 | 12.63 | A | C |
| ATOM | 919 | OG1 | THR | A | 126 | 7.121 | 13.560 | 3.334 | 1.00 | 12.18 | A | O |
| ATOM | 920 | CG2 | THR | A | 126 | 7.395 | 11.747 | 1.864 | 1.00 | 12.36 | A | C |
| ATOM | 921 | C | THR | A | 126 | 4.498 | 12.426 | 2.735 | 1.00 | 12.60 | A | C |
| ATOM | 922 | O | THR | A | 126 | 4.166 | 11.931 | 1.652 | 1.00 | 12.62 | A | O |
| ATOM | 923 | N | ASN | A | 127 | 4.010 | 13.572 | 3.200 | 1.00 | 12.52 | A | N |

| ATOM | 924 | CA | ASN | A | 127 | 2.778 | 14.189 | 2.696 | 1.00 | 12.84 | A | C |
|------|-----|-----|-----|---|-----|-------|--------|-------|------|-------|---|---|
| ATOM | 925 | CB | ASN | A | 127 | 1.599 | 13.811 | 3.605 | 1.00 | 13.04 | A | C |
| ATOM | 926 | CG | ASN | A | 127 | 1.433 | 12.325 | 3.720 | 1.00 | 13.25 | A | C |
| ATOM | 927 | OD1 | ASN | A | 127 | 1.916 | 11.686 | 4.690 | 1.00 | 13.15 | A | O |
| ATOM | 928 | ND2 | ASN | A | 127 | 0.814 | 11.740 | 2.712 | 1.00 | 9.82 | A | N |
| ATOM | 929 | C | ASN | A | 127 | 2.894 | 15.706 | 2.637 | 1.00 | 12.70 | A | C |
| ATOM | 930 | O | ASN | A | 127 | 2.798 | 16.390 | 3.661 | 1.00 | 13.27 | A | O |
| ATOM | 931 | N | SER | A | 128 | 3.103 | 16.211 | 1.435 | 1.00 | 12.76 | A | N |
| ATOM | 932 | CA | SER | A | 128 | 3.277 | 17.640 | 1.162 | 1.00 | 13.02 | A | C |
| ATOM | 933 | CB | SER | A | 128 | 4.308 | 17.831 | 0.043 | 1.00 | 12.57 | A | C |
| ATOM | 934 | OG | SER | A | 128 | 5.608 | 17.510 | 0.485 | 1.00 | 12.52 | A | O |
| ATOM | 935 | C | SER | A | 128 | 1.927 | 18.238 | 0.748 | 1.00 | 13.42 | A | C |
| ATOM | 936 | O | SER | A | 128 | 1.763 | 18.767 | -0.372 | 1.00 | 13.82 | A | O |
| ATOM | 937 | N | TRP | A | 129 | 0.968 | 18.129 | 1.663 | 1.00 | 13.86 | A | N |
| ATOM | 938 | CA | TRP | A | 129 | -0.392 | 18.616 | 1.465 | 1.00 | 13.67 | A | C |
| ATOM | 939 | CB | TRP | A | 129 | -1.215 | 17.648 | 0.602 | 1.00 | 13.88 | A | C |
| ATOM | 940 | CG | TRP | A | 129 | -1.130 | 16.180 | 0.964 | 1.00 | 13.08 | A | C |
| ATOM | 941 | CD1 | TRP | A | 129 | -0.305 | 15.232 | 0.391 | 1.00 | 14.80 | A | C |
| ATOM | 942 | NE1 | TRP | A | 129 | -0.531 | 13.997 | 0.956 | 1.00 | 12.40 | A | N |
| ATOM | 943 | CE2 | TRP | A | 129 | -1.518 | 14.122 | 1.900 | 1.00 | 13.40 | A | C |
| ATOM | 944 | CD2 | TRP | A | 129 | -1.924 | 15.480 | 1.921 | 1.00 | 12.64 | A | C |
| ATOM | 945 | CE3 | TRP | A | 129 | -2.948 | 15.857 | 2.806 | 1.00 | 14.15 | A | C |
| ATOM | 946 | CZ3 | TRP | A | 129 | -3.504 | 14.910 | 3.614 | 1.00 | 13.25 | A | C |
| ATOM | 947 | CH2 | TRP | A | 129 | -3.082 | 13.566 | 3.559 | 1.00 | 13.78 | A | C |
| ATOM | 948 | CZ2 | TRP | A | 129 | -2.101 | 13.158 | 2.711 | 1.00 | 12.56 | A | C |
| ATOM | 949 | C | TRP | A | 129 | -1.089 | 18.859 | 2.782 | 1.00 | 14.44 | A | C |
| ATOM | 950 | O | TRP | A | 129 | -0.612 | 18.460 | 3.876 | 1.00 | 14.19 | A | O |
| ATOM | 951 | N | GLY | A | 130 | -2.224 | 19.538 | 2.694 | 1.00 | 14.75 | A | N |
| ATOM | 952 | CA | GLY | A | 130 | -3.004 | 19.834 | 3.866 | 1.00 | 15.45 | A | C |
| ATOM | 953 | C | GLY | A | 130 | -4.173 | 20.744 | 3.563 | 1.00 | 16.66 | A | C |
| ATOM | 954 | O | GLY | A | 130 | -4.203 | 21.394 | 2.518 | 1.00 | 16.04 | A | O |
| ATOM | 955 | N | ALA | A | 131 | -5.139 | 20.754 | 4.478 | 1.00 | 16.87 | A | N |
| ATOM | 956 | CA | ALA | A | 131 | -6.222 | 21.733 | 4.484 | 1.00 | 18.19 | A | C |
| ATOM | 957 | CB | ALA | A | 131 | -7.515 | 21.097 | 4.983 | 1.00 | 17.33 | A | C |
| ATOM | 958 | C | ALA | A | 131 | -5.843 | 22.852 | 5.423 | 1.00 | 19.54 | A | C |
| ATOM | 959 | O | ALA | A | 131 | -5.562 | 22.590 | 6.592 | 1.00 | 20.18 | A | O |
| ATOM | 960 | N | PRO | A | 132 | -5.869 | 24.090 | 4.942 | 1.00 | 20.97 | A | N |
| ATOM | 961 | CA | PRO | A | 132 | -5.513 | 25.253 | 5.763 | 1.00 | 21.46 | A | C |
| ATOM | 962 | CB | PRO | A | 132 | -5.260 | 26.346 | 4.724 | 1.00 | 21.95 | A | C |
| ATOM | 963 | CG | PRO | A | 132 | -6.060 | 25.967 | 3.546 | 1.00 | 22.18 | A | C |
| ATOM | 964 | CD | PRO | A | 132 | -6.220 | 24.462 | 3.564 | 1.00 | 21.43 | A | C |
| ATOM | 965 | C | PRO | A | 132 | -6.595 | 25.676 | 6.753 | 1.00 | 22.74 | A | C |
| ATOM | 966 | O | PRO | A | 132 | -7.272 | 26.703 | 6.555 | 1.00 | 24.13 | A | O |
| ATOM | 967 | N | VAL | A | 133 | -6.708 | 24.912 | 7.833 | 1.00 | 22.71 | A | N |
| ATOM | 968 | CA | VAL | A | 133 | -7.723 | 25.086 | 8.850 | 1.00 | 23.39 | A | C |
| ATOM | 969 | CB | VAL | A | 133 | -8.349 | 23.712 | 9.223 | 1.00 | 23.48 | A | C |
| ATOM | 970 | CG1 | VAL | A | 133 | -9.115 | 23.133 | 8.045 | 1.00 | 25.68 | A | C |
| ATOM | 971 | CG2 | VAL | A | 133 | -7.269 | 22.750 | 9.687 | 1.00 | 24.53 | A | C |
| ATOM | 972 | C | VAL | A | 133 | -7.223 | 25.742 | 10.150 | 1.00 | 23.23 | A | C |
| ATOM | 973 | O | VAL | A | 133 | -7.855 | 25.599 | 11.185 | 1.00 | 22.57 | A | O |
| ATOM | 974 | N | ASN | A | 134 | -6.094 | 26.437 | 10.098 | 1.00 | 23.10 | A | N |
| ATOM | 975 | CA | ASN | A | 134 | -5.660 | 27.279 | 11.201 | 1.00 | 23.36 | A | C |
| ATOM | 976 | CB | ASN | A | 134 | -6.583 | 28.512 | 11.310 | 1.00 | 24.40 | A | C |
| ATOM | 977 | CG | ASN | A | 134 | -6.491 | 29.413 | 10.082 | 1.00 | 26.68 | A | C |
| ATOM | 978 | OD1 | ASN | A | 134 | -7.489 | 30.000 | 9.650 | 1.00 | 34.40 | A | O |
| ATOM | 979 | ND2 | ASN | A | 134 | -5.315 | 29.478 | 9.482 | 1.00 | 28.87 | A | N |
| ATOM | 980 | C | ASN | A | 134 | -5.588 | 26.561 | 12.535 | 1.00 | 22.43 | A | C |
| ATOM | 981 | O | ASN | A | 134 | -6.210 | 26.971 | 13.510 | 1.00 | 21.30 | A | O |
| ATOM | 982 | N | GLY | A | 135 | -4.844 | 25.458 | 12.574 | 1.00 | 21.06 | A | N |
| ATOM | 983 | CA | GLY | A | 135 | -4.548 | 24.840 | 13.846 | 1.00 | 20.36 | A | C |
| ATOM | 984 | C | GLY | A | 135 | -5.541 | 23.818 | 14.308 | 1.00 | 19.66 | A | C |
| ATOM | 985 | O | GLY | A | 135 | -5.320 | 23.200 | 15.327 | 1.00 | 18.95 | A | O |
| ATOM | 986 | N | ALA | A | 136 | -6.613 | 23.595 | 13.557 | 1.00 | 19.08 | A | N |

47

| ATOM | 987 | CA | ALA | A | 136 | -7.609 | 22.643 | 14.006 | 1.00 | 19.00 | A | C |
|------|------|-----|-----|---|-----|--------|--------|--------|------|-------|---|---|
| ATOM | 988 | CB | ALA | A | 136 | -8.925 | 22.778 | 13.199 | 1.00 | 19.34 | A | C |
| ATOM | 989 | C | ALA | A | 136 | -7.098 | 21.206 | 13.893 | 1.00 | 19.31 | A | C |
| ATOM | 990 | O | ALA | A | 136 | -6.354 | 20.851 | 12.952 | 1.00 | 18.44 | A | O |
| ATOM | 991 | N | TYR | A | 137 | -7.568 | 20.407 | 14.841 | 1.00 | 18.56 | A | N |
| ATOM | 992 | CA | TYR | A | 137 | -7.341 | 18.979 | 14.907 | 1.00 | 18.99 | A | C |
| ATOM | 993 | CB | TYR | A | 137 | -7.112 | 18.588 | 16.367 | 1.00 | 18.67 | A | C |
| ATOM | 994 | CG | TYR | A | 137 | -6.637 | 17.175 | 16.588 | 1.00 | 19.68 | A | C |
| ATOM | 995 | CD1 | TYR | A | 137 | -7.537 | 16.173 | 16.885 | 1.00 | 19.55 | A | C |
| ATOM | 996 | CE1 | TYR | A | 137 | -7.112 | 14.855 | 17.099 | 1.00 | 21.07 | A | C |
| ATOM | 997 | CZ | TYR | A | 137 | -5.765 | 14.548 | 17.045 | 1.00 | 20.92 | A | C |
| ATOM | 998 | OH | TYR | A | 137 | -5.371 | 13.250 | 17.265 | 1.00 | 20.20 | A | O |
| ATOM | 999 | CE2 | TYR | A | 137 | -4.837 | 15.538 | 16.754 | 1.00 | 20.35 | A | C |
| ATOM | 1000 | CD2 | TYR | A | 137 | -5.278 | 16.848 | 16.522 | 1.00 | 19.75 | A | C |
| ATOM | 1001 | C | TYR | A | 137 | -8.600 | 18.314 | 14.337 | 1.00 | 18.91 | A | C |
| ATOM | 1002 | O | TYR | A | 137 | -9.648 | 18.229 | 14.994 | 1.00 | 18.41 | A | O |
| ATOM | 1003 | N | THR | A | 138 | -8.481 | 17.872 | 13.091 | 1.00 | 18.62 | A | N |
| ATOM | 1004 | CA | THR | A | 138 | -9.608 | 17.401 | 12.329 | 1.00 | 17.86 | A | C |
| ATOM | 1005 | CB | THR | A | 138 | -9.480 | 17.836 | 10.897 | 1.00 | 18.02 | A | C |
| ATOM | 1006 | OG1 | THR | A | 138 | -8.271 | 17.308 | 10.321 | 1.00 | 16.63 | A | O |
| ATOM | 1007 | CG2 | THR | A | 138 | -9.308 | 19.330 | 10.788 | 1.00 | 17.63 | A | C |
| ATOM | 1008 | C | THR | A | 138 | -9.593 | 15.888 | 12.407 | 1.00 | 18.43 | A | C |
| ATOM | 1009 | O | THR | A | 138 | -8.662 | 15.296 | 12.954 | 1.00 | 17.82 | A | O |
| ATOM | 1010 | N | THR | A | 139 | -10.624 | 15.278 | 11.843 | 1.00 | 18.06 | A | N |
| ATOM | 1011 | CA | THR | A | 139 | -10.713 | 13.843 | 11.705 | 1.00 | 17.69 | A | C |
| ATOM | 1012 | CB | THR | A | 139 | -12.020 | 13.472 | 10.947 | 1.00 | 18.57 | A | C |
| ATOM | 1013 | OG1 | THR | A | 139 | -13.162 | 13.907 | 11.705 | 1.00 | 20.05 | A | O |
| ATOM | 1014 | CG2 | THR | A | 139 | -12.173 | 11.933 | 10.828 | 1.00 | 19.16 | A | C |
| ATOM | 1015 | C | THR | A | 139 | -9.496 | 13.285 | 10.989 | 1.00 | 16.78 | A | C |
| ATOM | 1016 | O | THR | A | 139 | -9.037 | 12.183 | 11.307 | 1.00 | 17.05 | A | O |
| ATOM | 1017 | N | ASP | A | 140 | -8.976 | 14.002 | 10.002 | 1.00 | 15.81 | A | N |
| ATOM | 1018 | CA | ASP | A | 140 | -7.758 | 13.544 | 9.351 | 1.00 | 15.65 | A | C |
| ATOM | 1019 | CB | ASP | A | 140 | -7.391 | 14.429 | 8.177 | 1.00 | 15.45 | A | C |
| ATOM | 1020 | CG | ASP | A | 140 | -8.279 | 14.209 | 6.984 | 1.00 | 16.65 | A | C |
| ATOM | 1021 | OD1 | ASP | A | 140 | -8.495 | 15.189 | 6.263 | 1.00 | 18.76 | A | O |
| ATOM | 1022 | OD2 | ASP | A | 140 | -8.781 | 13.102 | 6.702 | 1.00 | 16.74 | A | O |
| ATOM | 1023 | C | ASP | A | 140 | -6.567 | 13.504 | 10.352 | 1.00 | 15.55 | A | C |
| ATOM | 1024 | O | ASP | A | 140 | -5.823 | 12.532 | 10.395 | 1.00 | 15.72 | A | O |
| ATOM | 1025 | N | SER | A | 141 | -6.395 | 14.555 | 11.133 | 1.00 | 15.34 | A | N |
| ATOM | 1026 | CA | SER | A | 141 | -5.375 | 14.548 | 12.187 | 1.00 | 15.53 | A | C |
| ATOM | 1027 | CB | SER | A | 141 | -5.428 | 15.823 | 13.006 | 1.00 | 14.57 | A | C |
| ATOM | 1028 | OG | SER | A | 141 | -5.275 | 16.936 | 12.173 | 1.00 | 16.14 | A | O |
| ATOM | 1029 | C | SER | A | 141 | -5.514 | 13.375 | 13.157 | 1.00 | 15.46 | A | C |
| ATOM | 1030 | O | SER | A | 141 | -4.511 | 12.754 | 13.558 | 1.00 | 15.37 | A | O |
| ATOM | 1031 | N | ARG | A | 142 | -6.754 | 13.100 | 13.546 | 1.00 | 15.27 | A | N |
| ATOM | 1032 | CA | ARG | A | 142 | -7.053 | 11.998 | 14.462 | 1.00 | 15.97 | A | C |
| ATOM | 1033 | CB | ARG | A | 142 | -8.539 | 12.004 | 14.843 | 1.00 | 16.78 | A | C |
| ATOM | 1034 | CG | ARG | A | 142 | -8.882 | 11.091 | 16.022 | 1.00 | 18.90 | A | C |
| ATOM | 1035 | CD | ARG | A | 142 | -10.365 | 11.103 | 16.436 | 1.00 | 22.40 | A | C |
| ATOM | 1036 | NE | ARG | A | 142 | -10.533 | 10.384 | 17.704 | 1.00 | 25.70 | A | N |
| ATOM | 1037 | CZ | ARG | A | 142 | -10.549 | 9.057 | 17.839 | 1.00 | 29.38 | A | C |
| ATOM | 1038 | NH1 | ARG | A | 142 | -10.423 | 8.249 | 16.786 | 1.00 | 30.26 | A | N |
| ATOM | 1039 | NH2 | ARG | A | 142 | -10.685 | 8.524 | 19.048 | 1.00 | 30.56 | A | N |
| ATOM | 1040 | C | ARG | A | 142 | -6.703 | 10.643 | 13.860 | 1.00 | 15.43 | A | C |
| ATOM | 1041 | O | ARG | A | 142 | -6.107 | 9.778 | 14.534 | 1.00 | 14.36 | A | O |
| ATOM | 1042 | N | ASN | A | 143 | -7.068 | 10.437 | 12.593 | 1.00 | 14.96 | A | N |
| ATOM | 1043 | CA | ASN | A | 143 | -6.699 | 9.187 | 11.926 | 1.00 | 14.70 | A | C |
| ATOM | 1044 | CB | ASN | A | 143 | -7.451 | 9.062 | 10.593 | 1.00 | 15.54 | A | C |
| ATOM | 1045 | CG | ASN | A | 143 | -8.952 | 8.709 | 10.803 | 1.00 | 16.85 | A | C |
| ATOM | 1046 | OD1 | ASN | A | 143 | -9.842 | 9.204 | 10.096 | 1.00 | 20.66 | A | O |
| ATOM | 1047 | ND2 | ASN | A | 143 | -9.206 | 7.828 | 11.746 | 1.00 | 15.73 | A | N |
| ATOM | 1048 | C | ASN | A | 143 | -5.183 | 8.986 | 11.754 | 1.00 | 15.26 | A | C |
| ATOM | 1049 | O | ASN | A | 143 | -4.691 | 7.854 | 11.879 | 1.00 | 15.15 | A | O |

| ATOM | 1050 | N   | VAL | A | 144 | -4.438 | 10.060 | 11.450 | 1.00 | 14.90 | A | N |
|------|------|-----|-----|---|-----|--------|--------|--------|------|-------|---|---|
| ATOM | 1051 | CA  | VAL | A | 144 | -2.976 | 9.987  | 11.467 | 1.00 | 13.93 | A | C |
| ATOM | 1052 | CB  | VAL | A | 144 | -2.319 | 11.347 | 11.177 | 1.00 | 14.08 | A | C |
| ATOM | 1053 | CG1 | VAL | A | 144 | -0.803 | 11.272 | 11.422 | 1.00 | 12.32 | A | C |
| ATOM | 1054 | CG2 | VAL | A | 144 | -2.625 | 11.818 | 9.748  | 1.00 | 13.08 | A | C |
| ATOM | 1055 | C   | VAL | A | 144 | -2.478 | 9.507  | 12.843 | 1.00 | 14.23 | A | C |
| ATOM | 1056 | O   | VAL | A | 144 | -1.608 | 8.653  | 12.938 | 1.00 | 13.96 | A | O |
| ATOM | 1057 | N   | ASP | A | 145 | -3.021 | 10.077 | 13.916 | 1.00 | 14.48 | A | N |
| ATOM | 1058 | CA  | ASP | A | 145 | -2.548 | 9.745  | 15.256 | 1.00 | 14.49 | A | C |
| ATOM | 1059 | CB  | ASP | A | 145 | -3.123 | 10.711 | 16.249 | 1.00 | 14.81 | A | C |
| ATOM | 1060 | CG  | ASP | A | 145 | -2.406 | 12.033 | 16.218 | 1.00 | 15.70 | A | C |
| ATOM | 1061 | OD1 | ASP | A | 145 | -1.332 | 12.107 | 15.545 | 1.00 | 14.69 | A | O |
| ATOM | 1062 | OD2 | ASP | A | 145 | -2.845 | 13.048 | 16.803 | 1.00 | 14.46 | A | O |
| ATOM | 1063 | C   | ASP | A | 145 | -2.849 | 8.331  | 15.654 | 1.00 | 15.08 | A | C |
| ATOM | 1064 | O   | ASP | A | 145 | -1.999 | 7.622  | 16.183 | 1.00 | 14.89 | A | O |
| ATOM | 1065 | N   | ASP | A | 146 | -4.065 | 7.906  | 15.361 | 1.00 | 15.66 | A | N |
| ATOM | 1066 | CA  | ASP | A | 146 | -4.470 | 6.545  | 15.608 | 1.00 | 15.98 | A | C |
| ATOM | 1067 | CB  | ASP | A | 146 | -5.931 | 6.400  | 15.184 | 1.00 | 16.37 | A | C |
| ATOM | 1068 | CG  | ASP | A | 146 | -6.565 | 5.107  | 15.705 | 1.00 | 17.81 | A | C |
| ATOM | 1069 | OD1 | ASP | A | 146 | -6.337 | 4.735  | 16.879 | 1.00 | 18.17 | A | O |
| ATOM | 1070 | OD2 | ASP | A | 146 | -7.277 | 4.401  | 14.981 | 1.00 | 21.66 | A | O |
| ATOM | 1071 | C   | ASP | A | 146 | -3.562 | 5.571  | 14.849 | 1.00 | 16.07 | A | C |
| ATOM | 1072 | O   | ASP | A | 146 | -3.047 | 4.607  | 15.408 | 1.00 | 16.60 | A | O |
| ATOM | 1073 | N   | TYR | A | 147 | -3.324 | 5.842  | 13.576 | 1.00 | 15.73 | A | N |
| ATOM | 1074 | CA  | TYR | A | 147 | -2.463 | 4.988  | 12.772 | 1.00 | 15.49 | A | C |
| ATOM | 1075 | CB  | TYR | A | 147 | -2.387 | 5.486  | 11.314 | 1.00 | 15.22 | A | C |
| ATOM | 1076 | CG  | TYR | A | 147 | -1.759 | 4.421  | 10.459 | 1.00 | 16.31 | A | C |
| ATOM | 1077 | CD1 | TYR | A | 147 | -0.400 | 4.394  | 10.249 | 1.00 | 17.35 | A | C |
| ATOM | 1078 | CE1 | TYR | A | 147 | 0.180  | 3.380  | 9.506  | 1.00 | 17.49 | A | C |
| ATOM | 1079 | CZ  | TYR | A | 147 | -0.599 | 2.364  | 9.004  | 1.00 | 16.46 | A | C |
| ATOM | 1080 | OH  | TYR | A | 147 | -0.022 | 1.354  | 8.281  | 1.00 | 20.80 | A | O |
| ATOM | 1081 | CE2 | TYR | A | 147 | -1.944 | 2.346  | 9.227  | 1.00 | 16.19 | A | C |
| ATOM | 1082 | CD2 | TYR | A | 147 | -2.523 | 3.364  | 9.947  | 1.00 | 16.98 | A | C |
| ATOM | 1083 | C   | TYR | A | 147 | -1.025 | 4.833  | 13.309 | 1.00 | 15.68 | A | C |
| ATOM | 1084 | O   | TYR | A | 147 | -0.491 | 3.719  | 13.385 | 1.00 | 14.79 | A | O |
| ATOM | 1085 | N   | VAL | A | 148 | -0.399 | 5.953  | 13.652 | 1.00 | 16.33 | A | N |
| ATOM | 1086 | CA  | VAL | A | 148 | 0.975  | 5.950  | 14.144 | 1.00 | 16.18 | A | C |
| ATOM | 1087 | CB  | VAL | A | 148 | 1.534  | 7.390  | 14.262 | 1.00 | 16.37 | A | C |
| ATOM | 1088 | CG1 | VAL | A | 148 | 2.953  | 7.397  | 14.909 | 1.00 | 17.53 | A | C |
| ATOM | 1089 | CG2 | VAL | A | 148 | 1.600  | 8.044  | 12.899 | 1.00 | 16.39 | A | C |
| ATOM | 1090 | C   | VAL | A | 148 | 1.063  | 5.206  | 15.488 | 1.00 | 16.59 | A | C |
| ATOM | 1091 | O   | VAL | A | 148 | 2.022  | 4.481  | 15.765 | 1.00 | 16.63 | A | O |
| ATOM | 1092 | N   | ARG | A | 149 | 0.061  | 5.356  | 16.331 | 1.00 | 16.70 | A | N |
| ATOM | 1093 | CA  | ARG | A | 149 | 0.109  | 4.628  | 17.589 | 1.00 | 18.28 | A | C |
| ATOM | 1094 | CB  | ARG | A | 149 | -0.920 | 5.133  | 18.600 | 1.00 | 18.33 | A | C |
| ATOM | 1095 | CG  | ARG | A | 149 | -0.585 | 4.657  | 20.002 | 1.00 | 19.51 | A | C |
| ATOM | 1096 | CD  | ARG | A | 149 | -1.566 | 5.035  | 21.071 | 1.00 | 20.84 | A | C |
| ATOM | 1097 | NE  | ARG | A | 149 | -0.987 | 4.731  | 22.383 | 1.00 | 22.92 | A | N |
| ATOM | 1098 | CZ  | ARG | A | 149 | -1.661 | 4.491  | 23.504 | 1.00 | 24.06 | A | C |
| ATOM | 1099 | NH1 | ARG | A | 149 | -2.985 | 4.521  | 23.538 | 1.00 | 25.69 | A | N |
| ATOM | 1100 | NH2 | ARG | A | 149 | -0.987 | 4.221  | 24.616 | 1.00 | 23.61 | A | N |
| ATOM | 1101 | C   | ARG | A | 149 | -0.035 | 3.126  | 17.382 | 1.00 | 18.63 | A | C |
| ATOM | 1102 | O   | ARG | A | 149 | 0.517  | 2.346  | 18.156 | 1.00 | 18.97 | A | O |
| ATOM | 1103 | N   | LYS | A | 150 | -0.739 | 2.720  | 16.326 | 1.00 | 18.98 | A | N |
| ATOM | 1104 | CA  | LYS | A | 150 | -0.991 | 1.294  | 16.087 | 1.00 | 19.24 | A | C |
| ATOM | 1105 | CB  | LYS | A | 150 | -2.373 | 1.092  | 15.438 | 1.00 | 19.89 | A | C |
| ATOM | 1106 | CG  | LYS | A | 150 | -3.576 | 1.358  | 16.389 | 1.00 | 21.34 | A | C |
| ATOM | 1107 | CD  | LYS | A | 150 | -4.902 | 0.972  | 15.736 | 1.00 | 24.20 | A | C |
| ATOM | 1108 | CE  | LYS | A | 150 | -6.136 | 1.437  | 16.531 | 1.00 | 27.20 | A | C |
| ATOM | 1109 | NZ  | LYS | A | 150 | -7.373 | 1.614  | 15.668 | 1.00 | 30.36 | A | N |
| ATOM | 1110 | C   | LYS | A | 150 | 0.123  | 0.622  | 15.250 | 1.00 | 18.99 | A | C |
| ATOM | 1111 | O   | LYS | A | 150 | 0.296  | -0.577 | 15.305 | 1.00 | 17.16 | A | O |
| ATOM | 1112 | N   | ASN | A | 151 | 0.916  | 1.407  | 14.526 | 1.00 | 19.09 | A | N |

```
ATOM   1113  CA   ASN A 151     1.834   0.850  13.538  1.00 19.75      A    C
ATOM   1114  CB   ASN A 151     1.225   0.950  12.130  1.00 20.10      A    C
ATOM   1115  CG   ASN A 151    -0.141   0.299  12.025  1.00 19.86      A    C
ATOM   1116  OD1  ASN A 151    -0.239  -0.905  11.855  1.00 19.33      A    O
ATOM   1117  ND2  ASN A 151    -1.198   1.090  12.167  1.00 19.31      A    N
ATOM   1118  C    ASN A 151     3.150   1.599  13.557  1.00 20.21      A    C
ATOM   1119  O    ASN A 151     3.193   2.807  13.793  1.00 22.14      A    O
ATOM   1120  N    ASP A 152     4.239   0.911  13.299  1.00 20.04      A    N
ATOM   1121  CA   ASP A 152     5.508   1.595  13.319  1.00 20.51      A    C
ATOM   1122  CB  BASP A 152     6.571   0.640  13.830  0.35 20.16      A    C
ATOM   1123  CB  AASP A 152     6.645   0.666  13.762  0.65 21.56      A    C
ATOM   1124  CG  BASP A 152     6.199   0.067  15.205  0.35 19.09      A    C
ATOM   1125  CG  AASP A 152     7.225  -0.117  12.631  0.65 23.76      A    C
ATOM   1126  OD1 BASP A 152     5.318   0.654  15.901  0.35 15.06      A    O
ATOM   1127  OD1 AASP A 152     6.404  -0.719  11.924  0.65 27.77      A    O
ATOM   1128  OD2 BASP A 152     6.703  -0.977  15.653  0.35 16.81      A    O
ATOM   1129  OD2 AASP A 152     8.471  -0.170  12.353  0.65 27.40      A    O
ATOM   1130  C    ASP A 152     5.822   2.270  11.959  1.00 19.32      A    C
ATOM   1131  O    ASP A 152     6.748   1.916  11.253  1.00 20.60      A    O
ATOM   1132  N    MET A 153     4.988   3.250  11.628  1.00 16.58      A    N
ATOM   1133  CA   MET A 153     5.154   4.050  10.437  1.00 16.03      A    C
ATOM   1134  CB   MET A 153     3.876   4.007   9.619  1.00 16.23      A    C
ATOM   1135  CG   MET A 153     3.885   4.921   8.432  1.00 18.33      A    C
ATOM   1136  SD   MET A 153     4.694   4.182   7.030  1.00 21.72      A    S
ATOM   1137  CE   MET A 153     3.290   3.549   6.297  1.00 21.74      A    C
ATOM   1138  C    MET A 153     5.443   5.482  10.871  1.00 14.80      A    C
ATOM   1139  O    MET A 153     4.684   6.058  11.646  1.00 13.50      A    O
ATOM   1140  N    THR A 154     6.525   6.059  10.368  1.00 13.34      A    N
ATOM   1141  CA   THR A 154     6.813   7.482  10.638  1.00 13.08      A    C
ATOM   1142  CB   THR A 154     8.324   7.699  10.652  1.00 13.02      A    C
ATOM   1143  OG1  THR A 154     8.886   6.949  11.724  1.00 11.51      A    O
ATOM   1144  CG2  THR A 154     8.693   9.145  10.963  1.00 14.36      A    C
ATOM   1145  C    THR A 154     6.153   8.310   9.573  1.00 12.50      A    C
ATOM   1146  O    THR A 154     6.396   8.108   8.371  1.00 12.64      A    O
ATOM   1147  N    ILE A 155     5.290   9.227   9.987  1.00 12.34      A    N
ATOM   1148  CA   ILE A 155     4.492  10.002   9.037  1.00 13.00      A    C
ATOM   1149  CB   ILE A 155     2.983   9.799   9.346  1.00 13.05      A    C
ATOM   1150  CG1  ILE A 155     2.637   8.307   9.279  1.00 13.73      A    C
ATOM   1151  CD1  ILE A 155     1.121   8.017   9.274  1.00 12.93      A    C
ATOM   1152  CG2  ILE A 155     2.121  10.578   8.371  1.00 13.58      A    C
ATOM   1153  C    ILE A 155     4.861  11.480   9.137  1.00 13.01      A    C
ATOM   1154  O    ILE A 155     4.894  12.038  10.233  1.00 12.79      A    O
ATOM   1155  N    LEU A 156     5.125  12.117   8.001  1.00 12.16      A    N
ATOM   1156  CA   LEU A 156     5.509  13.528   7.982  1.00 12.88      A    C
ATOM   1157  CB   LEU A 156     6.903  13.692   7.354  1.00 12.18      A    C
ATOM   1158  CG   LEU A 156     8.089  12.960   8.007  1.00 13.25      A    C
ATOM   1159  CD1  LEU A 156     8.365  11.607   7.326  1.00 11.24      A    C
ATOM   1160  CD2  LEU A 156     9.339  13.796   7.910  1.00 13.01      A    C
ATOM   1161  C    LEU A 156     4.485  14.328   7.192  1.00 13.49      A    C
ATOM   1162  O    LEU A 156     3.982  13.850   6.160  1.00 14.76      A    O
ATOM   1163  N    PHE A 157     4.197  15.540   7.659  1.00 12.79      A    N
ATOM   1164  CA   PHE A 157     3.282  16.451   7.003  1.00 13.07      A    C
ATOM   1165  CB   PHE A 157     1.938  16.564   7.772  1.00 13.62      A    C
ATOM   1166  CG   PHE A 157     0.957  15.504   7.401  1.00 13.90      A    C
ATOM   1167  CD1  PHE A 157     0.191  15.636   6.272  1.00 11.22      A    C
ATOM   1168  CE1  PHE A 157    -0.678  14.632   5.896  1.00 14.22      A    C
ATOM   1169  CZ   PHE A 157    -0.743  13.441   6.630  1.00 14.61      A    C
ATOM   1170  CE2  PHE A 157     0.013  13.296   7.743  1.00 15.64      A    C
ATOM   1171  CD2  PHE A 157     0.891  14.312   8.122  1.00 14.56      A    C
ATOM   1172  C    PHE A 157     3.899  17.852   6.928  1.00 13.18      A    C
ATOM   1173  O    PHE A 157     4.527  18.318   7.867  1.00 12.04      A    O
ATOM   1174  N    ALA A 158     3.700  18.500   5.793  1.00 13.46      A    N
ATOM   1175  CA   ALA A 158     3.958  19.921   5.623  1.00 13.31      A    C
```

| ATOM | 1176 | CB | ALA A 158 | 3.509 | 20.334 | 4.235 | 1.00 | 14.02 | A | C |
|------|------|-----|-----------|-------|--------|-------|------|-------|---|---|
| ATOM | 1177 | C | ALA A 158 | 3.181 | 20.703 | 6.672 | 1.00 | 13.75 | A | C |
| ATOM | 1178 | O | ALA A 158 | 2.031 | 20.380 | 6.965 | 1.00 | 13.75 | A | O |
| ATOM | 1179 | N | ALA A 159 | 3.787 | 21.752 | 7.215 | 1.00 | 13.45 | A | N |
| ATOM | 1180 | CA | ALA A 159 | 3.122 | 22.582 | 8.210 | 1.00 | 13.81 | A | C |
| ATOM | 1181 | CB | ALA A 159 | 4.151 | 23.495 | 8.944 | 1.00 | 13.64 | A | C |
| ATOM | 1182 | C | ALA A 159 | 2.043 | 23.473 | 7.628 | 1.00 | 14.14 | A | C |
| ATOM | 1183 | O | ALA A 159 | 1.175 | 23.924 | 8.364 | 1.00 | 14.00 | A | O |
| ATOM | 1184 | N | GLY A 160 | 2.131 | 23.753 | 6.330 | 1.00 | 15.19 | A | N |
| ATOM | 1185 | CA | GLY A 160 | 1.230 | 24.680 | 5.652 | 1.00 | 15.34 | A | C |
| ATOM | 1186 | C | GLY A 160 | 1.957 | 25.941 | 5.236 | 1.00 | 14.93 | A | C |
| ATOM | 1187 | O | GLY A 160 | 3.041 | 26.238 | 5.736 | 1.00 | 14.62 | A | O |
| ATOM | 1188 | N | ASN A 161 | 1.371 | 26.686 | 4.307 | 1.00 | 15.04 | A | N |
| ATOM | 1189 | CA | ASN A 161 | 1.983 | 27.902 | 3.789 | 1.00 | 15.84 | A | C |
| ATOM | 1190 | CB | ASN A 161 | 2.072 | 27.872 | 2.261 | 1.00 | 15.91 | A | C |
| ATOM | 1191 | CG | ASN A 161 | 3.048 | 26.851 | 1.712 | 1.00 | 17.40 | A | C |
| ATOM | 1192 | OD1 | ASN A 161 | 3.001 | 26.550 | 0.490 | 1.00 | 21.70 | A | O |
| ATOM | 1193 | ND2 | ASN A 161 | 3.888 | 26.267 | 2.569 | 1.00 | 11.15 | A | N |
| ATOM | 1194 | C | ASN A 161 | 1.131 | 29.114 | 4.123 | 1.00 | 16.84 | A | C |
| ATOM | 1195 | O | ASN A 161 | 0.956 | 29.965 | 3.286 | 1.00 | 17.07 | A | O |
| ATOM | 1196 | N | GLU A 162 | 0.575 | 29.179 | 5.324 | 1.00 | 18.36 | A | N |
| ATOM | 1197 | CA | GLU A 162 | -0.392 | 30.213 | 5.668 | 1.00 | 18.85 | A | C |
| ATOM | 1198 | CB | GLU A 162 | -1.672 | 29.537 | 6.211 | 1.00 | 19.64 | A | C |
| ATOM | 1199 | CG | GLU A 162 | -2.431 | 28.723 | 5.150 | 1.00 | 22.12 | A | C |
| ATOM | 1200 | CD | GLU A 162 | -1.756 | 27.381 | 4.788 | 1.00 | 26.12 | A | C |
| ATOM | 1201 | OE1 | GLU A 162 | -1.585 | 26.545 | 5.702 | 1.00 | 28.48 | A | O |
| ATOM | 1202 | OE2 | GLU A 162 | -1.405 | 27.149 | 3.590 | 1.00 | 26.75 | A | O |
| ATOM | 1203 | C | GLU A 162 | 0.147 | 31.262 | 6.657 | 1.00 | 19.39 | A | C |
| ATOM | 1204 | O | GLU A 162 | -0.633 | 32.031 | 7.225 | 1.00 | 18.80 | A | O |
| ATOM | 1205 | N | GLY A 163 | 1.472 | 31.338 | 6.820 | 1.00 | 19.39 | A | N |
| ATOM | 1206 | CA | GLY A 163 | 2.082 | 32.322 | 7.705 | 1.00 | 20.03 | A | C |
| ATOM | 1207 | C | GLY A 163 | 2.224 | 33.699 | 7.048 | 1.00 | 21.41 | A | C |
| ATOM | 1208 | O | GLY A 163 | 1.822 | 33.866 | 5.877 | 1.00 | 20.72 | A | O |
| ATOM | 1209 | N | PRO A 164 | 2.835 | 34.671 | 7.737 | 1.00 | 21.93 | A | N |
| ATOM | 1210 | CA | PRO A 164 | 3.496 | 34.491 | 9.053 | 1.00 | 22.22 | A | C |
| ATOM | 1211 | CB | PRO A 164 | 4.575 | 35.577 | 9.050 | 1.00 | 22.99 | A | C |
| ATOM | 1212 | CG | PRO A 164 | 3.945 | 36.720 | 8.171 | 1.00 | 23.13 | A | C |
| ATOM | 1213 | CD | PRO A 164 | 2.976 | 36.047 | 7.209 | 1.00 | 22.03 | A | C |
| ATOM | 1214 | C | PRO A 164 | 2.681 | 34.621 | 10.329 | 1.00 | 22.18 | A | C |
| ATOM | 1215 | O | PRO A 164 | 3.289 | 34.603 | 11.414 | 1.00 | 21.36 | A | O |
| ATOM | 1216 | N | GLY A 165 | 1.363 | 34.702 | 10.239 | 1.00 | 21.66 | A | N |
| ATOM | 1217 | CA | GLY A 165 | 0.537 | 34.844 | 11.414 | 1.00 | 21.84 | A | C |
| ATOM | 1218 | C | GLY A 165 | 0.522 | 33.581 | 12.243 | 1.00 | 22.27 | A | C |
| ATOM | 1219 | O | GLY A 165 | 0.680 | 32.440 | 11.713 | 1.00 | 22.20 | A | O |
| ATOM | 1220 | N | SER A 166 | 0.305 | 33.762 | 13.543 | 1.00 | 22.06 | A | N |
| ATOM | 1221 | CA | SER A 166 | 0.290 | 32.645 | 14.470 | 1.00 | 21.96 | A | C |
| ATOM | 1222 | CB | SER A 166 | 0.344 | 33.167 | 15.917 | 1.00 | 23.25 | A | C |
| ATOM | 1223 | OG | SER A 166 | -0.948 | 33.579 | 16.367 | 1.00 | 25.13 | A | O |
| ATOM | 1224 | C | SER A 166 | -0.954 | 31.807 | 14.241 | 1.00 | 21.45 | A | C |
| ATOM | 1225 | O | SER A 166 | -1.949 | 32.311 | 13.716 | 1.00 | 21.15 | A | O |
| ATOM | 1226 | N | GLY A 167 | -0.879 | 30.515 | 14.574 | 1.00 | 20.07 | A | N |
| ATOM | 1227 | CA | GLY A 167 | -2.032 | 29.639 | 14.548 | 1.00 | 19.63 | A | C |
| ATOM | 1228 | C | GLY A 167 | -2.478 | 29.248 | 13.140 | 1.00 | 19.18 | A | C |
| ATOM | 1229 | O | GLY A 167 | -3.652 | 29.051 | 12.911 | 1.00 | 18.57 | A | O |
| ATOM | 1230 | N | THR A 168 | -1.541 | 29.140 | 12.200 | 1.00 | 18.31 | A | N |
| ATOM | 1231 | CA | THR A 168 | -1.893 | 28.857 | 10.810 | 1.00 | 17.13 | A | C |
| ATOM | 1232 | CB | THR A 168 | -1.295 | 29.958 | 9.908 | 1.00 | 17.32 | A | C |
| ATOM | 1233 | OG1 | THR A 168 | 0.077 | 30.172 | 10.261 | 1.00 | 14.44 | A | O |
| ATOM | 1234 | CG2 | THR A 168 | -1.988 | 31.299 | 10.156 | 1.00 | 17.60 | A | C |
| ATOM | 1235 | C | THR A 168 | -1.496 | 27.465 | 10.306 | 1.00 | 16.75 | A | C |
| ATOM | 1236 | O | THR A 168 | -1.462 | 27.213 | 9.091 | 1.00 | 16.26 | A | O |
| ATOM | 1237 | N | ILE A 169 | -1.265 | 26.540 | 11.234 | 1.00 | 15.95 | A | N |
| ATOM | 1238 | CA | ILE A 169 | -0.863 | 25.191 | 10.871 | 1.00 | 15.48 | A | C |

```
ATOM   1239  CB   ILE A 169     -0.454  24.378  12.127  1.00  15.09      A  C
ATOM   1240  CG1  ILE A 169      0.626  25.109  12.942  1.00  14.88      A  C
ATOM   1241  CD1  ILE A 169      2.021  25.201  12.267  1.00  16.38      A  C
ATOM   1242  CG2  ILE A 169      0.021  22.988  11.720  1.00  13.45      A  C
ATOM   1243  C    ILE A 169     -2.004  24.477  10.137  1.00  15.22      A  C
ATOM   1244  O    ILE A 169     -3.146  24.470  10.590  1.00  14.50      A  O
ATOM   1245  N    SER A 170     -1.681  23.857   9.018  1.00  14.94      A  N
ATOM   1246  CA   SER A 170     -2.665  23.060   8.310  1.00  15.41      A  C
ATOM   1247  CB   SER A 170     -2.299  23.004   6.821  1.00  16.02      A  C
ATOM   1248  OG   SER A 170     -1.040  22.404   6.585  1.00  16.03      A  O
ATOM   1249  C    SER A 170     -2.855  21.660   8.904  1.00  15.05      A  C
ATOM   1250  O    SER A 170     -1.986  21.137   9.616  1.00  13.80      A  O
ATOM   1251  N    ALA A 171     -3.992  21.036   8.582  1.00  14.65      A  N
ATOM   1252  CA   ALA A 171     -4.244  19.651   8.933  1.00  14.75      A  C
ATOM   1253  CB   ALA A 171     -5.700  19.507   9.443  1.00  15.62      A  C
ATOM   1254  C    ALA A 171     -4.043  18.750   7.740  1.00  15.02      A  C
ATOM   1255  O    ALA A 171     -4.475  19.096   6.652  1.00  14.12      A  O
ATOM   1256  N    PRO A 172     -3.482  17.548   7.899  1.00  15.48      A  N
ATOM   1257  CA   PRO A 172     -3.078  16.913   9.167  1.00  15.17      A  C
ATOM   1258  CB   PRO A 172     -3.080  15.411   8.796  1.00  15.90      A  C
ATOM   1259  CG   PRO A 172     -3.707  15.336   7.456  1.00  15.73      A  C
ATOM   1260  CD   PRO A 172     -3.401  16.614   6.768  1.00  15.43      A  C
ATOM   1261  C    PRO A 172     -1.724  17.260   9.788  1.00  14.85      A  C
ATOM   1262  O    PRO A 172     -1.284  16.499  10.651  1.00  16.20      A  O
ATOM   1263  N    GLY A 173     -1.086  18.352   9.382  1.00  14.01      A  N
ATOM   1264  CA   GLY A 173      0.078  18.895  10.064  1.00  13.48      A  C
ATOM   1265  C    GLY A 173     -0.158  19.147  11.553  1.00  13.91      A  C
ATOM   1266  O    GLY A 173      0.809  19.174  12.339  1.00  14.08      A  O
ATOM   1267  N    THR A 174     -1.419  19.291  11.956  1.00  13.50      A  N
ATOM   1268  CA   THR A 174     -1.758  19.475  13.375  1.00  13.19      A  C
ATOM   1269  CB   THR A 174     -3.137  20.125  13.530  1.00  13.66      A  C
ATOM   1270  OG1  THR A 174     -4.104  19.394  12.743  1.00  13.16      A  O
ATOM   1271  CG2  THR A 174     -3.114  21.510  12.957  1.00  14.22      A  C
ATOM   1272  C    THR A 174     -1.774  18.188  14.172  1.00  12.85      A  C
ATOM   1273  O    THR A 174     -1.909  18.227  15.390  1.00  12.08      A  O
ATOM   1274  N    ALA A 175     -1.696  17.040  13.505  1.00  12.94      A  N
ATOM   1275  CA   ALA A 175     -1.614  15.772  14.213  1.00  12.90      A  C
ATOM   1276  CB   ALA A 175     -1.422  14.641  13.211  1.00  13.54      A  C
ATOM   1277  C    ALA A 175     -0.484  15.740  15.264  1.00  12.44      A  C
ATOM   1278  O    ALA A 175      0.601  16.233  15.043  1.00  13.06      A  O
ATOM   1279  N    LYS A 176     -0.739  15.131  16.398  1.00  13.08      A  N
ATOM   1280  CA   LYS A 176      0.269  15.057  17.466  1.00  13.00      A  C
ATOM   1281  CB   LYS A 176     -0.383  14.511  18.719  1.00  12.78      A  C
ATOM   1282  CG   LYS A 176     -1.406  15.392  19.366  1.00  13.87      A  C
ATOM   1283  CD   LYS A 176     -2.044  14.693  20.553  1.00  15.77      A  C
ATOM   1284  CE   LYS A 176     -3.179  13.722  20.173  1.00  16.63      A  C
ATOM   1285  NZ   LYS A 176     -3.738  13.048  21.388  1.00  16.58      A  N
ATOM   1286  C    LYS A 176      1.433  14.107  17.115  1.00  13.10      A  C
ATOM   1287  O    LYS A 176      2.559  14.289  17.538  1.00  12.98      A  O
ATOM   1288  N    ASN A 177      1.119  13.047  16.390  1.00  12.81      A  N
ATOM   1289  CA   ASN A 177      2.047  11.933  16.187  1.00  12.78      A  C
ATOM   1290  CB   ASN A 177      1.278  10.628  16.301  1.00  12.48      A  C
ATOM   1291  CG   ASN A 177      0.733  10.382  17.718  1.00  12.12      A  C
ATOM   1292  OD1  ASN A 177      1.135  11.043  18.682  1.00  12.31      A  O
ATOM   1293  ND2  ASN A 177     -0.179   9.416  17.844  1.00  10.81      A  N
ATOM   1294  C    ASN A 177      2.822  11.966  14.876  1.00  12.68      A  C
ATOM   1295  O    ASN A 177      3.692  11.097  14.621  1.00  13.33      A  O
ATOM   1296  N    ALA A 178      2.483  12.933  14.029  1.00  12.80      A  N
ATOM   1297  CA   ALA A 178      3.234  13.208  12.801  1.00  12.70      A  C
ATOM   1298  CB   ALA A 178      2.382  13.938  11.817  1.00  12.83      A  C
ATOM   1299  C    ALA A 178      4.439  14.052  13.141  1.00  12.18      A  C
ATOM   1300  O    ALA A 178      4.471  14.685  14.188  1.00  12.29      A  O
ATOM   1301  N    ILE A 179      5.458  13.985  12.293  1.00  11.71      A  N
```

52

| | | | | | | | | | | | |
|---|---|---|---|---|---|---|---|---|---|---|---|
| ATOM | 1302 | CA | ILE | A | 179 | 6.531 | 14.966 | 12.283 | 1.00 | 11.72 | A | C |
| ATOM | 1303 | CB | ILE | A | 179 | 7.838 | 14.364 | 11.812 | 1.00 | 11.54 | A | C |
| ATOM | 1304 | CG1 | ILE | A | 179 | 8.251 | 13.222 | 12.712 | 1.00 | 13.45 | A | C |
| ATOM | 1305 | CD1 | ILE | A | 179 | 9.472 | 12.467 | 12.196 | 1.00 | 14.82 | A | C |
| ATOM | 1306 | CG2 | ILE | A | 179 | 8.927 | 15.437 | 11.783 | 1.00 | 12.00 | A | C |
| ATOM | 1307 | C | ILE | A | 179 | 6.085 | 16.076 | 11.317 | 1.00 | 11.98 | A | C |
| ATOM | 1308 | O | ILE | A | 179 | 5.943 | 15.852 | 10.109 | 1.00 | 11.31 | A | O |
| ATOM | 1309 | N | THR | A | 180 | 5.813 | 17.248 | 11.871 | 1.00 | 11.27 | A | N |
| ATOM | 1310 | CA | THR | A | 180 | 5.357 | 18.383 | 11.074 | 1.00 | 11.76 | A | C |
| ATOM | 1311 | CB | THR | A | 180 | 4.260 | 19.120 | 11.818 | 1.00 | 11.79 | A | C |
| ATOM | 1312 | OG1 | THR | A | 180 | 3.166 | 18.214 | 12.084 | 1.00 | 12.06 | A | O |
| ATOM | 1313 | CG2 | THR | A | 180 | 3.603 | 20.224 | 10.929 | 1.00 | 12.72 | A | C |
| ATOM | 1314 | C | THR | A | 180 | 6.530 | 19.306 | 10.690 | 1.00 | 11.82 | A | C |
| ATOM | 1315 | O | THR | A | 180 | 7.286 | 19.762 | 11.533 | 1.00 | 11.02 | A | O |
| ATOM | 1316 | N | VAL | A | 181 | 6.662 | 19.590 | 9.401 | 1.00 | 11.42 | A | N |
| ATOM | 1317 | CA | VAL | A | 181 | 7.830 | 20.305 | 8.899 | 1.00 | 11.43 | A | C |
| ATOM | 1318 | CB | VAL | A | 181 | 8.492 | 19.464 | 7.814 | 1.00 | 11.32 | A | C |
| ATOM | 1319 | CG1 | VAL | A | 181 | 9.744 | 20.118 | 7.309 | 1.00 | 12.42 | A | C |
| ATOM | 1320 | CG2 | VAL | A | 181 | 8.757 | 18.055 | 8.351 | 1.00 | 12.33 | A | C |
| ATOM | 1321 | C | VAL | A | 181 | 7.511 | 21.680 | 8.302 | 1.00 | 11.62 | A | C |
| ATOM | 1322 | O | VAL | A | 181 | 6.667 | 21.800 | 7.399 | 1.00 | 12.16 | A | O |
| ATOM | 1323 | N | GLY | A | 182 | 8.187 | 22.704 | 8.812 | 1.00 | 11.59 | A | N |
| ATOM | 1324 | CA | GLY | A | 182 | 8.095 | 24.042 | 8.273 | 1.00 | 12.80 | A | C |
| ATOM | 1325 | C | GLY | A | 182 | 9.296 | 24.352 | 7.391 | 1.00 | 13.72 | A | C |
| ATOM | 1326 | O | GLY | A | 182 | 10.243 | 23.574 | 7.344 | 1.00 | 14.13 | A | O |
| ATOM | 1327 | N | ALA | A | 183 | 9.264 | 25.492 | 6.700 | 1.00 | 13.43 | A | N |
| ATOM | 1328 | CA | ALA | A | 183 | 10.312 | 25.837 | 5.776 | 1.00 | 14.22 | A | C |
| ATOM | 1329 | CB | ALA | A | 183 | 9.709 | 26.166 | 4.401 | 1.00 | 14.53 | A | C |
| ATOM | 1330 | C | ALA | A | 183 | 11.205 | 27.001 | 6.238 | 1.00 | 14.32 | A | C |
| ATOM | 1331 | O | ALA | A | 183 | 10.717 | 28.110 | 6.498 | 1.00 | 14.13 | A | O |
| ATOM | 1332 | N | THR | A | 184 | 12.512 | 26.737 | 6.293 | 1.00 | 14.17 | A | N |
| ATOM | 1333 | CA | THR | A | 184 | 13.513 | 27.799 | 6.294 | 1.00 | 14.33 | A | C |
| ATOM | 1334 | CB | THR | A | 184 | 14.743 | 27.451 | 7.159 | 1.00 | 14.19 | A | C |
| ATOM | 1335 | OG1 | THR | A | 184 | 15.180 | 26.103 | 6.925 | 1.00 | 13.83 | A | O |
| ATOM | 1336 | CG2 | THR | A | 184 | 14.383 | 27.474 | 8.636 | 1.00 | 13.70 | A | C |
| ATOM | 1337 | C | THR | A | 184 | 13.905 | 28.018 | 4.841 | 1.00 | 15.47 | A | C |
| ATOM | 1338 | O | THR | A | 184 | 13.380 | 27.354 | 3.934 | 1.00 | 15.87 | A | O |
| ATOM | 1339 | N | GLU | A | 185 | 14.861 | 28.919 | 4.618 | 1.00 | 15.13 | A | N |
| ATOM | 1340 | CA | GLU | A | 185 | 15.328 | 29.246 | 3.290 | 1.00 | 14.03 | A | C |
| ATOM | 1341 | CB | GLU | A | 185 | 15.696 | 30.766 | 3.230 | 1.00 | 13.82 | A | C |
| ATOM | 1342 | CG | GLU | A | 185 | 14.492 | 31.673 | 3.495 | 1.00 | 15.09 | A | C |
| ATOM | 1343 | CD | GLU | A | 185 | 14.785 | 33.172 | 3.329 | 1.00 | 14.09 | A | C |
| ATOM | 1344 | OE1 | GLU | A | 185 | 15.911 | 33.541 | 2.985 | 1.00 | 15.60 | A | O |
| ATOM | 1345 | OE2 | GLU | A | 185 | 13.871 | 33.984 | 3.528 | 1.00 | 14.21 | A | O |
| ATOM | 1346 | C | GLU | A | 185 | 16.511 | 28.376 | 2.863 | 1.00 | 13.70 | A | C |
| ATOM | 1347 | O | GLU | A | 185 | 17.387 | 28.011 | 3.675 | 1.00 | 14.17 | A | O |
| ATOM | 1348 | N | ASN | A | 186 | 16.521 | 28.008 | 1.587 | 1.00 | 12.64 | A | N |
| ATOM | 1349 | CA | ASN | A | 186 | 17.707 | 27.452 | 0.959 | 1.00 | 13.44 | A | C |
| ATOM | 1350 | CB | ASN | A | 186 | 17.345 | 26.758 | -0.353 | 1.00 | 13.84 | A | C |
| ATOM | 1351 | CG | ASN | A | 186 | 18.293 | 25.630 | -0.717 | 1.00 | 14.82 | A | C |
| ATOM | 1352 | OD1 | ASN | A | 186 | 19.084 | 25.169 | 0.099 | 1.00 | 14.62 | A | O |
| ATOM | 1353 | ND2 | ASN | A | 186 | 18.189 | 25.156 | -1.970 | 1.00 | 15.51 | A | N |
| ATOM | 1354 | C | ASN | A | 186 | 18.652 | 28.603 | 0.681 | 1.00 | 13.83 | A | C |
| ATOM | 1355 | O | ASN | A | 186 | 18.244 | 29.769 | 0.711 | 1.00 | 14.50 | A | O |
| ATOM | 1356 | N | LEU | A | 187 | 19.920 | 28.298 | 0.470 | 1.00 | 14.83 | A | N |
| ATOM | 1357 | CA | LEU | A | 187 | 20.892 | 29.352 | 0.213 | 1.00 | 14.62 | A | C |
| ATOM | 1358 | CB | LEU | A | 187 | 22.144 | 29.144 | 1.018 | 1.00 | 15.48 | A | C |
| ATOM | 1359 | CG | LEU | A | 187 | 23.144 | 30.319 | 0.975 | 1.00 | 17.25 | A | C |
| ATOM | 1360 | CD1 | LEU | A | 187 | 22.504 | 31.587 | 1.469 | 1.00 | 18.56 | A | C |
| ATOM | 1361 | CD2 | LEU | A | 187 | 24.394 | 29.973 | 1.816 | 1.00 | 20.46 | A | C |
| ATOM | 1362 | C | LEU | A | 187 | 21.205 | 29.360 | -1.279 | 1.00 | 14.80 | A | C |
| ATOM | 1363 | O | LEU | A | 187 | 22.106 | 28.692 | -1.734 | 1.00 | 14.07 | A | O |
| ATOM | 1364 | N | ARG | A | 188 | 20.398 | 30.083 | -2.023 | 1.00 | 15.63 | A | N |

```
ATOM   1365  CA   ARG A 188      20.631  30.308  -3.454  1.00 17.55      A   C
ATOM   1366  CB   ARG A 188      19.658  29.484  -4.273  1.00 17.02      A   C
ATOM   1367  CG   ARG A 188      19.842  27.989  -4.168  1.00 17.82      A   C
ATOM   1368  CD   ARG A 188      19.063  27.213  -5.267  1.00 19.96      A   C
ATOM   1369  NE   ARG A 188      19.315  25.782  -5.224  1.00 18.26      A   N
ATOM   1370  CZ   ARG A 188      20.339  25.172  -5.814  1.00 19.52      A   C
ATOM   1371  NH1  ARG A 188      21.235  25.846  -6.530  1.00 17.91      A   N
ATOM   1372  NH2  ARG A 188      20.475  23.867  -5.693  1.00 19.41      A   N
ATOM   1373  C    ARG A 188      20.387  31.804  -3.694  1.00 17.89      A   C
ATOM   1374  O    ARG A 188      19.379  32.189  -4.251  1.00 18.33      A   O
ATOM   1375  N    PRO A 189      21.273  32.646  -3.181  1.00 19.58      A   N
ATOM   1376  CA   PRO A 189      20.990  34.082  -3.061  1.00 20.68      A   C
ATOM   1377  CB   PRO A 189      22.179  34.613  -2.239  1.00 21.07      A   C
ATOM   1378  CG   PRO A 189      23.271  33.608  -2.417  1.00 21.15      A   C
ATOM   1379  CD   PRO A 189      22.599  32.288  -2.657  1.00 20.12      A   C
ATOM   1380  C    PRO A 189      20.833  34.863  -4.373  1.00 21.39      A   C
ATOM   1381  O    PRO A 189      20.276  35.975  -4.347  1.00 20.51      A   O
ATOM   1382  N    SER A 190      21.285  34.307  -5.492  1.00 22.89      A   N
ATOM   1383  CA   SER A 190      21.033  34.940  -6.796  1.00 24.65      A   C
ATOM   1384  CB   SER A 190      21.685  34.135  -7.932  1.00 24.76      A   C
ATOM   1385  OG   SER A 190      21.082  32.831  -8.046  1.00 25.85      A   O
ATOM   1386  C    SER A 190      19.525  35.098  -7.028  1.00 25.23      A   C
ATOM   1387  O    SER A 190      19.080  35.918  -7.850  1.00 26.47      A   O
ATOM   1388  N    PHE A 191      18.723  34.365  -6.258  1.00 25.36      A   N
ATOM   1389  CA   PHE A 191      17.264  34.446  -6.389  1.00 25.28      A   C
ATOM   1390  CB   PHE A 191      16.643  33.046  -6.156  1.00 25.00      A   C
ATOM   1391  CG   PHE A 191      16.841  32.089  -7.310  1.00 23.34      A   C
ATOM   1392  CD1  PHE A 191      17.565  30.932  -7.159  1.00 21.81      A   C
ATOM   1393  CE1  PHE A 191      17.735  30.052  -8.218  1.00 22.63      A   C
ATOM   1394  CZ   PHE A 191      17.180  30.341  -9.470  1.00 21.46      A   C
ATOM   1395  CE2  PHE A 191      16.449  31.484  -9.631  1.00 22.61      A   C
ATOM   1396  CD2  PHE A 191      16.288  32.361  -8.562  1.00 25.47      A   C
ATOM   1397  C    PHE A 191      16.388  35.561  -5.720  1.00 25.71      A   C
ATOM   1398  O    PHE A 191      15.184  35.500  -5.877  1.00 27.33      A   O
ATOM   1399  N    GLY A 192      16.823  36.552  -4.944  1.00 26.98      A   N
ATOM   1400  CA   GLY A 192      17.639  36.484  -3.783  1.00 26.29      A   C
ATOM   1401  C    GLY A 192      16.795  36.445  -2.478  1.00 25.37      A   C
ATOM   1402  O    GLY A 192      17.008  35.528  -1.733  1.00 25.24      A   O
ATOM   1403  N    SER A 193      15.858  37.355  -2.179  1.00 24.49      A   N
ATOM   1404  CA   SER A 193      15.332  37.444  -0.778  1.00 24.39      A   C
ATOM   1405  CB   SER A 193      14.452  38.689  -0.554  1.00 24.51      A   C
ATOM   1406  OG   SER A 193      13.058  38.407  -0.623  1.00 25.19      A   O
ATOM   1407  C    SER A 193      14.664  36.176  -0.133  1.00 23.94      A   C
ATOM   1408  O    SER A 193      14.740  35.973   1.085  1.00 22.27      A   O
ATOM   1409  N    TYR A 194      14.037  35.331  -0.949  1.00 23.39      A   N
ATOM   1410  CA   TYR A 194      13.497  34.046  -0.477  1.00 23.08      A   C
ATOM   1411  CB   TYR A 194      12.407  33.559  -1.439  1.00 23.87      A   C
ATOM   1412  CG   TYR A 194      11.044  34.129  -1.144  1.00 27.80      A   C
ATOM   1413  CD1  TYR A 194      10.563  35.240  -1.832  1.00 31.12      A   C
ATOM   1414  CE1  TYR A 194       9.317  35.775  -1.554  1.00 32.91      A   C
ATOM   1415  CZ   TYR A 194       8.525  35.182  -0.591  1.00 34.12      A   C
ATOM   1416  OH   TYR A 194       7.282  35.696  -0.311  1.00 38.22      A   O
ATOM   1417  CE2  TYR A 194       8.974  34.076   0.108  1.00 33.28      A   C
ATOM   1418  CD2  TYR A 194      10.229  33.556  -0.169  1.00 31.26      A   C
ATOM   1419  C    TYR A 194      14.545  32.930  -0.289  1.00 21.59      A   C
ATOM   1420  O    TYR A 194      14.225  31.848   0.236  1.00 20.33      A   O
ATOM   1421  N    ALA A 195      15.785  33.185  -0.695  1.00 20.44      A   N
ATOM   1422  CA   ALA A 195      16.838  32.181  -0.610  1.00 20.47      A   C
ATOM   1423  CB   ALA A 195      16.915  31.365  -1.892  1.00 20.41      A   C
ATOM   1424  C    ALA A 195      18.222  32.757  -0.270  1.00 19.93      A   C
ATOM   1425  O    ALA A 195      19.230  32.354  -0.877  1.00 19.21      A   O
ATOM   1426  N    ASP A 196      18.264  33.615   0.750  1.00 19.15      A   N
ATOM   1427  CA   ASP A 196      19.472  34.355   1.126  1.00 19.86      A   C
```

| ATOM | 1428 | CB | ASP A 196 | 19.264 | 35.861 | 0.919 | 1.00 | 20.04 | A | C |
|------|------|-----|-----------|--------|--------|-------|------|-------|---|---|
| ATOM | 1429 | CG | ASP A 196 | 18.198 | 36.453 | 1.814 | 1.00 | 22.11 | A | C |
| ATOM | 1430 | OD1 | ASP A 196 | 18.040 | 37.693 | 1.696 | 1.00 | 23.87 | A | O |
| ATOM | 1431 | OD2 | ASP A 196 | 17.461 | 35.822 | 2.649 | 1.00 | 19.55 | A | O |
| ATOM | 1432 | C | ASP A 196 | 20.025 | 34.163 | 2.549 | 1.00 | 19.90 | A | C |
| ATOM | 1433 | O | ASP A 196 | 21.092 | 34.705 | 2.869 | 1.00 | 19.61 | A | O |
| ATOM | 1434 | N | ASN A 197 | 19.326 | 33.410 | 3.394 | 1.00 | 18.85 | A | N |
| ATOM | 1435 | CA | ASN A 197 | 19.790 | 33.177 | 4.757 | 1.00 | 18.29 | A | C |
| ATOM | 1436 | CB | ASN A 197 | 19.410 | 34.369 | 5.644 | 1.00 | 18.68 | A | C |
| ATOM | 1437 | CG | ASN A 197 | 20.123 | 34.360 | 7.001 | 1.00 | 19.48 | A | C |
| ATOM | 1438 | OD1 | ASN A 197 | 20.221 | 33.319 | 7.630 | 1.00 | 16.60 | A | O |
| ATOM | 1439 | ND2 | ASN A 197 | 20.603 | 35.541 | 7.455 | 1.00 | 14.55 | A | N |
| ATOM | 1440 | C | ASN A 197 | 19.198 | 31.861 | 5.304 | 1.00 | 17.30 | A | C |
| ATOM | 1441 | O | ASN A 197 | 17.986 | 31.734 | 5.463 | 1.00 | 16.96 | A | O |
| ATOM | 1442 | N | ILE A 198 | 20.066 | 30.901 | 5.608 | 1.00 | 16.67 | A | N |
| ATOM | 1443 | CA | ILE A 198 | 19.606 | 29.557 | 5.993 | 1.00 | 16.04 | A | C |
| ATOM | 1444 | CB | ILE A 198 | 20.771 | 28.571 | 6.020 | 1.00 | 15.64 | A | C |
| ATOM | 1445 | CG1 | ILE A 198 | 21.724 | 28.885 | 7.179 | 1.00 | 16.27 | A | C |
| ATOM | 1446 | CD1 | ILE A 198 | 22.734 | 27.781 | 7.490 | 1.00 | 18.08 | A | C |
| ATOM | 1447 | CG2 | ILE A 198 | 21.459 | 28.530 | 4.679 | 1.00 | 16.50 | A | C |
| ATOM | 1448 | C | ILE A 198 | 18.897 | 29.560 | 7.352 | 1.00 | 15.03 | A | C |
| ATOM | 1449 | O | ILE A 198 | 18.222 | 28.605 | 7.723 | 1.00 | 15.12 | A | O |
| ATOM | 1450 | N | ASN A 199 | 19.054 | 30.642 | 8.102 | 1.00 | 14.78 | A | N |
| ATOM | 1451 | CA | ASN A 199 | 18.316 | 30.794 | 9.344 | 1.00 | 14.68 | A | C |
| ATOM | 1452 | CB | ASN A 199 | 19.180 | 31.609 | 10.332 | 1.00 | 14.98 | A | C |
| ATOM | 1453 | CG | ASN A 199 | 20.487 | 30.948 | 10.708 | 1.00 | 15.69 | A | C |
| ATOM | 1454 | OD1 | ASN A 199 | 20.560 | 29.757 | 10.907 | 1.00 | 14.98 | A | O |
| ATOM | 1455 | ND2 | ASN A 199 | 21.526 | 31.768 | 10.903 | 1.00 | 20.04 | A | N |
| ATOM | 1456 | C | ASN A 199 | 17.036 | 31.597 | 9.311 | 1.00 | 14.49 | A | C |
| ATOM | 1457 | O | ASN A 199 | 16.239 | 31.490 | 10.227 | 1.00 | 14.69 | A | O |
| ATOM | 1458 | N | HIS A 200 | 16.736 | 32.328 | 8.241 | 1.00 | 15.73 | A | N |
| ATOM | 1459 | CA | HIS A 200 | 15.375 | 32.648 | 7.854 | 1.00 | 14.99 | A | C |
| ATOM | 1460 | CB | HIS A 200 | 15.338 | 33.612 | 6.641 | 1.00 | 15.17 | A | C |
| ATOM | 1461 | CG | HIS A 200 | 16.005 | 34.942 | 6.871 | 1.00 | 17.24 | A | C |
| ATOM | 1462 | ND1 | HIS A 200 | 16.242 | 35.840 | 5.842 | 1.00 | 16.94 | A | N |
| ATOM | 1463 | CE1 | HIS A 200 | 16.842 | 36.916 | 6.327 | 1.00 | 19.73 | A | C |
| ATOM | 1464 | NE2 | HIS A 200 | 17.009 | 36.751 | 7.628 | 1.00 | 17.79 | A | N |
| ATOM | 1465 | CD2 | HIS A 200 | 16.469 | 35.538 | 7.999 | 1.00 | 18.37 | A | C |
| ATOM | 1466 | C | HIS A 200 | 14.327 | 31.581 | 7.730 | 1.00 | 15.01 | A | C |
| ATOM | 1467 | O | HIS A 200 | 14.472 | 30.673 | 6.965 | 1.00 | 14.41 | A | O |
| ATOM | 1468 | N | VAL A 201 | 13.251 | 31.772 | 8.496 | 1.00 | 15.72 | A | N |
| ATOM | 1469 | CA | VAL A 201 | 12.004 | 31.059 | 8.294 | 1.00 | 16.37 | A | C |
| ATOM | 1470 | CB | VAL A 201 | 11.103 | 31.185 | 9.523 | 1.00 | 16.41 | A | C |
| ATOM | 1471 | CG1 | VAL A 201 | 9.780 | 30.428 | 9.297 | 1.00 | 16.12 | A | C |
| ATOM | 1472 | CG2 | VAL A 201 | 11.841 | 30.668 | 10.783 | 1.00 | 18.01 | A | C |
| ATOM | 1473 | C | VAL A 201 | 11.313 | 31.683 | 7.089 | 1.00 | 16.94 | A | C |
| ATOM | 1474 | O | VAL A 201 | 11.250 | 32.900 | 6.973 | 1.00 | 17.09 | A | O |
| ATOM | 1475 | N | ALA A 202 | 10.872 | 30.865 | 6.143 | 1.00 | 17.70 | A | N |
| ATOM | 1476 | CA | ALA A 202 | 10.233 | 31.396 | 4.949 | 1.00 | 17.52 | A | C |
| ATOM | 1477 | CB | ALA A 202 | 9.859 | 30.265 | 4.018 | 1.00 | 18.76 | A | C |
| ATOM | 1478 | C | ALA A 202 | 9.000 | 32.169 | 5.383 | 1.00 | 17.84 | A | C |
| ATOM | 1479 | O | ALA A 202 | 8.263 | 31.734 | 6.263 | 1.00 | 16.60 | A | O |
| ATOM | 1480 | N | GLN A 203 | 8.770 | 33.332 | 4.783 | 1.00 | 18.20 | A | N |
| ATOM | 1481 | CA | GLN A 203 | 7.629 | 34.135 | 5.192 | 1.00 | 19.45 | A | C |
| ATOM | 1482 | CB | BGLN A 203 | 7.542 | 35.347 | 4.260 | 0.40 | 19.94 | A | C |
| ATOM | 1483 | CB | AGLN A 203 | 7.529 | 35.467 | 4.445 | 0.60 | 20.39 | A | C |
| ATOM | 1484 | CG | BGLN A 203 | 7.527 | 36.681 | 4.943 | 0.40 | 22.36 | A | C |
| ATOM | 1485 | CG | AGLN A 203 | 6.748 | 36.514 | 5.261 | 0.60 | 24.58 | A | C |
| ATOM | 1486 | CD | BGLN A 203 | 6.379 | 37.556 | 4.452 | 0.40 | 25.52 | A | C |
| ATOM | 1487 | CD | AGLN A 203 | 7.553 | 37.090 | 6.439 | 0.60 | 28.39 | A | C |
| ATOM | 1488 | OE1 | BGLN A 203 | 5.568 | 37.122 | 3.624 | 0.40 | 27.97 | A | O |
| ATOM | 1489 | OE1 | AGLN A 203 | 8.525 | 37.816 | 6.236 | 0.60 | 33.69 | A | O |
| ATOM | 1490 | NE2 | BGLN A 203 | 6.299 | 38.772 | 4.972 | 0.40 | 24.61 | A | N |

55

| ATOM | 1491 | NE2A | GLN | A | 203 | 7.155 | 36.751 | 7.655 | 0.60 | 31.16 | A | N |
| ATOM | 1492 | C | GLN | A | 203 | 6.291 | 33.391 | 5.152 | 1.00 | 18.58 | A | C |
| ATOM | 1493 | O | GLN | A | 203 | 5.458 | 33.580 | 6.028 | 1.00 | 18.67 | A | O |
| ATOM | 1494 | N | PHE | A | 204 | 6.090 | 32.533 | 4.163 | 1.00 | 17.22 | A | N |
| ATOM | 1495 | CA | PHE | A | 204 | 4.805 | 31.833 | 4.027 | 1.00 | 16.75 | A | C |
| ATOM | 1496 | CB | PHE | A | 204 | 4.603 | 31.335 | 2.589 | 1.00 | 16.25 | A | C |
| ATOM | 1497 | CG | PHE | A | 204 | 5.720 | 30.475 | 2.093 | 1.00 | 16.52 | A | C |
| ATOM | 1498 | CD1 | PHE | A | 204 | 5.857 | 29.158 | 2.526 | 1.00 | 16.66 | A | C |
| ATOM | 1499 | CE1 | PHE | A | 204 | 6.893 | 28.378 | 2.083 | 1.00 | 17.13 | A | C |
| ATOM | 1500 | CZ | PHE | A | 204 | 7.854 | 28.909 | 1.234 | 1.00 | 14.67 | A | C |
| ATOM | 1501 | CE2 | PHE | A | 204 | 7.747 | 30.231 | 0.816 | 1.00 | 16.10 | A | C |
| ATOM | 1502 | CD2 | PHE | A | 204 | 6.693 | 31.009 | 1.267 | 1.00 | 15.39 | A | C |
| ATOM | 1503 | C | PHE | A | 204 | 4.670 | 30.647 | 5.018 | 1.00 | 16.21 | A | C |
| ATOM | 1504 | O | PHE | A | 204 | 3.570 | 30.150 | 5.198 | 1.00 | 15.42 | A | O |
| ATOM | 1505 | N | SER | A | 205 | 5.754 | 30.223 | 5.688 | 1.00 | 15.36 | A | N |
| ATOM | 1506 | CA | SER | A | 205 | 5.692 | 28.983 | 6.508 | 1.00 | 15.18 | A | C |
| ATOM | 1507 | CB | SER | A | 205 | 7.068 | 28.579 | 7.063 | 1.00 | 15.27 | A | C |
| ATOM | 1508 | OG | SER | A | 205 | 7.042 | 27.254 | 7.585 | 1.00 | 15.10 | A | O |
| ATOM | 1509 | C | SER | A | 205 | 4.657 | 29.103 | 7.615 | 1.00 | 14.84 | A | C |
| ATOM | 1510 | O | SER | A | 205 | 4.618 | 30.092 | 8.319 | 1.00 | 16.26 | A | O |
| ATOM | 1511 | N | SER | A | 206 | 3.764 | 28.141 | 7.753 | 1.00 | 15.82 | A | N |
| ATOM | 1512 | CA | SER | A | 206 | 2.751 | 28.237 | 8.818 | 1.00 | 15.73 | A | C |
| ATOM | 1513 | CB | SER | A | 206 | 1.714 | 27.117 | 8.735 | 1.00 | 16.10 | A | C |
| ATOM | 1514 | OG | SER | A | 206 | 0.811 | 27.350 | 7.655 | 1.00 | 14.83 | A | O |
| ATOM | 1515 | C | SER | A | 206 | 3.421 | 28.221 | 10.186 | 1.00 | 15.92 | A | C |
| ATOM | 1516 | O | SER | A | 206 | 4.486 | 27.589 | 10.362 | 1.00 | 15.30 | A | O |
| ATOM | 1517 | N | ARG | A | 207 | 2.786 | 28.928 | 11.113 | 1.00 | 15.72 | A | N |
| ATOM | 1518 | CA | ARG | A | 207 | 3.289 | 29.140 | 12.455 | 1.00 | 16.34 | A | C |
| ATOM | 1519 | CB | ARG | A | 207 | 3.511 | 30.636 | 12.715 | 1.00 | 16.50 | A | C |
| ATOM | 1520 | CG | ARG | A | 207 | 4.189 | 31.375 | 11.542 | 1.00 | 18.52 | A | C |
| ATOM | 1521 | CD | ARG | A | 207 | 5.604 | 30.916 | 11.227 | 1.00 | 20.29 | A | C |
| ATOM | 1522 | NE | ARG | A | 207 | 6.146 | 31.519 | 10.012 | 1.00 | 22.13 | A | N |
| ATOM | 1523 | CZ | ARG | A | 207 | 6.821 | 32.643 | 9.981 | 1.00 | 22.20 | A | C |
| ATOM | 1524 | NH1 | ARG | A | 207 | 7.056 | 33.294 | 11.080 | 1.00 | 25.41 | A | N |
| ATOM | 1525 | NH2 | ARG | A | 207 | 7.256 | 33.124 | 8.838 | 1.00 | 24.08 | A | N |
| ATOM | 1526 | C | ARG | A | 207 | 2.330 | 28.556 | 13.471 | 1.00 | 15.97 | A | C |
| ATOM | 1527 | O | ARG | A | 207 | 1.096 | 28.605 | 13.301 | 1.00 | 15.01 | A | O |
| ATOM | 1528 | N | GLY | A | 208 | 2.903 | 27.974 | 14.521 | 1.00 | 15.16 | A | N |
| ATOM | 1529 | CA | GLY | A | 208 | 2.139 | 27.523 | 15.655 | 1.00 | 16.14 | A | C |
| ATOM | 1530 | C | GLY | A | 208 | 1.622 | 28.688 | 16.476 | 1.00 | 16.50 | A | C |
| ATOM | 1531 | O | GLY | A | 208 | 1.753 | 29.830 | 16.059 | 1.00 | 17.36 | A | O |
| ATOM | 1532 | N | PRO | A | 209 | 0.999 | 28.423 | 17.617 | 1.00 | 17.42 | A | N |
| ATOM | 1533 | CA | PRO | A | 209 | 0.727 | 27.067 | 18.089 | 1.00 | 17.56 | A | C |
| ATOM | 1534 | CB | PRO | A | 209 | 0.407 | 27.269 | 19.579 | 1.00 | 17.58 | A | C |
| ATOM | 1535 | CG | PRO | A | 209 | -0.088 | 28.644 | 19.701 | 1.00 | 18.85 | A | C |
| ATOM | 1536 | CD | PRO | A | 209 | 0.477 | 29.457 | 18.545 | 1.00 | 17.85 | A | C |
| ATOM | 1537 | C | PRO | A | 209 | -0.483 | 26.483 | 17.368 | 1.00 | 17.50 | A | C |
| ATOM | 1538 | O | PRO | A | 209 | -1.094 | 27.157 | 16.558 | 1.00 | 18.18 | A | O |
| ATOM | 1539 | N | THR | A | 210 | -0.816 | 25.240 | 17.652 | 1.00 | 16.70 | A | N |
| ATOM | 1540 | CA | THR | A | 210 | -2.050 | 24.690 | 17.186 | 1.00 | 17.34 | A | C |
| ATOM | 1541 | CB | THR | A | 210 | -2.042 | 23.181 | 17.356 | 1.00 | 16.73 | A | C |
| ATOM | 1542 | OG1 | THR | A | 210 | -1.848 | 22.859 | 18.734 | 1.00 | 18.34 | A | O |
| ATOM | 1543 | CG2 | THR | A | 210 | -0.833 | 22.540 | 16.574 | 1.00 | 16.90 | A | C |
| ATOM | 1544 | C | THR | A | 210 | -3.206 | 25.327 | 17.987 | 1.00 | 17.55 | A | C |
| ATOM | 1545 | O | THR | A | 210 | -2.990 | 26.095 | 18.930 | 1.00 | 16.40 | A | O |
| ATOM | 1546 | N | ARG | A | 211 | -4.421 | 24.979 | 17.623 | 1.00 | 18.89 | A | N |
| ATOM | 1547 | CA | ARG | A | 211 | -5.595 | 25.577 | 18.264 | 1.00 | 20.56 | A | C |
| ATOM | 1548 | CB | ARG | A | 211 | -6.884 | 25.056 | 17.638 | 1.00 | 21.16 | A | C |
| ATOM | 1549 | CG | ARG | A | 211 | -8.149 | 25.719 | 18.255 | 1.00 | 25.92 | A | C |
| ATOM | 1550 | CD | ARG | A | 211 | -9.325 | 25.804 | 17.301 | 1.00 | 31.08 | A | C |
| ATOM | 1551 | NE | ARG | A | 211 | -8.956 | 26.457 | 16.042 | 1.00 | 35.63 | A | N |
| ATOM | 1552 | CZ | ARG | A | 211 | -9.626 | 26.296 | 14.905 | 1.00 | 38.91 | A | C |
| ATOM | 1553 | NH1 | ARG | A | 211 | -10.707 | 25.516 | 14.876 | 1.00 | 40.64 | A | N |

| | | | | | | | | | | | |
|------|------|-----|-----|---|-----|---------|--------|--------|------|-------|---|---|
| ATOM | 1554 | NH2 | ARG | A | 211 | -9.225  | 26.911 | 13.795 | 1.00 | 37.74 | A | N |
| ATOM | 1555 | C   | ARG | A | 211 | -5.591  | 25.308 | 19.768 | 1.00 | 20.16 | A | C |
| ATOM | 1556 | O   | ARG | A | 211 | -5.983  | 26.180 | 20.539 | 1.00 | 19.93 | A | O |
| ATOM | 1557 | N   | ASP | A | 212 | -5.120  | 24.121 | 20.185 | 1.00 | 19.40 | A | N |
| ATOM | 1558 | CA  | ASP | A | 212 | -5.031  | 23.791 | 21.616 | 1.00 | 18.64 | A | C |
| ATOM | 1559 | CB  | ASP | A | 212 | -5.346  | 22.306 | 21.877 | 1.00 | 18.58 | A | C |
| ATOM | 1560 | CG  | ASP | A | 212 | -4.318  | 21.356 | 21.254 | 1.00 | 16.59 | A | C |
| ATOM | 1561 | OD1 | ASP | A | 212 | -4.255  | 20.180 | 21.679 | 1.00 | 16.40 | A | O |
| ATOM | 1562 | OD2 | ASP | A | 212 | -3.545  | 21.688 | 20.339 | 1.00 | 17.56 | A | O |
| ATOM | 1563 | C   | ASP | A | 212 | -3.693  | 24.160 | 22.255 | 1.00 | 18.83 | A | C |
| ATOM | 1564 | O   | ASP | A | 212 | -3.387  | 23.707 | 23.370 | 1.00 | 19.22 | A | O |
| ATOM | 1565 | N   | GLY | A | 213 | -2.902  | 24.966 | 21.556 | 1.00 | 18.29 | A | N |
| ATOM | 1566 | CA  | GLY | A | 213 | -1.698  | 25.572 | 22.111 | 1.00 | 18.10 | A | C |
| ATOM | 1567 | C   | GLY | A | 213 | -0.439  | 24.713 | 22.065 | 1.00 | 17.75 | A | C |
| ATOM | 1568 | O   | GLY | A | 213 | 0.517   | 24.998 | 22.785 | 1.00 | 18.06 | A | O |
| ATOM | 1569 | N   | ARG | A | 214 | -0.431  | 23.665 | 21.242 | 1.00 | 16.43 | A | N |
| ATOM | 1570 | CA  | ARG | A | 214 | 0.757   | 22.826 | 21.110 | 1.00 | 16.72 | A | C |
| ATOM | 1571 | CB  | ARG | A | 214 | 0.403   | 21.461 | 20.536 | 1.00 | 16.17 | A | C |
| ATOM | 1572 | CG  | ARG | A | 214 | -0.276  | 20.553 | 21.473 | 1.00 | 16.40 | A | C |
| ATOM | 1573 | CD  | ARG | A | 214 | -0.753  | 19.301 | 20.814 | 1.00 | 16.61 | A | C |
| ATOM | 1574 | NE  | ARG | A | 214 | -1.771  | 19.613 | 19.826 | 1.00 | 16.67 | A | N |
| ATOM | 1575 | CZ  | ARG | A | 214 | -1.740  | 19.297 | 18.531 | 1.00 | 16.71 | A | C |
| ATOM | 1576 | NH1 | ARG | A | 214 | -0.720  | 18.628 | 17.981 | 1.00 | 16.55 | A | N |
| ATOM | 1577 | NH2 | ARG | A | 214 | -2.762  | 19.664 | 17.776 | 1.00 | 14.42 | A | N |
| ATOM | 1578 | C   | ARG | A | 214 | 1.772   | 23.493 | 20.203 | 1.00 | 16.16 | A | C |
| ATOM | 1579 | O   | ARG | A | 214 | 1.403   | 24.306 | 19.344 | 1.00 | 16.86 | A | O |
| ATOM | 1580 | N   | ILE | A | 215 | 3.046   | 23.168 | 20.396 | 1.00 | 15.82 | A | N |
| ATOM | 1581 | CA  | ILE | A | 215 | 4.107   | 23.640 | 19.516 | 1.00 | 15.40 | A | C |
| ATOM | 1582 | CB  | ILE | A | 215 | 5.503   | 23.498 | 20.175 | 1.00 | 16.18 | A | C |
| ATOM | 1583 | CG1 | ILE | A | 215 | 5.600   | 24.351 | 21.454 | 1.00 | 17.35 | A | C |
| ATOM | 1584 | CD1 | ILE | A | 215 | 5.526   | 25.842 | 21.181 | 1.00 | 20.01 | A | C |
| ATOM | 1585 | CG2 | ILE | A | 215 | 6.606   | 23.898 | 19.191 | 1.00 | 15.87 | A | C |
| ATOM | 1586 | C   | ILE | A | 215 | 4.100   | 22.834 | 18.214 | 1.00 | 15.40 | A | C |
| ATOM | 1587 | O   | ILE | A | 215 | 4.316   | 21.616 | 18.227 | 1.00 | 14.96 | A | O |
| ATOM | 1588 | N   | LYS | A | 216 | 3.841   | 23.536 | 17.117 | 1.00 | 14.61 | A | N |
| ATOM | 1589 | CA  | LYS | A | 216 | 4.072   | 23.062 | 15.745 | 1.00 | 14.64 | A | C |
| ATOM | 1590 | CB  | LYS | A | 216 | 2.765   | 22.616 | 15.067 | 1.00 | 13.98 | A | C |
| ATOM | 1591 | CG  | LYS | A | 216 | 2.190   | 21.271 | 15.526 | 1.00 | 13.46 | A | C |
| ATOM | 1592 | CD  | LYS | A | 216 | 3.073   | 20.102 | 15.117 | 1.00 | 14.36 | A | C |
| ATOM | 1593 | CE  | LYS | A | 216 | 2.427   | 18.754 | 15.453 | 1.00 | 13.24 | A | C |
| ATOM | 1594 | NZ  | LYS | A | 216 | 3.042   | 17.577 | 14.739 | 1.00 | 8.08  | A | N |
| ATOM | 1595 | C   | LYS | A | 216 | 4.632   | 24.269 | 14.984 | 1.00 | 14.70 | A | C |
| ATOM | 1596 | O   | LYS | A | 216 | 4.336   | 25.428 | 15.358 | 1.00 | 13.92 | A | O |
| ATOM | 1597 | N   | PRO | A | 217 | 5.410   | 24.032 | 13.921 | 1.00 | 14.59 | A | N |
| ATOM | 1598 | CA  | PRO | A | 217 | 5.788   | 22.691 | 13.468 | 1.00 | 13.65 | A | C |
| ATOM | 1599 | CB  | PRO | A | 217 | 6.452   | 22.944 | 12.115 | 1.00 | 14.58 | A | C |
| ATOM | 1600 | CG  | PRO | A | 217 | 6.934   | 24.356 | 12.178 | 1.00 | 15.23 | A | C |
| ATOM | 1601 | CD  | PRO | A | 217 | 6.012   | 25.077 | 13.086 | 1.00 | 14.44 | A | C |
| ATOM | 1602 | C   | PRO | A | 217 | 6.818   | 22.089 | 14.401 | 1.00 | 12.99 | A | C |
| ATOM | 1603 | O   | PRO | A | 217 | 7.262   | 22.738 | 15.379 | 1.00 | 12.11 | A | O |
| ATOM | 1604 | N   | ASP | A | 218 | 7.201   | 20.847 | 14.126 | 1.00 | 11.74 | A | N |
| ATOM | 1605 | CA  | ASP | A | 218 | 8.188   | 20.214 | 14.974 | 1.00 | 11.35 | A | C |
| ATOM | 1606 | CB  | ASP | A | 218 | 8.033   | 18.694 | 14.962 | 1.00 | 11.47 | A | C |
| ATOM | 1607 | CG  | ASP | A | 218 | 6.672   | 18.241 | 15.451 | 1.00 | 11.82 | A | C |
| ATOM | 1608 | OD1 | ASP | A | 218 | 6.440   | 18.370 | 16.680 | 1.00 | 10.57 | A | O |
| ATOM | 1609 | OD2 | ASP | A | 218 | 5.810   | 17.726 | 14.671 | 1.00 | 11.50 | A | O |
| ATOM | 1610 | C   | ASP | A | 218 | 9.619   | 20.566 | 14.610 | 1.00 | 11.30 | A | C |
| ATOM | 1611 | O   | ASP | A | 218 | 10.441  | 20.772 | 15.501 | 1.00 | 10.85 | A | O |
| ATOM | 1612 | N   | VAL | A | 219 | 9.928   | 20.516 | 13.314 | 1.00 | 11.61 | A | N |
| ATOM | 1613 | CA  | VAL | A | 219 | 11.254  | 20.829 | 12.815 | 1.00 | 12.37 | A | C |
| ATOM | 1614 | CB  | VAL | A | 219 | 12.118  | 19.589 | 12.602 | 1.00 | 12.19 | A | C |
| ATOM | 1615 | CG1 | VAL | A | 219 | 12.401  | 18.867 | 13.933 | 1.00 | 14.24 | A | C |
| ATOM | 1616 | CG2 | VAL | A | 219 | 11.485  | 18.660 | 11.587 | 1.00 | 13.54 | A | C |

| | | | | | | | | | | | | |
|---|---|---|---|---|---|---|---|---|---|---|---|---|
| ATOM | 1617 | C | VAL | A | 219 | 11.148 | 21.568 | 11.471 | 1.00 | 12.33 | A | C |
| ATOM | 1618 | O | VAL | A | 219 | 10.083 | 21.624 | 10.851 | 1.00 | 12.34 | A | O |
| ATOM | 1619 | N | MET | A | 220 | 12.266 | 22.139 | 11.057 | 1.00 | 11.78 | A | N |
| ATOM | 1620 | CA | MET | A | 220 | 12.365 | 22.930 | 9.852 | 1.00 | 11.89 | A | C |
| ATOM | 1621 | CB | MET | A | 220 | 12.798 | 24.371 | 10.167 | 1.00 | 11.30 | A | C |
| ATOM | 1622 | CG | MET | A | 220 | 12.025 | 25.058 | 11.255 | 1.00 | 11.64 | A | C |
| ATOM | 1623 | SD | MET | A | 220 | 10.310 | 25.322 | 10.860 | 1.00 | 12.02 | A | S |
| ATOM | 1624 | CE | MET | A | 220 | 10.416 | 26.727 | 9.791 | 1.00 | 11.35 | A | C |
| ATOM | 1625 | C | MET | A | 220 | 13.398 | 22.343 | 8.902 | 1.00 | 12.21 | A | C |
| ATOM | 1626 | O | MET | A | 220 | 14.368 | 21.731 | 9.321 | 1.00 | 12.24 | A | O |
| ATOM | 1627 | N | ALA | A | 221 | 13.175 | 22.556 | 7.613 | 1.00 | 12.60 | A | N |
| ATOM | 1628 | CA | ALA | A | 221 | 14.198 | 22.324 | 6.605 | 1.00 | 12.90 | A | C |
| ATOM | 1629 | CB | ALA | A | 221 | 14.098 | 20.912 | 6.081 | 1.00 | 12.23 | A | C |
| ATOM | 1630 | C | ALA | A | 221 | 14.064 | 23.341 | 5.464 | 1.00 | 13.77 | A | C |
| ATOM | 1631 | O | ALA | A | 221 | 13.029 | 24.027 | 5.312 | 1.00 | 14.46 | A | O |
| ATOM | 1632 | N | PRO | A | 222 | 15.116 | 23.487 | 4.687 | 1.00 | 13.99 | A | N |
| ATOM | 1633 | CA | PRO | A | 222 | 15.059 | 24.393 | 3.543 | 1.00 | 14.73 | A | C |
| ATOM | 1634 | CB | PRO | A | 222 | 16.387 | 24.159 | 2.845 | 1.00 | 13.69 | A | C |
| ATOM | 1635 | CG | PRO | A | 222 | 17.290 | 23.676 | 3.892 | 1.00 | 15.06 | A | C |
| ATOM | 1636 | CD | PRO | A | 222 | 16.433 | 22.855 | 4.830 | 1.00 | 14.39 | A | C |
| ATOM | 1637 | C | PRO | A | 222 | 13.896 | 24.044 | 2.622 | 1.00 | 14.82 | A | C |
| ATOM | 1638 | O | PRO | A | 222 | 13.719 | 22.847 | 2.284 | 1.00 | 15.21 | A | O |
| ATOM | 1639 | N | GLY | A | 223 | 13.178 | 25.069 | 2.193 | 1.00 | 14.57 | A | N |
| ATOM | 1640 | CA | GLY | A | 223 | 11.996 | 24.910 | 1.373 | 1.00 | 15.06 | A | C |
| ATOM | 1641 | C | GLY | A | 223 | 11.779 | 26.046 | 0.383 | 1.00 | 14.81 | A | C |
| ATOM | 1642 | O | GLY | A | 223 | 10.661 | 26.268 | -0.039 | 1.00 | 15.67 | A | O |
| ATOM | 1643 | N | THR | A | 224 | 12.822 | 26.799 | 0.049 | 1.00 | 13.98 | A | N |
| ATOM | 1644 | CA | THR | A | 224 | 12.706 | 27.772 | -1.007 | 1.00 | 14.01 | A | C |
| ATOM | 1645 | CB | THR | A | 224 | 12.912 | 29.229 | -0.517 | 1.00 | 13.98 | A | C |
| ATOM | 1646 | OG1 | THR | A | 224 | 14.220 | 29.350 | 0.047 | 1.00 | 13.39 | A | O |
| ATOM | 1647 | CG2 | THR | A | 224 | 11.952 | 29.585 | 0.597 | 1.00 | 14.59 | A | C |
| ATOM | 1648 | C | THR | A | 224 | 13.729 | 27.449 | -2.072 | 1.00 | 14.02 | A | C |
| ATOM | 1649 | O | THR | A | 224 | 14.813 | 26.932 | -1.791 | 1.00 | 14.13 | A | O |
| ATOM | 1650 | N | TYR | A | 225 | 13.389 | 27.786 | -3.308 | 1.00 | 14.73 | A | N |
| ATOM | 1651 | CA | TYR | A | 225 | 14.270 | 27.528 | -4.441 | 1.00 | 14.78 | A | C |
| ATOM | 1652 | CB | TYR | A | 225 | 15.197 | 28.726 | -4.686 | 1.00 | 15.26 | A | C |
| ATOM | 1653 | CG | TYR | A | 225 | 14.502 | 29.848 | -5.398 | 1.00 | 15.90 | A | C |
| ATOM | 1654 | CD1 | TYR | A | 225 | 14.027 | 30.940 | -4.692 | 1.00 | 18.12 | A | C |
| ATOM | 1655 | CE1 | TYR | A | 225 | 13.349 | 31.960 | -5.301 | 1.00 | 18.57 | A | C |
| ATOM | 1656 | CZ | TYR | A | 225 | 13.100 | 31.918 | -6.659 | 1.00 | 19.46 | A | C |
| ATOM | 1657 | OH | TYR | A | 225 | 12.391 | 32.974 | -7.207 | 1.00 | 20.70 | A | O |
| ATOM | 1658 | CE2 | TYR | A | 225 | 13.510 | 30.844 | -7.404 | 1.00 | 18.30 | A | C |
| ATOM | 1659 | CD2 | TYR | A | 225 | 14.225 | 29.788 | -6.771 | 1.00 | 19.15 | A | C |
| ATOM | 1660 | C | TYR | A | 225 | 15.022 | 26.196 | -4.331 | 1.00 | 14.79 | A | C |
| ATOM | 1661 | O | TYR | A | 225 | 16.252 | 26.119 | -4.395 | 1.00 | 15.52 | A | O |
| ATOM | 1662 | N | ILE | A | 226 | 14.248 | 25.130 | -4.186 | 1.00 | 15.07 | A | N |
| ATOM | 1663 | CA | ILE | A | 226 | 14.773 | 23.759 | -4.155 | 1.00 | 14.45 | A | C |
| ATOM | 1664 | CB | ILE | A | 226 | 13.904 | 22.866 | -3.254 | 1.00 | 14.35 | A | C |
| ATOM | 1665 | CG1 | ILE | A | 226 | 13.906 | 23.341 | -1.789 | 1.00 | 15.47 | A | C |
| ATOM | 1666 | CD1 | ILE | A | 226 | 15.239 | 23.250 | -1.085 | 1.00 | 16.51 | A | C |
| ATOM | 1667 | CG2 | ILE | A | 226 | 14.312 | 21.400 | -3.377 | 1.00 | 14.04 | A | C |
| ATOM | 1668 | C | ILE | A | 226 | 14.780 | 23.205 | -5.580 | 1.00 | 14.24 | A | C |
| ATOM | 1669 | O | ILE | A | 226 | 13.778 | 23.188 | -6.245 | 1.00 | 13.78 | A | O |
| ATOM | 1670 | N | LEU | A | 227 | 15.937 | 22.753 | -6.022 | 1.00 | 14.94 | A | N |
| ATOM | 1671 | CA | LEU | A | 227 | 16.141 | 22.230 | -7.359 | 1.00 | 14.97 | A | C |
| ATOM | 1672 | CB | LEU | A | 227 | 17.541 | 22.653 | -7.827 | 1.00 | 15.61 | A | C |
| ATOM | 1673 | CG | LEU | A | 227 | 17.950 | 22.137 | -9.196 | 1.00 | 16.60 | A | C |
| ATOM | 1674 | CD1 | LEU | A | 227 | 16.899 | 22.508 | -10.231 | 1.00 | 16.90 | A | C |
| ATOM | 1675 | CD2 | LEU | A | 227 | 19.340 | 22.669 | -9.559 | 1.00 | 18.98 | A | C |
| ATOM | 1676 | C | LEU | A | 227 | 16.010 | 20.708 | -7.284 | 1.00 | 14.76 | A | C |
| ATOM | 1677 | O | LEU | A | 227 | 16.803 | 20.038 | -6.602 | 1.00 | 15.34 | A | O |
| ATOM | 1678 | N | SER | A | 228 | 14.970 | 20.179 | -7.924 | 1.00 | 14.03 | A | N |
| ATOM | 1679 | CA | SER | A | 228 | 14.665 | 18.752 | -7.871 | 1.00 | 14.07 | A | C |

58

```
ATOM   1680  CB  SER A 228     13.701  18.448   -6.701  1.00  13.81      A    C
ATOM   1681  OG  SER A 228     13.631  17.038   -6.453  1.00  12.38      A    O
ATOM   1682  C   SER A 228     14.061  18.319   -9.208  1.00  14.98      A    C
ATOM   1683  O   SER A 228     13.971  19.115  -10.133  1.00  15.43      A    O
ATOM   1684  N   ALA A 229     13.626  17.067   -9.278  1.00  14.57      A    N
ATOM   1685  CA  ALA A 229     13.155  16.454  -10.516  1.00  14.87      A    C
ATOM   1686  CB  ALA A 229     12.824  14.945  -10.268  1.00  14.78      A    C
ATOM   1687  C   ALA A 229     11.939  17.135  -11.086  1.00  14.83      A    C
ATOM   1688  O   ALA A 229     10.939  17.411  -10.376  1.00  14.22      A    O
ATOM   1689  N   ARG A 230     12.027  17.381  -12.394  1.00  14.42      A    N
ATOM   1690  CA  ARG A 230     10.974  18.013  -13.155  1.00  14.49      A    C
ATOM   1691  CB  ARG A 230     11.553  19.008  -14.137  1.00  14.60      A    C
ATOM   1692  CG  ARG A 230     10.516  19.626  -15.065  1.00  16.55      A    C
ATOM   1693  CD  ARG A 230     11.044  20.792  -15.934  1.00  19.98      A    C
ATOM   1694  NE  ARG A 230      9.940  21.308  -16.751  1.00  19.63      A    N
ATOM   1695  CZ  ARG A 230      9.692  22.581  -16.995  1.00  21.34      A    C
ATOM   1696  NH1 ARG A 230     10.502  23.547  -16.545  1.00  21.55      A    N
ATOM   1697  NH2 ARG A 230      8.617  22.898  -17.730  1.00  20.71      A    N
ATOM   1698  C   ARG A 230     10.232  16.947  -13.948  1.00  14.81      A    C
ATOM   1699  O   ARG A 230     10.838  16.237  -14.762  1.00  14.40      A    O
ATOM   1700  N   SER A 231      8.931  16.837  -13.703  1.00  14.68      A    N
ATOM   1701  CA  SER A 231      8.106  15.937  -14.463  1.00  15.32      A    C
ATOM   1702  CB  SER A 231      6.660  16.034  -14.030  1.00  15.75      A    C
ATOM   1703  OG  SER A 231      5.836  15.317  -14.947  1.00  16.08      A    O
ATOM   1704  C   SER A 231      8.176  16.325  -15.956  1.00  15.44      A    C
ATOM   1705  O   SER A 231      8.087  17.494  -16.306  1.00  13.31      A    O
ATOM   1706  N   SER A 232      8.295  15.321  -16.802  1.00  15.69      A    N
ATOM   1707  CA  SER A 232      8.323  15.494  -18.255  1.00  16.52      A    C
ATOM   1708  CB  SER A 232      8.682  14.156  -18.906  1.00  16.29      A    C
ATOM   1709  OG  SER A 232      7.610  13.191  -18.730  1.00  16.72      A    O
ATOM   1710  C   SER A 232      7.004  16.050  -18.820  1.00  18.10      A    C
ATOM   1711  O   SER A 232      6.970  16.540  -19.945  1.00  18.08      A    O
ATOM   1712  N   LEU A 233      5.924  16.005  -18.040  1.00  18.99      A    N
ATOM   1713  CA  LEU A 233      4.647  16.550  -18.466  1.00  19.87      A    C
ATOM   1714  CB  LEU A 233      3.503  15.655  -17.989  1.00  20.52      A    C
ATOM   1715  CG  LEU A 233      3.579  14.202  -18.428  1.00  22.45      A    C
ATOM   1716  CD1 LEU A 233      2.344  13.472  -17.943  1.00  25.84      A    C
ATOM   1717  CD2 LEU A 233      3.683  14.146  -19.948  1.00  26.24      A    C
ATOM   1718  C   LEU A 233      4.357  17.956  -17.940  1.00  20.22      A    C
ATOM   1719  O   LEU A 233      3.365  18.546  -18.345  1.00  20.30      A    O
ATOM   1720  N   ALA A 234      5.164  18.485  -17.016  1.00  18.84      A    N
ATOM   1721  CA  ALA A 234      4.768  19.731  -16.365  1.00  19.34      A    C
ATOM   1722  CB  ALA A 234      5.297  19.781  -14.958  1.00  18.58      A    C
ATOM   1723  C   ALA A 234      5.197  20.991  -17.153  1.00  19.93      A    C
ATOM   1724  O   ALA A 234      6.300  21.037  -17.701  1.00  20.41      A    O
ATOM   1725  N   PRO A 235      4.325  21.989  -17.197  1.00  20.75      A    N
ATOM   1726  CA  PRO A 235      4.642  23.288  -17.802  1.00  21.88      A    C
ATOM   1727  CB  PRO A 235      3.271  23.921  -17.981  1.00  21.83      A    C
ATOM   1728  CG  PRO A 235      2.429  23.326  -16.902  1.00  21.93      A    C
ATOM   1729  CD  PRO A 235      2.947  21.944  -16.677  1.00  21.22      A    C
ATOM   1730  C   PRO A 235      5.495  24.199  -16.885  1.00  22.70      A    C
ATOM   1731  O   PRO A 235      5.513  23.970  -15.671  1.00  21.06      A    O
ATOM   1732  N   ASP A 236      6.150  25.204  -17.489  1.00  24.00      A    N
ATOM   1733  CA  ASP A 236      6.960  26.228  -16.795  1.00  24.41      A    C
ATOM   1734  CB  ASP A 236      7.455  27.332  -17.750  1.00  24.21      A    C
ATOM   1735  CG  ASP A 236      8.603  26.838  -18.636  1.00  25.42      A    C
ATOM   1736  OD1 ASP A 236      9.214  27.656  -19.365  1.00  25.18      A    O
ATOM   1737  OD2 ASP A 236      8.990  25.634  -18.674  1.00  23.87      A    O
ATOM   1738  C   ASP A 236      6.263  26.644  -15.520  1.00  24.33      A    C
ATOM   1739  O   ASP A 236      6.919  27.035  -14.558  1.00  24.16      A    O
ATOM   1740  N   SER A 237      4.933  26.677  -15.491  1.00  25.32      A    N
ATOM   1741  CA  SER A 237      4.179  27.723  -14.873  1.00  24.89      A    C
ATOM   1742  CB  SER A 237      2.801  27.926  -15.490  1.00  25.98      A    C
```

| ATOM | 1743 | OG | SER A 237 | 2.035 | 26.723 | -15.436 | 1.00 | 27.95 | A | O |
| ATOM | 1744 | C | SER A 237 | 4.027 | 26.960 | -13.487 | 1.00 | 24.14 | A | C |
| ATOM | 1745 | O | SER A 237 | 3.588 | 27.516 | -12.495 | 1.00 | 23.01 | A | O |
| ATOM | 1746 | N | SER A 238 | 4.363 | 25.660 | -13.448 | 1.00 | 22.59 | A | N |
| ATOM | 1747 | CA | SER A 238 | 4.313 | 24.861 | -12.201 | 1.00 | 22.41 | A | C |
| ATOM | 1748 | CB | SER A 238 | 4.238 | 23.344 | -12.501 | 1.00 | 22.11 | A | C |
| ATOM | 1749 | OG | SER A 238 | 3.046 | 22.968 | -13.146 | 1.00 | 22.38 | A | O |
| ATOM | 1750 | C | SER A 238 | 5.543 | 25.045 | -11.295 | 1.00 | 21.86 | A | C |
| ATOM | 1751 | O | SER A 238 | 5.550 | 24.542 | -10.184 | 1.00 | 22.29 | A | O |
| ATOM | 1752 | N | PHE A 239 | 6.568 | 25.744 | -11.789 | 1.00 | 21.32 | A | N |
| ATOM | 1753 | CA | PHE A 239 | 7.847 | 25.899 | -11.108 | 1.00 | 20.67 | A | C |
| ATOM | 1754 | CB | PHE A 239 | 8.966 | 25.299 | -11.966 | 1.00 | 20.17 | A | C |
| ATOM | 1755 | CG | PHE A 239 | 8.736 | 23.854 | -12.294 | 1.00 | 20.18 | A | C |
| ATOM | 1756 | CD1 | PHE A 239 | 8.964 | 22.881 | -11.344 | 1.00 | 18.34 | A | C |
| ATOM | 1757 | CE1 | PHE A 239 | 8.686 | 21.573 | -11.600 | 1.00 | 15.67 | A | C |
| ATOM | 1758 | CZ | PHE A 239 | 8.194 | 21.184 | -12.814 | 1.00 | 16.81 | A | C |
| ATOM | 1759 | CE2 | PHE A 239 | 7.924 | 22.128 | -13.775 | 1.00 | 16.81 | A | C |
| ATOM | 1760 | CD2 | PHE A 239 | 8.194 | 23.466 | -13.520 | 1.00 | 18.63 | A | C |
| ATOM | 1761 | C | PHE A 239 | 8.124 | 27.370 | -10.775 | 1.00 | 20.81 | A | C |
| ATOM | 1762 | O | PHE A 239 | 7.589 | 28.283 | -11.404 | 1.00 | 19.94 | A | O |
| ATOM | 1763 | N | TRP A 240 | 8.927 | 27.575 | -9.743 | 1.00 | 20.46 | A | N |
| ATOM | 1764 | CA | TRP A 240 | 9.420 | 28.913 | -9.382 | 1.00 | 21.13 | A | C |
| ATOM | 1765 | CB | TRP A 240 | 10.192 | 28.842 | -8.055 | 1.00 | 21.05 | A | C |
| ATOM | 1766 | CG | TRP A 240 | 9.324 | 28.850 | -6.857 | 1.00 | 22.76 | A | C |
| ATOM | 1767 | CD1 | TRP A 240 | 8.027 | 28.446 | -6.782 | 1.00 | 23.97 | A | C |
| ATOM | 1768 | NE1 | TRP A 240 | 7.548 | 28.624 | -5.509 | 1.00 | 24.62 | A | N |
| ATOM | 1769 | CE2 | TRP A 240 | 8.547 | 29.148 | -4.726 | 1.00 | 24.31 | A | C |
| ATOM | 1770 | CD2 | TRP A 240 | 9.677 | 29.302 | -5.537 | 1.00 | 23.24 | A | C |
| ATOM | 1771 | CE3 | TRP A 240 | 10.839 | 29.811 | -4.966 | 1.00 | 24.95 | A | C |
| ATOM | 1772 | CZ3 | TRP A 240 | 10.833 | 30.146 | -3.637 | 1.00 | 24.14 | A | C |
| ATOM | 1773 | CH2 | TRP A 240 | 9.682 | 29.991 | -2.857 | 1.00 | 23.89 | A | C |
| ATOM | 1774 | CZ2 | TRP A 240 | 8.542 | 29.483 | -3.378 | 1.00 | 25.05 | A | C |
| ATOM | 1775 | C | TRP A 240 | 10.355 | 29.466 | -10.461 | 1.00 | 20.95 | A | C |
| ATOM | 1776 | O | TRP A 240 | 10.419 | 30.673 | -10.703 | 1.00 | 20.42 | A | O |
| ATOM | 1777 | N | ALA A 241 | 11.097 | 28.566 | -11.080 | 1.00 | 21.11 | A | N |
| ATOM | 1778 | CA | ALA A 241 | 12.022 | 28.907 | -12.149 | 1.00 | 21.52 | A | C |
| ATOM | 1779 | CB | ALA A 241 | 13.243 | 29.629 | -11.606 | 1.00 | 21.93 | A | C |
| ATOM | 1780 | C | ALA A 241 | 12.466 | 27.641 | -12.801 | 1.00 | 21.79 | A | C |
| ATOM | 1781 | O | ALA A 241 | 12.440 | 26.569 | -12.169 | 1.00 | 22.05 | A | O |
| ATOM | 1782 | N | ASN A 242 | 12.929 | 27.769 | -14.040 | 1.00 | 22.09 | A | N |
| ATOM | 1783 | CA | ASN A 242 | 13.481 | 26.656 | -14.800 | 1.00 | 22.74 | A | C |
| ATOM | 1784 | CB | ASN A 242 | 13.397 | 26.962 | -16.322 | 1.00 | 22.69 | A | C |
| ATOM | 1785 | CG | ASN A 242 | 11.960 | 27.071 | -16.828 | 1.00 | 22.96 | A | C |
| ATOM | 1786 | OD1 | ASN A 242 | 11.024 | 26.578 | -16.198 | 1.00 | 21.33 | A | O |
| ATOM | 1787 | ND2 | ASN A 242 | 11.782 | 27.727 | -17.969 | 1.00 | 21.31 | A | N |
| ATOM | 1788 | C | ASN A 242 | 14.927 | 26.359 | -14.458 | 1.00 | 23.18 | A | C |
| ATOM | 1789 | O | ASN A 242 | 15.634 | 27.194 | -13.902 | 1.00 | 23.35 | A | O |
| ATOM | 1790 | N | HIS A 243 | 15.375 | 25.169 | -14.820 | 1.00 | 24.21 | A | N |
| ATOM | 1791 | CA | HIS A 243 | 16.802 | 24.875 | -14.862 | 1.00 | 25.06 | A | C |
| ATOM | 1792 | CB | HIS A 243 | 17.234 | 24.062 | -13.653 | 1.00 | 25.31 | A | C |
| ATOM | 1793 | CG | HIS A 243 | 18.703 | 23.809 | -13.595 | 1.00 | 27.16 | A | C |
| ATOM | 1794 | ND1 | HIS A 243 | 19.599 | 24.733 | -13.086 | 1.00 | 30.11 | A | N |
| ATOM | 1795 | CE1 | HIS A 243 | 20.820 | 24.231 | -13.152 | 1.00 | 30.29 | A | C |
| ATOM | 1796 | NE2 | HIS A 243 | 20.752 | 23.036 | -13.713 | 1.00 | 29.63 | A | N |
| ATOM | 1797 | CD2 | HIS A 243 | 19.442 | 22.754 | -14.008 | 1.00 | 28.73 | A | C |
| ATOM | 1798 | C | HIS A 243 | 17.158 | 24.144 | -16.162 | 1.00 | 26.02 | A | C |
| ATOM | 1799 | O | HIS A 243 | 17.851 | 24.726 | -17.003 | 1.00 | 25.77 | A | O |
| ATOM | 1800 | N | ASP A 244 | 16.711 | 22.880 | -16.299 | 1.00 | 26.20 | A | N |
| ATOM | 1801 | CA | ASP A 244 | 16.757 | 22.137 | -17.584 | 1.00 | 27.35 | A | C |
| ATOM | 1802 | CB | ASP A 244 | 17.972 | 21.252 | -17.646 | 1.00 | 28.02 | A | C |
| ATOM | 1803 | CG | ASP A 244 | 18.211 | 20.272 | -16.546 | 1.00 | 29.28 | A | C |
| ATOM | 1804 | OD1 | ASP A 244 | 19.393 | 20.188 | -16.099 | 1.00 | 34.73 | A | O |
| ATOM | 1805 | OD2 | ASP A 244 | 17.310 | 19.568 | -16.056 | 1.00 | 28.43 | A | O |

```
ATOM   1806  C    ASP A 244    15.427  21.429 -17.760  1.00 27.57      A    C
ATOM   1807  O    ASP A 244    14.751  21.208 -16.721  1.00 27.11      A    O
ATOM   1808  N    SER A 245    15.290  20.734 -18.836  1.00 27.83      A    N
ATOM   1809  CA   SER A 245    14.559  19.557 -19.209  1.00 26.73      A    C
ATOM   1810  CB   SER A 245    15.083  18.972 -20.483  1.00 26.99      A    C
ATOM   1811  OG   SER A 245    15.792  17.785 -20.481  1.00 27.12      A    O
ATOM   1812  C    SER A 245    14.234  18.594 -18.102  1.00 25.28      A    C
ATOM   1813  O    SER A 245    13.146  17.973 -18.152  1.00 24.66      A    O
ATOM   1814  N    LYS A 246    15.122  18.339 -17.176  1.00 23.51      A    N
ATOM   1815  CA   LYS A 246    14.918  17.304 -16.177  1.00 22.90      A    C
ATOM   1816  CB   LYS A 246    15.977  16.205 -16.332  1.00 23.64      A    C
ATOM   1817  CG   LYS A 246    15.852  15.384 -17.600  1.00 26.42      A    C
ATOM   1818  CD   LYS A 246    17.094  14.548 -17.859  1.00 29.39      A    C
ATOM   1819  CE   LYS A 246    16.880  13.584 -19.018  1.00 32.94      A    C
ATOM   1820  NZ   LYS A 246    18.070  13.501 -19.908  1.00 37.30      A    N
ATOM   1821  C    LYS A 246    14.812  17.762 -14.740  1.00 21.77      A    C
ATOM   1822  O    LYS A 246    14.396  17.059 -13.828  1.00 19.24      A    O
ATOM   1823  N    TYR A 247    15.126  19.026 -14.452  1.00 20.43      A    N
ATOM   1824  CA   TYR A 247    15.144  19.544 -13.079  1.00 19.83      A    C
ATOM   1825  CB   TYR A 247    16.541  19.398 -12.456  1.00 19.35      A    C
ATOM   1826  CG   TYR A 247    17.007  17.966 -12.434  1.00 19.14      A    C
ATOM   1827  CD1  TYR A 247    17.784  17.442 -13.482  1.00 21.08      A    C
ATOM   1828  CE1  TYR A 247    18.170  16.121 -13.489  1.00 17.97      A    C
ATOM   1829  CZ   TYR A 247    17.780  15.292 -12.458  1.00 19.97      A    C
ATOM   1830  OH   TYR A 247    18.159  13.964 -12.465  1.00 18.06      A    O
ATOM   1831  CE2  TYR A 247    16.999  15.781 -11.417  1.00 18.19      A    C
ATOM   1832  CD2  TYR A 247    16.630  17.109 -11.408  1.00 19.09      A    C
ATOM   1833  C    TYR A 247    14.697  21.003 -13.069  1.00 18.89      A    C
ATOM   1834  O    TYR A 247    15.017  21.761 -13.994  1.00 18.71      A    O
ATOM   1835  N    ALA A 248    13.936  21.385 -12.046  1.00 17.07      A    N
ATOM   1836  CA   ALA A 248    13.512  22.769 -11.893  1.00 16.28      A    C
ATOM   1837  CB   ALA A 248    12.294  23.035 -12.733  1.00 15.85      A    C
ATOM   1838  C    ALA A 248    13.253  23.110 -10.425  1.00 15.75      A    C
ATOM   1839  O    ALA A 248    13.358  22.236  -9.549  1.00 15.81      A    O
ATOM   1840  N    TYR A 249    12.956  24.384 -10.174  1.00 15.33      A    N
ATOM   1841  CA   TYR A 249    12.910  24.949  -8.832  1.00 15.08      A    C
ATOM   1842  CB   TYR A 249    13.520  26.336  -8.802  1.00 15.54      A    C
ATOM   1843  CG   TYR A 249    14.999  26.398  -9.087  1.00 15.33      A    C
ATOM   1844  CD1  TYR A 249    15.470  26.675 -10.370  1.00 17.29      A    C
ATOM   1845  CE1  TYR A 249    16.829  26.754 -10.640  1.00 16.19      A    C
ATOM   1846  CZ   TYR A 249    17.741  26.557  -9.608  1.00 18.72      A    C
ATOM   1847  OH   TYR A 249    19.088  26.649  -9.839  1.00 21.92      A    O
ATOM   1848  CE2  TYR A 249    17.306  26.287  -8.330  1.00 18.20      A    C
ATOM   1849  CD2  TYR A 249    15.930  26.207  -8.070  1.00 17.03      A    C
ATOM   1850  C    TYR A 249    11.497  25.078  -8.358  1.00 15.50      A    C
ATOM   1851  O    TYR A 249    10.599  25.480  -9.122  1.00 16.06      A    O
ATOM   1852  N    MET A 250    11.291  24.749  -7.082  1.00 15.10      A    N
ATOM   1853  CA   MET A 250    10.015  24.967  -6.430  1.00 15.40      A    C
ATOM   1854  CB   MET A 250     9.153  23.703  -6.542  1.00 15.90      A    C
ATOM   1855  CG   MET A 250     7.677  23.947  -6.729  1.00 19.64      A    C
ATOM   1856  SD   MET A 250     6.677  22.370  -6.869  1.00 23.44      A    S
ATOM   1857  CE   MET A 250     7.321  21.709  -8.163  1.00 22.96      A    C
ATOM   1858  C    MET A 250    10.274  25.318  -4.966  1.00 15.09      A    C
ATOM   1859  O    MET A 250    11.366  25.081  -4.440  1.00 15.78      A    O
ATOM   1860  N    GLY A 251     9.279  25.888  -4.314  1.00 14.69      A    N
ATOM   1861  CA   GLY A 251     9.373  26.203  -2.902  1.00 13.76      A    C
ATOM   1862  C    GLY A 251     8.026  26.058  -2.248  1.00 14.49      A    C
ATOM   1863  O    GLY A 251     6.984  26.057  -2.933  1.00 13.60      A    O
ATOM   1864  N    GLY A 252     8.056  25.926  -0.920  1.00 12.84      A    N
ATOM   1865  CA   GLY A 252     6.879  25.694  -0.101  1.00 13.22      A    C
ATOM   1866  C    GLY A 252     7.242  24.765   1.058  1.00 12.42      A    C
ATOM   1867  O    GLY A 252     8.354  24.185   1.073  1.00 11.46      A    O
ATOM   1868  N    THR A 253     6.328  24.598   2.008  1.00 12.37      A    N
```

| | | | | | | | | | | | | |
|---|---|---|---|---|---|---|---|---|---|---|---|---|
| ATOM | 1869 | CA | THR | A | 253 | 6.518 | 23.583 | 3.043 | 1.00 | 12.51 | A | C |
| ATOM | 1870 | CB | THR | A | 253 | 5.543 | 23.697 | 4.256 | 1.00 | 13.05 | A | C |
| ATOM | 1871 | OG1 | THR | A | 253 | 4.138 | 23.788 | 3.858 | 1.00 | 11.78 | A | O |
| ATOM | 1872 | CG2 | THR | A | 253 | 5.837 | 24.964 | 5.042 | 1.00 | 13.23 | A | C |
| ATOM | 1873 | C | THR | A | 253 | 6.463 | 22.211 | 2.396 | 1.00 | 12.60 | A | C |
| ATOM | 1874 | O | THR | A | 253 | 6.945 | 21.239 | 2.966 | 1.00 | 12.54 | A | O |
| ATOM | 1875 | N | SER | A | 254 | 5.902 | 22.158 | 1.187 | 1.00 | 12.69 | A | N |
| ATOM | 1876 | CA | SER | A | 254 | 5.905 | 20.957 | 0.357 | 1.00 | 12.54 | A | C |
| ATOM | 1877 | CB | SER | A | 254 | 5.228 | 21.233 | -0.994 | 1.00 | 12.41 | A | C |
| ATOM | 1878 | OG | SER | A | 254 | 3.822 | 21.002 | -0.960 | 1.00 | 11.90 | A | O |
| ATOM | 1879 | C | SER | A | 254 | 7.298 | 20.445 | 0.050 | 1.00 | 12.52 | A | C |
| ATOM | 1880 | O | SER | A | 254 | 7.459 | 19.253 | -0.150 | 1.00 | 12.45 | A | O |
| ATOM | 1881 | N | MET | A | 255 | 8.255 | 21.361 | -0.054 | 1.00 | 12.52 | A | N |
| ATOM | 1882 | CA | MET | A | 255 | 9.640 | 21.062 | -0.385 | 1.00 | 13.23 | A | C |
| ATOM | 1883 | CB | MET | A | 255 | 10.260 | 22.231 | -1.164 | 1.00 | 13.16 | A | C |
| ATOM | 1884 | CG | MET | A | 255 | 9.955 | 22.255 | -2.667 | 1.00 | 13.61 | A | C |
| ATOM | 1885 | SD | MET | A | 255 | 8.220 | 22.693 | -3.027 | 1.00 | 16.25 | A | S |
| ATOM | 1886 | CE | MET | A | 255 | 7.683 | 21.071 | -3.591 | 1.00 | 13.35 | A | C |
| ATOM | 1887 | C | MET | A | 255 | 10.478 | 20.759 | 0.873 | 1.00 | 13.32 | A | C |
| ATOM | 1888 | O | MET | A | 255 | 11.396 | 19.934 | 0.847 | 1.00 | 13.21 | A | O |
| ATOM | 1889 | N | ALA | A | 256 | 10.162 | 21.415 | 1.981 | 1.00 | 12.98 | A | N |
| ATOM | 1890 | CA | ALA | A | 256 | 10.904 | 21.161 | 3.213 | 1.00 | 12.47 | A | C |
| ATOM | 1891 | CB | ALA | A | 256 | 10.516 | 22.175 | 4.265 | 1.00 | 11.99 | A | C |
| ATOM | 1892 | C | ALA | A | 256 | 10.645 | 19.737 | 3.717 | 1.00 | 11.89 | A | C |
| ATOM | 1893 | O | ALA | A | 256 | 11.553 | 19.018 | 4.179 | 1.00 | 11.48 | A | O |
| ATOM | 1894 | N | THR | A | 257 | 9.390 | 19.341 | 3.629 | 1.00 | 11.55 | A | N |
| ATOM | 1895 | CA | THR | A | 257 | 8.944 | 18.065 | 4.146 | 1.00 | 11.50 | A | C |
| ATOM | 1896 | CB | THR | A | 257 | 7.423 | 17.938 | 3.908 | 1.00 | 12.07 | A | C |
| ATOM | 1897 | OG1 | THR | A | 257 | 6.754 | 19.013 | 4.569 | 1.00 | 13.08 | A | O |
| ATOM | 1898 | CG2 | THR | A | 257 | 6.838 | 16.661 | 4.540 | 1.00 | 12.46 | A | C |
| ATOM | 1899 | C | THR | A | 257 | 9.705 | 16.849 | 3.587 | 1.00 | 11.20 | A | C |
| ATOM | 1900 | O | THR | A | 257 | 10.172 | 16.018 | 4.382 | 1.00 | 11.03 | A | O |
| ATOM | 1901 | N | PRO | A | 258 | 9.781 | 16.686 | 2.259 | 1.00 | 11.22 | A | N |
| ATOM | 1902 | CA | PRO | A | 258 | 10.466 | 15.521 | 1.687 | 1.00 | 10.92 | A | C |
| ATOM | 1903 | CB | PRO | A | 258 | 10.200 | 15.644 | 0.182 | 1.00 | 10.45 | A | C |
| ATOM | 1904 | CG | PRO | A | 258 | 9.884 | 17.057 | -0.029 | 1.00 | 11.62 | A | C |
| ATOM | 1905 | CD | PRO | A | 258 | 9.164 | 17.504 | 1.207 | 1.00 | 10.92 | A | C |
| ATOM | 1906 | C | PRO | A | 258 | 11.969 | 15.503 | 1.976 | 1.00 | 10.83 | A | C |
| ATOM | 1907 | O | PRO | A | 258 | 12.524 | 14.417 | 2.020 | 1.00 | 9.95 | A | O |
| ATOM | 1908 | N | ILE | A | 259 | 12.605 | 16.665 | 2.160 | 1.00 | 11.19 | A | N |
| ATOM | 1909 | CA | ILE | A | 259 | 14.004 | 16.711 | 2.597 | 1.00 | 11.51 | A | C |
| ATOM | 1910 | CB | ILE | A | 259 | 14.439 | 18.183 | 2.712 | 1.00 | 11.81 | A | C |
| ATOM | 1911 | CG1 | ILE | A | 259 | 14.492 | 18.843 | 1.314 | 1.00 | 14.15 | A | C |
| ATOM | 1912 | CD1 | ILE | A | 259 | 15.690 | 18.403 | 0.504 | 1.00 | 17.31 | A | C |
| ATOM | 1913 | CG2 | ILE | A | 259 | 15.790 | 18.313 | 3.375 | 1.00 | 11.02 | A | C |
| ATOM | 1914 | C | ILE | A | 259 | 14.147 | 15.975 | 3.950 | 1.00 | 11.56 | A | C |
| ATOM | 1915 | O | ILE | A | 259 | 15.038 | 15.133 | 4.124 | 1.00 | 11.81 | A | O |
| ATOM | 1916 | N | VAL | A | 260 | 13.259 | 16.295 | 4.886 | 1.00 | 11.02 | A | N |
| ATOM | 1917 | CA | VAL | A | 260 | 13.244 | 15.668 | 6.199 | 1.00 | 12.26 | A | C |
| ATOM | 1918 | CB | VAL | A | 260 | 12.301 | 16.412 | 7.150 | 1.00 | 12.30 | A | C |
| ATOM | 1919 | CG1 | VAL | A | 260 | 12.286 | 15.743 | 8.557 | 1.00 | 12.78 | A | C |
| ATOM | 1920 | CG2 | VAL | A | 260 | 12.721 | 17.855 | 7.268 | 1.00 | 13.51 | A | C |
| ATOM | 1921 | C | VAL | A | 260 | 12.847 | 14.185 | 6.106 | 1.00 | 12.24 | A | C |
| ATOM | 1922 | O | VAL | A | 260 | 13.412 | 13.339 | 6.786 | 1.00 | 12.79 | A | O |
| ATOM | 1923 | N | ALA | A | 261 | 11.922 | 13.864 | 5.217 | 1.00 | 12.41 | A | N |
| ATOM | 1924 | CA | ALA | A | 261 | 11.530 | 12.480 | 4.997 | 1.00 | 11.93 | A | C |
| ATOM | 1925 | CB | ALA | A | 261 | 10.426 | 12.376 | 3.920 | 1.00 | 12.16 | A | C |
| ATOM | 1926 | C | ALA | A | 261 | 12.750 | 11.661 | 4.585 | 1.00 | 11.91 | A | C |
| ATOM | 1927 | O | ALA | A | 261 | 12.943 | 10.560 | 5.055 | 1.00 | 11.34 | A | O |
| ATOM | 1928 | N | GLY | A | 262 | 13.550 | 12.186 | 3.665 | 1.00 | 12.22 | A | N |
| ATOM | 1929 | CA | GLY | A | 262 | 14.794 | 11.533 | 3.291 | 1.00 | 12.29 | A | C |
| ATOM | 1930 | C | GLY | A | 262 | 15.786 | 11.431 | 4.447 | 1.00 | 12.34 | A | C |
| ATOM | 1931 | O | GLY | A | 262 | 16.414 | 10.386 | 4.660 | 1.00 | 11.90 | A | O |

62

| ATOM | 1932 | N | ASN A 263 | 15.901 | 12.490 | 5.243 | 1.00 | 12.20 | A | N |
|------|------|------|-----------|--------|--------|-------|------|-------|---|---|
| ATOM | 1933 | CA | ASN A 263 | 16.744 | 12.433 | 6.435 | 1.00 | 11.84 | A | C |
| ATOM | 1934 | CB | ASN A 263 | 16.772 | 13.773 | 7.170 | 1.00 | 12.31 | A | C |
| ATOM | 1935 | CG | ASN A 263 | 17.389 | 14.887 | 6.351 | 1.00 | 13.12 | A | C |
| ATOM | 1936 | OD1 | ASN A 263 | 18.326 | 14.681 | 5.525 | 1.00 | 15.95 | A | O |
| ATOM | 1937 | ND2 | ASN A 263 | 16.924 | 16.073 | 6.600 | 1.00 | 9.15 | A | N |
| ATOM | 1938 | C | ASN A 263 | 16.289 | 11.348 | 7.396 | 1.00 | 11.83 | A | C |
| ATOM | 1939 | O | ASN A 263 | 17.112 | 10.672 | 8.020 | 1.00 | 11.88 | A | O |
| ATOM | 1940 | N | VAL A 264 | 14.983 | 11.181 | 7.517 | 1.00 | 11.77 | A | N |
| ATOM | 1941 | CA | VAL A 264 | 14.425 | 10.138 | 8.367 | 1.00 | 12.33 | A | C |
| ATOM | 1942 | CB | VAL A 264 | 12.893 | 10.268 | 8.506 | 1.00 | 12.44 | A | C |
| ATOM | 1943 | CG1 | VAL A 264 | 12.280 | 9.045 | 9.178 | 1.00 | 12.44 | A | C |
| ATOM | 1944 | CG2 | VAL A 264 | 12.543 | 11.471 | 9.323 | 1.00 | 13.22 | A | C |
| ATOM | 1945 | C | VAL A 264 | 14.817 | 8.754 | 7.843 | 1.00 | 11.95 | A | C |
| ATOM | 1946 | O | VAL A 264 | 15.164 | 7.896 | 8.625 | 1.00 | 12.24 | A | O |
| ATOM | 1947 | N | ALA A 265 | 14.813 | 8.553 | 6.527 | 1.00 | 12.03 | A | N |
| ATOM | 1948 | CA | ALA A 265 | 15.279 | 7.292 | 5.966 | 1.00 | 11.47 | A | C |
| ATOM | 1949 | CB | ALA A 265 | 15.018 | 7.237 | 4.460 | 1.00 | 11.96 | A | C |
| ATOM | 1950 | C | ALA A 265 | 16.746 | 7.046 | 6.293 | 1.00 | 11.77 | A | C |
| ATOM | 1951 | O | ALA A 265 | 17.139 | 5.932 | 6.592 | 1.00 | 11.77 | A | O |
| ATOM | 1952 | N | GLN A 266 | 17.571 | 8.091 | 6.262 | 1.00 | 12.48 | A | N |
| ATOM | 1953 | CA | GLN A 266 | 18.999 | 7.940 | 6.586 | 1.00 | 11.99 | A | C |
| ATOM | 1954 | CB | GLN A 266 | 19.782 | 9.230 | 6.311 | 1.00 | 10.90 | A | C |
| ATOM | 1955 | CG | GLN A 266 | 19.786 | 9.691 | 4.865 | 1.00 | 12.48 | A | C |
| ATOM | 1956 | CD | GLN A 266 | 20.548 | 11.011 | 4.671 | 1.00 | 12.24 | A | C |
| ATOM | 1957 | OE1 | GLN A 266 | 21.762 | 11.028 | 4.352 | 1.00 | 16.02 | A | O |
| ATOM | 1958 | NE2 | GLN A 266 | 19.857 | 12.088 | 4.853 | 1.00 | 8.53 | A | N |
| ATOM | 1959 | C | GLN A 266 | 19.159 | 7.571 | 8.046 | 1.00 | 12.14 | A | C |
| ATOM | 1960 | O | GLN A 266 | 19.927 | 6.688 | 8.398 | 1.00 | 12.13 | A | O |
| ATOM | 1961 | N | LEU A 267 | 18.463 | 8.305 | 8.898 | 1.00 | 12.44 | A | N |
| ATOM | 1962 | CA | LEU A 267 | 18.473 | 8.049 | 10.317 | 1.00 | 12.06 | A | C |
| ATOM | 1963 | CB | LEU A 267 | 17.624 | 9.107 | 11.014 | 1.00 | 12.45 | A | C |
| ATOM | 1964 | CG | LEU A 267 | 17.550 | 9.097 | 12.540 | 1.00 | 12.15 | A | C |
| ATOM | 1965 | CD1 | LEU A 267 | 18.918 | 9.293 | 13.116 | 1.00 | 12.99 | A | C |
| ATOM | 1966 | CD2 | LEU A 267 | 16.616 | 10.187 | 13.009 | 1.00 | 12.84 | A | C |
| ATOM | 1967 | C | LEU A 267 | 17.984 | 6.649 | 10.654 | 1.00 | 12.72 | A | C |
| ATOM | 1968 | O | LEU A 267 | 18.581 | 5.972 | 11.497 | 1.00 | 12.91 | A | O |
| ATOM | 1969 | N | ARG A 268 | 16.872 | 6.219 | 10.044 | 1.00 | 12.54 | A | N |
| ATOM | 1970 | CA | ARG A 268 | 16.295 | 4.886 | 10.321 | 1.00 | 12.31 | A | C |
| ATOM | 1971 | CB | ARG A 268 | 14.961 | 4.722 | 9.577 | 1.00 | 12.21 | A | C |
| ATOM | 1972 | CG | ARG A 268 | 14.016 | 3.635 | 10.155 | 1.00 | 12.56 | A | C |
| ATOM | 1973 | CD | ARG A 268 | 12.652 | 3.605 | 9.510 | 1.00 | 14.20 | A | C |
| ATOM | 1974 | NE | ARG A 268 | 11.781 | 2.591 | 10.105 | 1.00 | 14.70 | A | N |
| ATOM | 1975 | CZ | ARG A 268 | 11.837 | 1.306 | 9.829 | 1.00 | 14.81 | A | C |
| ATOM | 1976 | NH1 | ARG A 268 | 12.697 | 0.829 | 8.942 | 1.00 | 14.11 | A | N |
| ATOM | 1977 | NH2 | ARG A 268 | 10.993 | 0.483 | 10.432 | 1.00 | 15.99 | A | N |
| ATOM | 1978 | C | ARG A 268 | 17.284 | 3.763 | 9.929 | 1.00 | 12.37 | A | C |
| ATOM | 1979 | O | ARG A 268 | 17.533 | 2.837 | 10.689 | 1.00 | 11.86 | A | O |
| ATOM | 1980 | N | GLU A 269 | 17.846 | 3.870 | 8.729 | 1.00 | 11.97 | A | N |
| ATOM | 1981 | CA | GLU A 269 | 18.965 | 3.026 | 8.306 | 1.00 | 12.12 | A | C |
| ATOM | 1982 | CB | GLU A 269 | 19.561 | 3.537 | 6.993 | 1.00 | 11.36 | A | C |
| ATOM | 1983 | CG | GLU A 269 | 20.764 | 2.715 | 6.542 | 1.00 | 12.78 | A | C |
| ATOM | 1984 | CD | GLU A 269 | 21.477 | 3.260 | 5.335 | 1.00 | 15.24 | A | C |
| ATOM | 1985 | OE1 | GLU A 269 | 21.277 | 4.447 | 5.007 | 1.00 | 16.05 | A | O |
| ATOM | 1986 | OE2 | GLU A 269 | 22.246 | 2.479 | 4.711 | 1.00 | 16.25 | A | O |
| ATOM | 1987 | C | GLU A 269 | 20.082 | 2.954 | 9.354 | 1.00 | 12.56 | A | C |
| ATOM | 1988 | O | GLU A 269 | 20.596 | 1.875 | 9.645 | 1.00 | 12.26 | A | O |
| ATOM | 1989 | N | HIS A 270 | 20.482 | 4.104 | 9.894 | 1.00 | 12.79 | A | N |
| ATOM | 1990 | CA | HIS A 270 | 21.556 | 4.119 | 10.859 | 1.00 | 12.79 | A | C |
| ATOM | 1991 | CB | HIS A 270 | 21.918 | 5.531 | 11.289 | 1.00 | 12.76 | A | C |
| ATOM | 1992 | CG | BHIS A 270 | 23.160 | 5.583 | 12.120 | 0.50 | 10.01 | A | C |
| ATOM | 1993 | CG | AHIS A 270 | 23.195 | 5.601 | 12.063 | 0.50 | 15.58 | A | C |
| ATOM | 1994 | ND1 | BHIS A 270 | 23.186 | 6.137 | 13.385 | 0.50 | 7.23 | A | N |

| ATOM | 1995 | ND1AHIS | A | 270 | 23.243 | 5.459 | 13.432 | 0.50 | 20.08 | A | N |
|------|------|---------|---|-----|--------|-------|--------|------|-------|---|---|
| ATOM | 1996 | CE1BHIS | A | 270 | 24.404 | 6.019 | 13.885 | 0.50 | 6.26 | A | C |
| ATOM | 1997 | CE1AHIS | A | 270 | 24.498 | 5.548 | 13.839 | 0.50 | 20.34 | A | C |
| ATOM | 1998 | NE2BHIS | A | 270 | 25.163 | 5.387 | 13.000 | 0.50 | 8.61 | A | N |
| ATOM | 1999 | NE2AHIS | A | 270 | 25.265 | 5.744 | 12.783 | 0.50 | 20.61 | A | N |
| ATOM | 2000 | CD2BHIS | A | 270 | 24.405 | 5.102 | 11.888 | 0.50 | 6.18 | A | C |
| ATOM | 2001 | CD2AHIS | A | 270 | 24.475 | 5.782 | 11.659 | 0.50 | 18.53 | A | C |
| ATOM | 2002 | C   HIS | A | 270 | 21.210 | 3.294 | 12.099 | 1.00 | 12.75 | A | C |
| ATOM | 2003 | O   HIS | A | 270 | 22.031 | 2.541 | 12.562 | 1.00 | 12.88 | A | O |
| ATOM | 2004 | N   PHE | A | 271 | 20.009 | 3.468 | 12.666 | 1.00 | 12.25 | A | N |
| ATOM | 2005 | CA  PHE | A | 271 | 19.642 | 2.680 | 13.834 | 1.00 | 12.36 | A | C |
| ATOM | 2006 | CB  PHE | A | 271 | 18.274 | 3.114 | 14.370 | 1.00 | 12.01 | A | C |
| ATOM | 2007 | CG  PHE | A | 271 | 18.328 | 4.292 | 15.293 | 1.00 | 11.54 | A | C |
| ATOM | 2008 | CD1 PHE | A | 271 | 18.557 | 4.127 | 16.643 | 1.00 | 12.44 | A | C |
| ATOM | 2009 | CE1 PHE | A | 271 | 18.601 | 5.229 | 17.500 | 1.00 | 13.55 | A | C |
| ATOM | 2010 | CZ  PHE | A | 271 | 18.400 | 6.479 | 17.016 | 1.00 | 11.92 | A | C |
| ATOM | 2011 | CE2 PHE | A | 271 | 18.145 | 6.655 | 15.663 | 1.00 | 15.14 | A | C |
| ATOM | 2012 | CD2 PHE | A | 271 | 18.096 | 5.567 | 14.820 | 1.00 | 13.90 | A | C |
| ATOM | 2013 | C   PHE | A | 271 | 19.620 | 1.178 | 13.492 | 1.00 | 13.05 | A | C |
| ATOM | 2014 | O   PHE | A | 271 | 20.147 | 0.341 | 14.240 | 1.00 | 15.06 | A | O |
| ATOM | 2015 | N   VAL | A | 272 | 19.007 | 0.850 | 12.371 | 1.00 | 12.88 | A | N |
| ATOM | 2016 | CA  VAL | A | 272 | 18.765 | -0.526 | 11.961 | 1.00 | 13.79 | A | C |
| ATOM | 2017 | CB  VAL | A | 272 | 17.856 | -0.539 | 10.706 | 1.00 | 13.45 | A | C |
| ATOM | 2018 | CG1 VAL | A | 272 | 17.977 | -1.840 | 9.953 | 1.00 | 15.25 | A | C |
| ATOM | 2019 | CG2 VAL | A | 272 | 16.429 | -0.264 | 11.112 | 1.00 | 13.55 | A | C |
| ATOM | 2020 | C   VAL | A | 272 | 20.068 | -1.276 | 11.689 | 1.00 | 14.17 | A | C |
| ATOM | 2021 | O   VAL | A | 272 | 20.242 | -2.415 | 12.162 | 1.00 | 14.67 | A | O |
| ATOM | 2022 | N   LYS | A | 273 | 20.992 | -0.619 | 10.990 | 1.00 | 14.25 | A | N |
| ATOM | 2023 | CA  LYS | A | 273 | 22.255 | -1.217 | 10.606 | 1.00 | 14.60 | A | C |
| ATOM | 2024 | CB  LYS | A | 273 | 22.759 | -0.664 | 9.267 | 1.00 | 14.93 | A | C |
| ATOM | 2025 | CG  LYS | A | 273 | 21.893 | -1.085 | 8.052 | 1.00 | 15.97 | A | C |
| ATOM | 2026 | CD  LYS | A | 273 | 22.432 | -0.488 | 6.729 | 1.00 | 14.97 | A | C |
| ATOM | 2027 | CE  LYS | A | 273 | 21.735 | -1.010 | 5.482 | 1.00 | 16.43 | A | C |
| ATOM | 2028 | NZ  LYS | A | 273 | 22.131 | -0.162 | 4.300 | 1.00 | 13.02 | A | N |
| ATOM | 2029 | C   LYS | A | 273 | 23.366 | -1.133 | 11.645 | 1.00 | 14.97 | A | C |
| ATOM | 2030 | O   LYS | A | 273 | 24.172 | -2.075 | 11.740 | 1.00 | 12.32 | A | O |
| ATOM | 2031 | N   ASN | A | 274 | 23.402 | -0.033 | 12.403 | 1.00 | 14.72 | A | N |
| ATOM | 2032 | CA  ASN | A | 274 | 24.556 | 0.298 | 13.225 | 1.00 | 15.62 | A | C |
| ATOM | 2033 | CB  ASN | A | 274 | 25.197 | 1.649 | 12.786 | 1.00 | 16.07 | A | C |
| ATOM | 2034 | CG  ASN | A | 274 | 25.555 | 1.662 | 11.290 | 1.00 | 17.59 | A | C |
| ATOM | 2035 | OD1 ASN | A | 274 | 25.285 | 2.647 | 10.543 | 1.00 | 18.70 | A | O |
| ATOM | 2036 | ND2 ASN | A | 274 | 26.124 | 0.561 | 10.839 | 1.00 | 13.05 | A | N |
| ATOM | 2037 | C   ASN | A | 274 | 24.253 | 0.365 | 14.694 | 1.00 | 15.60 | A | C |
| ATOM | 2038 | O   ASN | A | 274 | 25.165 | 0.449 | 15.465 | 1.00 | 15.70 | A | O |
| ATOM | 2039 | N   ARG | A | 275 | 22.979 | 0.348 | 15.092 | 1.00 | 14.80 | A | N |
| ATOM | 2040 | CA  ARG | A | 275 | 22.670 | 0.517 | 16.505 | 1.00 | 15.19 | A | C |
| ATOM | 2041 | CB  ARG | A | 275 | 22.046 | 1.883 | 16.723 | 1.00 | 14.89 | A | C |
| ATOM | 2042 | CG  ARG | A | 275 | 22.925 | 3.001 | 16.141 | 1.00 | 17.88 | A | C |
| ATOM | 2043 | CD  ARG | A | 275 | 22.682 | 4.354 | 16.748 | 1.00 | 17.97 | A | C |
| ATOM | 2044 | NE  ARG | A | 275 | 23.098 | 4.391 | 18.146 | 1.00 | 15.44 | A | N |
| ATOM | 2045 | CZ  ARG | A | 275 | 22.783 | 5.383 | 18.977 | 1.00 | 18.17 | A | C |
| ATOM | 2046 | NH1 ARG | A | 275 | 22.080 | 6.422 | 18.540 | 1.00 | 17.27 | A | N |
| ATOM | 2047 | NH2 ARG | A | 275 | 23.191 | 5.356 | 20.239 | 1.00 | 17.72 | A | N |
| ATOM | 2048 | C   ARG | A | 275 | 21.796 | -0.573 | 17.088 | 1.00 | 14.64 | A | C |
| ATOM | 2049 | O   ARG | A | 275 | 21.382 | -0.456 | 18.212 | 1.00 | 15.75 | A | O |
| ATOM | 2050 | N   GLY | A | 276 | 21.459 | -1.577 | 16.283 | 1.00 | 14.66 | A | N |
| ATOM | 2051 | CA  GLY | A | 276 | 20.880 | -2.825 | 16.771 | 1.00 | 14.63 | A | C |
| ATOM | 2052 | C   GLY | A | 276 | 19.403 | -2.811 | 17.060 | 1.00 | 14.40 | A | C |
| ATOM | 2053 | O   GLY | A | 276 | 18.863 | -3.751 | 17.664 | 1.00 | 13.52 | A | O |
| ATOM | 2054 | N   VAL | A | 277 | 18.729 | -1.745 | 16.638 | 1.00 | 14.10 | A | N |
| ATOM | 2055 | CA  VAL | A | 277 | 17.318 | -1.618 | 16.894 | 1.00 | 14.10 | A | C |
| ATOM | 2056 | CB  VAL | A | 277 | 17.021 | -0.657 | 18.097 | 1.00 | 14.71 | A | C |
| ATOM | 2057 | CG1 VAL | A | 277 | 17.768 | -1.058 | 19.354 | 1.00 | 14.49 | A | C |

| ATOM | 2058 | CG2 | VAL A 277 | 17.268 | 0.771 | 17.733 | 1.00 | 15.67 | A | C |
| ATOM | 2059 | C | VAL A 277 | 16.547 | -1.097 | 15.689 | 1.00 | 13.79 | A | C |
| ATOM | 2060 | O | VAL A 277 | 17.082 | -0.372 | 14.853 | 1.00 | 14.29 | A | O |
| ATOM | 2061 | N | THR A 278 | 15.273 | -1.472 | 15.607 | 1.00 | 14.21 | A | N |
| ATOM | 2062 | CA | THR A 278 | 14.325 | -0.778 | 14.749 | 1.00 | 14.50 | A | C |
| ATOM | 2063 | CB | THR A 278 | 13.187 | -1.700 | 14.301 | 1.00 | 15.23 | A | C |
| ATOM | 2064 | OG1 | THR A 278 | 13.744 | -2.825 | 13.607 | 1.00 | 19.10 | A | O |
| ATOM | 2065 | CG2 | THR A 278 | 12.304 | -0.986 | 13.245 | 1.00 | 17.53 | A | C |
| ATOM | 2066 | C | THR A 278 | 13.760 | 0.394 | 15.526 | 1.00 | 13.49 | A | C |
| ATOM | 2067 | O | THR A 278 | 13.028 | 0.210 | 16.485 | 1.00 | 13.51 | A | O |
| ATOM | 2068 | N | PRO A 279 | 14.104 | 1.612 | 15.134 | 1.00 | 12.55 | A | N |
| ATOM | 2069 | CA | PRO A 279 | 13.679 | 2.803 | 15.896 | 1.00 | 11.32 | A | C |
| ATOM | 2070 | CB | PRO A 279 | 14.520 | 3.920 | 15.277 | 1.00 | 11.71 | A | C |
| ATOM | 2071 | CG | PRO A 279 | 14.682 | 3.493 | 13.842 | 1.00 | 11.40 | A | C |
| ATOM | 2072 | CD | PRO A 279 | 14.817 | 1.975 | 13.895 | 1.00 | 12.28 | A | C |
| ATOM | 2073 | C | PRO A 279 | 12.211 | 3.055 | 15.672 | 1.00 | 11.90 | A | C |
| ATOM | 2074 | O | PRO A 279 | 11.786 | 3.053 | 14.516 | 1.00 | 12.19 | A | O |
| ATOM | 2075 | N | LYS A 280 | 11.438 | 3.212 | 16.743 | 1.00 | 11.50 | A | N |
| ATOM | 2076 | CA | LYS A 280 | 10.020 | 3.518 | 16.639 | 1.00 | 12.77 | A | C |
| ATOM | 2077 | CB | LYS A 280 | 9.354 | 3.389 | 18.024 | 1.00 | 13.46 | A | C |
| ATOM | 2078 | CG | LYS A 280 | 9.324 | 1.993 | 18.573 | 1.00 | 15.21 | A | C |
| ATOM | 2079 | CD | LYS A 280 | 8.273 | 1.192 | 17.861 | 1.00 | 20.42 | A | C |
| ATOM | 2080 | CE | LYS A 280 | 8.012 | -0.146 | 18.555 | 1.00 | 23.45 | A | C |
| ATOM | 2081 | NZ | LYS A 280 | 6.935 | -0.858 | 17.808 | 1.00 | 26.12 | A | N |
| ATOM | 2082 | C | LYS A 280 | 9.811 | 4.951 | 16.120 | 1.00 | 11.96 | A | C |
| ATOM | 2083 | O | LYS A 280 | 10.710 | 5.782 | 16.200 | 1.00 | 13.23 | A | O |
| ATOM | 2084 | N | PRO A 281 | 8.666 | 5.233 | 15.512 | 1.00 | 12.23 | A | N |
| ATOM | 2085 | CA | PRO A 281 | 8.370 | 6.608 | 15.073 | 1.00 | 11.69 | A | C |
| ATOM | 2086 | CB | PRO A 281 | 6.897 | 6.540 | 14.763 | 1.00 | 12.25 | A | C |
| ATOM | 2087 | CG | PRO A 281 | 6.755 | 5.162 | 14.210 | 1.00 | 12.66 | A | C |
| ATOM | 2088 | CD | PRO A 281 | 7.592 | 4.300 | 15.126 | 1.00 | 11.87 | A | C |
| ATOM | 2089 | C | PRO A 281 | 8.682 | 7.678 | 16.105 | 1.00 | 11.94 | A | C |
| ATOM | 2090 | O | PRO A 281 | 9.287 | 8.708 | 15.734 | 1.00 | 11.60 | A | O |
| ATOM | 2091 | N | SER A 282 | 8.303 | 7.447 | 17.374 | 1.00 | 11.91 | A | N |
| ATOM | 2092 | CA | SER A 282 | 8.579 | 8.404 | 18.442 | 1.00 | 11.78 | A | C |
| ATOM | 2093 | CB | SER A 282 | 8.017 | 7.930 | 19.789 | 1.00 | 11.85 | A | C |
| ATOM | 2094 | OG | SER A 282 | 8.503 | 6.639 | 20.117 | 1.00 | 12.30 | A | O |
| ATOM | 2095 | C | SER A 282 | 10.049 | 8.704 | 18.654 | 1.00 | 11.36 | A | C |
| ATOM | 2096 | O | SER A 282 | 10.402 | 9.835 | 19.014 | 1.00 | 11.10 | A | O |
| ATOM | 2097 | N | LEU A 283 | 10.896 | 7.696 | 18.498 | 1.00 | 11.81 | A | N |
| ATOM | 2098 | CA | LEU A 283 | 12.332 | 7.889 | 18.642 | 1.00 | 11.66 | A | C |
| ATOM | 2099 | CB | LEU A 283 | 13.042 | 6.532 | 18.856 | 1.00 | 11.73 | A | C |
| ATOM | 2100 | CG | LEU A 283 | 14.575 | 6.628 | 18.893 | 1.00 | 11.60 | A | C |
| ATOM | 2101 | CD1 | LEU A 283 | 15.029 | 7.501 | 20.001 | 1.00 | 10.08 | A | C |
| ATOM | 2102 | CD2 | LEU A 283 | 15.180 | 5.233 | 19.066 | 1.00 | 15.86 | A | C |
| ATOM | 2103 | C | LEU A 283 | 12.953 | 8.650 | 17.465 | 1.00 | 11.15 | A | C |
| ATOM | 2104 | O | LEU A 283 | 13.812 | 9.515 | 17.644 | 1.00 | 11.72 | A | O |
| ATOM | 2105 | N | LEU A 284 | 12.575 | 8.305 | 16.244 | 1.00 | 11.65 | A | N |
| ATOM | 2106 | CA | LEU A 284 | 13.056 | 9.058 | 15.088 | 1.00 | 10.89 | A | C |
| ATOM | 2107 | CB | LEU A 284 | 12.493 | 8.470 | 13.802 | 1.00 | 10.71 | A | C |
| ATOM | 2108 | CG | LEU A 284 | 13.010 | 7.059 | 13.442 | 1.00 | 10.82 | A | C |
| ATOM | 2109 | CD1 | LEU A 284 | 12.102 | 6.419 | 12.399 | 1.00 | 10.74 | A | C |
| ATOM | 2110 | CD2 | LEU A 284 | 14.425 | 7.107 | 12.953 | 1.00 | 10.79 | A | C |
| ATOM | 2111 | C | LEU A 284 | 12.741 | 10.568 | 15.245 | 1.00 | 10.24 | A | C |
| ATOM | 2112 | O | LEU A 284 | 13.591 | 11.414 | 15.013 | 1.00 | 9.64 | A | O |
| ATOM | 2113 | N | LYS A 285 | 11.527 | 10.868 | 15.682 | 1.00 | 10.77 | A | N |
| ATOM | 2114 | CA | LYS A 285 | 11.054 | 12.217 | 15.890 | 1.00 | 10.81 | A | C |
| ATOM | 2115 | CB | LYS A 285 | 9.544 | 12.188 | 16.152 | 1.00 | 10.59 | A | C |
| ATOM | 2116 | CG | LYS A 285 | 8.909 | 13.531 | 16.527 | 1.00 | 10.18 | A | C |
| ATOM | 2117 | CD | LYS A 285 | 7.372 | 13.380 | 16.583 | 1.00 | 12.54 | A | C |
| ATOM | 2118 | CE | LYS A 285 | 6.660 | 14.630 | 17.085 | 1.00 | 11.16 | A | C |
| ATOM | 2119 | NZ | LYS A 285 | 5.159 | 14.525 | 16.941 | 1.00 | 9.27 | A | N |
| ATOM | 2120 | C | LYS A 285 | 11.816 | 12.886 | 17.037 | 1.00 | 10.86 | A | C |

```
ATOM   2121  O    LYS A 285    12.287  13.995  16.888  1.00 11.16    A    O
ATOM   2122  N    ALA A 286    11.964  12.194  18.156  1.00 10.94    A    N
ATOM   2123  CA   ALA A 286    12.744  12.722  19.280  1.00 11.16    A    C
ATOM   2124  CB   ALA A 286    12.657  11.813  20.437  1.00 11.37    A    C
ATOM   2125  C    ALA A 286    14.206  12.952  18.897  1.00 10.98    A    C
ATOM   2126  O    ALA A 286    14.794  13.947  19.275  1.00 10.07    A    O
ATOM   2127  N    ALA A 287    14.778  12.048  18.115  1.00 11.61    A    N
ATOM   2128  CA   ALA A 287    16.175  12.206  17.679  1.00 12.13    A    C
ATOM   2129  CB   ALA A 287    16.692  10.922  17.034  1.00 11.58    A    C
ATOM   2130  C    ALA A 287    16.349  13.411  16.742  1.00 12.42    A    C
ATOM   2131  O    ALA A 287    17.310  14.165  16.873  1.00 11.82    A    O
ATOM   2132  N    LEU A 288    15.407  13.623  15.826  1.00 12.37    A    N
ATOM   2133  CA   LEU A 288    15.473  14.808  14.956  1.00 13.09    A    C
ATOM   2134  CB   LEU A 288    14.357  14.775  13.917  1.00 13.67    A    C
ATOM   2135  CG   LEU A 288    14.552  13.833  12.736  1.00 15.39    A    C
ATOM   2136  CD1  LEU A 288    13.379  14.033  11.840  1.00 19.44    A    C
ATOM   2137  CD2  LEU A 288    15.842  14.113  11.974  1.00 15.17    A    C
ATOM   2138  C    LEU A 288    15.329  16.105  15.747  1.00 12.69    A    C
ATOM   2139  O    LEU A 288    16.014  17.107  15.481  1.00 12.69    A    O
ATOM   2140  N    ILE A 289    14.412  16.096  16.704  1.00 12.33    A    N
ATOM   2141  CA   ILE A 289    14.195  17.261  17.546  1.00 12.69    A    C
ATOM   2142  CB   ILE A 289    12.920  17.084  18.397  1.00 12.55    A    C
ATOM   2143  CG1  ILE A 289    11.688  17.178  17.488  1.00 11.46    A    C
ATOM   2144  CD1  ILE A 289    10.404  16.725  18.104  1.00 10.00    A    C
ATOM   2145  CG2  ILE A 289    12.869  18.098  19.506  1.00 13.54    A    C
ATOM   2146  C    ILE A 289    15.412  17.596  18.426  1.00 12.74    A    C
ATOM   2147  O    ILE A 289    15.844  18.746  18.442  1.00 13.36    A    O
ATOM   2148  N    ALA A 290    15.975  16.612  19.132  1.00 11.71    A    N
ATOM   2149  CA   ALA A 290    17.132  16.854  19.976  1.00 11.73    A    C
ATOM   2150  CB   ALA A 290    17.529  15.593  20.698  1.00 12.08    A    C
ATOM   2151  C    ALA A 290    18.326  17.370  19.191  1.00 11.95    A    C
ATOM   2152  O    ALA A 290    19.114  18.198  19.689  1.00 10.65    A    O
ATOM   2153  N    GLY A 291    18.472  16.866  17.979  1.00 11.84    A    N
ATOM   2154  CA   GLY A 291    19.633  17.207  17.171  1.00 12.60    A    C
ATOM   2155  C    GLY A 291    19.466  18.506  16.400  1.00 12.44    A    C
ATOM   2156  O    GLY A 291    20.437  19.018  15.847  1.00 11.56    A    O
ATOM   2157  N    ALA A 292    18.249  19.056  16.378  1.00 12.96    A    N
ATOM   2158  CA   ALA A 292    17.960  20.238  15.562  1.00 13.11    A    C
ATOM   2159  CB   ALA A 292    16.454  20.468  15.434  1.00 12.99    A    C
ATOM   2160  C    ALA A 292    18.655  21.499  16.075  1.00 13.45    A    C
ATOM   2161  O    ALA A 292    18.954  21.612  17.252  1.00 13.95    A    O
ATOM   2162  N    ALA A 293    18.848  22.456  15.173  1.00 13.18    A    N
ATOM   2163  CA   ALA A 293    19.567  23.694  15.450  1.00 13.43    A    C
ATOM   2164  CB   ALA A 293    20.508  24.012  14.284  1.00 13.22    A    C
ATOM   2165  C    ALA A 293    18.611  24.866  15.637  1.00 13.54    A    C
ATOM   2166  O    ALA A 293    17.739  25.107  14.812  1.00 13.39    A    O
ATOM   2167  N    ASP A 294    18.838  25.626  16.691  1.00 13.60    A    N
ATOM   2168  CA   ASP A 294    18.167  26.895  16.899  1.00 14.04    A    C
ATOM   2169  CB   ASP A 294    18.590  27.428  18.270  1.00 14.33    A    C
ATOM   2170  CG   ASP A 294    17.918  28.728  18.634  1.00 14.83    A    C
ATOM   2171  OD1  ASP A 294    18.142  29.160  19.799  1.00 11.77    A    O
ATOM   2172  OD2  ASP A 294    17.144  29.360  17.861  1.00 10.92    A    O
ATOM   2173  C    ASP A 294    18.620  27.814  15.774  1.00 13.72    A    C
ATOM   2174  O    ASP A 294    19.801  28.075  15.636  1.00 14.66    A    O
ATOM   2175  N    VAL A 295    17.696  28.304  14.956  1.00 14.43    A    N
ATOM   2176  CA   VAL A 295    18.057  29.217  13.853  1.00 14.05    A    C
ATOM   2177  CB   VAL A 295    16.957  29.283  12.730  1.00 13.77    A    C
ATOM   2178  CG1  VAL A 295    16.670  27.907  12.184  1.00 13.93    A    C
ATOM   2179  CG2  VAL A 295    15.680  29.956  13.255  1.00 12.53    A    C
ATOM   2180  C    VAL A 295    18.352  30.622  14.346  1.00 14.82    A    C
ATOM   2181  O    VAL A 295    18.707  31.497  13.558  1.00 15.77    A    O
ATOM   2182  N    GLY A 296    18.190  30.857  15.646  1.00 14.99    A    N
ATOM   2183  CA   GLY A 296    18.450  32.159  16.208  1.00 15.10    A    C
```

```
ATOM   2184  C    GLY A 296   17.282  32.821  16.906  1.00  15.03    A  C
ATOM   2185  O    GLY A 296   17.458  33.886  17.489  1.00  15.00    A  O
ATOM   2186  N    LEU A 297   16.114  32.180  16.887  1.00  16.05    A  N
ATOM   2187  CA   LEU A 297   14.903  32.728  17.501  1.00  15.21    A  C
ATOM   2188  CB   LEU A 297   13.704  32.420  16.605  1.00  14.95    A  C
ATOM   2189  CG   LEU A 297   13.881  32.962  15.179  1.00  17.47    A  C
ATOM   2190  CD1  LEU A 297   12.718  32.507  14.330  1.00  19.73    A  C
ATOM   2191  CD2  LEU A 297   13.939  34.500  15.204  1.00  18.75    A  C
ATOM   2192  C    LEU A 297   14.645  32.187  18.903  1.00  15.26    A  C
ATOM   2193  O    LEU A 297   13.782  32.682  19.636  1.00  14.95    A  O
ATOM   2194  N    GLY A 298   15.380  31.152  19.266  1.00  15.28    A  N
ATOM   2195  CA   GLY A 298   15.251  30.546  20.570  1.00  15.27    A  C
ATOM   2196  C    GLY A 298   14.024  29.691  20.721  1.00  15.55    A  C
ATOM   2197  O    GLY A 298   13.173  29.572  19.817  1.00  15.07    A  O
ATOM   2198  N    PHE A 299   13.941  29.090  21.894  1.00  15.47    A  N
ATOM   2199  CA   PHE A 299   12.820  28.269  22.266  1.00  16.76    A  C
ATOM   2200  CB   PHE A 299   13.279  26.818  22.539  1.00  16.33    A  C
ATOM   2201  CG   PHE A 299   14.108  26.237  21.435  1.00  16.07    A  C
ATOM   2202  CD1  PHE A 299   13.572  26.047  20.167  1.00  15.70    A  C
ATOM   2203  CE1  PHE A 299   14.349  25.544  19.132  1.00  15.95    A  C
ATOM   2204  CZ   PHE A 299   15.660  25.233  19.357  1.00  15.27    A  C
ATOM   2205  CE2  PHE A 299   16.217  25.432  20.610  1.00  15.20    A  C
ATOM   2206  CD2  PHE A 299   15.436  25.929  21.642  1.00  17.28    A  C
ATOM   2207  C    PHE A 299   12.200  28.872  23.504  1.00  17.91    A  C
ATOM   2208  O    PHE A 299   12.886  29.548  24.280  1.00  19.79    A  O
ATOM   2209  N    PRO A 300   10.904  28.683  23.680  1.00  19.34    A  N
ATOM   2210  CA   PRO A 300   10.054  27.945  22.747  1.00  20.29    A  C
ATOM   2211  CB   PRO A 300    8.819  27.671  23.583  1.00  20.94    A  C
ATOM   2212  CG   PRO A 300    8.712  28.898  24.513  1.00  19.43    A  C
ATOM   2213  CD   PRO A 300   10.120  29.311  24.758  1.00  20.25    A  C
ATOM   2214  C    PRO A 300    9.672  28.834  21.581  1.00  20.84    A  C
ATOM   2215  O    PRO A 300    9.921  30.043  21.655  1.00  21.44    A  O
ATOM   2216  N    ASN A 301    9.076  28.282  20.524  1.00  21.28    A  N
ATOM   2217  CA   ASN A 301    8.874  29.078  19.319  1.00  21.53    A  C
ATOM   2218  CB   ASN A 301   10.198  29.173  18.567  1.00  21.34    A  C
ATOM   2219  CG   ASN A 301   10.320  30.458  17.764  1.00  20.14    A  C
ATOM   2220  OD1  ASN A 301    9.875  30.553  16.607  1.00  17.42    A  O
ATOM   2221  ND2  ASN A 301   10.920  31.454  18.371  1.00  22.84    A  N
ATOM   2222  C    ASN A 301    7.823  28.534  18.355  1.00  22.14    A  C
ATOM   2223  O    ASN A 301    7.868  27.379  17.978  1.00  23.49    A  O
ATOM   2224  N    GLY A 302    6.970  29.416  17.854  1.00  22.24    A  N
ATOM   2225  CA   GLY A 302    5.912  29.031  16.932  1.00  21.65    A  C
ATOM   2226  C    GLY A 302    6.292  29.165  15.475  1.00  20.83    A  C
ATOM   2227  O    GLY A 302    5.574  28.669  14.603  1.00  21.22    A  O
ATOM   2228  N    ASN A 303    7.446  29.787  15.225  1.00  19.62    A  N
ATOM   2229  CA   ASN A 303    7.981  30.005  13.886  1.00  17.66    A  C
ATOM   2230  CB   ASN A 303    8.705  31.349  13.816  1.00  17.45    A  C
ATOM   2231  CG   ASN A 303    7.872  32.473  14.381  1.00  20.84    A  C
ATOM   2232  OD1  ASN A 303    6.802  32.779  13.847  1.00  21.34    A  O
ATOM   2233  ND2  ASN A 303    8.305  33.033  15.530  1.00  22.58    A  N
ATOM   2234  C    ASN A 303    8.926  28.922  13.414  1.00  15.61    A  C
ATOM   2235  O    ASN A 303    8.831  28.481  12.239  1.00  13.70    A  O
ATOM   2236  N    GLN A 304    9.841  28.506  14.298  1.00  14.18    A  N
ATOM   2237  CA   GLN A 304   10.855  27.513  13.946  1.00  13.13    A  C
ATOM   2238  CB   GLN A 304   12.235  28.005  14.316  1.00  13.28    A  C
ATOM   2239  CG   GLN A 304   12.556  27.976  15.811  1.00  12.80    A  C
ATOM   2240  CD   GLN A 304   14.020  28.087  16.123  1.00  11.63    A  C
ATOM   2241  OE1  GLN A 304   14.842  27.582  15.386  1.00  13.19    A  O
ATOM   2242  NE2  GLN A 304   14.352  28.794  17.223  1.00   9.76    A  N
ATOM   2243  C    GLN A 304   10.601  26.128  14.582  1.00  13.52    A  C
ATOM   2244  O    GLN A 304   11.372  25.208  14.381  1.00  13.66    A  O
ATOM   2245  N    GLY A 305    9.537  25.972  15.343  1.00  12.81    A  N
ATOM   2246  CA   GLY A 305    9.351  24.745  16.101  1.00  12.54    A  C
```

```
ATOM   2247  C   GLY A 305   10.578  24.498  16.970  1.00  12.99      A    C
ATOM   2248  O   GLY A 305   11.062  25.409  17.633  1.00  11.36      A    O
ATOM   2249  N   TRP A 306   11.107  23.272  16.926  1.00  12.12      A    N
ATOM   2250  CA  TRP A 306   12.286  22.898  17.701  1.00  12.07      A    C
ATOM   2251  CB  TRP A 306   12.130  21.473  18.254  1.00  12.05      A    C
ATOM   2252  CG  TRP A 306   10.866  21.349  19.092  1.00  11.34      A    C
ATOM   2253  CD1 TRP A 306    9.755  20.611  18.805  1.00  12.28      A    C
ATOM   2254  NE1 TRP A 306    8.812  20.766  19.794  1.00  13.42      A    N
ATOM   2255  CE2 TRP A 306    9.316  21.592  20.761  1.00  12.23      A    C
ATOM   2256  CD2 TRP A 306   10.616  21.963  20.359  1.00  13.78      A    C
ATOM   2257  CE3 TRP A 306   11.344  22.822  21.183  1.00  13.41      A    C
ATOM   2258  CZ3 TRP A 306   10.780  23.247  22.365  1.00  12.96      A    C
ATOM   2259  CH2 TRP A 306    9.488  22.854  22.735  1.00  13.70      A    C
ATOM   2260  CZ2 TRP A 306    8.744  22.035  21.945  1.00  14.65      A    C
ATOM   2261  C   TRP A 306   13.562  23.046  16.890  1.00  12.73      A    C
ATOM   2262  O   TRP A 306   14.632  22.620  17.318  1.00  14.46      A    O
ATOM   2263  N   GLY A 307   13.479  23.716  15.741  1.00  13.10      A    N
ATOM   2264  CA  GLY A 307   14.679  24.111  15.015  1.00  12.18      A    C
ATOM   2265  C   GLY A 307   14.871  23.392  13.685  1.00  11.91      A    C
ATOM   2266  O   GLY A 307   14.008  22.628  13.238  1.00  11.20      A    O
ATOM   2267  N   ARG A 308   16.007  23.658  13.046  1.00  11.60      A    N
ATOM   2268  CA  ARG A 308   16.299  23.167  11.701  1.00  12.01      A    C
ATOM   2269  CB  ARG A 308   17.153  24.223  10.961  1.00  12.63      A    C
ATOM   2270  CG  ARG A 308   17.388  23.970   9.462  1.00  13.43      A    C
ATOM   2271  CD  ARG A 308   18.284  25.025   8.807  1.00  13.61      A    C
ATOM   2272  NE  ARG A 308   19.563  25.031   9.510  1.00  16.45      A    N
ATOM   2273  CZ  ARG A 308   20.127  26.086  10.086  1.00  16.43      A    C
ATOM   2274  NH1 ARG A 308   21.257  25.902  10.762  1.00  14.45      A    N
ATOM   2275  NH2 ARG A 308   19.660  27.319   9.902  1.00  13.23      A    N
ATOM   2276  C   ARG A 308   17.055  21.837  11.770  1.00  12.38      A    C
ATOM   2277  O   ARG A 308   18.094  21.742  12.434  1.00  12.82      A    O
ATOM   2278  N   VAL A 309   16.549  20.817  11.080  1.00  11.99      A    N
ATOM   2279  CA  VAL A 309   17.170  19.498  11.096  1.00  12.04      A    C
ATOM   2280  CB  VAL A 309   16.553  18.573  10.051  1.00  11.35      A    C
ATOM   2281  CG1 VAL A 309   17.354  17.272   9.961  1.00  11.45      A    C
ATOM   2282  CG2 VAL A 309   15.047  18.335  10.377  1.00  11.38      A    C
ATOM   2283  C   VAL A 309   18.667  19.545  10.868  1.00  12.28      A    C
ATOM   2284  O   VAL A 309   19.148  20.157   9.930  1.00  11.94      A    O
ATOM   2285  N   THR A 310   19.393  18.904  11.760  1.00  13.11      A    N
ATOM   2286  CA  THR A 310   20.854  18.814  11.675  1.00  12.86      A    C
ATOM   2287  CB  THR A 310   21.471  19.719  12.723  1.00  13.26      A    C
ATOM   2288  OG1 THR A 310   21.044  21.090  12.516  1.00  12.98      A    O
ATOM   2289  CG2 THR A 310   22.962  19.747  12.610  1.00  13.68      A    C
ATOM   2290  C   THR A 310   21.197  17.347  11.911  1.00  13.94      A    C
ATOM   2291  O   THR A 310   21.411  16.896  13.064  1.00  14.93      A    O
ATOM   2292  N   LEU A 311   21.269  16.601  10.818  1.00  13.28      A    N
ATOM   2293  CA  LEU A 311   21.110  15.149  10.879  1.00  14.11      A    C
ATOM   2294  CB  LEU A 311   20.956  14.589   9.463  1.00  13.67      A    C
ATOM   2295  CG  LEU A 311   20.879  13.078   9.311  1.00  13.50      A    C
ATOM   2296  CD1 LEU A 311   19.749  12.522  10.109  1.00  15.11      A    C
ATOM   2297  CD2 LEU A 311   20.749  12.688   7.849  1.00  14.22      A    C
ATOM   2298  C   LEU A 311   22.252  14.455  11.605  1.00  14.58      A    C
ATOM   2299  O   LEU A 311   22.012  13.521  12.345  1.00  14.74      A    O
ATOM   2300  N   ASP A 312   23.483  14.959  11.462  1.00  15.58      A    N
ATOM   2301  CA  ASP A 312   24.624  14.307  12.111  1.00  16.68      A    C
ATOM   2302  CB  ASP A 312   25.984  14.797  11.582  1.00  16.93      A    C
ATOM   2303  CG  ASP A 312   26.222  16.245  11.822  1.00  19.05      A    C
ATOM   2304  OD1 ASP A 312   27.394  16.614  11.755  1.00  25.94      A    O
ATOM   2305  OD2 ASP A 312   25.348  17.090  12.090  1.00  18.31      A    O
ATOM   2306  C   ASP A 312   24.560  14.330  13.615  1.00  16.48      A    C
ATOM   2307  O   ASP A 312   24.965  13.381  14.241  1.00  17.40      A    O
ATOM   2308  N   LYS A 313   23.976  15.364  14.202  1.00  16.32      A    N
ATOM   2309  CA  LYS A 313   23.755  15.366  15.641  1.00  16.35      A    C
```

68

| ATOM | 2310 | CB  | LYS A 313 | 23.363 | 16.773 | 16.130 | 1.00 | 17.66 | A | C |
| ATOM | 2311 | CG  | LYS A 313 | 24.430 | 17.848 | 15.938 | 1.00 | 20.65 | A | C |
| ATOM | 2312 | CD  | LYS A 313 | 24.735 | 18.590 | 17.250 | 1.00 | 28.29 | A | C |
| ATOM | 2313 | CE  | LYS A 313 | 25.619 | 17.746 | 18.162 | 1.00 | 31.10 | A | C |
| ATOM | 2314 | NZ  | LYS A 313 | 26.458 | 18.529 | 19.131 | 1.00 | 32.31 | A | N |
| ATOM | 2315 | C   | LYS A 313 | 22.666 | 14.379 | 16.100 | 1.00 | 15.36 | A | C |
| ATOM | 2316 | O   | LYS A 313 | 22.662 | 13.968 | 17.258 | 1.00 | 15.49 | A | O |
| ATOM | 2317 | N   | SER A 314 | 21.755 | 14.007 | 15.212 | 1.00 | 14.12 | A | N |
| ATOM | 2318 | CA  | SER A 314 | 20.700 | 13.047 | 15.537 | 1.00 | 13.37 | A | C |
| ATOM | 2319 | CB  | SER A 314 | 19.497 | 13.217 | 14.590 | 1.00 | 13.56 | A | C |
| ATOM | 2320 | OG  | SER A 314 | 18.889 | 14.489 | 14.723 | 1.00 | 11.16 | A | O |
| ATOM | 2321 | C   | SER A 314 | 21.148 | 11.581 | 15.505 | 1.00 | 14.53 | A | C |
| ATOM | 2322 | O   | SER A 314 | 20.507 | 10.717 | 16.112 | 1.00 | 14.30 | A | O |
| ATOM | 2323 | N   | LEU A 315 | 22.209 | 11.285 | 14.771 | 1.00 | 14.58 | A | N |
| ATOM | 2324 | CA  | LEU A 315 | 22.563 | 9.896  | 14.484 | 1.00 | 15.64 | A | C |
| ATOM | 2325 | CB  | LEU A 315 | 23.750 | 9.824  | 13.505 | 1.00 | 15.15 | A | C |
| ATOM | 2326 | CG  | LEU A 315 | 23.506 | 10.436 | 12.143 | 1.00 | 14.88 | A | C |
| ATOM | 2327 | CD1 | LEU A 315 | 24.834 | 10.445 | 11.360 | 1.00 | 16.67 | A | C |
| ATOM | 2328 | CD2 | LEU A 315 | 22.401 | 9.683  | 11.411 | 1.00 | 16.28 | A | C |
| ATOM | 2329 | C   | LEU A 315 | 22.905 | 9.095  | 15.733 | 1.00 | 16.16 | A | C |
| ATOM | 2330 | O   | LEU A 315 | 22.442 | 7.956  | 15.894 | 1.00 | 16.79 | A | O |
| ATOM | 2331 | N   | ASN A 316 | 23.692 | 9.686  | 16.631 | 1.00 | 17.60 | A | N |
| ATOM | 2332 | CA  | ASN A 316 | 24.235 | 8.915  | 17.749 | 1.00 | 17.99 | A | C |
| ATOM | 2333 | CB  | ASN A 316 | 25.757 | 8.837  | 17.694 | 1.00 | 19.59 | A | C |
| ATOM | 2334 | CG  | ASN A 316 | 26.264 | 7.879  | 16.601 | 1.00 | 22.56 | A | C |
| ATOM | 2335 | OD1 | ASN A 316 | 25.736 | 6.732  | 16.397 | 1.00 | 21.64 | A | O |
| ATOM | 2336 | ND2 | ASN A 316 | 27.321 | 8.320  | 15.910 | 1.00 | 23.13 | A | N |
| ATOM | 2337 | C   | ASN A 316 | 23.732 | 9.396  | 19.116 | 1.00 | 17.61 | A | C |
| ATOM | 2338 | O   | ASN A 316 | 24.390 | 9.212  | 20.152 | 1.00 | 15.93 | A | O |
| ATOM | 2339 | N   | VAL A 317 | 22.505 | 9.912  | 19.123 | 1.00 | 15.75 | A | N |
| ATOM | 2340 | CA  | VAL A 317 | 21.820 | 10.220 | 20.389 | 1.00 | 15.40 | A | C |
| ATOM | 2341 | CB  | VAL A 317 | 20.360 | 10.675 | 20.150 | 1.00 | 15.21 | A | C |
| ATOM | 2342 | CG1 | VAL A 317 | 20.335 | 11.940 | 19.350 | 1.00 | 16.28 | A | C |
| ATOM | 2343 | CG2 | VAL A 317 | 19.547 | 9.600  | 19.458 | 1.00 | 16.07 | A | C |
| ATOM | 2344 | C   | VAL A 317 | 21.803 | 8.995  | 21.323 | 1.00 | 14.67 | A | C |
| ATOM | 2345 | O   | VAL A 317 | 21.675 | 7.864  | 20.868 | 1.00 | 14.77 | A | O |
| ATOM | 2346 | N   | ALA A 318 | 21.932 | 9.225  | 22.627 | 1.00 | 13.95 | A | N |
| ATOM | 2347 | CA  | ALA A 318 | 21.623 | 8.186  | 23.603 | 1.00 | 13.64 | A | C |
| ATOM | 2348 | CB  | ALA A 318 | 22.196 | 8.523  | 24.940 | 1.00 | 13.50 | A | C |
| ATOM | 2349 | C   | ALA A 318 | 20.087 | 8.130  | 23.652 | 1.00 | 13.19 | A | C |
| ATOM | 2350 | O   | ALA A 318 | 19.416 | 9.168  | 23.498 | 1.00 | 13.46 | A | O |
| ATOM | 2351 | N   | PHE A 319 | 19.512 | 6.952  | 23.853 | 1.00 | 12.88 | A | N |
| ATOM | 2352 | CA  | PHE A 319 | 18.076 | 6.824  | 23.633 | 1.00 | 12.66 | A | C |
| ATOM | 2353 | CB  | PHE A 319 | 17.815 | 6.531  | 22.137 | 1.00 | 12.79 | A | C |
| ATOM | 2354 | CG  | PHE A 319 | 18.267 | 5.158  | 21.700 | 1.00 | 14.03 | A | C |
| ATOM | 2355 | CD1 | PHE A 319 | 19.504 | 4.980  | 21.107 | 1.00 | 12.44 | A | C |
| ATOM | 2356 | CE1 | PHE A 319 | 19.930 | 3.725  | 20.718 | 1.00 | 15.58 | A | C |
| ATOM | 2357 | CZ  | PHE A 319 | 19.115 | 2.619  | 20.921 | 1.00 | 15.26 | A | C |
| ATOM | 2358 | CE2 | PHE A 319 | 17.887 | 2.777  | 21.524 | 1.00 | 14.77 | A | C |
| ATOM | 2359 | CD2 | PHE A 319 | 17.461 | 4.047  | 21.905 | 1.00 | 15.05 | A | C |
| ATOM | 2360 | C   | PHE A 319 | 17.349 | 5.799  | 24.461 | 1.00 | 12.38 | A | C |
| ATOM | 2361 | O   | PHE A 319 | 17.947 | 4.918  | 25.055 | 1.00 | 12.11 | A | O |
| ATOM | 2362 | N   | VAL A 320 | 16.032 | 5.947  | 24.488 | 1.00 | 12.04 | A | N |
| ATOM | 2363 | CA  | VAL A 320 | 15.125 | 4.909  | 24.949 | 1.00 | 12.42 | A | C |
| ATOM | 2364 | CB  | VAL A 320 | 14.391 | 5.323  | 26.252 | 1.00 | 13.15 | A | C |
| ATOM | 2365 | CG1 | VAL A 320 | 13.237 | 4.331  | 26.589 | 1.00 | 13.46 | A | C |
| ATOM | 2366 | CG2 | VAL A 320 | 15.401 | 5.395  | 27.416 | 1.00 | 13.91 | A | C |
| ATOM | 2367 | C   | VAL A 320 | 14.137 | 4.758  | 23.802 | 1.00 | 11.88 | A | C |
| ATOM | 2368 | O   | VAL A 320 | 13.668 | 5.749  | 23.271 | 1.00 | 10.13 | A | O |
| ATOM | 2369 | N   | ASN A 321 | 13.824 | 3.520  | 23.441 | 1.00 | 11.82 | A | N |
| ATOM | 2370 | CA  | ASN A 321 | 13.018 | 3.205  | 22.261 | 1.00 | 11.75 | A | C |
| ATOM | 2371 | CB  | ASN A 321 | 13.858 | 2.323  | 21.313 | 1.00 | 11.75 | A | C |
| ATOM | 2372 | CG  | ASN A 321 | 13.214 | 2.117  | 19.944 | 1.00 | 12.10 | A | C |

| | | | | | | | | | | | | |
|---|---|---|---|---|---|---|---|---|---|---|---|---|
| ATOM | 2373 | OD1 | ASN | A | 321 | 12.506 | 3.005 | 19.437 | 1.00 | 12.86 | A | O |
| ATOM | 2374 | ND2 | ASN | A | 321 | 13.451 | 0.919 | 19.328 | 1.00 | 10.43 | A | N |
| ATOM | 2375 | C | ASN | A | 321 | 11.711 | 2.463 | 22.637 | 1.00 | 12.63 | A | C |
| ATOM | 2376 | O | ASN | A | 321 | 11.509 | 1.311 | 22.260 | 1.00 | 12.41 | A | O |
| ATOM | 2377 | N | GLU | A | 322 | 10.835 | 3.130 | 23.380 | 1.00 | 12.99 | A | N |
| ATOM | 2378 | CA | GLU | A | 322 | 9.542 | 2.552 | 23.760 | 1.00 | 13.23 | A | C |
| ATOM | 2379 | CB | GLU | A | 322 | 8.601 | 2.415 | 22.528 | 1.00 | 13.10 | A | C |
| ATOM | 2380 | CG | GLU | A | 322 | 8.146 | 3.794 | 22.013 | 1.00 | 12.18 | A | C |
| ATOM | 2381 | CD | GLU | A | 322 | 7.072 | 3.781 | 20.933 | 1.00 | 14.48 | A | C |
| ATOM | 2382 | OE1 | GLU | A | 322 | 6.747 | 4.884 | 20.429 | 1.00 | 15.20 | A | O |
| ATOM | 2383 | OE2 | GLU | A | 322 | 6.564 | 2.690 | 20.588 | 1.00 | 14.00 | A | O |
| ATOM | 2384 | C | GLU | A | 322 | 9.654 | 1.239 | 24.556 | 1.00 | 14.04 | A | C |
| ATOM | 2385 | O | GLU | A | 322 | 8.778 | 0.385 | 24.468 | 1.00 | 12.70 | A | O |
| ATOM | 2386 | N | THR | A | 323 | 10.688 | 1.122 | 25.387 | 1.00 | 13.33 | A | N |
| ATOM | 2387 | CA | THR | A | 323 | 10.907 | -0.101 | 26.145 | 1.00 | 14.72 | A | C |
| ATOM | 2388 | CB | THR | A | 323 | 12.375 | -0.466 | 26.173 | 1.00 | 14.45 | A | C |
| ATOM | 2389 | OG1 | THR | A | 323 | 13.168 | 0.721 | 26.401 | 1.00 | 16.74 | A | O |
| ATOM | 2390 | CG2 | THR | A | 323 | 12.813 | -0.980 | 24.810 | 1.00 | 15.39 | A | C |
| ATOM | 2391 | C | THR | A | 323 | 10.397 | -0.045 | 27.589 | 1.00 | 15.63 | A | C |
| ATOM | 2392 | O | THR | A | 323 | 10.572 | -1.011 | 28.319 | 1.00 | 14.71 | A | O |
| ATOM | 2393 | N | SER | A | 324 | 9.796 | 1.073 | 28.002 | 1.00 | 15.41 | A | N |
| ATOM | 2394 | CA | SER | A | 324 | 9.176 | 1.159 | 29.341 | 1.00 | 15.78 | A | C |
| ATOM | 2395 | CB | SER | A | 324 | 10.008 | 2.045 | 30.272 | 1.00 | 15.94 | A | C |
| ATOM | 2396 | OG | SER | A | 324 | 11.281 | 1.460 | 30.594 | 1.00 | 17.86 | A | O |
| ATOM | 2397 | C | SER | A | 324 | 7.739 | 1.723 | 29.257 | 1.00 | 15.74 | A | C |
| ATOM | 2398 | O | SER | A | 324 | 7.558 | 2.937 | 29.315 | 1.00 | 15.93 | A | O |
| ATOM | 2399 | N | PRO | A | 325 | 6.729 | 0.864 | 29.118 | 1.00 | 16.08 | A | N |
| ATOM | 2400 | CA | PRO | A | 325 | 5.326 | 1.306 | 29.170 | 1.00 | 17.17 | A | C |
| ATOM | 2401 | CB | PRO | A | 325 | 4.525 | 0.035 | 28.823 | 1.00 | 17.76 | A | C |
| ATOM | 2402 | CG | PRO | A | 325 | 5.454 | -1.106 | 29.084 | 1.00 | 17.45 | A | C |
| ATOM | 2403 | CD | PRO | A | 325 | 6.845 | -0.585 | 28.885 | 1.00 | 16.38 | A | C |
| ATOM | 2404 | C | PRO | A | 325 | 4.926 | 1.815 | 30.548 | 1.00 | 17.68 | A | C |
| ATOM | 2405 | O | PRO | A | 325 | 5.277 | 1.211 | 31.553 | 1.00 | 18.68 | A | O |
| ATOM | 2406 | N | LEU | A | 326 | 4.204 | 2.916 | 30.596 | 1.00 | 18.30 | A | N |
| ATOM | 2407 | CA | LEU | A | 326 | 3.796 | 3.491 | 31.871 | 1.00 | 18.53 | A | C |
| ATOM | 2408 | CB | LEU | A | 326 | 4.410 | 4.890 | 32.059 | 1.00 | 18.37 | A | C |
| ATOM | 2409 | CG | LEU | A | 326 | 5.938 | 5.027 | 32.161 | 1.00 | 19.30 | A | C |
| ATOM | 2410 | CD1 | LEU | A | 326 | 6.350 | 6.502 | 32.200 | 1.00 | 20.03 | A | C |
| ATOM | 2411 | CD2 | LEU | A | 326 | 6.471 | 4.338 | 33.387 | 1.00 | 17.55 | A | C |
| ATOM | 2412 | C | LEU | A | 326 | 2.287 | 3.605 | 31.982 | 1.00 | 19.08 | A | C |
| ATOM | 2413 | O | LEU | A | 326 | 1.589 | 3.926 | 30.989 | 1.00 | 18.42 | A | O |
| ATOM | 2414 | N | SER | A | 327 | 1.810 | 3.326 | 33.201 | 1.00 | 19.13 | A | N |
| ATOM | 2415 | CA | SER | A | 327 | 0.438 | 3.607 | 33.672 | 1.00 | 19.58 | A | C |
| ATOM | 2416 | CB | SER | A | 327 | -0.123 | 2.391 | 34.397 | 1.00 | 19.04 | A | C |
| ATOM | 2417 | OG | SER | A | 327 | -0.176 | 1.357 | 33.434 | 1.00 | 19.38 | A | O |
| ATOM | 2418 | C | SER | A | 327 | 0.558 | 4.902 | 34.476 | 1.00 | 19.58 | A | C |
| ATOM | 2419 | O | SER | A | 327 | 1.609 | 5.154 | 35.075 | 1.00 | 18.88 | A | O |
| ATOM | 2420 | N | THR | A | 328 | -0.505 | 5.696 | 34.595 | 1.00 | 20.38 | A | N |
| ATOM | 2421 | CA | THR | A | 328 | -0.861 | 6.454 | 35.789 | 1.00 | 19.96 | A | C |
| ATOM | 2422 | CB | THR | A | 328 | -2.343 | 6.678 | 35.951 | 1.00 | 20.43 | A | C |
| ATOM | 2423 | OG1 | THR | A | 328 | -2.870 | 7.047 | 34.681 | 1.00 | 20.32 | A | O |
| ATOM | 2424 | CG2 | THR | A | 328 | -2.602 | 7.922 | 36.842 | 1.00 | 21.84 | A | C |
| ATOM | 2425 | C | THR | A | 328 | -0.102 | 6.262 | 37.084 | 1.00 | 19.51 | A | C |
| ATOM | 2426 | O | THR | A | 328 | -0.223 | 5.222 | 37.739 | 1.00 | 19.59 | A | O |
| ATOM | 2427 | N | SER | A | 329 | 0.732 | 7.268 | 37.356 | 1.00 | 18.40 | A | N |
| ATOM | 2428 | CA | SER | A | 329 | 1.489 | 7.464 | 38.588 | 1.00 | 19.19 | A | C |
| ATOM | 2429 | CB | BSER | A | 329 | 0.679 | 7.017 | 39.818 | 0.50 | 19.34 | A | C |
| ATOM | 2430 | CB | ASER | A | 329 | 0.629 | 7.141 | 39.833 | 0.50 | 19.52 | A | C |
| ATOM | 2431 | OG | BSER | A | 329 | 0.653 | 5.599 | 39.887 | 0.50 | 18.73 | A | O |
| ATOM | 2432 | OG | ASER | A | 329 | -0.672 | 7.722 | 39.718 | 0.50 | 20.52 | A | O |
| ATOM | 2433 | C | SER | A | 329 | 2.792 | 6.686 | 38.588 | 1.00 | 18.71 | A | C |
| ATOM | 2434 | O | SER | A | 329 | 3.533 | 6.753 | 39.550 | 1.00 | 18.11 | A | O |
| ATOM | 2435 | N | GLN | A | 330 | 3.066 | 5.936 | 37.524 | 1.00 | 17.70 | A | N |

| ATOM | 2436 | CA | GLN A 330 | 4.339 | 5.250 | 37.420 | 1.00 | 17.71 | A | C |
|---|---|---|---|---|---|---|---|---|---|---|
| ATOM | 2437 | CB | GLN A 330 | 4.193 | 4.001 | 36.566 | 1.00 | 17.37 | A | C |
| ATOM | 2438 | CG | GLN A 330 | 3.233 | 2.970 | 37.168 | 1.00 | 17.60 | A | C |
| ATOM | 2439 | CD | GLN A 330 | 3.116 | 1.700 | 36.319 | 1.00 | 18.11 | A | C |
| ATOM | 2440 | OE1 | GLN A 330 | 3.305 | 1.763 | 35.119 | 1.00 | 16.08 | A | O |
| ATOM | 2441 | NE2 | GLN A 330 | 2.762 | 0.550 | 36.952 | 1.00 | 15.56 | A | N |
| ATOM | 2442 | C | GLN A 330 | 5.401 | 6.195 | 36.837 | 1.00 | 18.14 | A | C |
| ATOM | 2443 | O | GLN A 330 | 5.103 | 7.308 | 36.423 | 1.00 | 18.07 | A | O |
| ATOM | 2444 | N | LYS A 331 | 6.643 | 5.750 | 36.842 | 1.00 | 18.66 | A | N |
| ATOM | 2445 | CA | LYS A 331 | 7.710 | 6.524 | 36.276 | 1.00 | 19.23 | A | C |
| ATOM | 2446 | CB | LYS A 331 | 8.229 | 7.550 | 37.289 | 1.00 | 20.19 | A | C |
| ATOM | 2447 | CG | LYS A 331 | 8.972 | 6.934 | 38.450 | 1.00 | 23.65 | A | C |
| ATOM | 2448 | CD | LYS A 331 | 9.071 | 7.912 | 39.625 | 1.00 | 28.70 | A | C |
| ATOM | 2449 | CE | LYS A 331 | 9.954 | 7.332 | 40.754 | 1.00 | 31.64 | A | C |
| ATOM | 2450 | NZ | LYS A 331 | 10.409 | 8.388 | 41.729 | 1.00 | 34.93 | A | N |
| ATOM | 2451 | C | LYS A 331 | 8.804 | 5.589 | 35.820 | 1.00 | 18.87 | A | C |
| ATOM | 2452 | O | LYS A 331 | 8.887 | 4.410 | 36.261 | 1.00 | 18.18 | A | O |
| ATOM | 2453 | N | ALA A 332 | 9.587 | 6.091 | 34.873 | 1.00 | 17.37 | A | N |
| ATOM | 2454 | CA | ALA A 332 | 10.797 | 5.406 | 34.412 | 1.00 | 17.82 | A | C |
| ATOM | 2455 | CB | ALA A 332 | 10.689 | 5.068 | 32.941 | 1.00 | 17.06 | A | C |
| ATOM | 2456 | C | ALA A 332 | 11.991 | 6.324 | 34.650 | 1.00 | 17.33 | A | C |
| ATOM | 2457 | O | ALA A 332 | 11.999 | 7.480 | 34.213 | 1.00 | 16.52 | A | O |
| ATOM | 2458 | N | THR A 333 | 13.005 | 5.805 | 35.325 | 1.00 | 17.85 | A | N |
| ATOM | 2459 | CA | THR A 333 | 14.108 | 6.643 | 35.784 | 1.00 | 17.61 | A | C |
| ATOM | 2460 | CB | THR A 333 | 14.194 | 6.544 | 37.304 | 1.00 | 18.03 | A | C |
| ATOM | 2461 | OG1 | THR A 333 | 12.956 | 6.966 | 37.902 | 1.00 | 19.93 | A | O |
| ATOM | 2462 | CG2 | THR A 333 | 15.234 | 7.490 | 37.851 | 1.00 | 18.18 | A | C |
| ATOM | 2463 | C | THR A 333 | 15.410 | 6.186 | 35.159 | 1.00 | 17.36 | A | C |
| ATOM | 2464 | O | THR A 333 | 15.727 | 4.987 | 35.162 | 1.00 | 17.40 | A | O |
| ATOM | 2465 | N | TYR A 334 | 16.176 | 7.135 | 34.628 | 1.00 | 17.05 | A | N |
| ATOM | 2466 | CA | TYR A 334 | 17.437 | 6.840 | 33.986 | 1.00 | 17.42 | A | C |
| ATOM | 2467 | CB | TYR A 334 | 17.308 | 6.975 | 32.464 | 1.00 | 16.94 | A | C |
| ATOM | 2468 | CG | TYR A 334 | 16.144 | 6.230 | 31.860 | 1.00 | 16.41 | A | C |
| ATOM | 2469 | CD1 | TYR A 334 | 16.273 | 4.891 | 31.458 | 1.00 | 14.43 | A | C |
| ATOM | 2470 | CE1 | TYR A 334 | 15.205 | 4.205 | 30.912 | 1.00 | 14.63 | A | C |
| ATOM | 2471 | CZ | TYR A 334 | 13.977 | 4.846 | 30.772 | 1.00 | 15.02 | A | C |
| ATOM | 2472 | OH | TYR A 334 | 12.929 | 4.188 | 30.216 | 1.00 | 16.23 | A | O |
| ATOM | 2473 | CE2 | TYR A 334 | 13.819 | 6.147 | 31.153 | 1.00 | 15.62 | A | C |
| ATOM | 2474 | CD2 | TYR A 334 | 14.907 | 6.835 | 31.718 | 1.00 | 16.76 | A | C |
| ATOM | 2475 | C | TYR A 334 | 18.542 | 7.767 | 34.455 | 1.00 | 17.66 | A | C |
| ATOM | 2476 | O | TYR A 334 | 18.279 | 8.840 | 34.991 | 1.00 | 16.79 | A | O |
| ATOM | 2477 | N | SER A 335 | 19.783 | 7.375 | 34.169 | 1.00 | 17.44 | A | N |
| ATOM | 2478 | CA | SER A 335 | 20.939 | 8.218 | 34.442 | 1.00 | 18.62 | A | C |
| ATOM | 2479 | CB BSER A 335 | | 21.879 | 7.537 | 35.433 | 0.50 | 18.54 | A | C |
| ATOM | 2480 | CB ASER A 335 | | 21.916 | 7.518 | 35.393 | 0.50 | 18.59 | A | C |
| ATOM | 2481 | OG BSER A 335 | | 22.697 | 6.585 | 34.783 | 0.50 | 18.97 | A | O |
| ATOM | 2482 | OG ASER A 335 | | 21.316 | 7.174 | 36.629 | 0.50 | 19.43 | A | O |
| ATOM | 2483 | C | SER A 335 | 21.680 | 8.538 | 33.128 | 1.00 | 18.52 | A | C |
| ATOM | 2484 | O | SER A 335 | 21.698 | 7.720 | 32.221 | 1.00 | 18.14 | A | O |
| ATOM | 2485 | N | PHE A 336 | 22.298 | 9.715 | 33.049 | 1.00 | 17.92 | A | N |
| ATOM | 2486 | CA | PHE A 336 | 23.115 | 10.092 | 31.911 | 1.00 | 18.36 | A | C |
| ATOM | 2487 | CB | PHE A 336 | 22.324 | 10.949 | 30.900 | 1.00 | 18.52 | A | C |
| ATOM | 2488 | CG | PHE A 336 | 23.150 | 11.401 | 29.753 | 1.00 | 17.47 | A | C |
| ATOM | 2489 | CD1 | PHE A 336 | 23.733 | 12.667 | 29.739 | 1.00 | 18.88 | A | C |
| ATOM | 2490 | CE1 | PHE A 336 | 24.529 | 13.067 | 28.654 | 1.00 | 18.14 | A | C |
| ATOM | 2491 | CZ | PHE A 336 | 24.749 | 12.198 | 27.591 | 1.00 | 18.35 | A | C |
| ATOM | 2492 | CE2 | PHE A 336 | 24.174 | 10.936 | 27.601 | 1.00 | 18.49 | A | C |
| ATOM | 2493 | CD2 | PHE A 336 | 23.386 | 10.543 | 28.681 | 1.00 | 19.03 | A | C |
| ATOM | 2494 | C | PHE A 336 | 24.314 | 10.898 | 32.403 | 1.00 | 18.74 | A | C |
| ATOM | 2495 | O | PHE A 336 | 24.159 | 11.810 | 33.195 | 1.00 | 18.98 | A | O |
| ATOM | 2496 | N | THR A 337 | 25.504 | 10.547 | 31.938 | 1.00 | 19.28 | A | N |
| ATOM | 2497 | CA | THR A 337 | 26.733 | 11.219 | 32.364 | 1.00 | 19.77 | A | C |
| ATOM | 2498 | CB | THR A 337 | 27.879 | 10.201 | 32.343 | 1.00 | 20.11 | A | C |

| ATOM | 2499 | OG1 | THR A 337 | 27.609 | 9.175 | 33.321 | 1.00 | 19.75 | A | O |
|------|------|-----|-----------|--------|--------|--------|------|-------|---|---|
| ATOM | 2500 | CG2 | THR A 337 | 29.159 | 10.857 | 32.796 | 1.00 | 21.58 | A | C |
| ATOM | 2501 | C   | THR A 337 | 27.096 | 12.369 | 31.440 | 1.00 | 20.14 | A | C |
| ATOM | 2502 | O   | THR A 337 | 27.266 | 12.163 | 30.253 | 1.00 | 20.10 | A | O |
| ATOM | 2503 | N   | ALA A 338 | 27.181 | 13.571 | 32.000 | 1.00 | 19.74 | A | N |
| ATOM | 2504 | CA  | ALA A 338 | 27.487 | 14.793 | 31.259 | 1.00 | 20.23 | A | C |
| ATOM | 2505 | CB  | ALA A 338 | 26.468 | 15.858 | 31.588 | 1.00 | 19.43 | A | C |
| ATOM | 2506 | C   | ALA A 338 | 28.881 | 15.292 | 31.633 | 1.00 | 20.94 | A | C |
| ATOM | 2507 | O   | ALA A 338 | 29.389 | 14.991 | 32.710 | 1.00 | 19.43 | A | O |
| ATOM | 2508 | N   | GLN A 339 | 29.503 | 16.042 | 30.741 | 1.00 | 22.51 | A | N |
| ATOM | 2509 | CA  | GLN A 339 | 30.750 | 16.711 | 31.070 | 1.00 | 23.12 | A | C |
| ATOM | 2510 | CB  | GLN A 339 | 31.893 | 16.162 | 30.230 | 1.00 | 24.34 | A | C |
| ATOM | 2511 | CG  | GLN A 339 | 32.591 | 14.904 | 30.726 | 1.00 | 29.11 | A | C |
| ATOM | 2512 | CD  | GLN A 339 | 34.116 | 14.923 | 30.437 | 1.00 | 36.99 | A | C |
| ATOM | 2513 | OE1 | GLN A 339 | 34.841 | 13.991 | 30.825 | 1.00 | 41.39 | A | O |
| ATOM | 2514 | NE2 | GLN A 339 | 34.597 | 15.995 | 29.778 | 1.00 | 36.90 | A | N |
| ATOM | 2515 | C   | GLN A 339 | 30.543 | 18.167 | 30.722 | 1.00 | 23.27 | A | C |
| ATOM | 2516 | O   | GLN A 339 | 30.034 | 18.485 | 29.641 | 1.00 | 23.02 | A | O |
| ATOM | 2517 | N   | ALA A 340 | 30.922 | 19.061 | 31.619 | 1.00 | 22.60 | A | N |
| ATOM | 2518 | CA  | ALA A 340 | 30.793 | 20.492 | 31.347 | 1.00 | 22.71 | A | C |
| ATOM | 2519 | CB  | ALA A 340 | 31.311 | 21.296 | 32.535 | 1.00 | 22.84 | A | C |
| ATOM | 2520 | C   | ALA A 340 | 31.524 | 20.916 | 30.076 | 1.00 | 22.30 | A | C |
| ATOM | 2521 | O   | ALA A 340 | 32.474 | 20.270 | 29.650 | 1.00 | 22.44 | A | O |
| ATOM | 2522 | N   | GLY A 341 | 31.063 | 21.996 | 29.455 | 1.00 | 23.38 | A | N |
| ATOM | 2523 | CA  | GLY A 341 | 31.738 | 22.554 | 28.283 | 1.00 | 23.73 | A | C |
| ATOM | 2524 | C   | GLY A 341 | 30.989 | 22.425 | 26.956 | 1.00 | 24.15 | A | C |
| ATOM | 2525 | O   | GLY A 341 | 31.457 | 22.902 | 25.917 | 1.00 | 24.10 | A | O |
| ATOM | 2526 | N   | LYS A 342 | 29.829 | 21.774 | 26.970 | 1.00 | 24.05 | A | N |
| ATOM | 2527 | CA  | LYS A 342 | 29.038 | 21.637 | 25.743 | 1.00 | 24.39 | A | C |
| ATOM | 2528 | CB  | LYS A 342 | 29.643 | 20.545 | 24.861 | 1.00 | 25.08 | A | C |
| ATOM | 2529 | CG  | LYS A 342 | 29.610 | 19.148 | 25.496 | 1.00 | 27.13 | A | C |
| ATOM | 2530 | CD  | LYS A 342 | 30.471 | 18.173 | 24.723 | 1.00 | 29.40 | A | C |
| ATOM | 2531 | CE  | LYS A 342 | 30.254 | 16.725 | 25.182 | 1.00 | 29.96 | A | C |
| ATOM | 2532 | NZ  | LYS A 342 | 30.738 | 16.515 | 26.576 | 1.00 | 32.09 | A | N |
| ATOM | 2533 | C   | LYS A 342 | 27.552 | 21.373 | 26.058 | 1.00 | 23.40 | A | C |
| ATOM | 2534 | O   | LYS A 342 | 27.220 | 20.861 | 27.144 | 1.00 | 23.54 | A | O |
| ATOM | 2535 | N   | PRO A 343 | 26.652 | 21.755 | 25.151 | 1.00 | 22.09 | A | N |
| ATOM | 2536 | CA  | PRO A 343 | 25.219 | 21.683 | 25.450 | 1.00 | 21.01 | A | C |
| ATOM | 2537 | CB  | PRO A 343 | 24.557 | 22.206 | 24.163 | 1.00 | 21.58 | A | C |
| ATOM | 2538 | CG  | PRO A 343 | 25.613 | 23.026 | 23.492 | 1.00 | 22.06 | A | C |
| ATOM | 2539 | CD  | PRO A 343 | 26.902 | 22.348 | 23.820 | 1.00 | 22.30 | A | C |
| ATOM | 2540 | C   | PRO A 343 | 24.729 | 20.279 | 25.756 | 1.00 | 19.35 | A | C |
| ATOM | 2541 | O   | PRO A 343 | 25.311 | 19.298 | 25.317 | 1.00 | 17.82 | A | O |
| ATOM | 2542 | N   | LEU A 344 | 23.645 | 20.223 | 26.521 | 1.00 | 18.12 | A | N |
| ATOM | 2543 | CA  | LEU A 344 | 22.945 | 18.988 | 26.790 | 1.00 | 17.07 | A | C |
| ATOM | 2544 | CB  | LEU A 344 | 23.019 | 18.680 | 28.278 | 1.00 | 17.12 | A | C |
| ATOM | 2545 | CG  | LEU A 344 | 22.250 | 17.476 | 28.788 | 1.00 | 16.96 | A | C |
| ATOM | 2546 | CD1 | LEU A 344 | 22.743 | 16.188 | 28.128 | 1.00 | 16.29 | A | C |
| ATOM | 2547 | CD2 | LEU A 344 | 22.399 | 17.414 | 30.336 | 1.00 | 17.28 | A | C |
| ATOM | 2548 | C   | LEU A 344 | 21.484 | 19.168 | 26.360 | 1.00 | 16.41 | A | C |
| ATOM | 2549 | O   | LEU A 344 | 20.814 | 20.029 | 26.870 | 1.00 | 17.49 | A | O |
| ATOM | 2550 | N   | LYS A 345 | 21.013 | 18.336 | 25.440 | 1.00 | 15.23 | A | N |
| ATOM | 2551 | CA  | LYS A 345 | 19.638 | 18.405 | 24.943 | 1.00 | 14.51 | A | C |
| ATOM | 2552 | CB  | LYS A 345 | 19.644 | 18.807 | 23.474 | 1.00 | 14.84 | A | C |
| ATOM | 2553 | CG  | LYS A 345 | 20.104 | 20.235 | 23.248 | 1.00 | 13.82 | A | C |
| ATOM | 2554 | CD  | LYS A 345 | 19.987 | 20.664 | 21.795 | 1.00 | 16.32 | A | C |
| ATOM | 2555 | CE  | LYS A 345 | 18.599 | 21.126 | 21.423 | 1.00 | 13.96 | A | C |
| ATOM | 2556 | NZ  | LYS A 345 | 18.513 | 21.491 | 19.992 | 1.00 | 17.70 | A | N |
| ATOM | 2557 | C   | LYS A 345 | 18.929 | 17.066 | 25.135 | 1.00 | 14.25 | A | C |
| ATOM | 2558 | O   | LYS A 345 | 19.399 | 16.033 | 24.658 | 1.00 | 14.33 | A | O |
| ATOM | 2559 | N   | ILE A 346 | 17.821 | 17.084 | 25.870 | 1.00 | 13.41 | A | N |
| ATOM | 2560 | CA  | ILE A 346 | 17.031 | 15.888 | 26.116 | 1.00 | 13.30 | A | C |
| ATOM | 2561 | CB  | ILE A 346 | 16.983 | 15.589 | 27.619 | 1.00 | 13.25 | A | C |

| ATOM | 2562 | CG1 ILE A 346 | 18.376 | 15.444 | 28.197 | 1.00 13.74 | A | C |
|------|------|---------------|--------|--------|--------|-------------|---|---|
| ATOM | 2563 | CD1 ILE A 346 | 18.459 | 15.807 | 29.637 | 1.00 15.75 | A | C |
| ATOM | 2564 | CG2 ILE A 346 | 16.180 | 14.329 | 27.874 | 1.00 13.54 | A | C |
| ATOM | 2565 | C   ILE A 346 | 15.598 | 16.084 | 25.601 | 1.00 13.26 | A | C |
| ATOM | 2566 | O   ILE A 346 | 14.900 | 17.010 | 26.020 | 1.00 13.15 | A | O |
| ATOM | 2567 | N   SER A 347 | 15.159 | 15.197 | 24.714 | 1.00 12.65 | A | N |
| ATOM | 2568 | CA  SER A 347 | 13.795 | 15.262 | 24.172 | 1.00 12.47 | A | C |
| ATOM | 2569 | CB BSER A 347 | 13.838 | 15.473 | 22.654 | 0.35 12.36 | A | C |
| ATOM | 2570 | CB ASER A 347 | 13.813 | 15.524 | 22.662 | 0.65 12.73 | A | C |
| ATOM | 2571 | OG BSER A 347 | 12.569 | 15.297 | 22.042 | 0.35 10.59 | A | O |
| ATOM | 2572 | OG ASER A 347 | 14.655 | 16.634 | 22.329 | 0.65 13.18 | A | O |
| ATOM | 2573 | C   SER A 347 | 13.032 | 13.983 | 24.491 | 1.00 11.72 | A | C |
| ATOM | 2574 | O   SER A 347 | 13.511 | 12.881 | 24.219 | 1.00 11.81 | A | O |
| ATOM | 2575 | N   LEU A 348 | 11.830 | 14.165 | 25.026 | 1.00 11.03 | A | N |
| ATOM | 2576 | CA  LEU A 348 | 10.864 | 13.121 | 25.289 | 1.00 10.93 | A | C |
| ATOM | 2577 | CB  LEU A 348 | 10.302 | 13.274 | 26.706 | 1.00 10.96 | A | C |
| ATOM | 2578 | CG  LEU A 348 | 9.054  | 12.502 | 27.097 | 1.00 10.80 | A | C |
| ATOM | 2579 | CD1 LEU A 348 | 9.396  | 11.029 | 27.180 | 1.00 13.05 | A | C |
| ATOM | 2580 | CD2 LEU A 348 | 8.542  | 12.969 | 28.443 | 1.00 12.50 | A | C |
| ATOM | 2581 | C   LEU A 348 | 9.735  | 13.231 | 24.287 | 1.00 11.09 | A | C |
| ATOM | 2582 | O   LEU A 348 | 9.152  | 14.302 | 24.140 | 1.00 12.01 | A | O |
| ATOM | 2583 | N   VAL A 349 | 9.389  | 12.127 | 23.631 | 1.00 10.58 | A | N |
| ATOM | 2584 | CA  VAL A 349 | 8.327  | 12.142 | 22.638 | 1.00 11.32 | A | C |
| ATOM | 2585 | CB  VAL A 349 | 8.876  | 12.223 | 21.185 | 1.00 11.27 | A | C |
| ATOM | 2586 | CG1 VAL A 349 | 7.745  | 12.102 | 20.169 | 1.00 12.19 | A | C |
| ATOM | 2587 | CG2 VAL A 349 | 9.653  | 13.511 | 20.961 | 1.00 11.93 | A | C |
| ATOM | 2588 | C   VAL A 349 | 7.522  | 10.873 | 22.768 | 1.00 11.68 | A | C |
| ATOM | 2589 | O   VAL A 349 | 8.099  | 9.802  | 22.870 | 1.00 12.43 | A | O |
| ATOM | 2590 | N   TRP A 350 | 6.200  | 10.993 | 22.768 | 1.00 11.69 | A | N |
| ATOM | 2591 | CA  TRP A 350 | 5.354  | 9.813  | 22.662 | 1.00 11.48 | A | C |
| ATOM | 2592 | CB  TRP A 350 | 4.719  | 9.442  | 24.002 | 1.00 11.79 | A | C |
| ATOM | 2593 | CG  TRP A 350 | 3.822  | 10.448 | 24.628 | 1.00 11.11 | A | C |
| ATOM | 2594 | CD1 TRP A 350 | 2.457  | 10.378 | 24.720 | 1.00 12.11 | A | C |
| ATOM | 2595 | NE1 TRP A 350 | 1.961  | 11.469 | 25.386 | 1.00 12.24 | A | N |
| ATOM | 2596 | CE2 TRP A 350 | 3.015  | 12.262 | 25.774 | 1.00 13.16 | A | C |
| ATOM | 2597 | CD2 TRP A 350 | 4.208  | 11.640 | 25.311 | 1.00 13.33 | A | C |
| ATOM | 2598 | CE3 TRP A 350 | 5.440  | 12.249 | 25.593 | 1.00 12.51 | A | C |
| ATOM | 2599 | CZ3 TRP A 350 | 5.444  | 13.449 | 26.311 | 1.00 13.31 | A | C |
| ATOM | 2600 | CH2 TRP A 350 | 4.248  | 14.022 | 26.767 | 1.00 13.75 | A | C |
| ATOM | 2601 | CZ2 TRP A 350 | 3.022  | 13.427 | 26.507 | 1.00 13.59 | A | C |
| ATOM | 2602 | C   TRP A 350 | 4.314  | 9.883  | 21.536 | 1.00 11.50 | A | C |
| ATOM | 2603 | O   TRP A 350 | 3.905  | 10.953 | 21.077 | 1.00 11.90 | A | O |
| ATOM | 2604 | N   SER A 351 | 3.921  | 8.707  | 21.071 | 1.00 11.83 | A | N |
| ATOM | 2605 | CA  SER A 351 | 2.889  | 8.607  | 20.070 | 1.00 12.20 | A | C |
| ATOM | 2606 | CB  SER A 351 | 3.182  | 7.496  | 19.070 | 1.00 12.11 | A | C |
| ATOM | 2607 | OG  SER A 351 | 4.356  | 7.772  | 18.310 | 1.00 11.85 | A | O |
| ATOM | 2608 | C   SER A 351 | 1.636  | 8.378  | 20.884 | 1.00 12.65 | A | C |
| ATOM | 2609 | O   SER A 351 | 1.360  | 7.285  | 21.375 | 1.00 12.72 | A | O |
| ATOM | 2610 | N   ASP A 352 | 0.947  | 9.477  | 21.115 | 1.00 13.75 | A | N |
| ATOM | 2611 | CA  ASP A 352 | -0.205 | 9.532  | 21.982 | 1.00 14.48 | A | C |
| ATOM | 2612 | CB  ASP A 352 | -0.508 | 11.003 | 22.225 | 1.00 14.84 | A | C |
| ATOM | 2613 | CG  ASP A 352 | -1.480 | 11.251 | 23.385 | 1.00 16.70 | A | C |
| ATOM | 2614 | OD1 ASP A 352 | -1.655 | 10.366 | 24.260 | 1.00 15.50 | A | O |
| ATOM | 2615 | OD2 ASP A 352 | -2.115 | 12.329 | 23.458 | 1.00 15.19 | A | O |
| ATOM | 2616 | C   ASP A 352 | -1.427 | 8.842  | 21.389 | 1.00 15.19 | A | C |
| ATOM | 2617 | O   ASP A 352 | -1.569 | 8.678  | 20.155 | 1.00 15.52 | A | O |
| ATOM | 2618 | N   ALA A 353 | -2.331 | 8.434  | 22.273 | 1.00 15.26 | A | N |
| ATOM | 2619 | CA  ALA A 353 | -3.689 | 8.074  | 21.853 | 1.00 15.65 | A | C |
| ATOM | 2620 | CB  ALA A 353 | -4.526 | 7.801  | 23.051 | 1.00 15.59 | A | C |
| ATOM | 2621 | C   ALA A 353 | -4.325 | 9.197  | 21.018 | 1.00 15.42 | A | C |
| ATOM | 2622 | O   ALA A 353 | -4.076 | 10.374 | 21.264 | 1.00 15.11 | A | O |
| ATOM | 2623 | N   PRO A 354 | -5.157 | 8.840  | 20.041 | 1.00 16.50 | A | N |
| ATOM | 2624 | CA  PRO A 354 | -5.858 | 9.841  | 19.235 | 1.00 16.92 | A | C |

73

```
ATOM   2625   CB   PRO A 354    -6.724    9.003   18.287   1.00  17.36        A    C
ATOM   2626   CG   PRO A 354    -6.790    7.646   18.897   1.00  17.45        A    C
ATOM   2627   CD   PRO A 354    -5.499    7.456   19.640   1.00  16.81        A    C
ATOM   2628   C    PRO A 354    -6.723   10.771   20.073   1.00  18.05        A    C
ATOM   2629   O    PRO A 354    -7.420   10.293   20.957   1.00  17.51        A    O
ATOM   2630   N    GLY A 355    -6.629   12.074   19.819   1.00  18.60        A    N
ATOM   2631   CA   GLY A 355    -7.392   13.071   20.527   1.00  20.06        A    C
ATOM   2632   C    GLY A 355    -8.773   13.285   19.936   1.00  21.21        A    C
ATOM   2633   O    GLY A 355    -9.095   12.758   18.880   1.00  22.41        A    O
ATOM   2634   N    SER A 356    -9.598   14.050   20.628   1.00  22.87        A    N
ATOM   2635   CA   SER A 356   -10.939   14.377   20.145   1.00  23.97        A    C
ATOM   2636   CB   SER A 356   -11.924   14.555   21.319   1.00  24.97        A    C
ATOM   2637   OG   SER A 356   -12.771   15.696   21.117   1.00  26.85        A    O
ATOM   2638   C    SER A 356   -10.901   15.654   19.320   1.00  24.04        A    C
ATOM   2639   O    SER A 356   -10.151   16.583   19.635   1.00  23.54        A    O
ATOM   2640   N    THR A 357   -11.714   15.684   18.261   1.00  24.61        A    N
ATOM   2641   CA   THR A 357   -11.826   16.846   17.396   1.00  25.58        A    C
ATOM   2642   CB   THR A 357   -12.423   16.436   16.032   1.00  25.86        A    C
ATOM   2643   OG1  THR A 357   -13.673   15.748   16.218   1.00  25.91        A    O
ATOM   2644   CG2  THR A 357   -11.534   15.392   15.334   1.00  25.30        A    C
ATOM   2645   C    THR A 357   -12.687   17.982   18.000   1.00  26.58        A    C
ATOM   2646   O    THR A 357   -12.812   19.035   17.398   1.00  26.22        A    O
ATOM   2647   N    THR A 358   -13.276   17.771   19.175   1.00  27.59        A    N
ATOM   2648   CA   THR A 358   -14.113   18.816   19.779   1.00  28.37        A    C
ATOM   2649   CB   THR A 358   -15.575   18.335   19.938   1.00  28.27        A    C
ATOM   2650   OG1  THR A 358   -15.606   17.065   20.606   1.00  28.60        A    O
ATOM   2651   CG2  THR A 358   -16.192   18.066   18.587   1.00  27.97        A    C
ATOM   2652   C    THR A 358   -13.605   19.321   21.118   1.00  28.71        A    C
ATOM   2653   O    THR A 358   -13.954   20.424   21.524   1.00  29.22        A    O
ATOM   2654   N    ALA A 359   -12.758   18.548   21.795   1.00  28.63        A    N
ATOM   2655   CA   ALA A 359   -12.311   18.925   23.133   1.00  27.94        A    C
ATOM   2656   CB   ALA A 359   -11.668   17.739   23.814   1.00  28.64        A    C
ATOM   2657   C    ALA A 359   -11.349   20.100   23.099   1.00  27.98        A    C
ATOM   2658   O    ALA A 359   -10.738   20.393   22.060   1.00  27.61        A    O
ATOM   2659   N    SER A 360   -11.213   20.785   24.241   1.00  27.18        A    N
ATOM   2660   CA   SER A 360   -10.301   21.916   24.344   1.00  27.00        A    C
ATOM   2661   CB   SER A 360   -10.351   22.564   25.737   1.00  27.57        A    C
ATOM   2662   OG   SER A 360   -11.688   22.840   26.125   1.00  31.54        A    O
ATOM   2663   C    SER A 360    -8.858   21.485   24.060   1.00  25.14        A    C
ATOM   2664   O    SER A 360    -8.115   22.230   23.446   1.00  24.27        A    O
ATOM   2665   N    LEU A 361    -8.478   20.307   24.553   1.00  23.74        A    N
ATOM   2666   CA   LEU A 361    -7.100   19.812   24.453   1.00  23.54        A    C
ATOM   2667   CB   LEU A 361    -6.480   19.583   25.840   1.00  23.87        A    C
ATOM   2668   CG   LEU A 361    -6.119   20.802   26.702   1.00  27.30        A    C
ATOM   2669   CD1  LEU A 361    -5.434   20.335   27.980   1.00  28.61        A    C
ATOM   2670   CD2  LEU A 361    -5.217   21.827   25.975   1.00  28.84        A    C
ATOM   2671   C    LEU A 361    -7.097   18.493   23.701   1.00  21.78        A    C
ATOM   2672   O    LEU A 361    -7.942   17.611   23.961   1.00  21.73        A    O
ATOM   2673   N    THR A 362    -6.141   18.325   22.790   1.00  19.81        A    N
ATOM   2674   CA   THR A 362    -6.053   17.058   22.066   1.00  18.76        A    C
ATOM   2675   CB   THR A 362    -5.433   17.230   20.657   1.00  19.42        A    C
ATOM   2676   OG1  THR A 362    -4.100   17.707   20.786   1.00  17.51        A    O
ATOM   2677   CG2  THR A 362    -6.174   18.305   19.862   1.00  20.58        A    C
ATOM   2678   C    THR A 362    -5.261   16.023   22.819   1.00  17.21        A    C
ATOM   2679   O    THR A 362    -5.411   14.858   22.530   1.00  13.79        A    O
ATOM   2680   N    LEU A 363    -4.398   16.448   23.761   1.00  16.68        A    N
ATOM   2681   CA   LEU A 363    -3.560   15.505   24.484   1.00  16.39        A    C
ATOM   2682   CB   LEU A 363    -2.547   16.213   25.411   1.00  16.30        A    C
ATOM   2683   CG   LEU A 363    -1.460   15.318   25.990   1.00  16.62        A    C
ATOM   2684   CD1  LEU A 363    -0.380   14.960   24.939   1.00  16.31        A    C
ATOM   2685   CD2  LEU A 363    -0.838   15.936   27.236   1.00  15.84        A    C
ATOM   2686   C    LEU A 363    -4.424   14.536   25.280   1.00  17.11        A    C
ATOM   2687   O    LEU A 363    -5.404   14.936   25.911   1.00  17.15        A    O
```

74

```
ATOM   2688  N    VAL A 364   -4.068  13.253  25.249  1.00  16.50      A    N
ATOM   2689  CA   VAL A 364   -4.829  12.263  25.975  1.00  16.09      A    C
ATOM   2690  CB   VAL A 364   -5.285  11.121  25.030  1.00  15.79      A    C
ATOM   2691  CG1  VAL A 364   -5.871   9.933  25.826  1.00  17.24      A    C
ATOM   2692  CG2  VAL A 364   -6.288  11.651  24.020  1.00  16.26      A    C
ATOM   2693  C    VAL A 364   -3.983  11.744  27.139  1.00  15.68      A    C
ATOM   2694  O    VAL A 364   -4.329  11.942  28.309  1.00  15.17      A    O
ATOM   2695  N    ASN A 365   -2.875  11.085  26.809  1.00  14.41      A    N
ATOM   2696  CA   ASN A 365   -1.931  10.614  27.804  1.00  14.31      A    C
ATOM   2697  CB   ASN A 365   -1.302   9.286  27.354  1.00  14.31      A    C
ATOM   2698  CG   ASN A 365   -2.342   8.161  27.214  1.00  16.21      A    C
ATOM   2699  OD1  ASN A 365   -3.298   8.081  28.004  1.00  13.31      A    O
ATOM   2700  ND2  ASN A 365   -2.158   7.283  26.206  1.00  14.49      A    N
ATOM   2701  C    ASN A 365   -0.858  11.690  28.088  1.00  14.80      A    C
ATOM   2702  O    ASN A 365   -0.174  12.190  27.172  1.00  14.01      A    O
ATOM   2703  N    ASP A 366   -0.696  12.015  29.360  1.00  14.06      A    N
ATOM   2704  CA   ASP A 366    0.158  13.115  29.783  1.00  15.00      A    C
ATOM   2705  CB   ASP A 366   -0.632  14.068  30.672  1.00  14.40      A    C
ATOM   2706  CG   ASP A 366    0.105  15.346  30.990  1.00  15.13      A    C
ATOM   2707  OD1  ASP A 366    1.344  15.467  30.710  1.00  13.15      A    O
ATOM   2708  OD2  ASP A 366   -0.491  16.284  31.609  1.00  17.79      A    O
ATOM   2709  C    ASP A 366    1.367  12.568  30.548  1.00  14.89      A    C
ATOM   2710  O    ASP A 366    1.257  12.132  31.708  1.00  15.57      A    O
ATOM   2711  N    LEU A 367    2.501  12.562  29.865  1.00  14.16      A    N
ATOM   2712  CA   LEU A 367    3.772  12.248  30.462  1.00  14.65      A    C
ATOM   2713  CB   LEU A 367    4.581  11.333  29.520  1.00  14.37      A    C
ATOM   2714  CG   LEU A 367    3.990  10.005  29.077  1.00  13.06      A    C
ATOM   2715  CD1  LEU A 367    5.063   9.220  28.244  1.00  12.20      A    C
ATOM   2716  CD2  LEU A 367    3.485   9.123  30.239  1.00  15.25      A    C
ATOM   2717  C    LEU A 367    4.523  13.555  30.710  1.00  14.15      A    C
ATOM   2718  O    LEU A 367    4.271  14.546  30.045  1.00  14.17      A    O
ATOM   2719  N    ASP A 368    5.441  13.556  31.677  1.00  14.04      A    N
ATOM   2720  CA   ASP A 368    6.271  14.705  31.980  1.00  14.28      A    C
ATOM   2721  CB   ASP A 368    5.959  15.284  33.354  1.00  15.52      A    C
ATOM   2722  CG   ASP A 368    4.529  15.726  33.515  1.00  17.54      A    C
ATOM   2723  OD1  ASP A 368    3.909  16.253  32.540  1.00  14.48      A    O
ATOM   2724  OD2  ASP A 368    4.006  15.624  34.642  1.00  17.39      A    O
ATOM   2725  C    ASP A 368    7.724  14.275  32.057  1.00  14.49      A    C
ATOM   2726  O    ASP A 368    8.034  13.203  32.587  1.00  13.94      A    O
ATOM   2727  N    LEU A 369    8.603  15.108  31.520  1.00  14.32      A    N
ATOM   2728  CA   LEU A 369   10.044  14.937  31.645  1.00  14.31      A    C
ATOM   2729  CB   LEU A 369   10.735  15.448  30.380  1.00  13.72      A    C
ATOM   2730  CG   LEU A 369   12.238  15.284  30.298  1.00  12.96      A    C
ATOM   2731  CD1  LEU A 369   12.572  13.850  30.345  1.00  12.52      A    C
ATOM   2732  CD2  LEU A 369   12.749  15.953  28.980  1.00  14.11      A    C
ATOM   2733  C    LEU A 369   10.539  15.733  32.854  1.00  15.02      A    C
ATOM   2734  O    LEU A 369   10.218  16.922  33.012  1.00  15.50      A    O
ATOM   2735  N    VAL A 370   11.315  15.085  33.698  1.00  15.26      A    N
ATOM   2736  CA   VAL A 370   11.875  15.729  34.905  1.00  15.63      A    C
ATOM   2737  CB   VAL A 370   11.144  15.278  36.180  1.00  15.95      A    C
ATOM   2738  CG1  VAL A 370   11.679  16.020  37.425  1.00  17.73      A    C
ATOM   2739  CG2  VAL A 370    9.687  15.487  36.024  1.00  15.57      A    C
ATOM   2740  C    VAL A 370   13.359  15.388  34.975  1.00  15.46      A    C
ATOM   2741  O    VAL A 370   13.767  14.219  35.042  1.00  15.96      A    O
ATOM   2742  N    ILE A 371   14.174  16.422  34.908  1.00  15.15      A    N
ATOM   2743  CA   ILE A 371   15.608  16.261  34.858  1.00  14.98      A    C
ATOM   2744  CB   ILE A 371   16.171  17.036  33.669  1.00  14.71      A    C
ATOM   2745  CG1  ILE A 371   15.509  16.589  32.336  1.00  14.63      A    C
ATOM   2746  CD1  ILE A 371   15.600  15.075  32.072  1.00  14.66      A    C
ATOM   2747  CG2  ILE A 371   17.674  16.922  33.614  1.00  12.85      A    C
ATOM   2748  C    ILE A 371   16.145  16.835  36.155  1.00  15.95      A    C
ATOM   2749  O    ILE A 371   15.648  17.853  36.618  1.00  16.74      A    O
ATOM   2750  N    THR A 372   17.150  16.174  36.727  1.00  16.86      A    N
```

75

| ATOM | 2751 | CA | THR | A | 372 | 17.858 | 16.671 | 37.897 | 1.00 | 17.19 | A | C |
|------|------|-----|-----|---|-----|--------|--------|--------|------|-------|---|---|
| ATOM | 2752 | CB | THR | A | 372 | 17.618 | 15.748 | 39.089 | 1.00 | 17.36 | A | C |
| ATOM | 2753 | OG1 | THR | A | 372 | 16.212 | 15.514 | 39.265 | 1.00 | 18.17 | A | O |
| ATOM | 2754 | CG2 | THR | A | 372 | 18.044 | 16.409 | 40.372 | 1.00 | 17.84 | A | C |
| ATOM | 2755 | C | THR | A | 372 | 19.364 | 16.729 | 37.634 | 1.00 | 17.65 | A | C |
| ATOM | 2756 | O | THR | A | 372 | 19.962 | 15.725 | 37.262 | 1.00 | 18.44 | A | O |
| ATOM | 2757 | N | ALA | A | 373 | 19.971 | 17.891 | 37.870 | 1.00 | 17.80 | A | N |
| ATOM | 2758 | CA | ALA | A | 373 | 21.376 | 18.118 | 37.606 | 1.00 | 18.13 | A | C |
| ATOM | 2759 | CB | ALA | A | 373 | 21.643 | 19.607 | 37.475 | 1.00 | 18.43 | A | C |
| ATOM | 2760 | C | ALA | A | 373 | 22.175 | 17.545 | 38.767 | 1.00 | 19.16 | A | C |
| ATOM | 2761 | O | ALA | A | 373 | 21.601 | 17.188 | 39.780 | 1.00 | 18.57 | A | O |
| ATOM | 2762 | N | PRO | A | 374 | 23.479 | 17.368 | 38.581 | 1.00 | 19.84 | A | N |
| ATOM | 2763 | CA | PRO | A | 374 | 24.348 | 16.857 | 39.642 | 1.00 | 20.99 | A | C |
| ATOM | 2764 | CB | PRO | A | 374 | 25.727 | 16.884 | 39.001 | 1.00 | 20.60 | A | C |
| ATOM | 2765 | CG | PRO | A | 374 | 25.434 | 16.700 | 37.530 | 1.00 | 20.96 | A | C |
| ATOM | 2766 | CD | PRO | A | 374 | 24.174 | 17.460 | 37.286 | 1.00 | 20.29 | A | C |
| ATOM | 2767 | C | PRO | A | 374 | 24.266 | 17.647 | 40.948 | 1.00 | 22.06 | A | C |
| ATOM | 2768 | O | PRO | A | 374 | 24.303 | 17.039 | 42.011 | 1.00 | 23.95 | A | O |
| ATOM | 2769 | N | ASN | A | 375 | 24.024 | 18.954 | 40.873 | 1.00 | 23.03 | A | N |
| ATOM | 2770 | CA | ASN | A | 375 | 23.910 | 19.770 | 42.058 | 1.00 | 23.32 | A | C |
| ATOM | 2771 | CB | ASN | A | 375 | 24.515 | 21.165 | 41.790 | 1.00 | 24.03 | A | C |
| ATOM | 2772 | CG | ASN | A | 375 | 23.581 | 22.096 | 40.993 | 1.00 | 26.93 | A | C |
| ATOM | 2773 | OD1 | ASN | A | 375 | 22.515 | 21.689 | 40.492 | 1.00 | 28.51 | A | O |
| ATOM | 2774 | ND2 | ASN | A | 375 | 23.987 | 23.362 | 40.878 | 1.00 | 27.28 | A | N |
| ATOM | 2775 | C | ASN | A | 375 | 22.471 | 19.898 | 42.563 | 1.00 | 23.07 | A | C |
| ATOM | 2776 | O | ASN | A | 375 | 22.208 | 20.711 | 43.430 | 1.00 | 22.92 | A | O |
| ATOM | 2777 | N | GLY | A | 376 | 21.541 | 19.120 | 42.010 | 1.00 | 21.76 | A | N |
| ATOM | 2778 | CA | GLY | A | 376 | 20.166 | 19.165 | 42.469 | 1.00 | 21.43 | A | C |
| ATOM | 2779 | C | GLY | A | 376 | 19.197 | 20.077 | 41.724 | 1.00 | 20.91 | A | C |
| ATOM | 2780 | O | GLY | A | 376 | 17.990 | 20.006 | 41.937 | 1.00 | 19.94 | A | O |
| ATOM | 2781 | N | THR | A | 377 | 19.696 | 20.909 | 40.828 | 1.00 | 21.29 | A | N |
| ATOM | 2782 | CA | THR | A | 377 | 18.793 | 21.785 | 40.090 | 1.00 | 21.36 | A | C |
| ATOM | 2783 | CB | THR | A | 377 | 19.571 | 22.738 | 39.220 | 1.00 | 21.19 | A | C |
| ATOM | 2784 | OG1 | THR | A | 377 | 20.423 | 23.532 | 40.054 | 1.00 | 22.26 | A | O |
| ATOM | 2785 | CG2 | THR | A | 377 | 18.635 | 23.724 | 38.538 | 1.00 | 20.97 | A | C |
| ATOM | 2786 | C | THR | A | 377 | 17.818 | 20.971 | 39.239 | 1.00 | 20.56 | A | C |
| ATOM | 2787 | O | THR | A | 377 | 18.206 | 20.058 | 38.541 | 1.00 | 19.93 | A | O |
| ATOM | 2788 | N | LYS | A | 378 | 16.558 | 21.345 | 39.315 | 1.00 | 20.68 | A | N |
| ATOM | 2789 | CA | LYS | A | 378 | 15.488 | 20.630 | 38.644 | 1.00 | 21.64 | A | C |
| ATOM | 2790 | CB | LYS | A | 378 | 14.321 | 20.503 | 39.594 | 1.00 | 22.42 | A | C |
| ATOM | 2791 | CG | LYS | A | 378 | 13.709 | 19.168 | 39.611 | 1.00 | 28.13 | A | C |
| ATOM | 2792 | CD | LYS | A | 378 | 14.144 | 18.449 | 40.913 | 1.00 | 33.32 | A | C |
| ATOM | 2793 | CE | LYS | A | 378 | 13.743 | 17.001 | 40.854 | 1.00 | 34.92 | A | C |
| ATOM | 2794 | NZ | LYS | A | 378 | 14.605 | 16.111 | 41.699 | 1.00 | 38.61 | A | N |
| ATOM | 2795 | C | LYS | A | 378 | 14.990 | 21.344 | 37.397 | 1.00 | 20.06 | A | C |
| ATOM | 2796 | O | LYS | A | 378 | 14.902 | 22.553 | 37.388 | 1.00 | 19.59 | A | O |
| ATOM | 2797 | N | TYR | A | 379 | 14.623 | 20.568 | 36.378 | 1.00 | 18.59 | A | N |
| ATOM | 2798 | CA | TYR | A | 379 | 14.009 | 21.088 | 35.155 | 1.00 | 17.87 | A | C |
| ATOM | 2799 | CB | TYR | A | 379 | 15.030 | 21.111 | 34.011 | 1.00 | 17.25 | A | C |
| ATOM | 2800 | CG | TYR | A | 379 | 16.382 | 21.654 | 34.386 | 1.00 | 17.53 | A | C |
| ATOM | 2801 | CD1 | TYR | A | 379 | 17.297 | 20.864 | 35.052 | 1.00 | 18.58 | A | C |
| ATOM | 2802 | CE1 | TYR | A | 379 | 18.537 | 21.352 | 35.417 | 1.00 | 19.05 | A | C |
| ATOM | 2803 | CZ | TYR | A | 379 | 18.895 | 22.655 | 35.097 | 1.00 | 21.25 | A | C |
| ATOM | 2804 | OH | TYR | A | 379 | 20.160 | 23.104 | 35.465 | 1.00 | 20.46 | A | O |
| ATOM | 2805 | CE2 | TYR | A | 379 | 18.004 | 23.459 | 34.399 | 1.00 | 19.58 | A | C |
| ATOM | 2806 | CD2 | TYR | A | 379 | 16.751 | 22.953 | 34.060 | 1.00 | 18.07 | A | C |
| ATOM | 2807 | C | TYR | A | 379 | 12.852 | 20.198 | 34.732 | 1.00 | 17.42 | A | C |
| ATOM | 2808 | O | TYR | A | 379 | 12.967 | 18.973 | 34.766 | 1.00 | 18.13 | A | O |
| ATOM | 2809 | N | VAL | A | 380 | 11.732 | 20.787 | 34.340 | 1.00 | 16.44 | A | N |
| ATOM | 2810 | CA | VAL | A | 380 | 10.653 | 19.989 | 33.750 | 1.00 | 16.11 | A | C |
| ATOM | 2811 | CB | VAL | A | 380 | 9.320 | 20.114 | 34.514 | 1.00 | 16.66 | A | C |
| ATOM | 2812 | CG1 | VAL | A | 380 | 9.521 | 19.742 | 36.000 | 1.00 | 17.64 | A | C |
| ATOM | 2813 | CG2 | VAL | A | 380 | 8.716 | 21.505 | 34.369 | 1.00 | 16.52 | A | C |

| ATOM | 2814 | C | VAL | A | 380 | 10.466 | 20.353 | 32.283 | 1.00 | 14.93 | A | C |
|------|------|-----|-----|---|-----|--------|--------|--------|------|-------|---|---|
| ATOM | 2815 | O | VAL | A | 380 | 10.876 | 21.425 | 31.826 | 1.00 | 15.17 | A | O |
| ATOM | 2816 | N | GLY | A | 381 | 9.868 | 19.436 | 31.547 | 1.00 | 14.19 | A | N |
| ATOM | 2817 | CA | GLY | A | 381 | 9.761 | 19.541 | 30.101 | 1.00 | 13.85 | A | C |
| ATOM | 2818 | C | GLY | A | 381 | 9.132 | 20.847 | 29.647 | 1.00 | 13.74 | A | C |
| ATOM | 2819 | O | GLY | A | 381 | 8.096 | 21.259 | 30.153 | 1.00 | 13.57 | A | O |
| ATOM | 2820 | N | ASN | A | 382 | 9.813 | 21.509 | 28.729 | 1.00 | 13.87 | A | N |
| ATOM | 2821 | CA | ASN | A | 382 | 9.332 | 22.719 | 28.064 | 1.00 | 14.08 | A | C |
| ATOM | 2822 | CB | ASN | A | 382 | 8.002 | 22.451 | 27.344 | 1.00 | 13.91 | A | C |
| ATOM | 2823 | CG | ASN | A | 382 | 8.148 | 21.436 | 26.209 | 1.00 | 14.03 | A | C |
| ATOM | 2824 | OD1 | ASN | A | 382 | 9.250 | 21.181 | 25.757 | 1.00 | 13.23 | A | O |
| ATOM | 2825 | ND2 | ASN | A | 382 | 7.041 | 20.850 | 25.770 | 1.00 | 10.67 | A | N |
| ATOM | 2826 | C | ASN | A | 382 | 9.232 | 23.942 | 28.966 | 1.00 | 14.58 | A | C |
| ATOM | 2827 | O | ASN | A | 382 | 8.682 | 24.981 | 28.556 | 1.00 | 14.15 | A | O |
| ATOM | 2828 | N | ASP | A | 383 | 9.796 | 23.869 | 30.178 | 1.00 | 14.75 | A | N |
| ATOM | 2829 | CA | ASP | A | 383 | 9.813 | 25.057 | 31.017 | 1.00 | 14.97 | A | C |
| ATOM | 2830 | CB | ASP | A | 383 | 9.593 | 24.709 | 32.499 | 1.00 | 15.34 | A | C |
| ATOM | 2831 | CG | ASP | A | 383 | 9.580 | 25.952 | 33.388 | 1.00 | 15.81 | A | C |
| ATOM | 2832 | OD1 | ASP | A | 383 | 9.786 | 27.082 | 32.845 | 1.00 | 16.83 | A | O |
| ATOM | 2833 | OD2 | ASP | A | 383 | 9.394 | 25.897 | 34.636 | 1.00 | 15.84 | A | O |
| ATOM | 2834 | C | ASP | A | 383 | 11.127 | 25.813 | 30.810 | 1.00 | 15.13 | A | C |
| ATOM | 2835 | O | ASP | A | 383 | 12.160 | 25.490 | 31.398 | 1.00 | 15.46 | A | O |
| ATOM | 2836 | N | PHE | A | 384 | 11.074 | 26.859 | 30.000 | 1.00 | 15.11 | A | N |
| ATOM | 2837 | CA | PHE | A | 384 | 12.284 | 27.559 | 29.589 | 1.00 | 15.10 | A | C |
| ATOM | 2838 | CB | PHE | A | 384 | 12.178 | 27.818 | 28.086 | 1.00 | 16.52 | A | C |
| ATOM | 2839 | CG | PHE | A | 384 | 12.247 | 26.560 | 27.240 | 1.00 | 14.23 | A | C |
| ATOM | 2840 | CD1 | PHE | A | 384 | 13.440 | 25.910 | 27.059 | 1.00 | 19.41 | A | C |
| ATOM | 2841 | CE1 | PHE | A | 384 | 13.516 | 24.782 | 26.273 | 1.00 | 19.08 | A | C |
| ATOM | 2842 | CZ | PHE | A | 384 | 12.395 | 24.303 | 25.685 | 1.00 | 18.05 | A | C |
| ATOM | 2843 | CE2 | PHE | A | 384 | 11.208 | 24.943 | 25.845 | 1.00 | 14.73 | A | C |
| ATOM | 2844 | CD2 | PHE | A | 384 | 11.140 | 26.070 | 26.602 | 1.00 | 16.74 | A | C |
| ATOM | 2845 | C | PHE | A | 384 | 12.546 | 28.857 | 30.389 | 1.00 | 15.96 | A | C |
| ATOM | 2846 | O | PHE | A | 384 | 13.547 | 29.558 | 30.152 | 1.00 | 15.13 | A | O |
| ATOM | 2847 | N | THR | A | 385 | 11.666 | 29.151 | 31.350 | 1.00 | 16.18 | A | N |
| ATOM | 2848 | CA | THR | A | 385 | 11.820 | 30.294 | 32.264 | 1.00 | 17.37 | A | C |
| ATOM | 2849 | CB | THR | A | 385 | 10.519 | 31.097 | 32.400 | 1.00 | 17.19 | A | C |
| ATOM | 2850 | OG1 | THR | A | 385 | 9.520 | 30.295 | 33.030 | 1.00 | 17.98 | A | O |
| ATOM | 2851 | CG2 | THR | A | 385 | 9.922 | 31.491 | 31.028 | 1.00 | 18.21 | A | C |
| ATOM | 2852 | C | THR | A | 385 | 12.238 | 29.868 | 33.689 | 1.00 | 17.95 | A | C |
| ATOM | 2853 | O | THR | A | 385 | 11.703 | 28.890 | 34.252 | 1.00 | 17.41 | A | O |
| ATOM | 2854 | N | ALA | A | 386 | 13.197 | 30.599 | 34.250 | 1.00 | 18.54 | A | N |
| ATOM | 2855 | CA | ALA | A | 386 | 13.743 | 30.266 | 35.565 | 1.00 | 19.43 | A | C |
| ATOM | 2856 | CB | ALA | A | 386 | 15.056 | 30.971 | 35.792 | 1.00 | 19.87 | A | C |
| ATOM | 2857 | C | ALA | A | 386 | 12.728 | 30.697 | 36.594 | 1.00 | 20.06 | A | C |
| ATOM | 2858 | O | ALA | A | 386 | 12.078 | 31.735 | 36.409 | 1.00 | 21.03 | A | O |
| ATOM | 2859 | N | PRO | A | 387 | 12.525 | 29.897 | 37.635 | 1.00 | 19.71 | A | N |
| ATOM | 2860 | CA | PRO | A | 387 | 13.134 | 28.582 | 37.766 | 1.00 | 20.17 | A | C |
| ATOM | 2861 | CB | PRO | A | 387 | 12.951 | 28.269 | 39.250 | 1.00 | 20.90 | A | C |
| ATOM | 2862 | CG | PRO | A | 387 | 11.609 | 28.910 | 39.587 | 1.00 | 20.16 | A | C |
| ATOM | 2863 | CD | PRO | A | 387 | 11.670 | 30.223 | 38.796 | 1.00 | 21.11 | A | C |
| ATOM | 2864 | C | PRO | A | 387 | 12.413 | 27.549 | 36.890 | 1.00 | 19.33 | A | C |
| ATOM | 2865 | O | PRO | A | 387 | 11.237 | 27.688 | 36.612 | 1.00 | 18.07 | A | O |
| ATOM | 2866 | N | TYR | A | 388 | 13.144 | 26.521 | 36.491 | 1.00 | 19.24 | A | N |
| ATOM | 2867 | CA | TYR | A | 388 | 12.781 | 25.672 | 35.365 | 1.00 | 19.24 | A | C |
| ATOM | 2868 | CB | TYR | A | 388 | 14.059 | 25.211 | 34.661 | 1.00 | 18.91 | A | C |
| ATOM | 2869 | CG | TYR | A | 388 | 14.912 | 26.363 | 34.177 | 1.00 | 18.51 | A | C |
| ATOM | 2870 | CD1 | TYR | A | 388 | 16.128 | 26.625 | 34.761 | 1.00 | 16.61 | A | C |
| ATOM | 2871 | CE1 | TYR | A | 388 | 16.912 | 27.701 | 34.350 | 1.00 | 17.76 | A | C |
| ATOM | 2872 | CZ | TYR | A | 388 | 16.462 | 28.511 | 33.312 | 1.00 | 15.12 | A | C |
| ATOM | 2873 | OH | TYR | A | 388 | 17.242 | 29.565 | 32.918 | 1.00 | 17.69 | A | O |
| ATOM | 2874 | CE2 | TYR | A | 388 | 15.241 | 28.276 | 32.723 | 1.00 | 14.50 | A | C |
| ATOM | 2875 | CD2 | TYR | A | 388 | 14.462 | 27.229 | 33.154 | 1.00 | 14.33 | A | C |
| ATOM | 2876 | C | TYR | A | 388 | 11.934 | 24.467 | 35.745 | 1.00 | 19.73 | A | C |

| ATOM | 2877 | O   | TYR | A | 388 | 11.688 | 23.598 | 34.913 | 1.00 | 19.79 | A | O |
| ATOM | 2878 | N   | ASP | A | 389 | 11.422 | 24.442 | 36.972 | 1.00 | 20.38 | A | N |
| ATOM | 2879 | CA  | ASP | A | 389 | 10.605 | 23.327 | 37.430 | 1.00 | 21.41 | A | C |
| ATOM | 2880 | CB  | ASP | A | 389 | 11.346 | 22.547 | 38.507 | 1.00 | 22.04 | A | C |
| ATOM | 2881 | CG  | ASP | A | 389 | 11.504 | 23.343 | 39.796 | 1.00 | 24.83 | A | C |
| ATOM | 2882 | OD1 | ASP | A | 389 | 11.618 | 22.706 | 40.869 | 1.00 | 27.95 | A | O |
| ATOM | 2883 | OD2 | ASP | A | 389 | 11.523 | 24.595 | 39.822 | 1.00 | 24.63 | A | O |
| ATOM | 2884 | C   | ASP | A | 389 | 9.246  | 23.724 | 37.968 | 1.00 | 21.64 | A | C |
| ATOM | 2885 | O   | ASP | A | 389 | 8.629  | 22.947 | 38.709 | 1.00 | 22.12 | A | O |
| ATOM | 2886 | N   | ASN | A | 390 | 8.759  | 24.908 | 37.618 | 1.00 | 21.45 | A | N |
| ATOM | 2887 | CA  | ASN | A | 390 | 7.455  | 25.326 | 38.130 | 1.00 | 22.14 | A | C |
| ATOM | 2888 | CB  | ASN | A | 390 | 7.555  | 26.664 | 38.892 | 1.00 | 22.91 | A | C |
| ATOM | 2889 | CG  | ASN | A | 390 | 7.965  | 27.825 | 37.989 | 1.00 | 23.08 | A | C |
| ATOM | 2890 | OD1 | ASN | A | 390 | 8.404  | 27.620 | 36.847 | 1.00 | 22.98 | A | O |
| ATOM | 2891 | ND2 | ASN | A | 390 | 7.816  | 29.050 | 38.491 | 1.00 | 23.21 | A | N |
| ATOM | 2892 | C   | ASN | A | 390 | 6.356  | 25.402 | 37.060 | 1.00 | 22.16 | A | C |
| ATOM | 2893 | O   | ASN | A | 390 | 5.181  | 25.500 | 37.405 | 1.00 | 21.88 | A | O |
| ATOM | 2894 | N   | ASN | A | 391 | 6.717  | 25.340 | 35.772 | 1.00 | 21.51 | A | N |
| ATOM | 2895 | CA  | ASN | A | 391 | 5.705  | 25.411 | 34.713 | 1.00 | 20.98 | A | C |
| ATOM | 2896 | CB  | ASN | A | 391 | 5.986  | 26.567 | 33.728 | 1.00 | 21.22 | A | C |
| ATOM | 2897 | CG  | ASN | A | 391 | 6.221  | 27.924 | 34.426 | 1.00 | 22.34 | A | C |
| ATOM | 2898 | OD1 | ASN | A | 391 | 7.345  | 28.481 | 34.388 | 1.00 | 22.21 | A | O |
| ATOM | 2899 | ND2 | ASN | A | 391 | 5.151  | 28.490 | 35.029 | 1.00 | 23.08 | A | N |
| ATOM | 2900 | C   | ASN | A | 391 | 5.611  | 24.072 | 33.978 | 1.00 | 20.79 | A | C |
| ATOM | 2901 | O   | ASN | A | 391 | 6.295  | 23.818 | 32.978 | 1.00 | 21.08 | A | O |
| ATOM | 2902 | N   | TRP | A | 392 | 4.741  | 23.211 | 34.467 | 1.00 | 20.10 | A | N |
| ATOM | 2903 | CA  | TRP | A | 392 | 4.601  | 21.862 | 33.928 | 1.00 | 20.04 | A | C |
| ATOM | 2904 | CB  | TRP | A | 392 | 3.893  | 20.979 | 34.926 | 1.00 | 20.74 | A | C |
| ATOM | 2905 | CG  | TRP | A | 392 | 4.629  | 20.757 | 36.231 | 1.00 | 24.30 | A | C |
| ATOM | 2906 | CD1 | TRP | A | 392 | 4.687  | 21.605 | 37.309 | 1.00 | 28.54 | A | C |
| ATOM | 2907 | NE1 | TRP | A | 392 | 5.435  | 21.038 | 38.317 | 1.00 | 30.71 | A | N |
| ATOM | 2908 | CE2 | TRP | A | 392 | 5.870  | 19.804 | 37.902 | 1.00 | 28.29 | A | C |
| ATOM | 2909 | CD2 | TRP | A | 392 | 5.367  | 19.594 | 36.598 | 1.00 | 27.17 | A | C |
| ATOM | 2910 | CE3 | TRP | A | 392 | 5.695  | 18.400 | 35.937 | 1.00 | 29.38 | A | C |
| ATOM | 2911 | CZ3 | TRP | A | 392 | 6.456  | 17.447 | 36.608 | 1.00 | 28.54 | A | C |
| ATOM | 2912 | CH2 | TRP | A | 392 | 6.922  | 17.683 | 37.904 | 1.00 | 30.49 | A | C |
| ATOM | 2913 | CZ2 | TRP | A | 392 | 6.643  | 18.857 | 38.566 | 1.00 | 30.31 | A | C |
| ATOM | 2914 | C   | TRP | A | 392 | 3.767  | 21.890 | 32.661 | 1.00 | 19.03 | A | C |
| ATOM | 2915 | O   | TRP | A | 392 | 2.828  | 22.678 | 32.552 | 1.00 | 19.40 | A | O |
| ATOM | 2916 | N   | ASP | A | 393 | 4.107  | 21.020 | 31.709 | 1.00 | 17.66 | A | N |
| ATOM | 2917 | CA  | ASP | A | 393 | 3.416  | 20.958 | 30.424 | 1.00 | 16.59 | A | C |
| ATOM | 2918 | CB  | ASP | A | 393 | 4.431  | 20.669 | 29.332 | 1.00 | 16.47 | A | C |
| ATOM | 2919 | CG  | ASP | A | 393 | 3.813  | 20.660 | 27.930 | 1.00 | 15.80 | A | C |
| ATOM | 2920 | OD1 | ASP | A | 393 | 4.350  | 21.364 | 27.045 | 1.00 | 14.98 | A | O |
| ATOM | 2921 | OD2 | ASP | A | 393 | 2.817  | 19.975 | 27.629 | 1.00 | 15.12 | A | O |
| ATOM | 2922 | C   | ASP | A | 393 | 2.324  | 19.888 | 30.425 | 1.00 | 16.46 | A | C |
| ATOM | 2923 | O   | ASP | A | 393 | 2.606  | 18.716 | 30.648 | 1.00 | 15.65 | A | O |
| ATOM | 2924 | N   | GLY | A | 394 | 1.080  | 20.302 | 30.178 | 1.00 | 15.96 | A | N |
| ATOM | 2925 | CA  | GLY | A | 394 | -0.029 | 19.384 | 30.014 | 1.00 | 16.13 | A | C |
| ATOM | 2926 | C   | GLY | A | 394 | -0.747 | 19.498 | 28.675 | 1.00 | 16.27 | A | C |
| ATOM | 2927 | O   | GLY | A | 394 | -1.936 | 19.255 | 28.601 | 1.00 | 16.15 | A | O |
| ATOM | 2928 | N   | ARG | A | 395 | -0.030 | 19.864 | 27.617 | 1.00 | 17.31 | A | N |
| ATOM | 2929 | CA  | ARG | A | 395 | -0.607 | 19.978 | 26.264 | 1.00 | 17.68 | A | C |
| ATOM | 2930 | CB  | ARG | A | 395 | -0.588 | 21.437 | 25.783 | 1.00 | 19.08 | A | C |
| ATOM | 2931 | CG  | ARG | A | 395 | -1.434 | 22.408 | 26.518 | 1.00 | 26.02 | A | C |
| ATOM | 2932 | CD  | ARG | A | 395 | -1.172 | 23.839 | 26.066 | 1.00 | 31.34 | A | C |
| ATOM | 2933 | NE  | ARG | A | 395 | -1.802 | 24.813 | 26.969 | 1.00 | 37.30 | A | N |
| ATOM | 2934 | CZ  | ARG | A | 395 | -3.026 | 25.331 | 26.821 | 1.00 | 40.58 | A | C |
| ATOM | 2935 | NH1 | ARG | A | 395 | -3.478 | 26.216 | 27.717 | 1.00 | 44.12 | A | N |
| ATOM | 2936 | NH2 | ARG | A | 395 | -3.805 | 24.983 | 25.805 | 1.00 | 40.20 | A | N |
| ATOM | 2937 | C   | ARG | A | 395 | 0.176  | 19.240 | 25.165 | 1.00 | 15.80 | A | C |
| ATOM | 2938 | O   | ARG | A | 395 | -0.418 | 18.827 | 24.176 | 1.00 | 16.01 | A | O |
| ATOM | 2939 | N   | ASN | A | 396 | 1.502  | 19.212 | 25.282 | 1.00 | 14.41 | A | N |

```
ATOM   2940  CA   ASN A 396      2.389  18.645  24.251  1.00 13.89      A    C
ATOM   2941  CB   ASN A 396      3.662  19.483  24.133  1.00 13.10      A    C
ATOM   2942  CG   ASN A 396      3.408  20.889  23.585  1.00 14.63      A    C
ATOM   2943  OD1  ASN A 396      3.129  21.075  22.374  1.00 11.53      A    O
ATOM   2944  ND2  ASN A 396      3.550  21.897  24.463  1.00 11.89      A    N
ATOM   2945  C    ASN A 396      2.806  17.197  24.475  1.00 13.64      A    C
ATOM   2946  O    ASN A 396      2.995  16.743  25.634  1.00 14.92      A    O
ATOM   2947  N    ASN A 397      2.973  16.452  23.376  1.00 13.56      A    N
ATOM   2948  CA   ASN A 397      3.539  15.085  23.451  1.00 12.37      A    C
ATOM   2949  CB   ASN A 397      2.705  14.080  22.672  1.00 12.41      A    C
ATOM   2950  CG   ASN A 397      2.539  14.450  21.192  1.00 13.07      A    C
ATOM   2951  OD1  ASN A 397      2.243  15.594  20.849  1.00 12.07      A    O
ATOM   2952  ND2  ASN A 397      2.683  13.466  20.324  1.00 13.08      A    N
ATOM   2953  C    ASN A 397      5.011  15.077  23.010  1.00 13.13      A    C
ATOM   2954  O    ASN A 397      5.607  14.010  22.663  1.00 11.34      A    O
ATOM   2955  N    VAL A 398      5.577  16.291  23.028  1.00 12.66      A    N
ATOM   2956  CA   VAL A 398      6.992  16.524  22.914  1.00 12.58      A    C
ATOM   2957  CB   VAL A 398      7.329  17.261  21.626  1.00 13.06      A    C
ATOM   2958  CG1  VAL A 398      8.835  17.523  21.533  1.00 11.41      A    C
ATOM   2959  CG2  VAL A 398      6.846  16.476  20.408  1.00 12.98      A    C
ATOM   2960  C    VAL A 398      7.381  17.412  24.105  1.00 12.92      A    C
ATOM   2961  O    VAL A 398      6.819  18.501  24.272  1.00 13.08      A    O
ATOM   2962  N    GLU A 399      8.288  16.913  24.945  1.00 12.47      A    N
ATOM   2963  CA   GLU A 399      8.797  17.666  26.107  1.00 12.81      A    C
ATOM   2964  CB   GLU A 399      8.339  17.054  27.452  1.00 12.40      A    C
ATOM   2965  CG   GLU A 399      6.870  17.340  27.793  1.00 11.56      A    C
ATOM   2966  CD   GLU A 399      6.538  17.357  29.284  1.00 13.58      A    C
ATOM   2967  OE1  GLU A 399      5.312  17.324  29.635  1.00 14.48      A    O
ATOM   2968  OE2  GLU A 399      7.471  17.421  30.112  1.00 14.20      A    O
ATOM   2969  C    GLU A 399     10.307  17.680  26.052  1.00 12.44      A    C
ATOM   2970  O    GLU A 399     10.920  16.624  25.929  1.00 13.16      A    O
ATOM   2971  N    ASN A 400     10.890  18.883  26.174  1.00 12.69      A    N
ATOM   2972  CA   ASN A 400     12.326  19.098  26.073  1.00 12.36      A    C
ATOM   2973  CB   ASN A 400     12.636  19.953  24.822  1.00 12.37      A    C
ATOM   2974  CG   ASN A 400     12.185  19.302  23.559  1.00 14.26      A    C
ATOM   2975  OD1  ASN A 400     12.621  18.213  23.247  1.00 16.94      A    O
ATOM   2976  ND2  ASN A 400     11.302  19.964  22.817  1.00 14.56      A    N
ATOM   2977  C    ASN A 400     12.959  19.820  27.254  1.00 11.55      A    C
ATOM   2978  O    ASN A 400     12.363  20.716  27.867  1.00 11.03      A    O
ATOM   2979  N    VAL A 401     14.200  19.448  27.543  1.00 11.83      A    N
ATOM   2980  CA   VAL A 401     15.042  20.183  28.494  1.00 11.67      A    C
ATOM   2981  CB   VAL A 401     15.230  19.394  29.804  1.00 11.24      A    C
ATOM   2982  CG1  VAL A 401     16.317  20.017  30.668  1.00 13.18      A    C
ATOM   2983  CG2  VAL A 401     13.962  19.359  30.558  1.00 11.36      A    C
ATOM   2984  C    VAL A 401     16.351  20.372  27.792  1.00 12.10      A    C
ATOM   2985  O    VAL A 401     17.022  19.394  27.471  1.00 12.05      A    O
ATOM   2986  N    PHE A 402     16.693  21.634  27.528  1.00 12.69      A    N
ATOM   2987  CA   PHE A 402     17.841  22.019  26.744  1.00 14.06      A    C
ATOM   2988  CB   PHE A 402     17.401  22.853  25.517  1.00 14.70      A    C
ATOM   2989  CG   PHE A 402     16.602  22.079  24.464  1.00 12.42      A    C
ATOM   2990  CD1  PHE A 402     15.936  22.764  23.455  1.00 15.18      A    C
ATOM   2991  CE1  PHE A 402     15.222  22.069  22.468  1.00 12.37      A    C
ATOM   2992  CZ   PHE A 402     15.195  20.700  22.489  1.00 11.68      A    C
ATOM   2993  CE2  PHE A 402     15.841  20.022  23.493  1.00 14.06      A    C
ATOM   2994  CD2  PHE A 402     16.534  20.698  24.465  1.00 10.95      A    C
ATOM   2995  C    PHE A 402     18.725  22.896  27.641  1.00 15.85      A    C
ATOM   2996  O    PHE A 402     18.356  24.021  27.952  1.00 16.80      A    O
ATOM   2997  N    ILE A 403     19.886  22.379  28.028  1.00 16.60      A    N
ATOM   2998  CA   ILE A 403     20.787  23.062  28.963  1.00 17.41      A    C
ATOM   2999  CB   ILE A 403     21.088  22.167  30.160  1.00 16.74      A    C
ATOM   3000  CG1  ILE A 403     19.802  21.886  30.944  1.00 17.16      A    C
ATOM   3001  CD1  ILE A 403     19.946  20.733  31.931  1.00 15.43      A    C
ATOM   3002  CG2  ILE A 403     22.143  22.809  31.095  1.00 17.20      A    C
```

```
ATOM   3003  C    ILE A 403    22.064  23.395  28.240  1.00 17.62      A   C
ATOM   3004  O    ILE A 403    22.812  22.520  27.854  1.00 17.97      A   O
ATOM   3005  N    ASN A 404    22.299  24.678  28.026  1.00 18.39      A   N
ATOM   3006  CA   ASN A 404    23.429  25.112  27.231  1.00 19.61      A   C
ATOM   3007  CB   ASN A 404    23.255  26.599  26.874  1.00 20.94      A   C
ATOM   3008  CG   ASN A 404    24.297  27.071  25.913  1.00 26.59      A   C
ATOM   3009  OD1  ASN A 404    24.339  26.618  24.752  1.00 32.24      A   O
ATOM   3010  ND2  ASN A 404    25.177  27.980  26.381  1.00 32.91      A   N
ATOM   3011  C    ASN A 404    24.773  24.892  27.940  1.00 18.94      A   C
ATOM   3012  O    ASN A 404    25.769  24.575  27.296  1.00 18.69      A   O
ATOM   3013  N    ALA A 405    24.779  25.020  29.262  1.00 18.60      A   N
ATOM   3014  CA   ALA A 405    26.011  24.902  30.044  1.00 19.42      A   C
ATOM   3015  CB   ALA A 405    26.450  26.317  30.582  1.00 19.40      A   C
ATOM   3016  C    ALA A 405    25.787  23.934  31.217  1.00 19.12      A   C
ATOM   3017  O    ALA A 405    25.582  24.364  32.360  1.00 18.74      A   O
ATOM   3018  N    PRO A 406    25.782  22.632  30.936  1.00 19.16      A   N
ATOM   3019  CA   PRO A 406    25.508  21.629  31.977  1.00 19.37      A   C
ATOM   3020  CB   PRO A 406    25.266  20.351  31.156  1.00 19.25      A   C
ATOM   3021  CG   PRO A 406    26.120  20.546  29.977  1.00 19.80      A   C
ATOM   3022  CD   PRO A 406    26.033  22.010  29.631  1.00 18.90      A   C
ATOM   3023  C    PRO A 406    26.689  21.437  32.923  1.00 19.45      A   C
ATOM   3024  O    PRO A 406    27.815  21.833  32.607  1.00 19.95      A   O
ATOM   3025  N    GLN A 407    26.437  20.819  34.072  1.00 19.97      A   N
ATOM   3026  CA   GLN A 407    27.490  20.446  35.016  1.00 20.20      A   C
ATOM   3027  CB   GLN A 407    26.908  20.387  36.413  1.00 21.02      A   C
ATOM   3028  CG   GLN A 407    26.155  21.620  36.805  1.00 22.32      A   C
ATOM   3029  CD   GLN A 407    25.122  21.323  37.849  1.00 23.76      A   C
ATOM   3030  OE1  GLN A 407    25.320  20.443  38.713  1.00 21.20      A   O
ATOM   3031  NE2  GLN A 407    24.016  22.040  37.789  1.00 22.99      A   N
ATOM   3032  C    GLN A 407    28.062  19.075  34.675  1.00 20.27      A   C
ATOM   3033  O    GLN A 407    27.392  18.232  34.057  1.00 19.75      A   O
ATOM   3034  N    SER A 408    29.294  18.830  35.099  1.00 20.30      A   N
ATOM   3035  CA   SER A 408    29.869  17.491  35.033  1.00 20.08      A   C
ATOM   3036  CB   SER A 408    31.393  17.538  35.212  1.00 20.55      A   C
ATOM   3037  OG   SER A 408    32.042  18.067  34.072  1.00 19.34      A   O
ATOM   3038  C    SER A 408    29.269  16.615  36.120  1.00 19.97      A   C
ATOM   3039  O    SER A 408    29.130  17.043  37.268  1.00 20.96      A   O
ATOM   3040  N    GLY A 409    28.980  15.362  35.775  1.00 19.80      A   N
ATOM   3041  CA   GLY A 409    28.447  14.392  36.715  1.00 18.94      A   C
ATOM   3042  C    GLY A 409    27.216  13.697  36.160  1.00 19.28      A   C
ATOM   3043  O    GLY A 409    27.026  13.646  34.940  1.00 18.32      A   O
ATOM   3044  N    THR A 410    26.350  13.224  37.058  1.00 18.72      A   N
ATOM   3045  CA   THR A 410    25.226  12.396  36.678  1.00 18.26      A   C
ATOM   3046  CB   THR A 410    25.105  11.220  37.631  1.00 17.98      A   C
ATOM   3047  OG1  THR A 410    26.334  10.466  37.637  1.00 16.34      A   O
ATOM   3048  CG2  THR A 410    24.038  10.227  37.136  1.00 18.99      A   C
ATOM   3049  C    THR A 410    23.923  13.183  36.687  1.00 18.19      A   C
ATOM   3050  O    THR A 410    23.510  13.735  37.718  1.00 18.85      A   O
ATOM   3051  N    TYR A 411    23.274  13.241  35.524  1.00 17.34      A   N
ATOM   3052  CA   TYR A 411    21.942  13.783  35.430  1.00 16.46      A   C
ATOM   3053  CB   TYR A 411    21.731  14.459  34.067  1.00 16.84      A   C
ATOM   3054  CG   TYR A 411    22.286  15.869  34.025  1.00 16.33      A   C
ATOM   3055  CD1  TYR A 411    21.458  16.953  34.156  1.00 16.27      A   C
ATOM   3056  CE1  TYR A 411    21.956  18.231  34.131  1.00 16.41      A   C
ATOM   3057  CZ   TYR A 411    23.319  18.438  33.994  1.00 17.03      A   C
ATOM   3058  OH   TYR A 411    23.789  19.744  34.031  1.00 17.03      A   O
ATOM   3059  CE2  TYR A 411    24.172  17.380  33.880  1.00 16.15      A   C
ATOM   3060  CD2  TYR A 411    23.660  16.099  33.889  1.00 17.36      A   C
ATOM   3061  C    TYR A 411    20.956  12.627  35.606  1.00 16.14      A   C
ATOM   3062  O    TYR A 411    21.157  11.557  35.041  1.00 15.95      A   O
ATOM   3063  N    THR A 412    19.920  12.841  36.399  1.00 15.50      A   N
ATOM   3064  CA   THR A 412    18.760  11.959  36.418  1.00 16.39      A   C
ATOM   3065  CB   THR A 412    18.107  12.037  37.808  1.00 16.73      A   C
```

| ATOM | 3066 | OG1 | THR | A | 412 | 19.041 | 11.544 | 38.783 | 1.00 | 16.43 | A | O |
|------|------|-----|-----|---|-----|--------|--------|--------|------|-------|---|---|
| ATOM | 3067 | CG2 | THR | A | 412 | 16.877 | 11.115 | 37.946 | 1.00 | 18.16 | A | C |
| ATOM | 3068 | C | THR | A | 412 | 17.764 | 12.397 | 35.344 | 1.00 | 16.26 | A | C |
| ATOM | 3069 | O | THR | A | 412 | 17.404 | 13.568 | 35.286 | 1.00 | 15.84 | A | O |
| ATOM | 3070 | N | VAL | A | 413 | 17.313 | 11.444 | 34.516 | 1.00 | 16.67 | A | N |
| ATOM | 3071 | CA | VAL | A | 413 | 16.342 | 11.672 | 33.452 | 1.00 | 15.75 | A | C |
| ATOM | 3072 | CB | VAL | A | 413 | 16.924 | 11.246 | 32.066 | 1.00 | 16.41 | A | C |
| ATOM | 3073 | CG1 | VAL | A | 413 | 15.914 | 11.476 | 30.914 | 1.00 | 15.53 | A | C |
| ATOM | 3074 | CG2 | VAL | A | 413 | 18.240 | 11.946 | 31.773 | 1.00 | 15.42 | A | C |
| ATOM | 3075 | C | VAL | A | 413 | 15.134 | 10.811 | 33.791 | 1.00 | 16.95 | A | C |
| ATOM | 3076 | O | VAL | A | 413 | 15.232 | 9.574 | 33.667 | 1.00 | 18.21 | A | O |
| ATOM | 3077 | N | GLU | A | 414 | 14.040 | 11.439 | 34.256 | 1.00 | 16.21 | A | N |
| ATOM | 3078 | CA | GLU | A | 414 | 12.803 | 10.774 | 34.736 | 1.00 | 17.01 | A | C |
| ATOM | 3079 | CB | GLU | A | 414 | 12.467 | 11.202 | 36.134 | 1.00 | 16.06 | A | C |
| ATOM | 3080 | CG | GLU | A | 414 | 11.518 | 10.244 | 36.767 | 1.00 | 18.61 | A | C |
| ATOM | 3081 | CD | GLU | A | 414 | 11.626 | 10.318 | 38.265 | 1.00 | 17.99 | A | C |
| ATOM | 3082 | OE1 | GLU | A | 414 | 11.212 | 11.334 | 38.830 | 1.00 | 20.38 | A | O |
| ATOM | 3083 | OE2 | GLU | A | 414 | 12.198 | 9.404 | 38.810 | 1.00 | 18.55 | A | O |
| ATOM | 3084 | C | GLU | A | 414 | 11.715 | 11.142 | 33.729 | 1.00 | 16.05 | A | C |
| ATOM | 3085 | O | GLU | A | 414 | 11.428 | 12.301 | 33.528 | 1.00 | 15.27 | A | O |
| ATOM | 3086 | N | VAL | A | 415 | 11.037 | 10.172 | 33.145 | 1.00 | 16.93 | A | N |
| ATOM | 3087 | CA | VAL | A | 415 | 9.622 | 10.184 | 32.847 | 1.00 | 16.73 | A | C |
| ATOM | 3088 | CB | VAL | A | 415 | 9.472 | 9.367 | 31.526 | 1.00 | 17.17 | A | C |
| ATOM | 3089 | CG1 | VAL | A | 415 | 8.168 | 9.660 | 30.813 | 1.00 | 16.00 | A | C |
| ATOM | 3090 | CG2 | VAL | A | 415 | 10.652 | 9.660 | 30.622 | 1.00 | 16.72 | A | C |
| ATOM | 3091 | C | VAL | A | 415 | 8.540 | 9.769 | 33.787 | 1.00 | 16.71 | A | C |
| ATOM | 3092 | O | VAL | A | 415 | 8.463 | 8.634 | 34.185 | 1.00 | 16.19 | A | O |
| ATOM | 3093 | N | GLN | A | 416 | 7.684 | 10.736 | 34.077 | 1.00 | 16.05 | A | N |
| ATOM | 3094 | CA | GLN | A | 416 | 6.553 | 10.579 | 34.989 | 1.00 | 16.43 | A | C |
| ATOM | 3095 | CB | GLN | A | 416 | 6.519 | 11.747 | 35.981 | 1.00 | 16.01 | A | C |
| ATOM | 3096 | CG | GLN | A | 416 | 7.786 | 11.832 | 36.802 | 1.00 | 16.27 | A | C |
| ATOM | 3097 | CD | GLN | A | 416 | 7.821 | 12.929 | 37.827 | 1.00 | 17.22 | A | C |
| ATOM | 3098 | OE1 | GLN | A | 416 | 6.912 | 13.762 | 37.905 | 1.00 | 16.47 | A | O |
| ATOM | 3099 | NE2 | GLN | A | 416 | 8.933 | 12.972 | 38.601 | 1.00 | 16.72 | A | N |
| ATOM | 3100 | C | GLN | A | 416 | 5.232 | 10.504 | 34.235 | 1.00 | 17.01 | A | C |
| ATOM | 3101 | O | GLN | A | 416 | 4.899 | 11.388 | 33.440 | 1.00 | 16.94 | A | O |
| ATOM | 3102 | N | ALA | A | 417 | 4.461 | 9.462 | 34.522 | 1.00 | 17.66 | A | N |
| ATOM | 3103 | CA | ALA | A | 417 | 3.122 | 9.307 | 33.953 | 1.00 | 18.17 | A | C |
| ATOM | 3104 | CB | ALA | A | 417 | 2.770 | 7.857 | 33.891 | 1.00 | 18.16 | A | C |
| ATOM | 3105 | C | ALA | A | 417 | 2.092 | 10.083 | 34.790 | 1.00 | 19.19 | A | C |
| ATOM | 3106 | O | ALA | A | 417 | 1.542 | 9.565 | 35.775 | 1.00 | 18.61 | A | O |
| ATOM | 3107 | N | TYR | A | 418 | 1.859 | 11.338 | 34.437 | 1.00 | 19.27 | A | N |
| ATOM | 3108 | CA | TYR | A | 418 | 0.944 | 12.153 | 35.234 | 1.00 | 20.10 | A | C |
| ATOM | 3109 | CB | TYR | A | 418 | 0.985 | 13.618 | 34.803 | 1.00 | 20.65 | A | C |
| ATOM | 3110 | CG | TYR | A | 418 | 0.021 | 14.496 | 35.570 | 1.00 | 23.36 | A | C |
| ATOM | 3111 | CD1 | TYR | A | 418 | 0.255 | 14.818 | 36.908 | 1.00 | 26.20 | A | C |
| ATOM | 3112 | CE1 | TYR | A | 418 | -0.645 | 15.610 | 37.625 | 1.00 | 29.96 | A | C |
| ATOM | 3113 | CZ | TYR | A | 418 | -1.772 | 16.099 | 36.990 | 1.00 | 31.45 | A | C |
| ATOM | 3114 | OH | TYR | A | 418 | -2.659 | 16.888 | 37.685 | 1.00 | 34.45 | A | O |
| ATOM | 3115 | CE2 | TYR | A | 418 | -2.018 | 15.804 | 35.652 | 1.00 | 29.32 | A | C |
| ATOM | 3116 | CD2 | TYR | A | 418 | -1.123 | 15.002 | 34.957 | 1.00 | 26.35 | A | C |
| ATOM | 3117 | C | TYR | A | 418 | -0.477 | 11.623 | 35.158 | 1.00 | 19.95 | A | C |
| ATOM | 3118 | O | TYR | A | 418 | -1.142 | 11.445 | 36.190 | 1.00 | 19.83 | A | O |
| ATOM | 3119 | N | ASN | A | 419 | -0.928 | 11.332 | 33.945 | 1.00 | 19.39 | A | N |
| ATOM | 3120 | CA | ASN | A | 419 | -2.284 | 10.855 | 33.708 | 1.00 | 19.40 | A | C |
| ATOM | 3121 | CB | ASN | A | 419 | -3.243 | 12.051 | 33.629 | 1.00 | 20.03 | A | C |
| ATOM | 3122 | CG | ASN | A | 419 | -4.705 | 11.625 | 33.611 | 1.00 | 21.53 | A | C |
| ATOM | 3123 | OD1 | ASN | A | 419 | -5.094 | 10.758 | 34.354 | 1.00 | 23.42 | A | O |
| ATOM | 3124 | ND2 | ASN | A | 419 | -5.493 | 12.212 | 32.727 | 1.00 | 22.67 | A | N |
| ATOM | 3125 | C | ASN | A | 419 | -2.374 | 10.079 | 32.402 | 1.00 | 19.75 | A | C |
| ATOM | 3126 | O | ASN | A | 419 | -2.186 | 10.646 | 31.317 | 1.00 | 19.24 | A | O |
| ATOM | 3127 | N | VAL | A | 420 | -2.703 | 8.795 | 32.486 | 1.00 | 19.24 | A | N |
| ATOM | 3128 | CA | VAL | A | 420 | -2.744 | 7.948 | 31.295 | 1.00 | 18.57 | A | C |

| ATOM | 3129 | CB | VAL A 420 | -1.533 | 6.986 | 31.288 | 1.00 | 18.55 | A | C |
|------|------|------|-----------|--------|-------|--------|------|-------|---|---|
| ATOM | 3130 | CG1 | VAL A 420 | -1.504 | 6.086 | 30.040 | 1.00 | 17.15 | A | C |
| ATOM | 3131 | CG2 | VAL A 420 | -0.196 | 7.799 | 31.402 | 1.00 | 20.43 | A | C |
| ATOM | 3132 | C | VAL A 420 | -4.067 | 7.165 | 31.234 | 1.00 | 18.55 | A | C |
| ATOM | 3133 | O | VAL A 420 | -4.109 | 5.996 | 31.606 | 1.00 | 18.55 | A | O |
| ATOM | 3134 | N | PRO A 421 | -5.132 | 7.816 | 30.776 | 1.00 | 18.79 | A | N |
| ATOM | 3135 | CA | PRO A 421 | -6.444 | 7.169 | 30.635 | 1.00 | 19.54 | A | C |
| ATOM | 3136 | CB | PRO A 421 | -7.397 | 8.324 | 30.288 | 1.00 | 19.65 | A | C |
| ATOM | 3137 | CG | PRO A 421 | -6.540 | 9.394 | 29.746 | 1.00 | 19.70 | A | C |
| ATOM | 3138 | CD | PRO A 421 | -5.175 | 9.239 | 30.396 | 1.00 | 18.68 | A | C |
| ATOM | 3139 | C | PRO A 421 | -6.507 | 6.141 | 29.530 | 1.00 | 19.68 | A | C |
| ATOM | 3140 | O | PRO A 421 | -7.411 | 5.318 | 29.565 | 1.00 | 20.15 | A | O |
| ATOM | 3141 | N | VAL A 422 | -5.592 | 6.178 | 28.566 | 1.00 | 19.04 | A | N |
| ATOM | 3142 | CA | VAL A 422 | -5.594 | 5.180 | 27.505 | 1.00 | 18.85 | A | C |
| ATOM | 3143 | CB | VAL A 422 | -5.990 | 5.781 | 26.146 | 1.00 | 18.35 | A | C |
| ATOM | 3144 | CG1 | VAL A 422 | -6.200 | 4.653 | 25.091 | 1.00 | 18.91 | A | C |
| ATOM | 3145 | CG2 | VAL A 422 | -7.264 | 6.616 | 26.285 | 1.00 | 18.28 | A | C |
| ATOM | 3146 | C | VAL A 422 | -4.226 | 4.509 | 27.448 | 1.00 | 19.38 | A | C |
| ATOM | 3147 | O | VAL A 422 | -3.435 | 4.713 | 26.505 | 1.00 | 18.38 | A | O |
| ATOM | 3148 | N | GLY A 423 | -3.957 | 3.707 | 28.480 | 1.00 | 19.46 | A | N |
| ATOM | 3149 | CA | GLY A 423 | -2.642 | 3.150 | 28.702 | 1.00 | 19.62 | A | C |
| ATOM | 3150 | C | GLY A 423 | -2.510 | 1.665 | 28.496 | 1.00 | 19.72 | A | C |
| ATOM | 3151 | O | GLY A 423 | -3.464 | 0.954 | 28.162 | 1.00 | 21.71 | A | O |
| ATOM | 3152 | N | PRO A 424 | -1.307 | 1.174 | 28.695 | 1.00 | 19.10 | A | N |
| ATOM | 3153 | CA | PRO A 424 | -0.142 | 1.999 | 29.040 | 1.00 | 17.55 | A | C |
| ATOM | 3154 | CB | PRO A 424 | 0.876 | 0.969 | 29.467 | 1.00 | 18.01 | A | C |
| ATOM | 3155 | CG | PRO A 424 | 0.510 | -0.258 | 28.696 | 1.00 | 19.87 | A | C |
| ATOM | 3156 | CD | PRO A 424 | -0.988 | -0.267 | 28.649 | 1.00 | 19.26 | A | C |
| ATOM | 3157 | C | PRO A 424 | 0.396 | 2.842 | 27.899 | 1.00 | 16.87 | A | C |
| ATOM | 3158 | O | PRO A 424 | 0.038 | 2.660 | 26.733 | 1.00 | 17.20 | A | O |
| ATOM | 3159 | N | GLN A 425 | 1.248 | 3.798 | 28.239 | 1.00 | 15.38 | A | N |
| ATOM | 3160 | CA | GLN A 425 | 1.848 | 4.678 | 27.240 | 1.00 | 14.36 | A | C |
| ATOM | 3161 | CB | GLN A 425 | 1.507 | 6.140 | 27.559 | 1.00 | 14.88 | A | C |
| ATOM | 3162 | CG | GLN A 425 | 2.070 | 7.202 | 26.576 | 1.00 | 14.70 | A | C |
| ATOM | 3163 | CD | GLN A 425 | 1.512 | 7.043 | 25.180 | 1.00 | 16.71 | A | C |
| ATOM | 3164 | OE1 | GLN A 425 | 0.321 | 7.321 | 24.956 | 1.00 | 15.27 | A | O |
| ATOM | 3165 | NE2 | GLN A 425 | 2.349 | 6.580 | 24.235 | 1.00 | 11.93 | A | N |
| ATOM | 3166 | C | GLN A 425 | 3.341 | 4.470 | 27.252 | 1.00 | 13.10 | A | C |
| ATOM | 3167 | O | GLN A 425 | 3.987 | 4.662 | 28.283 | 1.00 | 12.18 | A | O |
| ATOM | 3168 | N | THR A 426 | 3.887 | 4.036 | 26.112 | 1.00 | 12.88 | A | N |
| ATOM | 3169 | CA | THR A 426 | 5.320 | 4.008 | 25.913 | 1.00 | 12.90 | A | C |
| ATOM | 3170 | CB | THR A 426 | 5.737 | 2.949 | 24.890 | 1.00 | 12.37 | A | C |
| ATOM | 3171 | OG1 | THR A 426 | 5.134 | 3.254 | 23.626 | 1.00 | 12.70 | A | O |
| ATOM | 3172 | CG2 | THR A 426 | 5.232 | 1.573 | 25.283 | 1.00 | 13.56 | A | C |
| ATOM | 3173 | C | THR A 426 | 5.796 | 5.370 | 25.413 | 1.00 | 13.16 | A | C |
| ATOM | 3174 | O | THR A 426 | 4.986 | 6.223 | 25.037 | 1.00 | 14.38 | A | O |
| ATOM | 3175 | N | PHE A 427 | 7.115 | 5.551 | 25.401 | 1.00 | 12.86 | A | N |
| ATOM | 3176 | CA | PHE A 427 | 7.741 | 6.823 | 25.036 | 1.00 | 12.44 | A | C |
| ATOM | 3177 | CB | PHE A 427 | 7.802 | 7.778 | 26.240 | 1.00 | 12.42 | A | C |
| ATOM | 3178 | CG | PHE A 427 | 8.612 | 7.235 | 27.366 | 1.00 | 12.82 | A | C |
| ATOM | 3179 | CD1 | PHE A 427 | 9.988 | 7.361 | 27.365 | 1.00 | 15.21 | A | C |
| ATOM | 3180 | CE1 | PHE A 427 | 10.768 | 6.801 | 28.381 | 1.00 | 15.74 | A | C |
| ATOM | 3181 | CZ | PHE A 427 | 10.161 | 6.102 | 29.408 | 1.00 | 14.88 | A | C |
| ATOM | 3182 | CE2 | PHE A 427 | 8.766 | 5.987 | 29.427 | 1.00 | 16.39 | A | C |
| ATOM | 3183 | CD2 | PHE A 427 | 8.000 | 6.538 | 28.407 | 1.00 | 14.33 | A | C |
| ATOM | 3184 | C | PHE A 427 | 9.149 | 6.532 | 24.549 | 1.00 | 12.14 | A | C |
| ATOM | 3185 | O | PHE A 427 | 9.694 | 5.444 | 24.807 | 1.00 | 11.55 | A | O |
| ATOM | 3186 | N | SER A 428 | 9.721 | 7.523 | 23.867 | 1.00 | 11.59 | A | N |
| ATOM | 3187 | CA | SER A 428 | 11.116 | 7.528 | 23.480 | 1.00 | 11.99 | A | C |
| ATOM | 3188 | CB | SER A 428 | 11.292 | 7.463 | 21.965 | 1.00 | 12.17 | A | C |
| ATOM | 3189 | OG | SER A 428 | 10.837 | 6.219 | 21.442 | 1.00 | 12.32 | A | O |
| ATOM | 3190 | C | SER A 428 | 11.804 | 8.776 | 24.031 | 1.00 | 12.57 | A | C |
| ATOM | 3191 | O | SER A 428 | 11.174 | 9.829 | 24.263 | 1.00 | 11.91 | A | O |

```
ATOM   3192  N   LEU A 429    13.103   8.620  24.278  1.00 12.35      A  N
ATOM   3193  CA  LEU A 429    13.950   9.712  24.714  1.00 12.57      A  C
ATOM   3194  CB  LEU A 429    14.508   9.476  26.135  1.00 12.84      A  C
ATOM   3195  CG  LEU A 429    13.542   9.648  27.296  1.00 13.77      A  C
ATOM   3196  CD1 LEU A 429    14.046   8.907  28.520  1.00 15.69      A  C
ATOM   3197  CD2 LEU A 429    13.348  11.110  27.609  1.00 15.64      A  C
ATOM   3198  C   LEU A 429    15.098   9.756  23.768  1.00 11.82      A  C
ATOM   3199  O   LEU A 429    15.593   8.707  23.372  1.00 11.44      A  O
ATOM   3200  N   ALA A 430    15.532  10.957  23.405  1.00 11.55      A  N
ATOM   3201  CA  ALA A 430    16.805  11.139  22.699  1.00 11.87      A  C
ATOM   3202  CB  ALA A 430    16.581  11.528  21.235  1.00 12.63      A  C
ATOM   3203  C   ALA A 430    17.613  12.215  23.404  1.00 12.76      A  C
ATOM   3204  O   ALA A 430    17.072  13.256  23.776  1.00 12.14      A  O
ATOM   3205  N   ILE A 431    18.907  11.943  23.584  1.00 12.52      A  N
ATOM   3206  CA  ILE A 431    19.813  12.835  24.287  1.00 13.32      A  C
ATOM   3207  CB  ILE A 431    20.325  12.179  25.593  1.00 13.00      A  C
ATOM   3208  CG1 ILE A 431    19.175  11.882  26.542  1.00 14.30      A  C
ATOM   3209  CD1 ILE A 431    19.575  11.061  27.776  1.00 16.21      A  C
ATOM   3210  CG2 ILE A 431    21.292  13.123  26.288  1.00 14.86      A  C
ATOM   3211  C   ILE A 431    21.005  13.176  23.392  1.00 12.92      A  C
ATOM   3212  O   ILE A 431    21.728  12.288  22.937  1.00 11.26      A  O
ATOM   3213  N   VAL A 432    21.192  14.464  23.134  1.00 13.80      A  N
ATOM   3214  CA  VAL A 432    22.387  14.966  22.483  1.00 15.20      A  C
ATOM   3215  CB  VAL A 432    22.028  15.996  21.387  1.00 15.89      A  C
ATOM   3216  CG1 VAL A 432    23.293  16.591  20.809  1.00 15.25      A  C
ATOM   3217  CG2 VAL A 432    21.167  15.361  20.293  1.00 15.39      A  C
ATOM   3218  C   VAL A 432    23.346  15.634  23.498  1.00 16.38      A  C
ATOM   3219  O   VAL A 432    22.923  16.472  24.298  1.00 16.49      A  O
ATOM   3220  N   HIS A 433    24.633  15.257  23.458  1.00 17.45      A  N
ATOM   3221  CA  HIS A 433    25.669  15.872  24.306  1.00 18.55      A  C
ATOM   3222  CB  HIS A 433    25.637  15.240  25.711  1.00 19.21      A  C
ATOM   3223  CG  HIS A 433    26.553  15.885  26.707  1.00 19.32      A  C
ATOM   3224  ND1 HIS A 433    26.497  17.233  27.015  1.00 18.22      A  N
ATOM   3225  CE1 HIS A 433    27.378  17.497  27.969  1.00 18.90      A  C
ATOM   3226  NE2 HIS A 433    27.999  16.380  28.289  1.00 16.51      A  N
ATOM   3227  CD2 HIS A 433    27.502  15.353  27.513  1.00 17.65      A  C
ATOM   3228  C   HIS A 433    27.031  15.627  23.684  1.00 19.74      A  C
ATOM   3229  O   HIS A 433    27.664  16.546  23.133  1.00 21.68      A  O
ATOM   3230  OXT HIS A 433    27.463  14.480  23.735  1.00 19.29      A  O
TER    3230      HIS A 433
HETATM 3231  CA  CA  A 601    15.429  35.876   3.369  1.00 16.92      A  CA
HETATM 3232  CA  CA  A 602     3.346  16.597  30.346  1.00 13.45      A  CA
HETATM 3233  CA  CA  A 603     9.615  28.353  34.891  1.00 17.30      A  CA
ATOM   3234  N   ASP B  16     3.955  53.303 -10.201  1.00 49.01      B  N
ATOM   3235  CA  ASP B  16     4.171  51.870  -9.771  1.00 49.32      B  C
ATOM   3236  CB  ASP B  16     5.553  51.425 -10.270  1.00 49.78      B  C
ATOM   3237  CG  ASP B  16     6.176  52.438 -11.248  1.00 52.12      B  C
ATOM   3238  OD1 ASP B  16     5.667  52.549 -12.399  1.00 54.86      B  O
ATOM   3239  OD2 ASP B  16     7.151  53.181 -10.957  1.00 52.51      B  O
ATOM   3240  C   ASP B  16     4.009  51.690  -8.232  1.00 48.45      B  C
ATOM   3241  O   ASP B  16     4.793  50.996  -7.567  1.00 47.87      B  O
ATOM   3242  N   ARG B  17     2.959  52.301  -7.687  1.00 47.87      B  N
ATOM   3243  CA  ARG B  17     2.863  52.592  -6.247  1.00 47.30      B  C
ATOM   3244  CB  ARG B  17     2.430  54.064  -6.059  1.00 46.77      B  C
ATOM   3245  CG  ARG B  17     3.107  55.055  -7.028  1.00 44.50      B  C
ATOM   3246  CD  ARG B  17     2.860  56.528  -6.691  1.00 39.98      B  C
ATOM   3247  NE  ARG B  17     3.266  56.891  -5.335  1.00 33.05      B  N
ATOM   3248  CZ  ARG B  17     4.483  57.334  -5.001  1.00 29.36      B  C
ATOM   3249  NH1 ARG B  17     5.440  57.459  -5.915  1.00 28.07      B  N
ATOM   3250  NH2 ARG B  17     4.752  57.650  -3.740  1.00 24.57      B  N
ATOM   3251  C   ARG B  17     1.917  51.699  -5.415  1.00 48.01      B  C
ATOM   3252  O   ARG B  17     1.463  52.120  -4.342  1.00 47.42      B  O
ATOM   3253  N   HIS B  18     1.616  50.486  -5.885  1.00 48.51      B  N
```

| ATOM | 3254 | CA | HIS | B | 18 | 0.770 | 49.573 | -5.108 | 1.00 | 48.98 | B | C |
|------|------|-----|-----|---|----|---------|--------|--------|------|-------|---|---|
| ATOM | 3255 | CB | HIS | B | 18 | 0.515 | 48.266 | -5.875 | 1.00 | 49.29 | B | C |
| ATOM | 3256 | CG | HIS | B | 18 | -0.510 | 48.388 | -6.961 | 1.00 | 50.05 | B | C |
| ATOM | 3257 | ND1 | HIS | B | 18 | -0.195 | 48.803 | -8.238 | 1.00 | 51.24 | B | N |
| ATOM | 3258 | CE1 | HIS | B | 18 | -1.291 | 48.814 | -8.979 | 1.00 | 51.27 | B | C |
| ATOM | 3259 | NE2 | HIS | B | 18 | -2.305 | 48.419 | -8.228 | 1.00 | 50.69 | B | N |
| ATOM | 3260 | CD2 | HIS | B | 18 | -1.844 | 48.147 | -6.962 | 1.00 | 50.64 | B | C |
| ATOM | 3261 | C | HIS | B | 18 | 1.429 | 49.229 | -3.770 | 1.00 | 49.16 | B | C |
| ATOM | 3262 | O | HIS | B | 18 | 2.598 | 48.822 | -3.738 | 1.00 | 49.03 | B | O |
| ATOM | 3263 | N | ASN | B | 19 | 0.690 | 49.386 | -2.667 | 1.00 | 49.15 | B | N |
| ATOM | 3264 | CA | ASN | B | 19 | 1.167 | 48.868 | -1.384 | 1.00 | 49.11 | B | C |
| ATOM | 3265 | CB | ASN | B | 19 | 0.276 | 49.313 | -0.205 | 1.00 | 49.60 | B | C |
| ATOM | 3266 | CG | ASN | B | 19 | 0.951 | 49.099 | 1.176 | 1.00 | 51.24 | B | C |
| ATOM | 3267 | OD1 | ASN | B | 19 | 0.415 | 48.415 | 2.058 | 1.00 | 53.59 | B | O |
| ATOM | 3268 | ND2 | ASN | B | 19 | 2.123 | 49.705 | 1.363 | 1.00 | 54.56 | B | N |
| ATOM | 3269 | C | ASN | B | 19 | 1.241 | 47.332 | -1.459 | 1.00 | 48.22 | B | C |
| ATOM | 3270 | O | ASN | B | 19 | 0.443 | 46.685 | -2.138 | 1.00 | 47.43 | B | O |
| ATOM | 3271 | N | LEU | B | 20 | 2.221 | 46.772 | -0.762 | 1.00 | 47.20 | B | N |
| ATOM | 3272 | CA | LEU | B | 20 | 2.393 | 45.333 | -0.689 | 1.00 | 46.38 | B | C |
| ATOM | 3273 | CB | LEU | B | 20 | 3.743 | 45.000 | -0.055 | 1.00 | 46.97 | B | C |
| ATOM | 3274 | CG | LEU | B | 20 | 4.896 | 45.800 | -0.684 | 1.00 | 48.40 | B | C |
| ATOM | 3275 | CD1 | LEU | B | 20 | 6.201 | 45.666 | 0.117 | 1.00 | 49.54 | B | C |
| ATOM | 3276 | CD2 | LEU | B | 20 | 5.076 | 45.391 | -2.158 | 1.00 | 48.69 | B | C |
| ATOM | 3277 | C | LEU | B | 20 | 1.235 | 44.792 | 0.141 | 1.00 | 44.75 | B | C |
| ATOM | 3278 | O | LEU | B | 20 | 1.113 | 45.092 | 1.342 | 1.00 | 45.26 | B | O |
| ATOM | 3279 | N | LYS | B | 21 | 0.338 | 44.073 | -0.523 | 1.00 | 41.97 | B | N |
| ATOM | 3280 | CA | LYS | B | 21 | -0.740 | 43.395 | 0.170 | 1.00 | 39.98 | B | C |
| ATOM | 3281 | CB | LYS | B | 21 | -2.088 | 44.025 | -0.183 | 1.00 | 40.54 | B | C |
| ATOM | 3282 | CG | LYS | B | 21 | -3.225 | 43.550 | 0.700 | 1.00 | 41.63 | B | C |
| ATOM | 3283 | CD | LYS | B | 21 | -4.257 | 44.620 | 0.878 | 1.00 | 43.44 | B | C |
| ATOM | 3284 | CE | LYS | B | 21 | -5.391 | 44.131 | 1.718 | 1.00 | 44.82 | B | C |
| ATOM | 3285 | NZ | LYS | B | 21 | -4.992 | 44.004 | 3.147 | 1.00 | 47.17 | B | N |
| ATOM | 3286 | C | LYS | B | 21 | -0.710 | 41.917 | -0.214 | 1.00 | 37.09 | B | C |
| ATOM | 3287 | O | LYS | B | 21 | -0.679 | 41.588 | -1.395 | 1.00 | 35.80 | B | O |
| ATOM | 3288 | N | THR | B | 22 | -0.685 | 41.045 | 0.796 | 1.00 | 33.91 | B | N |
| ATOM | 3289 | CA | THR | B | 22 | -0.642 | 39.592 | 0.593 | 1.00 | 31.44 | B | C |
| ATOM | 3290 | CB | THR | B | 22 | 0.734 | 39.046 | 1.030 | 1.00 | 31.70 | B | C |
| ATOM | 3291 | OG1 | THR | B | 22 | 1.002 | 39.436 | 2.387 | 1.00 | 31.47 | B | O |
| ATOM | 3292 | CG2 | THR | B | 22 | 1.857 | 39.681 | 0.211 | 1.00 | 31.30 | B | C |
| ATOM | 3293 | C | THR | B | 22 | -1.739 | 38.843 | 1.342 | 1.00 | 29.57 | B | C |
| ATOM | 3294 | O | THR | B | 22 | -1.830 | 37.617 | 1.246 | 1.00 | 28.17 | B | O |
| ATOM | 3295 | N | GLU | B | 23 | -2.542 | 39.576 | 2.107 | 1.00 | 27.46 | B | N |
| ATOM | 3296 | CA | GLU | B | 23 | -3.672 | 39.011 | 2.828 | 1.00 | 27.22 | B | C |
| ATOM | 3297 | CB | BGLU | B | 23 | -3.280 | 38.728 | 4.282 | 0.50 | 27.51 | B | C |
| ATOM | 3298 | CB | AGLU | B | 23 | -3.287 | 38.646 | 4.277 | 0.50 | 27.17 | B | C |
| ATOM | 3299 | CG | BGLU | B | 23 | -2.826 | 37.304 | 4.512 | 0.50 | 29.10 | B | C |
| ATOM | 3300 | CG | AGLU | B | 23 | -3.050 | 39.822 | 5.223 | 0.50 | 27.55 | B | C |
| ATOM | 3301 | CD | BGLU | B | 23 | -2.236 | 37.062 | 5.891 | 0.50 | 30.84 | B | C |
| ATOM | 3302 | CD | AGLU | B | 23 | -3.020 | 39.396 | 6.689 | 0.50 | 28.07 | B | C |
| ATOM | 3303 | OE1 | BGLU | B | 23 | -1.959 | 38.040 | 6.614 | 0.50 | 32.02 | B | O |
| ATOM | 3304 | OE1 | AGLU | B | 23 | -2.853 | 38.186 | 6.954 | 0.50 | 28.41 | B | O |
| ATOM | 3305 | OE2 | BGLU | B | 23 | -2.054 | 35.879 | 6.241 | 0.50 | 31.16 | B | O |
| ATOM | 3306 | OE2 | AGLU | B | 23 | -3.182 | 40.264 | 7.579 | 0.50 | 28.60 | B | O |
| ATOM | 3307 | C | GLU | B | 23 | -4.842 | 39.988 | 2.799 | 1.00 | 25.94 | B | C |
| ATOM | 3308 | O | GLU | B | 23 | -4.631 | 41.199 | 2.805 | 1.00 | 25.17 | B | O |
| ATOM | 3309 | N | TRP | B | 24 | -6.065 | 39.462 | 2.765 | 1.00 | 24.57 | B | N |
| ATOM | 3310 | CA | TRP | B | 24 | -7.264 | 40.300 | 2.708 | 1.00 | 23.89 | B | C |
| ATOM | 3311 | CB | TRP | B | 24 | -7.910 | 40.174 | 1.304 | 1.00 | 23.59 | B | C |
| ATOM | 3312 | CG | TRP | B | 24 | -7.105 | 40.786 | 0.245 | 1.00 | 21.71 | B | C |
| ATOM | 3313 | CD1 | TRP | B | 24 | -7.232 | 42.050 | -0.239 | 1.00 | 21.02 | B | C |
| ATOM | 3314 | NE1 | TRP | B | 24 | -6.293 | 42.276 | -1.211 | 1.00 | 18.62 | B | N |
| ATOM | 3315 | CE2 | TRP | B | 24 | -5.544 | 41.148 | -1.396 | 1.00 | 19.02 | B | C |
| ATOM | 3316 | CD2 | TRP | B | 24 | -6.006 | 40.190 | -0.480 | 1.00 | 20.72 | B | C |

| ATOM | 3317 | CE3 | TRP B | 24 | -5.387 | 38.941 | -0.454 | 1.00 | 19.49 | B | C |
|------|------|-----|-------|----|--------|--------|--------|------|-------|---|---|
| ATOM | 3318 | CZ3 | TRP B | 24 | -4.326 | 38.694 | -1.313 | 1.00 | 20.25 | B | C |
| ATOM | 3319 | CH2 | TRP B | 24 | -3.883 | 39.662 | -2.207 | 1.00 | 21.63 | B | C |
| ATOM | 3320 | CZ2 | TRP B | 24 | -4.477 | 40.911 | -2.257 | 1.00 | 22.09 | B | C |
| ATOM | 3321 | C | TRP B | 24 | -8.294 | 39.948 | 3.789 | 1.00 | 23.91 | B | C |
| ATOM | 3322 | O | TRP B | 24 | -9.369 | 39.456 | 3.467 | 1.00 | 22.74 | B | O |
| ATOM | 3323 | N | PRO B | 25 | -7.986 | 40.196 | 5.070 | 1.00 | 24.64 | B | N |
| ATOM | 3324 | CA | PRO B | 25 | -8.918 | 39.850 | 6.161 | 1.00 | 25.12 | B | C |
| ATOM | 3325 | CB | PRO B | 25 | -8.176 | 40.312 | 7.448 | 1.00 | 25.87 | B | C |
| ATOM | 3326 | CG | PRO B | 25 | -7.011 | 41.163 | 7.002 | 1.00 | 26.08 | B | C |
| ATOM | 3327 | CD | PRO B | 25 | -6.737 | 40.807 | 5.562 | 1.00 | 25.49 | B | C |
| ATOM | 3328 | C | PRO B | 25 | -10.307 | 40.520 | 6.029 | 1.00 | 25.18 | B | C |
| ATOM | 3329 | O | PRO B | 25 | -11.310 | 39.978 | 6.469 | 1.00 | 24.94 | B | O |
| ATOM | 3330 | N | GLU B | 26 | -10.350 | 41.668 | 5.364 | 1.00 | 25.43 | B | N |
| ATOM | 3331 | CA | GLU B | 26 | -11.581 | 42.416 | 5.141 | 1.00 | 25.93 | B | C |
| ATOM | 3332 | CB | GLU B | 26 | -11.243 | 43.829 | 4.627 | 1.00 | 26.70 | B | C |
| ATOM | 3333 | CG | GLU B | 26 | -10.690 | 43.922 | 3.189 | 1.00 | 28.62 | B | C |
| ATOM | 3334 | CD | GLU B | 26 | -9.169 | 43.775 | 3.077 | 1.00 | 29.61 | B | C |
| ATOM | 3335 | OE1 | GLU B | 26 | -8.535 | 43.174 | 3.985 | 1.00 | 28.68 | B | O |
| ATOM | 3336 | OE2 | GLU B | 26 | -8.608 | 44.252 | 2.057 | 1.00 | 31.71 | B | O |
| ATOM | 3337 | C | GLU B | 26 | -12.571 | 41.705 | 4.193 | 1.00 | 25.60 | B | C |
| ATOM | 3338 | O | GLU B | 26 | -13.746 | 42.060 | 4.139 | 1.00 | 24.75 | B | O |
| ATOM | 3339 | N | LEU B | 27 | -12.119 | 40.672 | 3.483 | 1.00 | 24.52 | B | N |
| ATOM | 3340 | CA | LEU B | 27 | -12.957 | 40.024 | 2.483 | 1.00 | 23.54 | B | C |
| ATOM | 3341 | CB | LEU B | 27 | -12.104 | 39.593 | 1.287 | 1.00 | 23.84 | B | C |
| ATOM | 3342 | CG | LEU B | 27 | -11.506 | 40.722 | 0.430 | 1.00 | 23.08 | B | C |
| ATOM | 3343 | CD1 | LEU B | 27 | -10.702 | 40.165 | -0.732 | 1.00 | 22.31 | B | C |
| ATOM | 3344 | CD2 | LEU B | 27 | -12.603 | 41.624 | -0.097 | 1.00 | 23.37 | B | C |
| ATOM | 3345 | C | LEU B | 27 | -13.716 | 38.829 | 3.042 | 1.00 | 23.75 | B | C |
| ATOM | 3346 | O | LEU B | 27 | -14.504 | 38.205 | 2.334 | 1.00 | 23.33 | B | O |
| ATOM | 3347 | N | VAL B | 28 | -13.490 | 38.504 | 4.312 | 1.00 | 23.89 | B | N |
| ATOM | 3348 | CA | VAL B | 28 | -14.143 | 37.357 | 4.918 | 1.00 | 24.64 | B | C |
| ATOM | 3349 | CB | VAL B | 28 | -13.571 | 37.050 | 6.359 | 1.00 | 24.78 | B | C |
| ATOM | 3350 | CG1 | VAL B | 28 | -14.359 | 35.963 | 7.027 | 1.00 | 25.39 | B | C |
| ATOM | 3351 | CG2 | VAL B | 28 | -12.099 | 36.634 | 6.272 | 1.00 | 25.78 | B | C |
| ATOM | 3352 | C | VAL B | 28 | -15.612 | 37.694 | 4.992 | 1.00 | 24.75 | B | C |
| ATOM | 3353 | O | VAL B | 28 | -15.952 | 38.791 | 5.424 | 1.00 | 25.68 | B | O |
| ATOM | 3354 | N | GLY B | 29 | -16.468 | 36.797 | 4.516 | 1.00 | 24.86 | B | N |
| ATOM | 3355 | CA | GLY B | 29 | -17.916 | 37.000 | 4.539 | 1.00 | 24.80 | B | C |
| ATOM | 3356 | C | GLY B | 29 | -18.493 | 37.638 | 3.274 | 1.00 | 25.07 | B | C |
| ATOM | 3357 | O | GLY B | 29 | -19.692 | 37.598 | 3.061 | 1.00 | 25.27 | B | O |
| ATOM | 3358 | N | LYS B | 30 | -17.630 | 38.203 | 2.429 | 1.00 | 25.21 | B | N |
| ATOM | 3359 | CA | LYS B | 30 | -18.025 | 38.782 | 1.146 | 1.00 | 24.62 | B | C |
| ATOM | 3360 | CB | LYS B | 30 | -16.952 | 39.780 | 0.679 | 1.00 | 25.65 | B | C |
| ATOM | 3361 | CG | LYS B | 30 | -16.716 | 40.964 | 1.606 | 1.00 | 28.42 | B | C |
| ATOM | 3362 | CD | LYS B | 30 | -16.577 | 42.245 | 0.785 | 1.00 | 34.26 | B | C |
| ATOM | 3363 | CE | LYS B | 30 | -16.462 | 43.527 | 1.631 | 1.00 | 35.92 | B | C |
| ATOM | 3364 | NZ | LYS B | 30 | -15.996 | 43.273 | 3.011 | 1.00 | 37.72 | B | N |
| ATOM | 3365 | C | LYS B | 30 | -18.188 | 37.728 | 0.065 | 1.00 | 23.30 | B | C |
| ATOM | 3366 | O | LYS B | 30 | -17.670 | 36.623 | 0.166 | 1.00 | 22.29 | B | O |
| ATOM | 3367 | N | SER B | 31 | -18.884 | 38.089 | -1.001 | 1.00 | 21.94 | B | N |
| ATOM | 3368 | CA | SER B | 31 | -19.036 | 37.204 | -2.145 | 1.00 | 20.89 | B | C |
| ATOM | 3369 | CB | SER B | 31 | -20.046 | 37.776 | -3.143 | 1.00 | 21.21 | B | C |
| ATOM | 3370 | OG | SER B | 31 | -19.519 | 38.912 | -3.815 | 1.00 | 20.40 | B | O |
| ATOM | 3371 | C | SER B | 31 | -17.726 | 37.017 | -2.865 | 1.00 | 19.88 | B | C |
| ATOM | 3372 | O | SER B | 31 | -16.828 | 37.843 | -2.800 | 1.00 | 18.67 | B | O |
| ATOM | 3373 | N | VAL B | 32 | -17.649 | 35.920 | -3.588 | 1.00 | 20.34 | B | N |
| ATOM | 3374 | CA | VAL B | 32 | -16.487 | 35.617 | -4.393 | 1.00 | 20.89 | B | C |
| ATOM | 3375 | CB | BVAL B | 32 | -16.717 | 34.256 | -5.141 | 0.50 | 20.94 | B | C |
| ATOM | 3376 | CB | AVAL B | 32 | -16.555 | 34.234 | -5.043 | 0.50 | 21.07 | B | C |
| ATOM | 3377 | CG1 | BVAL B | 32 | -16.023 | 34.221 | -6.524 | 0.50 | 20.85 | B | C |
| ATOM | 3378 | CG1 | AVAL B | 32 | -17.648 | 34.180 | -6.069 | 0.50 | 20.79 | B | C |
| ATOM | 3379 | CG2 | BVAL B | 32 | -16.276 | 33.087 | -4.281 | 0.50 | 20.73 | B | C |

| ATOM | 3380 | CG2A | VAL | B | 32 | -15.193 | 33.903 | -5.657 | 0.50 | 21.36 | B | C |
| ATOM | 3381 | C | VAL | B | 32 | -16.238 | 36.732 | -5.431 | 1.00 | 20.83 | B | C |
| ATOM | 3382 | O | VAL | B | 32 | -15.100 | 37.105 | -5.681 | 1.00 | 20.37 | B | O |
| ATOM | 3383 | N | GLU | B | 33 | -17.316 | 37.263 | -6.011 | 1.00 | 21.19 | B | N |
| ATOM | 3384 | CA | GLU | B | 33 | -17.205 | 38.264 | -7.072 | 1.00 | 21.20 | B | C |
| ATOM | 3385 | CB | GLU | B | 33 | -18.553 | 38.478 | -7.767 | 1.00 | 21.59 | B | C |
| ATOM | 3386 | CG | GLU | B | 33 | -19.045 | 37.271 | -8.543 | 1.00 | 24.67 | B | C |
| ATOM | 3387 | CD | GLU | B | 33 | -19.799 | 36.219 | -7.708 | 1.00 | 29.01 | B | C |
| ATOM | 3388 | OE1 | GLU | B | 33 | -20.001 | 35.123 | -8.275 | 1.00 | 36.54 | B | O |
| ATOM | 3389 | OE2 | GLU | B | 33 | -20.187 | 36.437 | -6.517 | 1.00 | 27.78 | B | O |
| ATOM | 3390 | C | GLU | B | 33 | -16.688 | 39.571 | -6.497 | 1.00 | 20.62 | B | C |
| ATOM | 3391 | O | GLU | B | 33 | -15.885 | 40.255 | -7.130 | 1.00 | 20.20 | B | O |
| ATOM | 3392 | N | GLU | B | 34 | -17.124 | 39.910 | -5.283 | 1.00 | 20.61 | B | N |
| ATOM | 3393 | CA | GLU | B | 34 | -16.627 | 41.131 | -4.634 | 1.00 | 20.97 | B | C |
| ATOM | 3394 | CB | GLU | B | 34 | -17.456 | 41.533 | -3.407 | 1.00 | 21.10 | B | C |
| ATOM | 3395 | CG | GLU | B | 34 | -18.778 | 42.224 | -3.722 | 1.00 | 25.82 | B | C |
| ATOM | 3396 | CD | GLU | B | 34 | -18.615 | 43.546 | -4.481 | 1.00 | 31.16 | B | C |
| ATOM | 3397 | OE1 | GLU | B | 34 | -17.968 | 44.484 | -3.932 | 1.00 | 32.84 | B | O |
| ATOM | 3398 | OE2 | GLU | B | 34 | -19.135 | 43.645 | -5.626 | 1.00 | 33.71 | B | O |
| ATOM | 3399 | C | GLU | B | 34 | -15.156 | 40.951 | -4.257 | 1.00 | 19.67 | B | C |
| ATOM | 3400 | O | GLU | B | 34 | -14.340 | 41.858 | -4.438 | 1.00 | 18.91 | B | O |
| ATOM | 3401 | N | ALA | B | 35 | -14.809 | 39.775 | -3.765 | 1.00 | 19.75 | B | N |
| ATOM | 3402 | CA | ALA | B | 35 | -13.414 | 39.485 | -3.401 | 1.00 | 19.10 | B | C |
| ATOM | 3403 | CB | ALA | B | 35 | -13.311 | 38.127 | -2.749 | 1.00 | 19.66 | B | C |
| ATOM | 3404 | C | ALA | B | 35 | -12.457 | 39.581 | -4.582 | 1.00 | 18.90 | B | C |
| ATOM | 3405 | O | ALA | B | 35 | -11.387 | 40.183 | -4.470 | 1.00 | 18.82 | B | O |
| ATOM | 3406 | N | LYS | B | 36 | -12.839 | 38.993 | -5.716 | 1.00 | 18.68 | B | N |
| ATOM | 3407 | CA | LYS | B | 36 | -11.991 | 38.978 | -6.894 | 1.00 | 18.17 | B | C |
| ATOM | 3408 | CB | LYS | B | 36 | -12.659 | 38.220 | -8.063 | 1.00 | 18.44 | B | C |
| ATOM | 3409 | CG | LYS | B | 36 | -12.714 | 36.693 | -7.928 | 1.00 | 19.56 | B | C |
| ATOM | 3410 | CD | LYS | B | 36 | -13.304 | 36.026 | -9.159 | 1.00 | 20.72 | B | C |
| ATOM | 3411 | CE | LYS | B | 36 | -13.194 | 34.496 | -9.136 | 1.00 | 22.32 | B | C |
| ATOM | 3412 | NZ | LYS | B | 36 | -13.963 | 33.865 | -10.274 | 1.00 | 20.54 | B | N |
| ATOM | 3413 | C | LYS | B | 36 | -11.648 | 40.406 | -7.316 | 1.00 | 17.67 | B | C |
| ATOM | 3414 | O | LYS | B | 36 | -10.500 | 40.694 | -7.681 | 1.00 | 17.81 | B | O |
| ATOM | 3415 | N | LYS | B | 37 | -12.614 | 41.316 | -7.254 | 1.00 | 17.52 | B | N |
| ATOM | 3416 | CA | LYS | B | 37 | -12.345 | 42.667 | -7.746 | 1.00 | 17.89 | B | C |
| ATOM | 3417 | CB | LYS | B | 37 | -13.621 | 43.519 | -7.870 | 1.00 | 17.35 | B | C |
| ATOM | 3418 | CG | LYS | B | 37 | -14.544 | 43.165 | -9.036 | 1.00 | 17.43 | B | C |
| ATOM | 3419 | CD | LYS | B | 37 | -15.847 | 44.074 | -9.064 | 1.00 | 15.06 | B | C |
| ATOM | 3420 | CE | LYS | B | 37 | -16.801 | 43.812 | -7.921 | 1.00 | 16.48 | B | C |
| ATOM | 3421 | NZ | LYS | B | 37 | -18.031 | 44.685 | -7.989 | 1.00 | 15.66 | B | N |
| ATOM | 3422 | C | LYS | B | 37 | -11.333 | 43.372 | -6.852 | 1.00 | 18.04 | B | C |
| ATOM | 3423 | O | LYS | B | 37 | -10.499 | 44.126 | -7.354 | 1.00 | 18.33 | B | O |
| ATOM | 3424 | N | VAL | B | 38 | -11.436 | 43.174 | -5.535 | 1.00 | 17.93 | B | N |
| ATOM | 3425 | CA | VAL | B | 38 | -10.525 | 43.824 | -4.595 | 1.00 | 18.53 | B | C |
| ATOM | 3426 | CB | VAL | B | 38 | -11.024 | 43.636 | -3.136 | 1.00 | 19.32 | B | C |
| ATOM | 3427 | CG1 | VAL | B | 38 | -9.975 | 44.055 | -2.128 | 1.00 | 21.52 | B | C |
| ATOM | 3428 | CG2 | VAL | B | 38 | -12.310 | 44.445 | -2.919 | 1.00 | 20.73 | B | C |
| ATOM | 3429 | C | VAL | B | 38 | -9.122 | 43.270 | -4.742 | 1.00 | 18.91 | B | C |
| ATOM | 3430 | O | VAL | B | 38 | -8.135 | 44.013 | -4.797 | 1.00 | 17.79 | B | O |
| ATOM | 3431 | N | ILE | B | 39 | -9.033 | 41.947 | -4.830 | 1.00 | 19.11 | B | N |
| ATOM | 3432 | CA | ILE | B | 39 | -7.747 | 41.304 | -5.009 | 1.00 | 19.64 | B | C |
| ATOM | 3433 | CB | ILE | B | 39 | -7.919 | 39.764 | -4.973 | 1.00 | 19.43 | B | C |
| ATOM | 3434 | CG1 | ILE | B | 39 | -8.288 | 39.324 | -3.573 | 1.00 | 20.37 | B | C |
| ATOM | 3435 | CD1 | ILE | B | 39 | -8.994 | 37.995 | -3.564 | 1.00 | 21.36 | B | C |
| ATOM | 3436 | CG2 | ILE | B | 39 | -6.657 | 39.024 | -5.470 | 1.00 | 19.76 | B | C |
| ATOM | 3437 | C | ILE | B | 39 | -7.077 | 41.759 | -6.287 | 1.00 | 19.71 | B | C |
| ATOM | 3438 | O | ILE | B | 39 | -5.877 | 42.087 | -6.266 | 1.00 | 19.70 | B | O |
| ATOM | 3439 | N | LEU | B | 40 | -7.816 | 41.785 | -7.404 | 1.00 | 19.22 | B | N |
| ATOM | 3440 | CA | LEU | B | 40 | -7.205 | 42.231 | -8.664 | 1.00 | 19.62 | B | C |
| ATOM | 3441 | CB | LEU | B | 40 | -8.100 | 41.927 | -9.888 | 1.00 | 19.18 | B | C |
| ATOM | 3442 | CG | LEU | B | 40 | -8.145 | 40.416 | -10.190 | 1.00 | 20.23 | B | C |

```
ATOM   3443  CD1 LEU B  40      -9.235  40.047 -11.123  1.00 19.43           B   C
ATOM   3444  CD2 LEU B  40      -6.799  39.947 -10.725  1.00 21.26           B   C
ATOM   3445  C   LEU B  40      -6.840  43.716  -8.608  1.00 19.20           B   C
ATOM   3446  O   LEU B  40      -5.939  44.144  -9.300  1.00 19.48           B   O
ATOM   3447  N   GLN B  41      -7.553  44.494  -7.803  1.00 19.70           B   N
ATOM   3448  CA  GLN B  41      -7.216  45.914  -7.622  1.00 20.69           B   C
ATOM   3449  CB  GLN B  41      -8.286  46.641  -6.813  1.00 20.30           B   C
ATOM   3450  CG  GLN B  41      -8.068  48.173  -6.731  1.00 20.90           B   C
ATOM   3451  CD  GLN B  41      -8.159  48.842  -8.083  1.00 21.54           B   C
ATOM   3452  OE1 GLN B  41      -9.070  48.529  -8.858  1.00 23.88           B   O
ATOM   3453  NE2 GLN B  41      -7.224  49.762  -8.384  1.00 20.95           B   N
ATOM   3454  C   GLN B  41      -5.880  46.050  -6.906  1.00 22.10           B   C
ATOM   3455  O   GLN B  41      -5.105  46.941  -7.213  1.00 22.64           B   O
ATOM   3456  N   ASP B  42      -5.625  45.149  -5.955  1.00 22.70           B   N
ATOM   3457  CA  ASP B  42      -4.396  45.178  -5.161  1.00 23.28           B   C
ATOM   3458  CB  ASP B  42      -4.654  44.531  -3.800  1.00 22.77           B   C
ATOM   3459  CG  ASP B  42      -5.531  45.369  -2.928  1.00 24.12           B   C
ATOM   3460  OD1 ASP B  42      -5.619  46.599  -3.174  1.00 27.20           B   O
ATOM   3461  OD2 ASP B  42      -6.206  44.899  -1.991  1.00 25.78           B   O
ATOM   3462  C   ASP B  42      -3.273  44.438  -5.859  1.00 23.19           B   C
ATOM   3463  O   ASP B  42      -2.103  44.761  -5.700  1.00 24.27           B   O
ATOM   3464  N   LYS B  43      -3.629  43.444  -6.655  1.00 22.96           B   N
ATOM   3465  CA  LYS B  43      -2.634  42.541  -7.203  1.00 23.10           B   C
ATOM   3466  CB  LYS B  43      -2.508  41.291  -6.299  1.00 23.07           B   C
ATOM   3467  CG  LYS B  43      -1.376  40.306  -6.701  1.00 23.16           B   C
ATOM   3468  CD  LYS B  43      -1.348  39.100  -5.750  1.00 24.15           B   C
ATOM   3469  CE  LYS B  43      -0.391  37.996  -6.217  1.00 25.40           B   C
ATOM   3470  NZ  LYS B  43       1.031  38.403  -6.170  1.00 25.72           B   N
ATOM   3471  C   LYS B  43      -3.067  42.157  -8.593  1.00 23.00           B   C
ATOM   3472  O   LYS B  43      -3.672  41.107  -8.782  1.00 22.17           B   O
ATOM   3473  N   PRO B  44      -2.772  43.010  -9.571  1.00 24.07           B   N
ATOM   3474  CA  PRO B  44      -3.282  42.826 -10.948  1.00 24.35           B   C
ATOM   3475  CB  PRO B  44      -2.632  43.985 -11.735  1.00 24.65           B   C
ATOM   3476  CG  PRO B  44      -2.197  44.997 -10.702  1.00 25.50           B   C
ATOM   3477  CD  PRO B  44      -1.960  44.238  -9.415  1.00 24.73           B   C
ATOM   3478  C   PRO B  44      -2.929  41.486 -11.583  1.00 24.65           B   C
ATOM   3479  O   PRO B  44      -3.680  40.967 -12.409  1.00 25.49           B   O
ATOM   3480  N   GLU B  45      -1.778  40.935 -11.206  1.00 25.20           B   N
ATOM   3481  CA  GLU B  45      -1.310  39.651 -11.725  1.00 25.70           B   C
ATOM   3482  CB  GLU B  45       0.226  39.599 -11.602  1.00 26.83           B   C
ATOM   3483  CG  GLU B  45       0.764  39.243 -10.206  1.00 28.74           B   C
ATOM   3484  CD  GLU B  45       0.925  40.423  -9.262  1.00 32.92           B   C
ATOM   3485  OE1 GLU B  45       1.667  40.252  -8.253  1.00 34.10           B   O
ATOM   3486  OE2 GLU B  45       0.316  41.511  -9.488  1.00 33.16           B   O
ATOM   3487  C   GLU B  45      -1.945  38.404 -11.048  1.00 25.32           B   C
ATOM   3488  O   GLU B  45      -1.679  37.270 -11.452  1.00 25.52           B   O
ATOM   3489  N   ALA B  46      -2.788  38.593 -10.034  1.00 24.59           B   N
ATOM   3490  CA  ALA B  46      -3.327  37.441  -9.309  1.00 23.90           B   C
ATOM   3491  CB  ALA B  46      -4.271  37.895  -8.229  1.00 23.95           B   C
ATOM   3492  C   ALA B  46      -4.015  36.426 -10.216  1.00 23.75           B   C
ATOM   3493  O   ALA B  46      -4.777  36.788 -11.103  1.00 22.67           B   O
ATOM   3494  N   GLN B  47      -3.717  35.150  -9.982  1.00 23.77           B   N
ATOM   3495  CA  GLN B  47      -4.438  34.035 -10.568  1.00 24.59           B   C
ATOM   3496  CB  GLN B  47      -3.479  32.976 -11.105  1.00 25.29           B   C
ATOM   3497  CG  GLN B  47      -2.425  33.498 -12.080  1.00 28.88           B   C
ATOM   3498  CD  GLN B  47      -3.025  33.975 -13.393  1.00 35.56           B   C
ATOM   3499  OE1 GLN B  47      -3.624  33.176 -14.144  1.00 40.01           B   O
ATOM   3500  NE2 GLN B  47      -2.869  35.278 -13.686  1.00 38.46           B   N
ATOM   3501  C   GLN B  47      -5.298  33.425  -9.460  1.00 23.84           B   C
ATOM   3502  O   GLN B  47      -4.786  32.790  -8.517  1.00 24.12           B   O
ATOM   3503  N   ILE B  48      -6.597  33.644  -9.559  1.00 22.91           B   N
ATOM   3504  CA  ILE B  48      -7.502  33.329  -8.463  1.00 23.01           B   C
ATOM   3505  CB  ILE B  48      -8.486  34.462  -8.235  1.00 22.88           B   C
```

| ATOM | 3506 | CG1 | ILE | B | 48 | -7.708 | 35.747 | -7.988 | 1.00 | 22.10 | B | C |
|------|------|-----|-----|---|----|--------|--------|--------|------|-------|---|---|
| ATOM | 3507 | CD1 | ILE | B | 48 | -8.568 | 36.992 | -7.917 | 1.00 | 22.38 | B | C |
| ATOM | 3508 | CG2 | ILE | B | 48 | -9.391 | 34.161 | -7.036 | 1.00 | 21.57 | B | C |
| ATOM | 3509 | C | ILE | B | 48 | -8.230 | 32.047 | -8.746 | 1.00 | 23.69 | B | C |
| ATOM | 3510 | O | ILE | B | 48 | -8.685 | 31.820 | -9.877 | 1.00 | 23.31 | B | O |
| ATOM | 3511 | N | ILE | B | 49 | -8.277 | 31.206 | -7.716 | 1.00 | 23.55 | B | N |
| ATOM | 3512 | CA | ILE | B | 49 | -8.894 | 29.894 | -7.746 | 1.00 | 24.95 | B | C |
| ATOM | 3513 | CB | ILE | B | 49 | -7.803 | 28.812 | -7.480 | 1.00 | 26.22 | B | C |
| ATOM | 3514 | CG1 | ILE | B | 49 | -6.723 | 28.868 | -8.575 | 1.00 | 29.02 | B | C |
| ATOM | 3515 | CD1 | ILE | B | 49 | -7.264 | 28.733 | -9.982 | 1.00 | 29.18 | B | C |
| ATOM | 3516 | CG2 | ILE | B | 49 | -8.409 | 27.422 | -7.364 | 1.00 | 28.74 | B | C |
| ATOM | 3517 | C | ILE | B | 49 | -9.903 | 29.851 | -6.610 | 1.00 | 23.74 | B | C |
| ATOM | 3518 | O | ILE | B | 49 | -9.620 | 30.348 | -5.511 | 1.00 | 24.09 | B | O |
| ATOM | 3519 | N | VAL | B | 50 | -11.045 | 29.224 | -6.847 | 1.00 | 22.75 | B | N |
| ATOM | 3520 | CA | VAL | B | 50 | -12.088 | 29.110 | -5.838 | 1.00 | 22.00 | B | C |
| ATOM | 3521 | CB | VAL | B | 50 | -13.441 | 29.682 | -6.364 | 1.00 | 21.55 | B | C |
| ATOM | 3522 | CG1 | VAL | B | 50 | -14.583 | 29.378 | -5.388 | 1.00 | 22.01 | B | C |
| ATOM | 3523 | CG2 | VAL | B | 50 | -13.338 | 31.190 | -6.581 | 1.00 | 21.04 | B | C |
| ATOM | 3524 | C | VAL | B | 50 | -12.273 | 27.639 | -5.439 | 1.00 | 21.99 | B | C |
| ATOM | 3525 | O | VAL | B | 50 | -12.375 | 26.780 | -6.291 | 1.00 | 21.98 | B | O |
| ATOM | 3526 | N | LEU | B | 51 | -12.318 | 27.363 | -4.141 | 1.00 | 21.70 | B | N |
| ATOM | 3527 | CA | LEU | B | 51 | -12.460 | 26.003 | -3.643 | 1.00 | 22.26 | B | C |
| ATOM | 3528 | CB | LEU | B | 51 | -11.110 | 25.407 | -3.219 | 1.00 | 22.70 | B | C |
| ATOM | 3529 | CG | LEU | B | 51 | -10.067 | 25.113 | -4.267 | 1.00 | 25.11 | B | C |
| ATOM | 3530 | CD1 | LEU | B | 51 | -8.762 | 24.764 | -3.495 | 1.00 | 25.85 | B | C |
| ATOM | 3531 | CD2 | LEU | B | 51 | -10.513 | 23.968 | -5.183 | 1.00 | 27.79 | B | C |
| ATOM | 3532 | C | LEU | B | 51 | -13.312 | 25.997 | -2.406 | 1.00 | 21.80 | B | C |
| ATOM | 3533 | O | LEU | B | 51 | -13.289 | 26.962 | -1.646 | 1.00 | 21.46 | B | O |
| ATOM | 3534 | N | PRO | B | 52 | -14.006 | 24.886 | -2.163 | 1.00 | 21.76 | B | N |
| ATOM | 3535 | CA | PRO | B | 52 | -14.750 | 24.709 | -0.921 | 1.00 | 22.13 | B | C |
| ATOM | 3536 | CB | PRO | B | 52 | -15.340 | 23.290 | -1.071 | 1.00 | 23.06 | B | C |
| ATOM | 3537 | CG | PRO | B | 52 | -15.389 | 23.059 | -2.525 | 1.00 | 22.20 | B | C |
| ATOM | 3538 | CD | PRO | B | 52 | -14.145 | 23.724 | -3.058 | 1.00 | 22.25 | B | C |
| ATOM | 3539 | C | PRO | B | 52 | -13.836 | 24.809 | 0.290 | 1.00 | 22.83 | B | C |
| ATOM | 3540 | O | PRO | B | 52 | -12.682 | 24.367 | 0.252 | 1.00 | 22.05 | B | O |
| ATOM | 3541 | N | VAL | B | 53 | -14.340 | 25.400 | 1.365 | 1.00 | 23.43 | B | N |
| ATOM | 3542 | CA | VAL | B | 53 | -13.579 | 25.504 | 2.581 | 1.00 | 23.96 | B | C |
| ATOM | 3543 | CB | VAL | B | 53 | -14.297 | 26.368 | 3.643 | 1.00 | 24.41 | B | C |
| ATOM | 3544 | CG1 | VAL | B | 53 | -15.583 | 25.692 | 4.134 | 1.00 | 25.13 | B | C |
| ATOM | 3545 | CG2 | VAL | B | 53 | -13.360 | 26.671 | 4.805 | 1.00 | 25.93 | B | C |
| ATOM | 3546 | C | VAL | B | 53 | -13.324 | 24.083 | 3.068 | 1.00 | 24.12 | B | C |
| ATOM | 3547 | O | VAL | B | 53 | -14.153 | 23.193 | 2.859 | 1.00 | 24.69 | B | O |
| ATOM | 3548 | N | GLY | B | 54 | -12.158 | 23.867 | 3.657 | 1.00 | 22.87 | B | N |
| ATOM | 3549 | CA | GLY | B | 54 | -11.765 | 22.548 | 4.117 | 1.00 | 22.48 | B | C |
| ATOM | 3550 | C | GLY | B | 54 | -11.067 | 21.662 | 3.092 | 1.00 | 21.22 | B | C |
| ATOM | 3551 | O | GLY | B | 54 | -10.597 | 20.606 | 3.453 | 1.00 | 21.63 | B | O |
| ATOM | 3552 | N | THR | B | 55 | -10.977 | 22.091 | 1.837 | 1.00 | 20.24 | B | N |
| ATOM | 3553 | CA | THR | B | 55 | -10.295 | 21.324 | 0.809 | 1.00 | 19.46 | B | C |
| ATOM | 3554 | CB | THR | B | 55 | -10.469 | 22.006 | -0.573 | 1.00 | 19.86 | B | C |
| ATOM | 3555 | OG1 | THR | B | 55 | -11.866 | 22.158 | -0.875 | 1.00 | 22.41 | B | O |
| ATOM | 3556 | CG2 | THR | B | 55 | -9.957 | 21.139 | -1.701 | 1.00 | 19.94 | B | C |
| ATOM | 3557 | C | THR | B | 55 | -8.788 | 21.125 | 1.077 | 1.00 | 17.93 | B | C |
| ATOM | 3558 | O | THR | B | 55 | -8.057 | 22.042 | 1.417 | 1.00 | 16.91 | B | O |
| ATOM | 3559 | N | ILE | B | 56 | -8.336 | 19.911 | 0.849 | 1.00 | 17.04 | B | N |
| ATOM | 3560 | CA | ILE | B | 56 | -6.929 | 19.571 | 0.943 | 1.00 | 16.44 | B | C |
| ATOM | 3561 | CB | ILE | B | 56 | -6.800 | 18.076 | 1.232 | 1.00 | 15.71 | B | C |
| ATOM | 3562 | CG1 | ILE | B | 56 | -7.439 | 17.782 | 2.600 | 1.00 | 17.41 | B | C |
| ATOM | 3563 | CD1 | ILE | B | 56 | -7.353 | 16.319 | 3.098 | 1.00 | 17.17 | B | C |
| ATOM | 3564 | CG2 | ILE | B | 56 | -5.347 | 17.684 | 1.247 | 1.00 | 16.77 | B | C |
| ATOM | 3565 | C | ILE | B | 56 | -6.217 | 19.981 | -0.336 | 1.00 | 16.51 | B | C |
| ATOM | 3566 | O | ILE | B | 56 | -6.701 | 19.691 | -1.434 | 1.00 | 17.03 | B | O |
| ATOM | 3567 | N | VAL | B | 57 | -5.088 | 20.678 | -0.203 | 1.00 | 15.94 | B | N |
| ATOM | 3568 | CA | VAL | B | 57 | -4.342 | 21.200 | -1.361 | 1.00 | 16.45 | B | C |

| ATOM | 3569 | CB | VAL | B | 57 | -4.511 | 22.726 | -1.488 | 1.00 | 16.21 | B | C |
|------|------|-----|------|---|----|--------|--------|--------|------|-------|---|---|
| ATOM | 3570 | CG1 | VAL | B | 57 | -6.012 | 23.092 | -1.672 | 1.00 | 16.73 | B | C |
| ATOM | 3571 | CG2 | VAL | B | 57 | -3.991 | 23.435 | -0.236 | 1.00 | 18.16 | B | C |
| ATOM | 3572 | C | VAL | B | 57 | -2.853 | 20.910 | -1.205 | 1.00 | 16.63 | B | C |
| ATOM | 3573 | O | VAL | B | 57 | -2.393 | 20.624 | -0.099 | 1.00 | 16.42 | B | O |
| ATOM | 3574 | N | THR | B | 58 | -2.106 | 20.982 | -2.299 | 1.00 | 17.06 | B | N |
| ATOM | 3575 | CA | THR | B | 58 | -0.658 | 20.801 | -2.247 | 1.00 | 16.63 | B | C |
| ATOM | 3576 | CB | THR | B | 58 | -0.069 | 20.712 | -3.654 | 1.00 | 17.18 | B | C |
| ATOM | 3577 | OG1 | THR | B | 58 | -0.660 | 21.718 | -4.494 | 1.00 | 15.16 | B | O |
| ATOM | 3578 | CG2 | THR | B | 58 | -0.423 | 19.426 | -4.292 | 1.00 | 19.58 | B | C |
| ATOM | 3579 | C | THR | B | 58 | -0.093 | 22.017 | -1.536 | 1.00 | 16.72 | B | C |
| ATOM | 3580 | O | THR | B | 58 | -0.756 | 23.071 | -1.492 | 1.00 | 16.30 | B | O |
| ATOM | 3581 | N | MET | B | 59 | 1.103 | 21.885 | -0.960 | 1.00 | 16.38 | B | N |
| ATOM | 3582 | CA | MET | B | 59 | 1.692 | 22.982 | -0.180 | 1.00 | 16.07 | B | C |
| ATOM | 3583 | CB | MET | B | 59 | 1.960 | 22.552 | 1.254 | 1.00 | 15.92 | B | C |
| ATOM | 3584 | CG | MET | B | 59 | 0.668 | 22.365 | 2.012 | 1.00 | 16.14 | B | C |
| ATOM | 3585 | SD | MET | B | 59 | -0.197 | 23.961 | 2.251 | 1.00 | 17.09 | B | S |
| ATOM | 3586 | CE | MET | B | 59 | -1.612 | 23.424 | 3.218 | 1.00 | 17.23 | B | C |
| ATOM | 3587 | C | MET | B | 59 | 2.911 | 23.607 | -0.816 | 1.00 | 15.83 | B | C |
| ATOM | 3588 | O | MET | B | 59 | 3.884 | 23.983 | -0.134 | 1.00 | 15.99 | B | O |
| ATOM | 3589 | N | GLU | B | 60 | 2.837 | 23.794 | -2.127 | 1.00 | 15.11 | B | N |
| ATOM | 3590 | CA | GLU | B | 60 | 3.838 | 24.609 | -2.795 | 1.00 | 15.22 | B | C |
| ATOM | 3591 | CB | GLU | B | 60 | 4.155 | 24.079 | -4.187 | 1.00 | 15.39 | B | C |
| ATOM | 3592 | CG | GLU | B | 60 | 3.299 | 24.609 | -5.334 | 1.00 | 15.29 | B | C |
| ATOM | 3593 | CD | GLU | B | 60 | 1.845 | 24.166 | -5.270 | 1.00 | 19.22 | B | C |
| ATOM | 3594 | OE1 | GLU | B | 60 | 1.435 | 23.486 | -4.308 | 1.00 | 18.05 | B | O |
| ATOM | 3595 | OE2 | GLU | B | 60 | 1.087 | 24.526 | -6.195 | 1.00 | 18.12 | B | O |
| ATOM | 3596 | C | GLU | B | 60 | 3.344 | 26.081 | -2.771 | 1.00 | 15.50 | B | C |
| ATOM | 3597 | O | GLU | B | 60 | 2.133 | 26.356 | -2.798 | 1.00 | 15.17 | B | O |
| ATOM | 3598 | N | TYR | B | 61 | 4.292 | 27.011 | -2.721 | 1.00 | 16.00 | B | N |
| ATOM | 3599 | CA | TYR | B | 61 | 3.982 | 28.431 | -2.654 | 1.00 | 17.37 | B | C |
| ATOM | 3600 | CB | TYR | B | 61 | 4.938 | 29.138 | -1.699 | 1.00 | 17.67 | B | C |
| ATOM | 3601 | CG | TYR | B | 61 | 4.671 | 30.623 | -1.493 | 1.00 | 18.81 | B | C |
| ATOM | 3602 | CD1 | TYR | B | 61 | 5.474 | 31.572 | -2.103 | 1.00 | 21.63 | B | C |
| ATOM | 3603 | CE1 | TYR | B | 61 | 5.233 | 32.895 | -1.943 | 1.00 | 21.96 | B | C |
| ATOM | 3604 | CZ | TYR | B | 61 | 4.207 | 33.299 | -1.134 | 1.00 | 22.89 | B | C |
| ATOM | 3605 | OH | TYR | B | 61 | 4.007 | 34.644 | -0.972 | 1.00 | 29.91 | B | O |
| ATOM | 3606 | CE2 | TYR | B | 61 | 3.407 | 32.394 | -0.507 | 1.00 | 20.81 | B | C |
| ATOM | 3607 | CD2 | TYR | B | 61 | 3.637 | 31.059 | -0.697 | 1.00 | 19.36 | B | C |
| ATOM | 3608 | C | TYR | B | 61 | 4.067 | 29.037 | -4.048 | 1.00 | 18.22 | B | C |
| ATOM | 3609 | O | TYR | B | 61 | 5.126 | 29.041 | -4.654 | 1.00 | 18.04 | B | O |
| ATOM | 3610 | N | ARG | B | 62 | 2.943 | 29.531 | -4.564 | 1.00 | 19.75 | B | N |
| ATOM | 3611 | CA | ARG | B | 62 | 2.938 | 30.226 | -5.861 | 1.00 | 21.16 | B | C |
| ATOM | 3612 | CB | ARG | B | 62 | 1.909 | 29.625 | -6.814 | 1.00 | 21.92 | B | C |
| ATOM | 3613 | CG | ARG | B | 62 | 2.189 | 28.185 | -7.196 | 1.00 | 25.65 | B | C |
| ATOM | 3614 | CD | ARG | B | 62 | 1.385 | 27.694 | -8.421 | 1.00 | 29.39 | B | C |
| ATOM | 3615 | NE | ARG | B | 62 | 1.516 | 26.235 | -8.639 | 1.00 | 32.04 | B | N |
| ATOM | 3616 | CZ | ARG | B | 62 | 0.982 | 25.593 | -9.675 | 1.00 | 33.94 | B | C |
| ATOM | 3617 | NH1 | ARG | B | 62 | 1.129 | 24.287 | -9.798 | 1.00 | 37.42 | B | N |
| ATOM | 3618 | NH2 | ARG | B | 62 | 0.292 | 26.255 | -10.586 | 1.00 | 34.66 | B | N |
| ATOM | 3619 | C | ARG | B | 62 | 2.619 | 31.687 | -5.652 | 1.00 | 21.86 | B | C |
| ATOM | 3620 | O | ARG | B | 62 | 1.491 | 32.033 | -5.296 | 1.00 | 21.34 | B | O |
| ATOM | 3621 | N | ILE | B | 63 | 3.609 | 32.547 | -5.894 | 1.00 | 23.01 | B | N |
| ATOM | 3622 | CA | ILE | B | 63 | 3.503 | 33.950 | -5.502 | 1.00 | 24.71 | B | C |
| ATOM | 3623 | CB | ILE | B | 63 | 4.824 | 34.707 | -5.709 | 1.00 | 25.05 | B | C |
| ATOM | 3624 | CG1 | ILE | B | 63 | 4.790 | 36.033 | -4.919 | 1.00 | 28.83 | B | C |
| ATOM | 3625 | CD1 | ILE | B | 63 | 6.158 | 36.609 | -4.546 | 1.00 | 31.61 | B | C |
| ATOM | 3626 | CG2 | ILE | B | 63 | 5.047 | 34.966 | -7.207 | 1.00 | 26.58 | B | C |
| ATOM | 3627 | C | ILE | B | 63 | 2.367 | 34.699 | -6.216 | 1.00 | 24.34 | B | C |
| ATOM | 3628 | O | ILE | B | 63 | 1.860 | 35.675 | -5.672 | 1.00 | 25.45 | B | O |
| ATOM | 3629 | N | ASP | B | 64 | 1.961 | 34.240 | -7.398 | 1.00 | 24.15 | B | N |
| ATOM | 3630 | CA | ASP | B | 64 | 0.901 | 34.919 | -8.153 | 1.00 | 24.63 | B | C |
| ATOM | 3631 | CB | BASP | B | 64 | 1.214 | 34.889 | -9.664 | 0.40 | 24.94 | B | C |

| | | | | | | | | | | | | |
|---|---|---|---|---|---|---|---|---|---|---|---|---|
| ATOM | 3632 | CB | AASP | B | 64 | 1.201 | 34.873 | -9.650 | 0.60 | 24.96 | B | C |
| ATOM | 3633 | CG | BASP | B | 64 | 0.987 | 33.514 | -10.307 | 0.40 | 25.65 | B | C |
| ATOM | 3634 | CG | AASP | B | 64 | 2.403 | 35.712 | -10.026 | 0.60 | 25.91 | B | C |
| ATOM | 3635 | OD1 | BASP | B | 64 | 0.745 | 33.465 | -11.536 | 0.40 | 27.17 | B | O |
| ATOM | 3636 | OD1 | AASP | B | 64 | 3.136 | 35.290 | -10.950 | 0.60 | 27.52 | B | O |
| ATOM | 3637 | OD2 | BASP | B | 64 | 1.054 | 32.426 | -9.686 | 0.40 | 28.49 | B | O |
| ATOM | 3638 | OD2 | AASP | B | 64 | 2.704 | 36.782 | -9.439 | 0.60 | 26.67 | B | O |
| ATOM | 3639 | C | ASP | B | 64 | -0.514 | 34.361 | -7.896 | 1.00 | 24.47 | B | C |
| ATOM | 3640 | O | ASP | B | 64 | -1.515 | 34.928 | -8.392 | 1.00 | 24.83 | B | O |
| ATOM | 3641 | N | ARG | B | 65 | -0.601 | 33.269 | -7.136 | 1.00 | 21.92 | B | N |
| ATOM | 3642 | CA | ARG | B | 65 | -1.876 | 32.615 | -6.893 | 1.00 | 21.12 | B | C |
| ATOM | 3643 | CB | ARG | B | 65 | -1.677 | 31.101 | -6.737 | 1.00 | 19.95 | B | C |
| ATOM | 3644 | CG | ARG | B | 65 | -2.946 | 30.305 | -6.463 | 1.00 | 18.99 | B | C |
| ATOM | 3645 | CD | ARG | B | 65 | -2.730 | 28.808 | -6.572 | 1.00 | 19.09 | B | C |
| ATOM | 3646 | NE | ARG | B | 65 | -1.784 | 28.369 | -5.554 | 1.00 | 18.66 | B | N |
| ATOM | 3647 | CZ | ARG | B | 65 | -1.130 | 27.214 | -5.534 | 1.00 | 19.41 | B | C |
| ATOM | 3648 | NH1 | ARG | B | 65 | -1.279 | 26.300 | -6.470 | 1.00 | 19.74 | B | N |
| ATOM | 3649 | NH2 | ARG | B | 65 | -0.311 | 26.963 | -4.522 | 1.00 | 21.82 | B | N |
| ATOM | 3650 | C | ARG | B | 65 | -2.556 | 33.168 | -5.662 | 1.00 | 20.36 | B | C |
| ATOM | 3651 | O | ARG | B | 65 | -1.896 | 33.515 | -4.682 | 1.00 | 21.06 | B | O |
| ATOM | 3652 | N | VAL | B | 66 | -3.875 | 33.284 | -5.727 | 1.00 | 20.04 | B | N |
| ATOM | 3653 | CA | VAL | B | 66 | -4.692 | 33.540 | -4.560 | 1.00 | 19.79 | B | C |
| ATOM | 3654 | CB | VAL | B | 66 | -5.266 | 34.962 | -4.510 | 1.00 | 19.88 | B | C |
| ATOM | 3655 | CG1 | VAL | B | 66 | -6.036 | 35.160 | -3.204 | 1.00 | 21.06 | B | C |
| ATOM | 3656 | CG2 | VAL | B | 66 | -4.194 | 36.004 | -4.607 | 1.00 | 20.96 | B | C |
| ATOM | 3657 | C | VAL | B | 66 | -5.846 | 32.525 | -4.551 | 1.00 | 20.19 | B | C |
| ATOM | 3658 | O | VAL | B | 66 | -6.733 | 32.512 | -5.444 | 1.00 | 20.12 | B | O |
| ATOM | 3659 | N | ARG | B | 67 | -5.832 | 31.654 | -3.557 | 1.00 | 20.01 | B | N |
| ATOM | 3660 | CA | ARG | B | 67 | -6.916 | 30.713 | -3.394 | 1.00 | 20.45 | B | C |
| ATOM | 3661 | CB | ARG | B | 67 | -6.416 | 29.437 | -2.740 | 1.00 | 20.71 | B | C |
| ATOM | 3662 | CG | ARG | B | 67 | -5.572 | 28.538 | -3.626 | 1.00 | 21.28 | B | C |
| ATOM | 3663 | CD | ARG | B | 67 | -5.471 | 27.144 | -3.016 | 1.00 | 23.63 | B | C |
| ATOM | 3664 | NE | ARG | B | 67 | -4.539 | 26.204 | -3.632 | 1.00 | 22.50 | B | N |
| ATOM | 3665 | CZ | ARG | B | 67 | -3.326 | 25.921 | -3.160 | 1.00 | 24.19 | B | C |
| ATOM | 3666 | NH1 | ARG | B | 67 | -2.589 | 24.992 | -3.759 | 1.00 | 22.65 | B | N |
| ATOM | 3667 | NH2 | ARG | B | 67 | -2.837 | 26.571 | -2.106 | 1.00 | 22.06 | B | N |
| ATOM | 3668 | C | ARG | B | 67 | -8.001 | 31.368 | -2.543 | 1.00 | 20.49 | B | C |
| ATOM | 3669 | O | ARG | B | 67 | -7.704 | 31.990 | -1.529 | 1.00 | 20.17 | B | O |
| ATOM | 3670 | N | LEU | B | 68 | -9.255 | 31.253 | -2.970 | 1.00 | 20.39 | B | N |
| ATOM | 3671 | CA | LEU | B | 68 | -10.373 | 31.705 | -2.148 | 1.00 | 21.05 | B | C |
| ATOM | 3672 | CB | LEU | B | 68 | -11.323 | 32.604 | -2.957 | 1.00 | 21.11 | B | C |
| ATOM | 3673 | CG | LEU | B | 68 | -10.701 | 33.830 | -3.627 | 1.00 | 21.59 | B | C |
| ATOM | 3674 | CD1 | LEU | B | 68 | -11.745 | 34.523 | -4.508 | 1.00 | 22.82 | B | C |
| ATOM | 3675 | CD2 | LEU | B | 68 | -10.187 | 34.785 | -2.595 | 1.00 | 22.44 | B | C |
| ATOM | 3676 | C | LEU | B | 68 | -11.132 | 30.493 | -1.648 | 1.00 | 21.12 | B | C |
| ATOM | 3677 | O | LEU | B | 68 | -11.663 | 29.709 | -2.432 | 1.00 | 21.87 | B | O |
| ATOM | 3678 | N | PHE | B | 69 | -11.209 | 30.345 | -0.345 | 1.00 | 21.61 | B | N |
| ATOM | 3679 | CA | PHE | B | 69 | -11.937 | 29.226 | 0.233 | 1.00 | 21.97 | B | C |
| ATOM | 3680 | CB | PHE | B | 69 | -11.151 | 28.647 | 1.422 | 1.00 | 22.17 | B | C |
| ATOM | 3681 | CG | PHE | B | 69 | -9.896 | 27.905 | 1.028 | 1.00 | 19.89 | B | C |
| ATOM | 3682 | CD1 | PHE | B | 69 | -9.938 | 26.557 | 0.737 | 1.00 | 21.41 | B | C |
| ATOM | 3683 | CE1 | PHE | B | 69 | -8.788 | 25.862 | 0.370 | 1.00 | 20.29 | B | C |
| ATOM | 3684 | CZ | PHE | B | 69 | -7.598 | 26.525 | 0.290 | 1.00 | 21.78 | B | C |
| ATOM | 3685 | CE2 | PHE | B | 69 | -7.542 | 27.879 | 0.575 | 1.00 | 21.52 | B | C |
| ATOM | 3686 | CD2 | PHE | B | 69 | -8.691 | 28.557 | 0.950 | 1.00 | 19.33 | B | C |
| ATOM | 3687 | C | PHE | B | 69 | -13.321 | 29.725 | 0.664 | 1.00 | 23.11 | B | C |
| ATOM | 3688 | O | PHE | B | 69 | -13.414 | 30.651 | 1.506 | 1.00 | 22.87 | B | O |
| ATOM | 3689 | N | VAL | B | 70 | -14.371 | 29.105 | 0.111 | 1.00 | 24.00 | B | N |
| ATOM | 3690 | CA | VAL | B | 70 | -15.755 | 29.578 | 0.289 | 1.00 | 25.82 | B | C |
| ATOM | 3691 | CB | VAL | B | 70 | -16.442 | 29.958 | -1.054 | 1.00 | 25.72 | B | C |
| ATOM | 3692 | CG1 | VAL | B | 70 | -15.686 | 31.063 | -1.756 | 1.00 | 26.70 | B | C |
| ATOM | 3693 | CG2 | VAL | B | 70 | -16.599 | 28.756 | -1.958 | 1.00 | 26.63 | B | C |
| ATOM | 3694 | C | VAL | B | 70 | -16.691 | 28.602 | 0.996 | 1.00 | 26.63 | B | C |

| ATOM | 3695 | O   | VAL | B | 70 | -16.532 | 27.380 | 0.899  | 1.00 | 27.14 | B | O |
|------|------|-----|-----|---|----|---------|--------|--------|------|-------|---|---|
| ATOM | 3696 | N   | ASP | B | 71 | -17.664 | 29.155 | 1.714  | 1.00 | 27.52 | B | N |
| ATOM | 3697 | CA  | ASP | B | 71 | -18.686 | 28.352 | 2.399  | 1.00 | 29.00 | B | C |
| ATOM | 3698 | CB  | ASP | B | 71 | -19.247 | 29.132 | 3.591  | 1.00 | 28.91 | B | C |
| ATOM | 3699 | CG  | ASP | B | 71 | -20.019 | 30.392 | 3.171  | 1.00 | 28.74 | B | C |
| ATOM | 3700 | OD1 | ASP | B | 71 | -20.161 | 31.290 | 4.018  | 1.00 | 29.94 | B | O |
| ATOM | 3701 | OD2 | ASP | B | 71 | -20.509 | 30.572 | 2.032  | 1.00 | 27.69 | B | O |
| ATOM | 3702 | C   | ASP | B | 71 | -19.796 | 27.931 | 1.413  | 1.00 | 30.37 | B | C |
| ATOM | 3703 | O   | ASP | B | 71 | -19.646 | 28.098 | 0.217  | 1.00 | 29.90 | B | O |
| ATOM | 3704 | N   | LYS | B | 72 | -20.898 | 27.376 | 1.906  | 1.00 | 33.42 | B | N |
| ATOM | 3705 | CA  | LYS | B | 72 | -21.949 | 26.822 | 1.022  | 1.00 | 34.98 | B | C |
| ATOM | 3706 | CB  | LYS | B | 72 | -22.951 | 26.004 | 1.834  | 1.00 | 35.44 | B | C |
| ATOM | 3707 | CG  | LYS | B | 72 | -22.190 | 24.925 | 2.916  | 0.00 | 40.00 | B | C |
| ATOM | 3708 | CD  | LYS | B | 72 | -22.489 | 23.435 | 2.595  | 0.00 | 40.00 | B | C |
| ATOM | 3709 | CE  | LYS | B | 72 | -21.240 | 22.528 | 2.667  | 0.00 | 40.00 | B | C |
| ATOM | 3710 | NZ  | LYS | B | 72 | -21.120 | 21.662 | 1.456  | 0.00 | 40.00 | B | N |
| ATOM | 3711 | C   | LYS | B | 72 | -22.709 | 27.904 | 0.261  | 1.00 | 36.38 | B | C |
| ATOM | 3712 | O   | LYS | B | 72 | -23.332 | 27.627 | -0.766 | 1.00 | 37.77 | B | O |
| ATOM | 3713 | N   | LEU | B | 73 | -22.640 | 29.138 | 0.753  | 1.00 | 37.07 | B | N |
| ATOM | 3714 | CA  | LEU | B | 73 | -23.306 | 30.286 | 0.114  | 1.00 | 37.22 | B | C |
| ATOM | 3715 | CB  | LEU | B | 73 | -23.759 | 31.265 | 1.201  | 1.00 | 37.50 | B | C |
| ATOM | 3716 | CG  | LEU | B | 73 | -24.711 | 30.678 | 2.254  | 1.00 | 40.20 | B | C |
| ATOM | 3717 | CD1 | LEU | B | 73 | -25.387 | 31.796 | 3.053  | 1.00 | 41.53 | B | C |
| ATOM | 3718 | CD2 | LEU | B | 73 | -25.782 | 29.775 | 1.612  | 1.00 | 41.89 | B | C |
| ATOM | 3719 | C   | LEU | B | 73 | -22.406 | 31.008 | -0.890 | 1.00 | 36.59 | B | C |
| ATOM | 3720 | O   | LEU | B | 73 | -22.781 | 32.029 | -1.482 | 1.00 | 36.18 | B | O |
| ATOM | 3721 | N   | ASP | B | 74 | -21.203 | 30.468 | -1.076 | 1.00 | 36.33 | B | N |
| ATOM | 3722 | CA  | ASP | B | 74 | -20.192 | 31.084 | -1.923 | 1.00 | 35.65 | B | C |
| ATOM | 3723 | CB  | ASP | B | 74 | -20.709 | 31.252 | -3.342 | 1.00 | 36.81 | B | C |
| ATOM | 3724 | CG  | ASP | B | 74 | -20.063 | 30.283 | -4.286 | 1.00 | 40.36 | B | C |
| ATOM | 3725 | OD1 | ASP | B | 74 | -19.429 | 30.756 | -5.259 | 1.00 | 45.75 | B | O |
| ATOM | 3726 | OD2 | ASP | B | 74 | -20.108 | 29.032 | -4.101 | 1.00 | 45.61 | B | O |
| ATOM | 3727 | C   | ASP | B | 74 | -19.646 | 32.399 | -1.381 | 1.00 | 33.94 | B | C |
| ATOM | 3728 | O   | ASP | B | 74 | -19.147 | 33.249 | -2.136 | 1.00 | 34.59 | B | O |
| ATOM | 3729 | N   | ASN | B | 75 | -19.696 | 32.546 | -0.066 | 1.00 | 31.85 | B | N |
| ATOM | 3730 | CA  | ASN | B | 75 | -19.000 | 33.634 | 0.597  | 1.00 | 30.66 | B | C |
| ATOM | 3731 | CB  | ASN | B | 75 | -19.877 | 34.185 | 1.714  | 1.00 | 30.45 | B | C |
| ATOM | 3732 | CG  | ASN | B | 75 | -21.170 | 34.793 | 1.176  | 1.00 | 30.77 | B | C |
| ATOM | 3733 | OD1 | ASN | B | 75 | -21.169 | 35.401 | 0.102  | 1.00 | 30.57 | B | O |
| ATOM | 3734 | ND2 | ASN | B | 75 | -22.275 | 34.615 | 1.907  | 1.00 | 30.32 | B | N |
| ATOM | 3735 | C   | ASN | B | 75 | -17.628 | 33.213 | 1.127  | 1.00 | 29.26 | B | C |
| ATOM | 3736 | O   | ASN | B | 75 | -17.433 | 32.073 | 1.553  | 1.00 | 29.07 | B | O |
| ATOM | 3737 | N   | ILE | B | 76 | -16.685 | 34.143 | 1.086  | 1.00 | 27.83 | B | N |
| ATOM | 3738 | CA  | ILE | B | 76 | -15.337 | 33.893 | 1.540  | 1.00 | 26.88 | B | C |
| ATOM | 3739 | CB  | ILE | B | 76 | -14.511 | 35.182 | 1.455  | 1.00 | 26.51 | B | C |
| ATOM | 3740 | CG1 | ILE | B | 76 | -14.474 | 35.737 | 0.020  | 1.00 | 26.61 | B | C |
| ATOM | 3741 | CD1 | ILE | B | 76 | -14.130 | 34.728 | -1.039 | 1.00 | 26.87 | B | C |
| ATOM | 3742 | CG2 | ILE | B | 76 | -13.137 | 34.927 | 1.993  | 1.00 | 25.62 | B | C |
| ATOM | 3743 | C   | ILE | B | 76 | -15.401 | 33.382 | 2.993  | 1.00 | 26.59 | B | C |
| ATOM | 3744 | O   | ILE | B | 76 | -15.994 | 34.017 | 3.838  | 1.00 | 26.12 | B | O |
| ATOM | 3745 | N   | ALA | B | 77 | -14.795 | 32.237 | 3.263  | 1.00 | 26.58 | B | N |
| ATOM | 3746 | CA  | ALA | B | 77 | -14.899 | 31.582 | 4.576  | 1.00 | 26.79 | B | C |
| ATOM | 3747 | CB  | ALA | B | 77 | -15.129 | 30.088 | 4.386  | 1.00 | 26.56 | B | C |
| ATOM | 3748 | C   | ALA | B | 77 | -13.670 | 31.815 | 5.467  | 1.00 | 26.89 | B | C |
| ATOM | 3749 | O   | ALA | B | 77 | -13.764 | 31.751 | 6.677  | 1.00 | 27.73 | B | O |
| ATOM | 3750 | N   | GLU | B | 78 | -12.518 | 32.072 | 4.871  | 1.00 | 26.32 | B | N |
| ATOM | 3751 | CA  | GLU | B | 78 | -11.355 | 32.446 | 5.647  | 1.00 | 26.33 | B | C |
| ATOM | 3752 | CB  | GLU | B | 78 | -10.508 | 31.216 | 6.041  | 1.00 | 26.80 | B | C |
| ATOM | 3753 | CG  | GLU | B | 78 | -10.106 | 30.290 | 4.921  | 1.00 | 27.82 | B | C |
| ATOM | 3754 | CD  | GLU | B | 78 | -9.956  | 28.827 | 5.352  | 1.00 | 28.92 | B | C |
| ATOM | 3755 | OE1 | GLU | B | 78 | -9.785  | 28.493 | 6.559  | 1.00 | 36.40 | B | O |
| ATOM | 3756 | OE2 | GLU | B | 78 | -10.001 | 27.977 | 4.472  | 1.00 | 28.50 | B | O |
| ATOM | 3757 | C   | GLU | B | 78 | -10.530 | 33.478 | 4.895  | 1.00 | 25.94 | B | C |

```
ATOM   3758  O    GLU  B  78   -10.807  33.805   3.712  1.00  25.71      B   O
ATOM   3759  N    VAL  B  79    -9.527  33.996   5.594  1.00  24.85      B   N
ATOM   3760  CA   VAL  B  79    -8.712  35.088   5.102  1.00  24.88      B   C
ATOM   3761  CB   VAL  B  79    -7.692  35.578   6.167  1.00  25.77      B   C
ATOM   3762  CG1  VAL  B  79    -6.814  36.672   5.583  1.00  25.38      B   C
ATOM   3763  CG2  VAL  B  79    -8.396  36.073   7.456  1.00  26.10      B   C
ATOM   3764  C    VAL  B  79    -7.957  34.652   3.835  1.00  24.07      B   C
ATOM   3765  O    VAL  B  79    -7.137  33.742   3.883  1.00  23.32      B   O
ATOM   3766  N    PRO  B  80    -8.275  35.271   2.699  1.00  23.27      B   N
ATOM   3767  CA   PRO  B  80    -7.533  35.029   1.466  1.00  22.93      B   C
ATOM   3768  CB   PRO  B  80    -8.296  35.856   0.426  1.00  23.15      B   C
ATOM   3769  CG   PRO  B  80    -9.645  36.042   1.005  1.00  23.83      B   C
ATOM   3770  CD   PRO  B  80    -9.404  36.194   2.480  1.00  23.67      B   C
ATOM   3771  C    PRO  B  80    -6.101  35.512   1.601  1.00  22.05      B   C
ATOM   3772  O    PRO  B  80    -5.891  36.588   2.136  1.00  20.76      B   O
ATOM   3773  N    ARG  B  81    -5.147  34.691   1.190  1.00  21.54      B   N
ATOM   3774  CA   ARG  B  81    -3.765  35.135   1.074  1.00  22.93      B   C
ATOM   3775  CB  BARG  B  81    -2.937  34.683   2.298  0.40  22.99      B   C
ATOM   3776  CB  AARG  B  81    -2.865  34.764   2.275  0.60  23.27      B   C
ATOM   3777  CG  BARG  B  81    -3.684  34.672   3.645  0.40  24.27      B   C
ATOM   3778  CG  AARG  B  81    -3.278  33.619   3.175  0.60  25.79      B   C
ATOM   3779  CD  BARG  B  81    -2.996  33.818   4.744  0.40  26.79      B   C
ATOM   3780  CD  AARG  B  81    -2.615  33.723   4.575  0.60  27.67      B   C
ATOM   3781  NE  BARG  B  81    -3.975  33.234   5.664  0.40  28.96      B   N
ATOM   3782  NE  AARG  B  81    -3.283  32.907   5.581  0.60  29.38      B   N
ATOM   3783  CZ  BARG  B  81    -4.567  32.053   5.508  0.40  29.28      B   C
ATOM   3784  CZ  AARG  B  81    -3.934  33.381   6.642  0.60  30.17      B   C
ATOM   3785  NH1BARG  B  81    -4.284  31.274   4.471  0.40  31.06      B   N
ATOM   3786  NH1AARG  B  81    -4.020  34.680   6.867  0.60  31.08      B   N
ATOM   3787  NH2BARG  B  81    -5.448  31.645   6.408  0.40  30.62      B   N
ATOM   3788  NH2AARG  B  81    -4.510  32.545   7.486  0.60  31.16      B   N
ATOM   3789  C    ARG  B  81    -3.115  34.616  -0.178  1.00  21.97      B   C
ATOM   3790  O    ARG  B  81    -3.560  33.632  -0.761  1.00  21.68      B   O
ATOM   3791  N    VAL  B  82    -2.029  35.286  -0.559  1.00  20.91      B   N
ATOM   3792  CA   VAL  B  82    -1.224  34.884  -1.679  1.00  21.03      B   C
ATOM   3793  CB   VAL  B  82    -0.126  35.943  -1.952  1.00  21.89      B   C
ATOM   3794  CG1  VAL  B  82     0.869  35.435  -2.906  1.00  22.93      B   C
ATOM   3795  CG2  VAL  B  82    -0.734  37.243  -2.483  1.00  21.44      B   C
ATOM   3796  C    VAL  B  82    -0.583  33.532  -1.371  1.00  20.69      B   C
ATOM   3797  O    VAL  B  82    -0.235  33.255  -0.221  1.00  19.36      B   O
ATOM   3798  N    GLY  B  83    -0.469  32.694  -2.389  1.00  19.91      B   N
ATOM   3799  CA   GLY  B  83     0.382  31.525  -2.331  1.00  20.16      B   C
ATOM   3800  C    GLY  B  83    -0.236  30.295  -2.955  1.00  20.11      B   C
ATOM   3801  O    GLY  B  83    -1.416  30.328  -3.319  1.00  20.36      B   O
ATOM   3802  OXT  GLY  B  83     0.468  29.294  -3.104  1.00  18.79      B   O
```

**Claims**

1. A JP170 type subtilase variant comprising at least one modification in an amino acid residue in a position located at a distance of 10Å or less to the ion-binding site, preferably positions located at a distance of 6 Å or less.

**2.** The variant of claim 1, wherein modifications are made in at least one of the positions:

a) ion-binding site 1: 183, 184, 185, 186, 187, 188, 189, 191, 193, 195, 196, 197, 198, 199, 200, 201, 202, 203, 224 and 225,

b) ion-binding site 2: 378, 379, 380, 381, 382, 383, 384, 385, 386, 387, 388, 389, 390, 391, 392 and 393,

c) ion-binding site 3: 348, 350, 352, 363, 364, 365, 366, 367, 369, 370, 380, 381, 382, 383, 391, 392, 393, 394, 395, 396, 397, 398, 399, 400, 414, 415, 416, 417, 418, 419, 420, preferably the modifications: S193Q,Y; H200D, N; H200D,N+D196N; N390D; N391D; G394N,Q,F,Y,S and W392S,N,Q.

**3.** A JP170 type subtilase variant comprising the introduction of a ion-binding site corresponding to the Strong ion-binding site of the BPN' like family subtilases, wherein said variant has a partial or full deletion of the region N79-N82 of SEQ ID NO:1 and subsequent insertion of one or more amino acid residues, preferably insertion of the sequence LNNSIQV (SEQ ID NO:5) followed by the substitution A45D,N and optionally the substitutions E44P,T and/or R47Q.

**4.** A JP170 type subtilase variant in which one or more ion-binding sites have been removed, wherein said variant comprises partial or full deletion of the region N186-N199 of SEQ ID NO:1 and subsequent insertion of one or more amino acid residues, preferably insertion of the sequence SSN (SEQ ID NO:6), and preferably further comprising one or both of the substitutions I7Q and V3Y.

**5.** A BPN' type subtilase variant in which the ion-binding sites have been removed, wherein said variant comprises:

a) partial or full deletion of the region A194-L196 (Savinase in BPN' numbering) or a corresponding region in another BPN' like subtilase and insertion of three or more amino acid residues, preferably insertion of P209-P217 from JP170 or a corresponding region in another JP170 like subtilase and

partial or full deletion of the region L75-L82 (Savinase in BPN' numbering) or a corresponding region in said other BPN' like subtilase and insertion of one or more amino acid residues, preferably insertion of H83-Y92 from TY145 or a corresponding region in another TY145 like subtilase or

b) partial or full deletion of the region A194-L196 (Savinase in BPN' numbering) or a corresponding region in another BPN' like subtilase and insertion of three or more amino acid residues, preferably insertion of P209-P217 from JP170 or a corresponding region in another JP170 like subtilase and

partial or full deletion of the L75-L82 (Savinase in BPN' numbering) or a corresponding region in said other BPN' like subtilase and insertion of one or more amino acid residues, preferably insertion of N79-K83 from JP170 or a corresponding region in another JP170 like subtilase.

**6.** A JP170 type subtilase variant comprising at least one modification in a position selected from the group comprising positions:

13, 14, 15, 16, 17, 18, 37, 38, 39, 40, 41, 42, 43, 47, 48, 49, 50, 58, 59, 60, 67, 96, 97, 98, 99, 107, 108, 109, 110, 111, 131, 132, 133, 134, 152, 153, 163, 164, 165, 166, 188, 189, 190, 191, 193, 195, 210, 234, 235, 236, 237, 238, 239, 240, 241, 242, 243, 244, 245, 326, 327, 328, 329, 330, 331, 332, 337, 338, 339, 340, 342, 355, 356, 357, 359, 360, 372, 373, 374, 375, 376, 377, 378, 384, 385, 387, 388, 389, 390, 391, 392, 404, 405, 406, 407, 408, 409, 410, 411 and 419.

**7.** The subtilase variant according to claim 6 comprising one or more of the modifications: W240H,Y; G355A,S; S356T, N; T357N,Q,D,E,P; A359S,T,N,Q or S360T,N.

**8.** A variant subtilase comprising an alteration in one or more positions which are within a distance of 10Å from a Cl2 inhibitor which is bound to the active site of JP170, wherein the positions, as specified in SEQ ID NO:1 are:

29, 30, 31, 32, 64, 67, 68, 69, 70, 71, 72, 93, 96, 97, 98, 107, 108, 109, 110, 113, 114, 127, 128, 129, 130, 131, 132, 133, 134, 136, 138, 139, 140, 141, 144, 157, 174, 180, 181, 182, 183, 191, 193, 202, 203, 205, 206, 207, 211, 223, 224, 225, 226, 234, 235, 236, 237, 238, 239, 240, 241, 249, 250, 251, 252, 253, 254, 257, 258, preferably comprising the substitution W129L.

**9.** A JP170 like subtilase variant comprising one or more disulfide bridges introduced by one or more of the following modifications: G21C+A86C, V26C+A265C, G57C+G105C, G74C+A229C, Q111C+N143C, G160C+S170C, A286C+V349C, A27C+A122C, A45C+G78C, V72C+P258C, G78C+A229C, D98C+G104C, Q111C+Y147C, G135C+G167C, R142C+P354C, V144C+A178C, G182C+P217C, A183C+G223C, A195C+Y225C, F271C+P279C, A287C+A430C, A293C+T310C, E322C+S428C, S324C+A332C, S327C+P424C, D352C+N397C, G355C+T362C, G291C+S314C, Preferably one or more of the substitutions: G21C+A86C, V26C+A265C, G57C+G105C, G74C+A229C, Q111C+Y143C, G160C+S170C, A286C+V349C, A4C+P222C and A27C+A117C wherein the positions correspond to the positions in SEQ ID NO:1.

10. A JP170 type subtilase variant comprising an alteration in one or more of the positions N79, N316, L381, K9, and K313, preferably comprising one or more of the substitutions N79D, N316D, L381 D, K9R, and K313R of SEQ ID NO:1.

11. A JP170 type subtilase variant comprising an alteration in one or more of the positions 22, 44, 110, 139, 140, 166, 198, 201, 203, 231, 282, 356, 357 and 378, preferably comprising one or more of the substitutions: Q22P, E44P, L110P, T139P, D140P, S166P I198P, V201 P, Q203P, S231 P, S282P, S356P, T357P and K378P.

12. A JP170 like subtilase variant comprising a deletion of the region 311-433, preferably deletion of positions 317-433 or 315-433, further comprising one or more of the substitutions L283N,Q; A290S,N and W306H,Y,K.

13. A detergent composition comprising a JP170 type subtilase variant or a BPN' type subtilase variant of any of claims 1-12.

14. Use of a JP170 type subtilase variant or a BPN' type subtilase variant of claims 1-12 in cleaning or washing applications.

```
     1                                                            60
a)  NDVARGIVKA DVAQNNYGLY GQGQVVAVAD TGLDTGRNDS SMHEAFRGKI TALYALGRTN
b)  NDVARGIVKA DVAQNNYGLY GQGQLVAVAD TGLDTGRNDS SMHEAFRGKI TALYALGRTN
c)  NDVARGIVKA DVAQNNFGLY GQGQIVAVAD TGLDTGRNDS SMHEAFRGKI TALYALGRTN


    61                                                           120
a)  NANDPNGHGT HVAGSVLGNA LNKGMAPQAN LVFQSIMDSS GGLGGLPSNL NTLFSQAWNA
b)  NASDPNGHGT HVAGSVLGNA LNKGMAPQAN LVFQSIMDSS GGLGGLPSNL NTLFSQAWNA
c)  NANDPNGHGT HVAGSVLGNA TNKGMAPQAN LVFQSIMDSG GGLGGLPANL QTLFSQAYSA


   121                                                           180
a)  GARIHTNSWG APVNGAYTAN SRQVDEYVRN NDMTVLFAAG NEGPNSGTIS APGTAKNAIT
b)  GARIHTNSWG APVNGAYTAN SRQVDEYVRN NDMTVLFAAG NEGPNSGTIS APGTAKNAIT
c)  GARIHTNSWG APVNGAYTTD SRNVDDYVRK NDMTILFAAG NEGPGSGTIS APGTAKNAIT


   181                                                           240
a)  VGATENYRPS FGSLADNPNH IAQFSSRGAT RDGRIKPDVT APGTFILSAR SSLAPDSSFW
b)  VGATENYRPS FGSIADNPNH IAQFSSRGAT RDGRIKPDVT APGTFILSAR SSLAPDSSFW
c)  VGATENLRPS FGSYADNINH VAQFSSRGPT RDGRIKPDVM APGTYILSAR SSLAPDSSFW


   241                                                           300
a)  ANYNSKYAYM GGTSMATPIV AGNVAQLREH FIKNRGITPK PSLIKAALIA GATDVGLGYP
b)  ANYNSKYAYM GGTSMATPIV AGNVAQLREH FIKNRGITPK PSLIKAALIA GATDVGLGYP
c)  ANHDSKYAYM GGTSMATPIV AGNVAQLREH FVKNRGVTPK PSLLKAALIA GAADVGLGFP


   301                                                           360
a)  SGDQGWGRVT LDKSLNVAYV NEATALATGQ KATYSFQAQA GKPLKISLVW TDAPGSTTAS
b)  SGDQGWGRVT LDKSLNVAYV NEATALATGQ KATYSFQAQA GKPLKISLVW TDAPGSTTAS
c)  NGNQGWGRVT LDKSLNVAFV NETSPLSTSQ KATYSFTAQA GKPLKISLVW SDAPGSTTAS


   361                                                           420
a)  YTLVNDLDLV ITAPNGQKYV GNDFSYPYDN NWDGRNNVEN VFINAPQSGT YTIEVQAYNV
b)  YTLVNDLDLV ITAPNGQKYV GNDFSYPYDN NWDGRNNVEN VFINAPQSGT YIIEVQAYNV
c)  LTLVNDLDLV ITAPNGTKYV GNDFTAPYDN NWDGRNNVEN VFINAPQSGT YTVEVQAYNV


   421          433
a)  PSGPQRFSLA IVH
b)  PSGPQRFSLA IVH
c)  PVSPQTFSLA IVH
```

## Fig. 1

**Fig. 2**

| | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| 1 | **100** | **94** | 53 | 53 | 51 | 53 | 53 | 52 | 52 | 53 | 55 | 52 | 52 | 51 | 51 | 51 | 50 |
| 2 | | **100** | 52 | 53 | 53 | 51 | 50 | 51 | 55 | 51 | 54 | 54 | 54 | 53 | 54 | 54 | 54 |
| 3 | | | 100 | 93 | 76 | 52 | 52 | 51 | 50 | 52 | 46 | 58 | 58 | 59 | 57 | 60 | 60 |
| 4 | | | | 100 | 75 | 60 | 60 | 60 | 56 | 51 | 55 | 58 | 57 | 59 | 59 | 62 | 61 |
| 5 | | | | | 100 | 60 | 60 | 58 | 58 | 60 | 62 | 58 | 58 | 66 | 66 | 74 | 59 |
| 6 | | | | | | 100 | 99 | 99 | 97 | 91 | 76 | 69 | 74 | 74 | 74 | 74 | 74 |
| 7 | | | | | | | 100 | 99 | 97 | 90 | 77 | 63 | 69 | 74 | 67 | 73 | 73 |
| 8 | | | | | | | | 100 | 98 | 91 | 77 | 69 | 74 | 66 | 74 | 74 | 74 |
| 9 | | | | | | | | | 100 | 88 | 79 | 66 | 71 | 74 | 67 | 74 | 74 |
| 10 | | | | | | | | | | 100 | 77 | 64 | 69 | 74 | 67 | 74 | 74 |
| 11 | | | | | | | | | | | 100 | 99 | 99 | 76 | 72 | 76 | 76 |
| 12 | | | | | | | | | | | | 100 | 76 | 76 | 76 | 76 | 76 |
| 13 | | | | | | | | | | | | | 100 | 76 | 76 | 76 | 76 |
| 14 | | | | | | | | | | | | | | 100 | 99 | 99 | 99 |
| 15 | | | | | | | | | | | | | | | 100 | 99 | 99 |
| 16 | | | | | | | | | | | | | | | | 100 | 98 |
| 17 | | | | | | | | | | | | | | | | | 100 |

**Fig. 3**

```
1)    AVPS-TQTPW GIKSIYNDQS ITK-TTGGSG IKVAVLDTGV YT------SH LDLAGSAEQC
2)    ----AQSVPY GVSQIKAPAL HSQ-GYTGSN VKVAVIDSGI DS------SH PDLK--VAGG
3)    ----AQSVPW GISRVQAPAA HNR-GLTGSG VKVAVLDTGI S-------TH PDL--NIRGG
4)    -------NDV ARGIVKADVA QNNFGLYGQG QIVAVADTGL DTGRNDSSMH EAFRGKITAL
            1                                                         53


1)    KDFTQSNPLV DGSC-TDRQG HGTHVAGTVL AHGGSNGQGV YGVAPQAKLW AYKVLGD-NG
2)    ASMVPSET-- --PNFQDDNS HGTHVAGTVA ALN--NSIGV LGVAPSSALY AVKVLGD AG
3)    ASFVPGEP-- --ST-QDGNG HGTHVAGTIA ALN--NSIGV LGVAPSAELY AVKVLGA-SG
4)    YALG-RTN-- --NA-NDPNG HGTHVAGSVL GN---ATNKG MAPQANLVFQ SIMD-SGGGL
       54                                                        106


1)    SGY--SDD-I AAAIRHVADE ASRTGSKVVI NMSLGSSA-- -KDSLIASAV DYAY-GKGVL
2)    SGQ--YSW-I INGIEWAIAN N-----MDVI NMSLGGPS-- -GSAALKAAV DKAV-ASGVV
3)    SGS--VSS-I AQGLEWAGNN G-----MHVA NLSLGSPS-- -PSATLEQAV NSAT-SRGVL
4)    GGLPA-NLQT LFSQAYSAGA R-----IHTN SWGAPVNGAY TTDSRN-VDD YVRKNDMTIL
      107                                                        156


1)    IVAAAGNSGS G---SNTIGF PGGLVNAVAV AALEN----- -----VQQNG TYRVADFSSR
2)    VVAAAGNEG- STGSSSTVGY PGKYPSVIAV GAVDS----- -----S---- -NQRASFSSV
3)    VVAASGNSGA -----GSISY PARYANAMAV GATDQ----- -----N---- -NNRASFSQY
4)    FAAGNEGPGS G---TISAPG TAKNAITVGA TENLRPSFGS YADNIN---- -HVAQFSSRG
      157                                                        208


1)    GNPATAGDYI IQERDIEVSA PGASVESTWY T--------- ---GGYNTIS GTSMATPHVA
2)    GP-------- ----ELDVMA PGVSIQSTLP G--------- ---NKYGAYN GTSMASPHVA
3)    GA-------- ----GLDIVA PGVNVQSTYP G--------- ---STYASLN GTSMATPHVA
4)    PTRDGRIK-- ----PDVMAP GTYILSARSS LAPDSSFWAN HDSKYAYMGG TSMATPIVAG
      209                                                        262


1)    GLAAKIWSAN T-----SLSH SQLRTELQNR AKVYDIKGGI GAGTGDDYAS GFGYPRVK--
2)    GAAALILSKH P-----NWTN TQVRSSLQNT T--TKLG--- -----DSFYY GKGLINVQAA
3)    GAAALVKQKN P-----SWSN VQIRNHLKNT A--TSLG--- -----STNLY GSGLVNAEAA
4)    NVAQLREHFV KNRGVTPKPS LLKAALIAGA A--DVGLGFP -----NGNQG WGRVTLDKSL
      263                                                        315


1)    ---------- ---------- ---------- ---------- ---------- ----------
2)    AQ-------- ---------- ---------- ---------- ---------- ----------
3)    TR-------- ---------- ---------- ---------- ---------- ----------
4)    NVAFVNETSP LSTSQKATYS FTAQAGKPLK ISLVWSDAPG STTASLTLVN DLDLVITAPN
      316                                                        375


1)    ---------- ---------- ---------- ---------- ---------- --------
2)    ---------- ---------- ---------- ---------- ---------- --------
3)    ---------- ---------- ---------- ---------- ---------- --------
4)    GTKYVGNDFT APYDNNWDGR NNVENVFINA PQSGTYTVEV QAYNVPVGPQ TFSLAIVH
      376                                                        433
```

# Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 170 A
- WO 9217577 A
- DK 2004000066 W
- WO 8801293 A
- WO 9856927 A
- EP 204342 A
- JP 7062152 A
- JP 4197182 B
- US 6376227 B
- EP 1209233 A
- WO 9967370 A
- WO 0071691 A
- US 5118623 A
- WO 9634946 A
- US 4288627 A
- US 4599311 A
- US 4745051 A
- EP 397485 A
- US 5155037 A
- US 5162222 A
- WO 9117243 A
- US 4882279 A
- US 4879231 A
- EP 272277 A
- EP 230023 A
- US 5077214 A
- WO 8901029 A
- WO 8901028 A
- US 4775624 A
- US 4879236 A
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B

- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- GB 1296839 A
- WO 9402597 A
- WO 9418314 A
- WO 9623873 A
- WO 9743424 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 9324618 A
- WO 9510602 A
- WO 9815257 A
- US 4106991 A
- US 4661452 A
- GB 1483591 A
- EP 238216 A
- WO 9219709 A
- WO 9219708 A
- WO 0242740 A

**Non-patent literature cited in the description**

- **Siezen RJ ; Leunissen JAM.** *Protein Science,* 1997, vol. 6, 501-523
- **Davail S et al.** *The Journal of Biological Chemistry,* 1994, vol. 269 (26), 1 7448-17453
- **Narinx E et al.** *Protein Engineering,* 1997, vol. 10 (11), 1271-1279
- **Siezen et al.** *Protein Engng.,* 1991, vol. 4, 719-737
- **Siezen et al.** *Protein Science,* 1997, vol. 6, 501-523
- **S.B. Needleman ; C.D. Wunsch.** *Journal of Molecular Biology,* 1970, vol. 48, 443-445
- **Feinberg, A. P. ; Vogelstein, B.** *Anal. Biochem.,* 1983, vol. 132, 6-13
- **R.J. Siezeri ; J.A.M Leunissen.** *Protein science,* 1997, vol. 6 (3), 501-523
- **Davail S et al.** *J. Biol. Chem.,* 1994, vol. 269, 17448-17453
- **Stout, G.K. ; Jensen, L.H.** X-Ray Structure Determination. John Wiley & Sons, Inc, 1989
- **Kabsch ; Sander.** *Biopolymers,* 1983, vol. 22, 2577-2637
- **Sambrook et al.** Molecular cloning: A laboratory manual. Cold Spring Harbor lab, 1989
- Current protocols in Molecular Biology. John Wiley and Sons, 1995
- Molecular Biological Methods for Bacillus. John Wiley and Sons, 1990
- DNA Cloning: A Practical Approach. vol. I, II
- Oligonucleotide Synthesis. 1984
- Nucleic Acid Hybridization. 1985
- Transcription And Translation. 1984
- Animal Cell Culture. 1986
- Immobilized Cells And Enzymes. IRL Press, 1986
- **B. Perbal.** A Practical Guide To Molecular Cloning. 1984
- **L.J. Jensen et al.** *Nucleic Acid Research,* 1998, vol. 26, 697-702
- **Leung et al.** *Technique,* 1989, vol. 1, 11-15
- **Tomandl, D. et al.** *Journal of Computer-Aided Molecular Design,* 1997, vol. 11, 29-38
- **Jensen, LJ ; Andersen, KV ; Svendsen, A ; Kretzschmar, T.** *Nucleic Acids Research,* 1998, vol. 26, 697-702
- **Ehrlich.** *Proceedings of the National Academy of Sciences USA,* 1978, vol. 75, 1433
- **Villa-Kamaroff et al.** *Proceedings of the National Academy of Sciences USA,* 1978, vol. 75, 3727-3731
- Useful proteins from recombinant bacteria. *Scientific American,* 1980, vol. 242, 74-94
- **McKnight et al.** *EMBO J.,* 1985, vol. 4, 2093-2099
- **Hitzeman et al.** *J. Biol. Chem.,* 1980, vol. 255, 12073-12080
- **Alber ; Kawasaki.** *J. Mol. Appl. Gen.,* 1982, vol. 1, 419-434
- **Young et al.** Genetic Engineering of Microorganisms for Chemicals. Plenum Press, 1982
- **Russell et al.** *Nature,* 1983, vol. 304, 652-654
- **Romanos et al.** *Yeast,* 1992, vol. 8, 423-488
- **Subramani et al.** *Mol. Cell Biol.,* 1981, vol. 1, 854-864
- **Palmiter et al.** *Science,* 1983, vol. 222, 809-814
- **Vasuvedan et al.** *FEBS Lett.,* 1992, vol. 311, 7-11
- **J.M. Vlak et al.** *J. Gen. Virology,* 1988, vol. 69, 765-776
- **Gleeson et al.** *J. Gen. Microbiol.,* 1986, vol. 132, 3459-3465
- Biology and Activities of Yeast. Soc. App. Bacteriol. Symposium Series No. 9, 1980
- Biochemistry and Genetics of Yeast. 1987
- The Yeasts. 1987
- The Molecular Biology of the Yeast Saccharomyces. 1981
- Guide to Yeast Genetics and Molecular Biology. **Becker ; Guarente.** Methods in Enzymology. Academic Press, Inc, vol. 194, 182-187
- **Ito et al.** *Journal of Bacteriology,* 1983, vol. 153, 163
- **Hinnen et al.** *Proceedings of the National Academy of Sciences USA,* 1978, vol. 75, 1920
- **Kaufman ; Sharp.** *J. Mol. Biol.,* 1982, vol. 159, 601-621
- **Southern ; Berg.** *J. Mol. Appl. Genet.,* 1982, vol. 1, 327-341
- **Loyter et al.** *Proc. Natl. Acad. Sci. USA,* 1982, vol. 79, 422-426
- **Wigler et al.** *Cell,* 1978, vol. 14, 725
- **Corsaro ; Pearson.** *Somatic Cell Genetics,* 1981, vol. 7, 603
- **Ausubel et al.** Current Protocols in Molecular Biology. John Wiley and Sons, Inc, 1987
- **Hawley-Nelson et al.** *Focus,* 1993, vol. 15, 73
- **Ciccarone et al.** *Focus,* vol. 15, 80
- **Graham ; van der Eb.** *Virology,* 1973, vol. 52, 456
- **Neumann et al.** *EMBO J.,* 1982, vol. 1, 841-845
- **Graham ; Van der Eb.** *Virology,* 1978, vol. 52, 546
- More Gene Manipulations in Fungi. Academic Press, 1991
- Protein Purification. VCH Publishers, 1989
- **Dartois et al.** *Biochemica et Biophysica Acta,* 1993, vol. 1131, 253-360
- **Sambrook et al.** Molecular Cloning: A Laboratory Manual. Cold Spring Harbor, 1989

- **Dubnau et al.** *J. M ol. Biol.,* 1971, vol. 56, 209-221